(19) Europäisches Patentamt
European Patent Office
Office européen des brevets

(11) EP 3 984 523 B1

(12) EUROPEAN PATENT SPECIFICATION

(45) Date of publication and mention of the grant of the patent:
**03.09.2025 Bulletin 2025/36**

(21) Application number: **21213854.9**

(22) Date of filing: **06.12.2019**

(51) International Patent Classification (IPC):
***A61K 9/00*** *(2006.01)* ***A61K 31/395*** *(2006.01)*
***A61K 31/426*** *(2006.01)* ***A61K 31/573*** *(2006.01)*
***A61P 5/00*** *(2006.01)*

(52) Cooperative Patent Classification (CPC):
(C-Sets available)
**A61K 9/0095; A61K 31/426; A61K 31/573; A61P 5/00; C07C 209/52; C07C 249/02; C07C 259/06; C07D 277/42** (Cont.)

# (54) CRF1 RECEPTOR ANTAGONIST, PHARMACEUTICAL FORMULATIONS AND SOLID FORMS THEREOF FOR THE TREATMENT OF CONGENITAL ADRENAL HYPERPLASIA

CRF1-REZEPTOR-ANTAGONIST, PHARMAZEUTISCHE FORMULIERUNGEN UND FESTE FORMEN DAVON ZUR BEHANDLUNG VON KONGENITALER ADRENALER HYPERPLASIE

ANTAGONISTE DU RÉCEPTEUR CRF1, FORMULATIONS PHARMACEUTIQUES ET SES FORMES SOLIDES POUR LE TRAITEMENT DE L’HYPERPLASIE SURRÉNALE CONGÉNITALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **07.12.2018 US 201862776763 P**
**11.03.2019 US 201962816674 P**

(43) Date of publication of application:
**20.04.2022 Bulletin 2022/16**

(62) Document number(s) of the earlier application(s) in accordance with Art. 76 EPC:
**19848938.7 / 3 890 706**

(73) Proprietors:
- **Neurocrine Biosciences, Inc.**
  **San Diego, California 92130 (US)**
- **Sanofi**
  **75017 Paris (FR)**

(72) Inventors:
- **FARBER, Robert H.**
  **San Diego, 92130 (US)**
- **LOEWEN, Gordon Raphael**
  **San Diego, 92130 (US)**
- **ZHANG, Xiaoping**
  **San Diego, 92130 (US)**
- **GIRI, Nagdeep**
  **San Diego, 92130 (US)**
- **CHAN, Jean L.**
  **San Diego, 92130 (US)**
- **STIRN, Scott**
  **San Diego, 92130 (US)**
- **SAYERS, Brian**
  **San Diego, 92130 (US)**
- **TAYLOR, Graeme**
  **San Diego, 92130 (US)**
- **COSTA, Christina Marie**
  **San Diego, 92130 (US)**
- **PARKS, Stacy**
  **Oregon, 97701 (US)**
- **VICKERY, Anthony D.**
  **Oregon, 97701 (US)**
- **DOWNING, Kristie M.**
  **Oregon, 97701 (US)**
- **IYOHA, Kingsley**
  **Nottingham, NG11 6JS (GB)**
- **NGWENYA-JONES, Ayanda**
  **Nottingham, NG11 6JS (GB)**
- **CHARLIER, Anne**
  **75008 Paris (FR)**
- **MEHTON, Gurvinder Singh**
  **Nottingham, NG11 6JS (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Carpmaels & Ransford LLP**
**One Southampton Row**
**London WC1B 5HA (GB)**

(56) References cited:
**WO-A1-2010/125414** **WO-A1-2015/112642**
**WO-A1-2018/219804**

(52) Cooperative Patent Classification (CPC): (Cont.)

C-Sets
**A61K 31/426, A61K 2300/00;**
**A61K 31/573, A61K 2300/00;**
**C07C 209/52, C07C 211/29;**
**C07C 249/02, C07C 251/24**

## Description

## TECHNICAL FIELD

The present disclosure relates to pharmaceutical formulations of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, and their use in the treatment of congenital adrenal hyperplasia (CAH).

## BACKGROUND

Classic congenital adrenal hyperplasia (CAH) is a disease that includes a group of autosomal recessive disorders that result in an enzyme deficiency that alters the production of adrenal steroids due to 21-hydroxylase deficiency, a condition that results in little or no cortisol biosynthesis. One clinical manifestation of the absence of cortisol is the lack of feedback inhibition of pituitary adrenocorticotropic hormone (ACTH) secretion. Increased ACTH levels cause adrenal hyperplasia and the enzyme mutation causes a shunting of cortisol precursor steroids to alternate pathways. Most notably, the shunting of androgens leads to virilization and other developmental complications in females and the over-accumulation of ACTH is associated with the formation of testicular adrenal rest tumors in males. In addition, since the same enzyme (21-hydroxylase) is used in the pathway for the biosynthesis of the mineralocorticoids, a number of these patients suffer from aldosterone deficiency which can result in dehydration and death due to salt-wasting. The prevalence of classic 21-hydroxylase deficiency CAH in the US general population, based on newborn screening, has been documented as 1:10,000 to 1:20,800 (Trakakis et al., "An update to 21-hydroxylase deficient congenital adrenal hyperplasia," Gynecol. Endocrinol. (2010) 26(1):63-71; Hertzberg et al., "Birth prevalence rates of newborn screening disorders in relation to screening practices in the United States," J. Pediatr. (2011) 159(4):555-560).

Pediatric patients from birth through adolescence, and females in particular, appear to be the most vulnerable population of CAH sufferers and represent the subgroup of patients with the greatest unmet medical need (Cheng and Speiser, "Treatment outcomes in congenital adrenal hyperplasia," Adv. Pediatr. (2012) 59(1):269-281; Merke and Poppas, "Management of adolescents with congenital adrenal hyperplasia," Lancet Diabetes Endocrinol. (2013) 1(4):341-352). Excessive androgen production in these younger patients results in early onset puberty and adrenarche, changes in skeletal maturation patterns, short stature caused by premature growth plate fusion, as well as significant hirsutism and acne problems. While survival is properly ensured through steroid replacement strategies based on physiologic dosing of glucocorticoids (e.g., hydrocortisone) and mineralocorticoids (e.g., fludrocortisone), these doses are often inadequate to suppress the accumulating ACTH and overproduction of progestogens and androgens (e.g., 17-hydroxyprogesterone [17-OHP], androstenedione, and testosterone). The uncontrolled symptoms of androgen excess, indeed, have a substantial impact on the day-to-day functioning and development of these patients.

Currently, exogenous corticosteroids are the standard of care for treating patients with classic CAH. This treatment is used to correct the cortisol deficiency and reduce the excessive ACTH levels and androgen excess. However, the dose and duration of steroid use required to suppress ACTH are typically well above the normal physiological level used for cortisol replacement alone (as in patients with Addison's disease). This increased exposure to glucocorticoids can lead to iatrogenic Cushing's syndrome, increased cardiovascular risk factors, glucose intolerance, reduced growth velocity, and decreased bone mineral density in CAH patients (Elnecave et al., "Bone mineral density in girls with classical congenital adrenal hyperplasia due to CYP21 deficiency," J. Pediatr. Endocrinol. Metab. (2008) 21(12):1155-1162; King et al., "Long-term corticosteroid replacement and bone mineral density in adult women with classical congenital adrenal hyperplasia," J. Clin. Endocrinol. Metab. (2006) 91(3):865-869; Migeon and Wisniewski, "Congenital adrenal hyperplasia owing to 21-hydroxylase deficiency. Growth, development, and therapeutic considerations," Endocrinol. Metab. Clin. North Am. (2001) 30(1): 193-206).

Corticotropin releasing factor (CRF) is a hypothalamic hormone released directly into the hypophyseal portal vasculature and acts on specific corticotropin releasing factor 1 (CRF1) receptors on corticotropes in the anterior pituitary to stimulate the release of ACTH. Blockade of these receptors has been shown to decrease the release of ACTH in both animals and humans. Therefore, compounds that block CRF1 receptors have the potential to directly inhibit the excessive ACTH release that occurs in CAH and thereby allow for normalization of androgen production while using lower, more physiologic doses of hydrocortisone.

The compound of Formula (I)

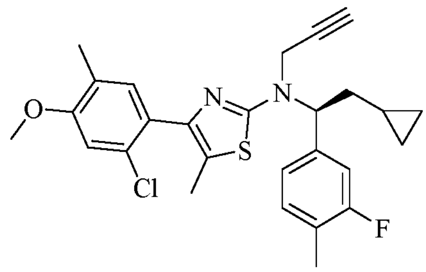

(I)

4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, is a selective CRF1 receptor antagonist. The compound of Formula (I) can be prepared according to the methods described in U.S. Patent Nos. 6,586,456 and 8,314,249. The compound of Formula (I) is a low solubility compound with low bioavailability. Partially due to its low solubility, attempts at formulating the compound of Formula (I) have proven to be difficult, particularly for formulations suitable for pediatric administration.

Thus, there is a need for a treatment for CAH that avoids the severe complications associated with corticosteroid therapy. There is also a need for a formulation of the compound of Formula (I) with increased bioavailability and a need for a formulation of the compound of Formula (I) that is suitable for pediatric administration. The formulations of the present invention help address these and other needs.WO 2015/112642 A1 relates to CRF1 receptor antagonists which have the potential to directly inhibit ACTH release in patients with CAH and thereby allow normalization of androgen production while using lower, more physiologic dose of hydrocortisone, and thus reducing treatment-associated side effects.

## SUMMARY

The invention is defined by the appended claims. In particular, the invention provides a pharmaceutical composition in oral solution dosage form comprising:

(a) a compound of Formula (I):

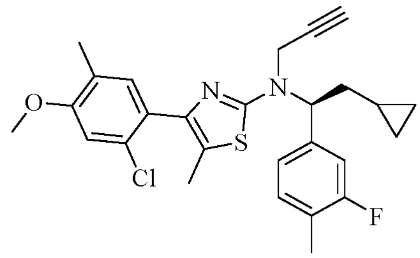

(I)

(4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine),
or a pharmaceutically acceptable salt thereof;
(b) one or more of a sweetener, an anti-oxidant, and a flavor; and
(c) a liquid vehicle, wherein the liquid vehicle is selected from medium-chain triglycerides, propylene glycol dicaprylate/dicaprate, glycerin, propylene glycol, polyethylene glycol, olive oil, soybean oil, corn oil, and diethylene glycol monoethyl ether.

The invention also provides a pharmaceutical composition in oral solution dosage form of the present invention for use in a method of treating congenital adrenal hyperplasia (CAH) in a subject.

In some embodiments, the subject is in a fed state.

In some embodiments, the pharmaceutical composition of the invention is administered to the subject with a nutritional composition. In some embodiments, the nutritional composition is a liquid dietary supplement comprising about 1500 calories per liter with a caloric distribution of about 14.7% protein, about 32% fat and about 53.3% carbohydrate. In some embodiments, the nutritional composition is administered in an amount of about 8 fluid ounces. In some embodiments, the nutritional composition is administered within 30 minutes of administration of the pharmaceutical composition.

In some embodiments, administering the pharmaceutical composition of the invention exhibits a positive food effect.

In some embodiments, the subject is a pediatric subject.

Other features and advantages of the methods, processes, formulations, and uses described herein will be apparent from the following detailed description and figures, and from the claims.

## DESCRIPTION OF DRAWINGS

FIG. 1 shows the dissolution performance of several reference spray-dried dispersion formulations in 0.5 wt% simulated intestinal fluid (SIF) in phosphate buffered saline (PBS), pH 6.5.

FIG. 2 shows the vertical membrane flux cell integrated in the μDiss Profiler™ used for the membrane flux assay.

FIG. 3 shows non-sink dissolution data for several reference spray-dried dispersion formulations and the compound of Formula (I) in 0.5 wt% SIF in PBS, pH 6.5.

FIG. 4 is a graph showing membrane flux of 1 mg/mL GB/IB 0.5 wt% SIF doses of the compound of Formula (I) and various reference spray-dried dispersion formulations over time. The solid lines indicate flux ($\mu g\ min^{-1}\ cm^{-2}$) and the broken lines indicate concentration (μg/mL) in 0.5% SIF.

FIG. 5 is a flow diagram of the reference spray drying manufacturing process used to prepare a 1000 g batch of a reference SDD containing 25% of the compound of Formula (I) and 75% PVP/VA 64.

FIGS. 6A and 6B are line graphs showing the pharmacokinetic results of a bioavailability and food effect study in dogs. FIG. 6A shows the results from Cohort 1 and FIG. 6B shows the results from Cohort 2.

FIG. 7 is a flow chart showing the study design of a Phase 1 study of the pharmacokinetics and food effect of the compound of Formula (I) in healthy adult subjects.

FIGS. 8A and 8B are line graphs showing the mean plasma concentration versus time profiles for the compound of Formula (I) under fasted and fed conditions, respectively, in healthy adult subjects.

FIGS. 9A-9C are spaghetti plots of the pharmacokinetics of the compound of Formula (I) in healthy adult subjects under fasted and fed conditions. FIG. 9A shows the $AUC_{0\text{-tlast}}$ values. FIG. 9B shows the $AUC_{0\text{-}\infty}$ values. FIG. 9C shows the $C_{max}$ values.

FIG. 10 is a flow chart showing the study design of a Phase 1 study of the bioavailability, pharmacokinetics and food effect of the compound of Formula (I) in healthy adult subjects.

FIG. 11 shows the study design of a Phase 2 study of the compound of Formula (I) in adult subjects with congenital adrenal hyperplasia.

FIGs. 12A and 12B show the arithmetic mean values for adrenocorticotropic hormone (ACTH) (FIG. 12A) and 17-hydroxyprogesterone (17-OHP) (FIG. 12B) for all 8 Cohort 1 subjects plotted at each time point for pre-treatment baseline (circles), day 1 (squares), and day 14 (triangles).

FIGs. 13A and 13B show arithmetic mean values for androstenedione (FIG. 13A) and testosterone (FIG. 13B) for all 8 Cohort 1 subjects were plotted at each timepoint for pre-treatment baseline (circles), day 1 (squares), and day 14 (triangles).

FIGs. 14A and 14B show the reduction of ACTH at timepoints 8-, 10-, and 12-hours postdose. FIG 14A shows the values for each time point as compared to baseline. FIG. 14B shows the mean values across all three timepoints.

FIGs. 15A and 15B show the reduction of 17-OHP at timepoints 8-, 10-, and 12-hours postdose. FIG 15A shows the values for each time point as compared to baseline. FIG. 15B shows the mean values across all three timepoints.

FIGs. 16A and 16B show the reduction of androstenedione at timepoints 8-, 10-, and 12-hours postdose. FIG 16A shows the values for each time point as compared to baseline. FIG. 16B shows the mean values across all three timepoints.

FIG. 17A shows the plasma ACTH Mean Blood Concentrations following the compound of Formula (I) 50 mg Dose qhs (Cohort 1; n=8). Error bars represent the standard error of the mean for each morning window timepoint. ACTH normal ranges: Female 6 to 58 pg/mL; Male 7 to 69 pg/mL.

FIG. 17B shows the serum 17-OHP Mean Blood Concentrations following the compound of Formula (I) 50 mg Dose qhs (Cohort 1; n=8). Error bars represent the standard error of the mean for each morning window timepoint. 17-OHP normal ranges: Female < 207 ng/dL; Male < 139 ng/dL.

FIG. 17C: shows the serum Androstenedione Mean Blood Concentrations following the compound of Formula (I) 50 mg Dose qhs (Cohort 1; n=8). Error bars represent the standard error of the mean for each morning window timepoint. Androstenedione normal ranges: Female 26 to 214 ng/mL; Male 33 to 134 ng/mL.

FIG. 18A shows the plasma ACTH Mean Blood Concentrations following the compound of Formula (I) 100 mg Dose qhs (Cohort 2; n=4). Error bars represent the standard error of the mean for each morning window timepoint. ACTH normal ranges: Female 6 to 58 pg/mL; Male 7 to 69 pg/mL.

FIG. 18B shows the serum 17-OHP Mean Blood Concentrations following the compound of Formula (I) 100 mg Dose qhs (Cohort 2; n=4). Error bars represent the standard error of the mean for each morning window timepoint. 17-OHP normal ranges: Female < 207 ng/dL; Male < 139 ng/dL.

FIG. 18C shows the serum Androstenedione Mean Blood Concentrations following the compound of Formula (I) 100 mg Dose qhs (Cohort 2; n=4). Error bars represent the standard error of the mean for each morning window timepoint. Androstenedione normal ranges: Female 26 to 214 ng/mL; Male 33 to 134 ng/mL.

FIG. 19A shows the plasma ACTH Mean Blood Concentrations following the compound of Formula (I) 100 mg Dose with Evening Meal (Cohort 3). Error bars represent the standard error of the mean for each morning window timepoint. ACTH normal ranges: Female 6 to 58 pg/mL; Male 7 to 69 pg/mL.

FIG. 19B shows the serum 17-OHP Mean Blood Concentrations following the compound of Formula (I) 100 mg Dose with Evening Meal (Cohort 3). Error bars represent the standard error of the mean for each morning window timepoint. 17-OHP normal ranges: Female < 207 ng/dL; Male < 139 ng/dL.

FIG. 19C shows the serum Androstenedione Mean Blood Concentrations following the compound of Formula (I) 100 mg Dose with Evening Meal (Cohort 3). Error bars represent the standard error of the mean for each morning window timepoint. Androstenedione normal ranges: Female 26 to 214 ng/mL; Male 33 to 134 ng/mL.

FIG. 20 is a scheme showing the manufacturing process for forming 50 mg reference capsules of the compound of Formula (I).

FIG. 21 is an alternative scheme showing the manufacturing process for forming 50 mg reference capsules of the compound of Formula (I).

FIGs. 22A and 22B show a scheme showing the manufacturing process for forming SDD reference granules of the compound of Formula (I).

FIG. 23 is a scheme showing the manufacturing process for forming 50 mg/nL liquid formulation 1 of the compound of Formula (I).

FIG. 24 is a scheme showing the manufacturing process for forming 50 mg/nL liquid formulation 2 of the compound of Formula (I).

FIG. 25 is an XRPD spectrum of the compound of Formula (I) free base crystalline form I.

FIG. 26 is a DSC spectrum of the compound of Formula (I) free base crystalline form I.

FIG. 27 is an XRPD spectrum of the compound of Formula (I) tosylate crystalline form 1.

FIG. 28 is a DSC and TGA spectrum of the compound of Formula (I) tosylate crystalline form 1.

## DETAILED DESCRIPTION

As described herein, 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine having the Formula (I):

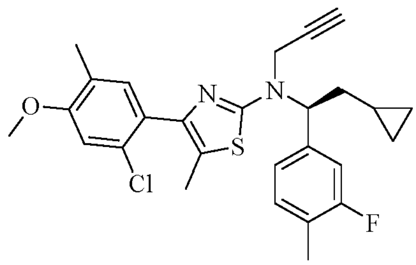

(I)

or a pharmaceutically acceptable salt thereof, is a selective CRF1 receptor antagonist that has been found to be effective treating congenital adrenal hyperplasia. Specifically, the compound of Formula (I) has been found to effectively reduce several biomarkers associated with congenital adrenal hyperplasia.

Newborn screening for CAH is performed by immunoassay to measure 17-OHP levels in heel-stick capillary blood specimens obtained within the first 72 hours of life. The blood sample is analyzed for 17-OHP by commercially available dissociation-enhanced lanthanide fluoroimmunoassay (DELFIA; PerkinElmer, Waltham Massachusetts) (White et al., J. Pediatr. 163:10-12 (2013)). Second-tier screening tests utilizing biochemical and molecular genetic testing methods, performed between 8 and 14 days of life, are employed by nine states in the United States and strongly recommended by an additional 5 states. The biochemical method includes immunoassay with organic solvent extraction or liquid chromatography followed by tandem mass spectrometry to measure steroid ratios of 17-OHP, androstenedione, and 21-deoxycortisol to cortisol *(see, e.g.,* Speiser et al., Int. J. Pediatr. Endocrinol. 2010:494173, 2010). The genetic screen looks for *CYP21A2* mutations that are associated with CAH. While not widely employed in the U.S., the addition of a

second screening could potentially improve the sensitivity of the overall screening process, where sensitivity of the first screen alone is approximately 72%.

In absence of results from the newborn screening, female infants with classical CAH are typically identified due to the presence of ambiguous genitalia. Males have normal genitalia at birth and therefore are not diagnosed unless newborn screening is conducted or other medical complications come to attention. Infants who are not initially diagnosed with CAH and suffer from the salt-wasting form of the disease are later diagnosed in the setting of poor weight gain, vomiting, hyperkalemia and hyponatremia within the first few weeks of life.

Treatment of CAH is based on normalization of hormone and steroid levels using a variety of medications from diagnosis in infancy through adulthood. Glucocorticoids are the current standard treatment in CAH and are used both to correct the endogenous cortisol deficiency and for reducing the elevated ACTH levels from the pituitary, which drives increased androgen production. Unlike the treatment of Addison's disease (adrenal insufficiency), in which cortisol replacement is sufficient, the treatment of CAH must also reduce ACTH production, to control the subsequent androgen excess as well. Thus, the goals of glucocorticoid treatment include cortisol replacement and suppression of ACTH to prevent virilization and menstrual disturbances in women and to inhibit testicular adrenal rest tumors in men. Mineralocorticoid replacement is needed to achieve normal plasma renin activity for maintenance of regular blood pressure, electrolyte balance, and volume status in those patients with the salt-wasting form of CAH.

The regimen of glucocorticoid treatment must support normal physiology and also ensure that sufficient cortisol is available during events that may elicit a strong stress response (e.g., intercurrent illness, exercise, hypotension). Careful monitoring is also necessary to avoid the development of iatrogenic Cushing's syndrome due to glucocorticoid overtreatment in an effort to adequately suppress androgen production, or Addisonian syndrome due to under-treatment.

Overtreatment with mineralocorticoids may cause hypertension while under-treatment may lead to low blood pressure, salt loss, fatigue and increased requirements for glucocorticoids. Typical laboratory tests for monitoring treatment efficacy include measurement of plasma concentrations of 17-OHP, androstenedione, testosterone, renin activity, and electrolytes.

Adult patients with CAH have an increased prevalence of risk factors for cardiovascular disease including obesity, hypertension, and insulin resistance *(see, e.g.,* Kim et al., Semin. Reprod. Med. 27(4):316-21 (2009)). A study of a large cohort of pediatric and adult CAH patients (n=244) demonstrated that patients are prescribed a variety of glucocorticoid treatment regimens yet frequently suffer from poor hormonal control and the aforementioned adverse outcomes *(see, e.g.,* Finkielstain et al., J. Clin. Endocrinol Metab. 97(12):4429-38 (2012)).

Treatment of CAH includes efforts to normalize the cortisol deficiency with glucocorticoids (usually hydrocortisone in children but often more potent agents with narrow therapeutic indices, such as dexamethasone, in adults) and, if necessary for salt-wasting, mineralocorticoids (usually fludrocortisone). The glucocorticoid doses required to achieve sufficient suppression of excess androgens, however, are usually well above the normal physiologic dose used for cortisol replacement alone as in patients with Addison's disease. This increased exposure to glucocorticoids can lead to iatrogenic Cushing's syndrome, increased cardiovascular risk factors, glucose intolerance, and decreased bone mineral density in CAH patients *(see, e.g.,* Elnecave et al., J. Pediatr. Endocrinol. Metab. 21:1155-62 (2008); King et al., J. Clin. Endocrinol. Metab. 91(3):8656-59 (2006); Migeon et al., Endocrinol. Metab. Clin. North Am. 30:193-206 (2001)). Recently, best practices for the clinical management of congenital adrenal hyperplasia were published in the Journal of Clinical Endocrinology and Metabolism (Speiser, P.W., et al. J. Clin. Endocrinol. Metab. November 2018, 103(11): 1-46).

Corticotropin-releasing factor (CRF) was isolated from ovine hypothalami and identified as a 41-amino acid peptide. CRF has been found to produce profound alterations in endocrine, nervous, and immune system function. CRF is believed to be the major physiological regulator of the basal and stress-induced release of adrenocorticotropic hormone ("ACTH"), β-endorphin, and other pro-opiomelanocortin ("POMC")-derived peptides from the anterior pituitary *(see, e.g.,* Vale et al., Science 213:1394-1397, 1981). Secretion of CRF causes release of ACTH from corticotrophs in the anterior pituitary via binding to the $CRF_1$ receptor, a member of the class B family of G-protein coupled receptors.

Due to the physiological significance of CRF, the development of biologically-active small molecules having significant $CRF_1$ receptor binding activity and which are capable of antagonizing the $CRF_1$ receptor remains a desirable goal and has been the subject of ongoing research and development for the treatment of anxiety, depression, irritable bowel syndrome, post-traumatic stress disorder, and substance abuse.

The pituitary hormone ACTH, under the control of hypothalamic corticotropin-releasing factor (CRF), stimulates uptake of cholesterol and drives the synthesis of pregnenolone initiating steroidogenesis in the adrenal gland. The adrenal cortex is comprised of three zones, which produce distinct classes of hormones many of which are driven by ACTH mobilizing cholesterol through this pathway. Deficiencies in these enzymes as a result of mutation or deletion cause the substrate concentrations to increase. In the most common form of CAH resulting from mutations or deletions in the 21-hydroxylase gene (CYP21A2), potent androgens are produced by the adrenal because of the accumulation of the steroid precursors, progesterone and 17-hydroxyprogesterone (17-OHP). Plasma levels of 17-OHP can reach 10-1000 times the normal concentration in these cases. These increases result in the overproduction of androgens, specifically androstenedione, testosterone, and dihydroxytestosterone causing virilization in females. In addition, 21-hydroxylase deficiency

in CAH causes insufficient biosynthesis of glucocorticoids and mineralocorticoids, specifically cortisol and aldosterone. Cortisol is a critical negative feedback regulator of hypothalamic CRF secretion and pituitary ACTH release. The lack of glucocorticoid synthesis and release eliminates the restraint on the hypothalamus and pituitary, which causes ACTH levels to increase. The excessive ACTH stimulation causes hypertrophy of the zona fasciculata and zona reticularis resulting in adrenal hyperplasia.

**Definitions**

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting.

The term "about" preceding a value for DSC, TGA, or $T_g$, which are reported as degrees Celsius, have an allowable variability of ±5°C. In all other instances, unless otherwise specified, the term "about" preceding a stated value includes the stated value and also includes ±20% of the stated value, and includes more specifically values of ±10%, ±5%, ±2%, and ±1% of the stated value.

To provide a more concise description, some of the quantitative expressions herein are recited as a range from about amount X to about amount Y. It is understood that when a range is recited, the range is not limited to the recited upper and lower bounds, but rather includes the full range from about amount X through about amount Y, or any range therein.

"Room temperature" or "RT" refers to the ambient temperature of a typical laboratory, which is typically around 25°C.

"Spray-drying" refers to the method of producing a dry powder from a solution or slurry. The solution or slurry is atomized or rapidly dried with a hot gas, e.g., air or nitrogen, that causes the solvent to evaporate quickly and uniformly. A "spray-dried dispersion" refers to the powder obtained from the spray-drying process.

The term "pharmaceutically acceptable carrier" or "pharmaceutically acceptable excipient" includes any and all solvents, co-solvents, complexing agents, dispersion media, coatings, antibacterial and antifungal agents, isotonic and absorption delaying agents, and the like, which are not biologically or otherwise undesirable. The use of such media and agents for pharmaceutically active substances is well known in the art. Except insofar as any conventional media or agent is incompatible with the active ingredient, its use in the therapeutic formulations is contemplated. Supplementary active ingredients can also be incorporated into the formulations. In addition, various excipients, such as are commonly used in the art, can be included. These and other such compounds are described in the literature, e.g., in the Merck Index, Merck & Company, Rahway, NJ. Considerations for the inclusion of various components in pharmaceutical compositions are described, e.g., in Gilman et al. (Eds.) (2010); Goodman and Gilman's: The Pharmacological Basis of Therapeutics, 12th Ed., The McGraw-Hill Companies.

"Subject," as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate. In some embodiments, the subject is a human.

In some embodiments, the subject has experienced and/or exhibited at least one symptom of the disease or disorder to be treated and/or prevented. In some embodiments, the subject has been identified or diagnosed as having congenital adrenal hyperplasia (CAH). In some embodiments, the subject is suspected of having CAH. In some embodiments, the subject has a clinical record indicating that the subject has CAH (and optionally the clinical record indicates that the subject should be treated with any of the compositions provided herein). In some embodiments, the subject is a pediatric subject.

The term "pediatric subject" as used herein refers to a subject under the age of 21 years at the time of diagnosis or treatment. The term "pediatric" can be further divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman et al., Textbook of Pediatrics, 15th Ed. Philadelphia: W.B. Saunders Company, 1996; Rudolph et al., Rudolph's Pediatrics, 21st Ed. New York: McGraw-Hill, 2002; and Avery et al., Pediatric Medicine, 2nd Ed. Baltimore: Williams & Wilkins; 1994. In some embodiments, a pediatric subject is from birth through the first 28 days of life, from 29 days of age to less than two years of age, from two years of age to less than 12 years of age, or 12 years of age through 21 years of age (up to, but not including, the twenty-second birthday). In some embodiments, a pediatric subject is from birth through the first 28 days of life, from 29 days of age to less than 1 year of age, from one month of age to less than four months of age, from three months of age to less than seven months of age, from six months of age to less than 1 year of age, from 1 year of age to less than 2 years of age, from 2 years of age to less than 3 years of age, from 2 years of age to less than seven years of age, from 3 years of age to less than 5 years of age, from 5 years of age to less than 10 years of age, from 6 years of age to less than 13 years of age, from 10 years of age to less than 15 years of age, or from 15 years of age to less than 22 years of age.

As used herein, the terms "treat" or "treatment" refer to therapeutic or palliative measures. Beneficial or desired

clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment.

The term "preventing," as used herein, means the prevention of the onset, recurrence or spread, in whole or in part, of the disease or condition as described herein, or a symptom thereof.

The term "administration" or "administering" refers to a method of giving a dosage of a compound or pharmaceutical formulation to a vertebrate or invertebrate, including a mammal, a bird, a fish, or an amphibian. The preferred method of administration can vary depending on various factors, e.g., the components of the pharmaceutical formulation, the site of the disease, and the severity of the disease.

As used herein, "therapeutically effective amount" is an amount of the compound of Formula (I), or a pharmaceutically acceptable salt thereof, or an amount of a pharmaceutical composition comprising the compound of Formula (I), which is sufficient to achieve the desired effect and can vary according to the nature and severity of the disease condition, and the potency of the compound. A therapeutic effect is the relief, to some extent, of one or more of the symptoms of the disease, and can include curing a disease. "Curing" means that the symptoms of active disease are eliminated. However, certain long-term or permanent effects of the disease can exist even after a cure is obtained (such as, e.g., extensive tissue damage).

The term "amorphous" means a solid in a solid state that is a non-crystalline state. Amorphous solids are disordered arrangements of molecules and therefore possess no distinguishable crystal lattice or unit cell and consequently have no definable long range ordering. The solid state form of a solid may be determined by polarized light microscopy, X-ray powder diffraction (XRPD), differential scanning calorimetry (DSC), or other standard techniques known to those of skill in the art.

As used herein, "time of day window" refers to a period of time defined by a window start time and a window stop time. These times all refer to local times where a sample was taken. The phrase "same time of day window" when referring to samples taken from the subject mean, e.g., that a sample taken at 8:15 a.m. and a sample taken at 9:15 a.m. are considered to be taken in the same time of day window of, e.g., 2 a.m. to 10 a.m. or 6 a.m. to 10 a.m.

**Methods**

Any references to methods of treatment and medical uses are to be interpreted as references to pharmaceutical compositions of the invention for use in those methods or uses.

The present disclosure relates to methods of treating congenital adrenal hyperplasia (CAH). The methods include administering to a subject a therapeutically effective amount of a compound of Formula (I), or pharmaceutically acceptable salt thereof.

Provided herein is a method of treating congenital adrenal hyperplasia (CAH) comprising administering a compound of Formula (I), or a pharmaceutically acceptable salt thereof to normalize or partially normalize levels of biomarkers associated with congenital adrenal hyperplasia. In some embodiments, normalizing or partially normalizing levels of biomarkers comprises reducing levels of elevated biomarkers or increasing levels of depressed biomarkers as compared to subject without CAH.

Provided herein is a method of treating congenital adrenal hyperplasia in a subject in need thereof comprising administering a compound of Formula (I), or a pharmaceutically acceptable salt thereof, in an amount sufficient to reduce the level of one or more biomarkers associated with congenital adrenal hyperplasia. In some embodiments, the biomarkers are selected from (a) 17-hydroxyprogesterone (17-OHP); (b) adrenocorticotropic hormone (ACTH); and (c) androstenedione in the subject.

In some embodiments, the reduction in level of any of the biomarkers (e.g., any of 17-OHP, ACTH, and androstenedione) is determined by comparing the level of the biomarker as measured during the circadian release on a day prior to administering the compound of Formula (I), or a pharmaceutically acceptable salt thereof and the level of the biomarker as measured during the circadian release on the day after administering the compound of Formula (I), or a pharmaceutically acceptable salt thereof. A day prior to administering the compound of Formula (I) applies to a subject that has not previously been administered the compound of Formula (I) within at least the past 24 hours.

In some embodiments, the circadian release of biomarkers associated with CAH occurs between the hours of 2 a.m. and 10 a.m. In other embodiments, the circadian release of biomarkers associated with CAH occurs between the hours of 6 a.m. and 10 a.m.

In some embodiments of any of the methods disclosed herein, the compound of Formula (I), or a pharmaceutically acceptable salt, is administered to the subject at nighttime or administration prior to sleep (i.e., bedtime administration). In some embodiments, the compound of Formula (I), or a pharmaceutically acceptable salt thereof, is administered three to eight hours prior to the circadian release of the biomarker. In some embodiments, the compound

of Formula (I), or a pharmaceutically acceptable salt thereof, is administered six to eight hours prior to the circadian release of the biomarker. Administration prior to the circadian release may be adapted for shift workers (e.g., those who work at night and sleep during the day), in which case administration will not necessarily occur at nighttime. Administration is therefore dependent upon the expected circadian release of the biomarker, and can vary depending upon the individual's (*i.e.,* subject, patient) particular work and sleep patterns.

**In** some embodiments of the methods provided herein, the level of 17-hydroxyprogesterone is reduced by at least 10%, at least 15%, at least 20%, at least 25%, at least 30%, at lease 35%, at least 40%, at least 50%, at least 55% or at least 60% from pre-administration levels. **In** some embodiments, the level of 17-hydroxyprogesterone is reduced by at least 25%. **In** some embodiments, the level of 17-hydroxyprogesterone is reduced by at least 50%. **In** some embodiments of the methods provided herein, the level of 17-hydroxyprogesterone is reduced by an amount of from about 10% to about 90%, about 15% to about 90%, about 20% to about 90%, about 25% to about 90%, about 30% to about 90%, about 35% to about 90%, about 40% to about 90%, about 50% to about 90%, about 55% to about 90%, or about 60% to about 90% from pre-administration levels.

**In** some embodiments, the level of 17-hydroxyprogesterone is reduced to a level within the range of 17-hydroxyprogesterone expected for a subject without CAH, i.e., less than 1,000 ng/dL or less than 200 ng/dL.

**In** some embodiments of the methods provided herein, the level of adrenocorticotropic hormone is reduced by at least 10%, at least 15%, at least 20%, at least 25%, at least 30%, at lease 35%, at least 40%, at least 50%, at least 55% or at least 60% from pre-administration levels. **In** some embodiments, the level of adrenocorticotropic hormone is reduced by at least 25%. **In** some embodiments, the level of adrenocorticotropic hormone is reduced by at least 40%. **In** some embodiments, the level of adrenocorticotropic hormone is reduced by at least 50%.

**In** some embodiments of the methods provided herein, the level of adrenocorticotropic hormone is reduced by an amount of from about 10% to about 90%, about 15% to about 90%, about 20% to about 90%, about 25% to about 90%, about 30% to about 90%, about 35% to about 90%, about 40% to about 90%, about 50% to about 90%, about 55% to about 90%, or about 60% to about 90% from pre-administration levels.

**In** some embodiments, the level of adrenocorticotropic hormone is reduced to a level within the range of adrenocorticotropic hormone expected for a subject without CAH.

**In** some embodiments of the methods provided herein, the level of androstenedione is reduced by at least 10%, at least 15%, at least 20%, at least 25%, at least 30%, at lease 35%, at least 40%, at least 50%, at least 55% or at least 60% from pre-administration levels. **In** some embodiments, the level of androstenedione is reduced by at least 25%. **In** some embodiments, the level of androstenedione is reduced by at least 30%. In some embodiments, the level of androstenedione is reduced by at least 50%.

In some embodiments of the methods provided herein, the level of androstenedione is reduced by an amount of from about 10% to about 90%, about 15% to about 90%, about 20% to about 90%, about 25% to about 90%, about 30% to about 90%, about 35% to about 90%, about 40% to about 90%, about 50% to about 90%, about 55% to about 90%, or about 60% to about 90% from pre-administration levels.

In some embodiments, the level of androstenedione is reduced to a level within the range of androstenedione expected for a subject without CAH, i.e., less than 200 ng/dL.

Also provided herein is a method for reducing the severity of one or more symptoms selected from hirsutism, precocious puberty, fertility problems, acne, and growth impairment in a subject having classic congenital adrenal hyperplasia, comprising administering a compound of Formula (I), or a pharmaceutically acceptable salt thereof, in an amount sufficient to reduce one or more biomarker of CAH in a subject, e.g. reduce the androstenedione in the subject. Growth impairment can refer to, e.g., accelerated height velocity, accelerated weight velocity, and/or accelerated bone age.

Provided herein is a method for reducing the level of one or more biomarkers of congenital adrenal hyperplasia in a subject having congenital adrenal hyperplasia comprising administering to the subject a compound of Formula (I), or a pharmaceutically acceptable salt thereof. In some embodiments, the one or more biomarkers of congenital adrenal hyperplasia are selected from (a) 17-hydroxyprogesterone (17-OHP); (b) adrenocorticotropic hormone (ACTH); and (c) androstenedione.

Provided herein is a method for reducing the dosage of corticosteroid administered to a subject having congenital adrenal hyperplasia for controlling congenital adrenal hyperplasia comprising administering to the subject a compound of Formula (I), or a pharmaceutically acceptable salt thereof. In some embodiments, the corticosteroid is a glucocorticoid.

Also provided herein is a method of reducing the severity of one or more side effects of glucocorticoid treatment in a subject having congenital adrenal hyperplasia comprising administering to the subject a compound of Formula (I), or a pharmaceutically acceptable salt thereof. The long-term effects of glucocorticoid treatment are well documented in the art (see, e.g., Oray, M. et al. (2016): Long-term effect of glucocorticoids, Expert Opinion on Drug Safety. DOI: 10.1517/14740338.2016.1140743). Such side effects are associated with every biological system, e.g., musculoskeletal (e.g., osteoporosis, avascular necrosis of bone, and myopathy), endocrine and metabolic (e.g., hyperglycemia, diabetes mellitus, dyslipidemia, weight gain, Cushing syndrome, Cushingoid features, growth suppression, adrenal suppression),

gastrointestinal (e.g., gastritis, peptic ulcer, gastrointestinal bleeding, visceral perforation, hepatic steatosis, pancreatitis), cardiovascular (e.g., hypertension, coronary heart disease, ischemic heart disease, heart failure), dermatologic (e.g., dermatoprosis, skin atrophy, ecchymosis, purpura, erosions, striae, delayed wound healing, easy bruising, acne, hirsutism, and hair loss), neuropsychiatric (e.g., mood changes, depression, euphoria, mood lability, irritability, akathisia, anxiety, cognitive impairment, psychosis, dementia, and delirium), ophthalmologic (e.g., cataract, glaucoma, ptosis, mydriasis, opportunistic ocular infections, and central serous chorioretinopathy), and immunologic (e.g., suppression of cell-mediated immunity, predisposition to infections, and reactivation of latent infections).

Accordingly, in some embodiments, the side effects of glucocorticoid treatment are selected from osteoporosis, avascular necrosis of bone, myopathy, hyperglycemia, diabetes mellitus, dyslipidemia, weight gain, Cushing syndrome, Cushingoid features, growth suppression, adrenal suppression, gastritis, peptic ulcer, gastrointestinal bleeding, visceral perforation, hepatic steatosis, pancreatitis, hypertension, coronary heart disease, ischemic heart disease, heart failure, dermatoprosis, skin atrophy, ecchymosis, purpura, erosions, striae, delayed wound healing, easy bruising, acne, hirsutism, hair loss, mood changes, depression, euphoria, mood lability, irritability, akathisia, anxiety, cognitive impairment, psychosis, dementia, delirium, cataract, glaucoma, ptosis, mydriasis, opportunistic ocular infections, central serous chorioretinopathy, suppression of cell-mediated immunity, predisposition to infections, reactivation of latent infections, and any combination thereof.

Provided herein is a method of treating congenital adrenal hyperplasia in a subject comprising

(i) measuring the level of one or more biomarkers selected from (a) 17-hydroxyprogesterone (17-OHP); (b) adrenocorticotropic hormone (ACTH); and (c) androstenedione in a biological sample obtained from the subject;
(ii) analyzing the level of the one or more biomarkers to determine if the level of the one or more biomarkers is elevated compared to a healthy subject not having congenital adrenal hyperplasia; and
(iii) administering to the subject a compound of Formula (I), or a pharmaceutically acceptable salt thereof if the subject is determined to have elevated levels of the one or more biomarkers.

In some embodiments, the method further comprises (iv) measuring the level of the one or more biomarkers after administering a compound of Formula (I), or a pharmaceutically acceptable salt thereof, in a biological sample obtained from the subject to determine whether the subject has reduced levels of the one or more biomarkers as compared with the measurement of step (i). In some embodiments, the method further comprises (v) continuing the administration of the compound of Formula (I), or a pharmaceutically acceptable salt thereof if the subject has reduced levels of the one or more biomarkers.

In some embodiments, steps (i) and (iv) are performed on biological samples taken from the subject in a similar manner and within a same time of day window. In some embodiments, steps (i) and (iv) are performed on biological samples taken from the subject within the time of day window from 2 a.m. to 10 a.m. In some embodiments, steps (i) and (iv) are performed on biological samples taken from the subject within the time of day window from 6 a.m. to 10 a.m.

In some embodiments, steps (i) and (iv) comprise measuring the levels of at least two biomarkers selected from (a) 17-hydroxyprogesterone (17-OHP); (b) adrenocorticotropic hormone (ACTH); and (c) androstenedione.

In some embodiments, steps (i) and (iv) comprise measuring the levels of (a) 17-hydroxyprogesterone (17-OHP); (b) adrenocorticotropic hormone (ACTH); and (c) androstenedione.

In some embodiments, step (i) comprises measuring the level of 17-hydroxyprogesterone (17-OHP), wherein the level of 17-hydroxyprogesterone (17-OHP) is elevated when it is greater than or equal to 1,000 ng/dL.

In some embodiments, step (i) comprises measuring the level of androstenedione, wherein the level of androstenedione is elevated when it is greater than 200 ng/dL.

In some embodiments of the methods of the present disclosure, the compound of Formula (I) is administered at an amount equivalent to from about 25 mg to about 150 mg of the compound of Formula (I) free base. In some embodiments, compound of Formula (I), or a pharmaceutically acceptable salt thereof, is administered at an amount equivalent to about 50 mg or about 100 mg of the compound of Formula (I) free base.

In some embodiments of the methods disclosed herein, compound of Formula (I) is administered in the free base form.

In some embodiments of the methods disclosed herein, the compound of Formula (I) is administered once daily.

Also provided herein is a method of treating CAH in a pediatric subject. The methods include administering to a pediatric subject a therapeutically effective amount of a compound of Formula (I), or pharmaceutically acceptable salt thereof. In some embodiments, the pediatric subject is a neonate. In some embodiments, the pediatric subject is an infant. In some embodiments, the pediatric subject is a child. In some embodiments, the pediatric subject is an adolescent.

In some embodiments of the methods of the present disclosure, the compound of Formula (I), or a pharmaceutically acceptable salt thereof, is administered to the subject in a fed state. The term "fed state," as used herein, refers to administration of the compound of Formula (I), or a pharmaceutically acceptable salt thereof from about 1 hour before consumption of food or a nutritional composition to about 1 hour after consumption of food or a nutritional composition. The

term "fasted state," as used herein, refers to a gap of at least two hours between consumption of food or a nutritional composition and administration of the compound of Formula (I), or a pharmaceutically acceptable salt thereof. In some embodiments, the compound of Formula (I), or a pharmaceutically acceptable salt thereof is administered to the subject with food or a nutritional composition, such as a nutritional supplement or formula, a meal replacement beverage, a liquid dietary supplement, or a high caloric liquid meal. In some embodiments, the compound of Formula (I), or a pharmaceutically acceptable salt thereof is administered to the subject within about 1 hour before the subject has consumed food or a nutritional composition. In some embodiments, the compound of Formula (I), or a pharmaceutically acceptable salt thereof is administered to the subject within about 1 hour after the subject has consumed food or a nutritional composition. Examples of suitable nutritional compositions include, but are not limited to, infant formulas, dietary supplements, dietary substitutes, and rehydration compositions. In some embodiments, the food is a product containing concentrated calories and protein. In some embodiments, the nutritional composition is a composition utilized for enteral and parenteral supplementation for infants, specialty infant formulas, supplements for the elderly, and supplements for those with gastrointestinal difficulties and/or malabsorption. Adult and pediatric nutritional formulas are well known in the art and are commercially available (e.g., Similac®, Ensure®, Jevity® and Alimentum® from Ross Products Division, Abbott Laboratories, Columbus, Ohio).

In some embodiments, the nutritional composition is in liquid form. The energy density of the nutritional compositions, when in liquid form, can range from about 0.6 Kcal to about 3 Kcal per mL. In some embodiments, the nutritional composition is in solid or powdered form. When in solid or powdered form, the nutritional supplements can contain from about 1.2 to more than 9 Kcals per gram, such as about 3 to 7 Kcals per gram.

In some embodiments, the nutritional composition is a meal replacement bar. Examples include PowerBar®, Glucerna® bars, Choice DM® bars, Ensure® bars, and Boost® bars. In some embodiments, the nutritional composition is a nutrition shake or meal replacement beverage. Commercially available examples include the Ensure® branded adult products (such as Ensure® Original, Ensure® Plus, Ensure® Enlive, Ensure® High Protein, Ensure® Clear, and Ensure® Light), Glucerna®, Choice DM®, Slim Fast®, Pediasure®, Glytrol®, and Resource®. In some embodiments, the nutritional composition is Ensure® Plus. In some embodiments, the nutritional composition is vanilla-flavored Ensure® Plus. Ensure Plus® is a high calorie liquid dietary supplement that contains 1500 calories per liter with a caloric distribution of 14.7% protein, 32% fat and 53.3% carbohydrate.

In some embodiments of the disclosed methods, the compound of Formula (I), or a pharmaceutically acceptable salt thereof is administered to the subject with 8 fluid ounces (237 mL) of Ensure® Plus. In some embodiments, the Ensure® Plus is vanilla-flavored.

In some embodiments of the methods, the compound of Formula (I), or a pharmaceutically acceptable salt thereof is administered to the subject after administration of the nutritional composition. In some embodiments, the compound of Formula (I), or a pharmaceutically acceptable salt thereof is administered to the subject before administration of the nutritional composition. In some embodiments, the compound of Formula (I), or a pharmaceutically acceptable salt thereof, is administered to the subject at the same time as administration of the nutritional composition.

In some embodiments, the compound of Formula (I), or a pharmaceutically acceptable salt thereof is administered to the subject, followed by administration of the nutritional composition. In some embodiments, the nutritional composition is administered about 1 minute, about 5 minutes, about 10 minutes, about 15 minutes, about 20 minutes, about 25 minutes, about 30 minutes, about 35 minutes, about 40 minutes, about 45 minutes, or about 60 minutes, or within a range defined by any of the preceding values after administration of the compound of Formula (I), or a pharmaceutically acceptable salt thereof. In some embodiments, the nutritional composition is administered 1 minute, 5 minutes, 10 minutes, 15 minutes, 20 minutes, 25 minutes, 30 minutes, 35 minutes, 40 minutes, 45 minutes, or 60 minutes, or within a range defined by any of the preceding values after administration of the compound of Formula (I), or a pharmaceutically acceptable salt thereof. In some embodiments, the nutritional composition is administered within 30 minutes of administering the compound of Formula (I), or a pharmaceutically acceptable salt thereof. In some embodiments of the disclosed methods, the compound of Formula (I), or a pharmaceutically acceptable salt thereof, is administered to the subject with 8 fluid ounces (237 mL) of Ensure® Plus. In some embodiments, the Ensure® Plus is vanilla-flavored.

In some embodiments, the nutritional composition is administered to the subject, followed by administration of the compound of Formula (I), or a pharmaceutically acceptable salt thereof. In some embodiments, compound of Formula (I), or a pharmaceutically acceptable salt thereof is administered about 1 minute, about 5 minutes, about 10 minutes, about 15 minutes, about 20 minutes, about 25 minutes, about 30 minutes, about 35 minutes, about 40 minutes, about 45 minutes, or about 60 minutes, or within a range defined by any of the preceding values after administration of the nutritional composition. In some embodiments, the compound of Formula (I), or a pharmaceutically acceptable salt thereof, is administered 1 minute, 5 minutes, 10 minutes, 15 minutes, 20 minutes, 25 minutes, 30 minutes, 35 minutes, 40 minutes, 45 minutes, or 60 minutes, or within a range defined by any of the preceding values after administration of the nutritional composition. In some embodiments, the compound of Formula (I), or a pharmaceutically acceptable salt thereof, is administered within 30 minutes of administering the nutritional composition. In some embodiments of the disclosed methods, the compound of Formula (I), or a pharmaceutically acceptable salt thereof, is administered to the subject with 8

fluid ounces (237 mL) of Ensure® Plus. In some embodiments, the Ensure® Plus is vanilla-flavored.

In some embodiments of the methods, a food effect is observed between administration of the compound of Formula (I), or a pharmaceutically acceptable salt thereof in a fed state versus a fasted state. The term "food effect," as used herein, refers to the relative difference in AUC (area under the curve $AUC_{(0-t)}$ and/or $AUC_{(0-\infty)}$) or $C_{max}$ (maximum plasma concentration or peak plasma concentration) of an active substance, when the compound of Formula (I), or a pharmaceutically acceptable salt thereof, is administered orally to a subject, concomitantly with food or in a fed state as compared to the same values when the same compound of Formula (I), or a pharmaceutically acceptable salt thereof is administered in a fasted state. The food effect (F) is calculated as:

$$F\% = [(X_{fasted} - X_{fed}) / X_{fasted}] \times 100$$

where $X_{fed}$ and $X_{fasted}$ are the values of AUC ($AUC_{(0-t)}$ and/or $AUC_{(0-\infty)}$) or $C_{max}$ in the fed and fasted state, respectively. In some embodiments, an increased, or positive, food effect is observed when the compound of Formula (I), or a pharmaceutically acceptable salt thereof is administered to a subject in a fed state. In some embodiments, administration of the compound of Formula (I), or a pharmaceutically acceptable salt thereof, results in an increased, or positive, food effect, whereby an increased $C_{max}$ and/or AUC are observed when administered orally in the fed state as compared to the fasting state.

In some embodiments of the methods, the ratio of the AUC in the fed state to the AUC in the fasted state is about 5 to about 10, such as about 5 to about 9, about 5 to about 8, about 5 to about 7, about 5 to about 6, about 6 to about 10, about 6 to about 9, about 6 to about 8, about 6 to about 7, about 7 to about 10, about 7 to about 9, about 7 to about 8, about 8 to about 10, about 8 to about 9, or about 8 to about 10. In some embodiments, the ratio of the AUC in the fed state to the AUC in the fasted state is about 5, about 6, about 7, about 8, about 9, or about 10, or within a range defined by any of the preceding values. In some embodiments of the methods, the ratio of the AUC in the fed state to the AUC in the fasted state is about 10 to about 20.

In some embodiments of the methods, the ratio of the AUC in the fed state to the AUC in the fasted state is 5 to 10, such as 5 to 9, 5 to 8, 5 to 7, 5 to 6, 6 to 10, 6 to 9, 6 to 8, 6 to 7, 7 to 10, 7 to 9, 7 to 8, 8 to 10, 8 to 9, or 8 to 10. In some embodiments, the ratio of the AUC in the fed state to the AUC in the fasted state is 5, 6, 7, 8, 9, or 10, or within a range defined by any of the preceding values.

In some embodiments of the methods, the ratio of the $C_{max}$ in the fed state to the $C_{max}$ in the fasted state is about 5 to about 10, such as about 5 to about 9, about 5 to about 8, about 5 to about 7, about 5 to about 6, about 6 to about 10, about 6 to about 9, about 6 to about 8, about 6 to about 7, about 7 to about 10, about 7 to about 9, about 7 to about 8, about 8 to about 10, about 8 to about 9, or about 8 to about 10. In some embodiments, the ratio of the $C_{max}$ in the fed state to the $C_{max}$ in the fasted state is about 5, about 6, about 7, about 8, about 9, or about 10, or within a range defined by any of the preceding values. In some embodiments, the mean $C_{max}$ of the compound of Formula (I), or pharmaceutically acceptable salt thereof, is about 1.5 to about 3 times higher in the fed stated compared to the fasted state. In some embodiments of the methods, the ratio of the $C_{max}$ in the fed state to the $C_{max}$ in the fasted state is 5 to 10, such as 5 to 9, 5 to 8, 5 to 7, 5 to 6, 6 to 10, 6 to 9, 6 to 8, 6 to 7, 7 to 10, 7 to 9, 7 to 8, 8 to 10, 8 to 9, or 8 to 10. In some embodiments, the ratio of the $C_{max}$ in the fed state to the $C_{max}$ in the fasted state is 5, 6, 7, 8, 9, or 10, or within a range defined by any of the preceding values. In some embodiments, the mean $C_{max}$ of the compound of Formula (I), or pharmaceutically acceptable salt thereof, is 1.5 to 3 times higher in the fed stated compared to the fasted state. In some embodiments, the mean $C_{max}$ of the compound of Formula (I), or pharmaceutically acceptable salt thereof, is about 2 times higher in the fed stated compared to the fasted state. In some embodiments of the methods, the ratio of the $C_{max}$ in the fed state to the $C_{max}$ in the fasted state is about 10 to about 20.

In some embodiments, the 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, is administered to the subject with a meal. In some embodiments, the meal is a high fat, high caloric meal. In some embodiments, the meal is a low fat, low caloric meal. In some embodiments, the 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, is administered within approximately 5 minutes after the start of the meal. In some embodiments, the meal is an evening meal. In some embodiments, the meal is a morning meal.

In some embodiments, the fed state is with a high fat meal. In some embodiments, the fed state is with a low fat meal. The FDA has provided draft guidelines regarding high fat and low fat meals ("Assessing the Effects of Food on Drugs in INDs and NDAs - Clinical Pharmacology Considerations Guidance for Industry," U.S. Department of Health and Human Services, Food and Drug Administration, Center for Drug Evaluation and Research (CDER), February 2019, Clinical Pharmacology). Table 1 shows test meal definitions provided by the FDA guidance.

**Table 1**

| Meal Type | Total Kcal | Fat | | |
|---|---|---|---|---|
| | | Kcal | Grams | Percent |
| High-Fat | 800-1000 | 500-600 | 55-65 | 50 |
| Low-Fat | 400-500 | 100-125 | 11-14 | 25 |

The composition of a high fat meal provided by the FDA guidance is depicted in Table 2.

**Table 2. Composition of a High Fat Meal***

| | |
|---|---|
| Total Calories | 800-1000 |
| Calories from Protein | 150 |
| Calories from Carbohydrates | 250 |
| Calories from Fat | 500-600 |
| An Example of a High Fat Breakfast | • Two eggs fried in butter<br>• Two strips of bacon<br>• Two slices of toast with butter<br>• Four ounces of hash brown potatoes<br>• Eight ounces of whole milk |
| *50 percent of calories are derived from fat. Substitutions can be made to this meal, if the content, volume, and viscosity are maintained. | |

The composition of a low fat meal provided by the FDA guidance is depicted in Table 3.

Table 3. Composition of a Low Fat Meal

| | |
|---|---|
| Total Calories | 400-500 |
| Fat (g) | 250 |
| Percent Calories from Fat | 25 |
| An Example of a Low Fat Breakfast* | • Eight ounces milk (1 percent fat)<br>• One boiled egg<br>• One packet flavored instant oatmeal made with water |
| *This low-fat breakfast contains 387 calories and has 10 grams of fat. | |

In some embodiments, a high fat meal contains 800-1000 total Kcal and 500-600 fat Kcal. In some embodiments, a low fat meal contains 400-500 total Kcal and 100-125 fat Kcal.

Also provided herein is a method of improving gastrointestinal absorption of a compound of Formula (I), or pharmaceutically acceptable salt thereof, in a subject. The method includes orally administering to the subject a pharmaceutical composition of the present invention, wherein the improvement is relative to oral administration of the compound of Formula (I), or pharmaceutically acceptable salt thereof, which has not been prepared as a spray-dried dispersion. In some embodiments, the subject is a pediatric subject.

Also provided herein is a method of improving oral bioavailability of a compound of Formula (I), or pharmaceutically acceptable salt thereof, in a subject. The method includes orally administering to the subject a pharmaceutical composition of the present disclosure, wherein the improvement is relative to oral administration of the compound of Formula (I), or pharmaceutically acceptable salt thereof, which has not been prepared as a spray-dried dispersion.

In some embodiments of the methods provided herein, the subject is a pediatric subject.

Also provided herein is a method of treating congenital adrenal hyperplasia (CAH), in a subject in need thereof, comprising administering to the subject a pharmaceutical composition of the present disclosure, wherein the pharmaceutical composition comprises a therapeutically effective amount of the compound of Formula (I), or a pharmaceutically acceptable salt thereof. According to the invention, the pharmaceutical composition is a liquid formulation. In some embodiments, the pharmaceutical composition is administered to the subject in a fed state.

Also provided herein is a pharmaceutical composition of the present invention for use in a method of treating congenital adrenal hyperplasia (CAH) in a subject. In some embodiments, the subject is in a fed state.

In some embodiments, the pharmaceutical composition is administered to the subject with a nutritional composition. In some embodiments, the nutritional composition is a liquid dietary supplement comprising about 1000 to about 2000 calories per liter with a fat content greater than about 30%. In some embodiments, the nutritional composition is a liquid dietary supplement comprising 1500 calories per liter with a caloric distribution of 14.7% protein, 32% fat and 53.3% carbohydrate. In some embodiments, the nutritional composition is administered in an amount of about 6 to about 12 fluid ounces. In some embodiments, the nutritional composition is administered in an amount of about 8 fluid ounces. In some embodiments, the nutritional composition is administered within 30 minutes of administration of the pharmaceutical composition.

In some embodiments, the pharmaceutical composition exhibits a positive food effect. In some embodiments, the positive food effect is measured in terms of $C_{max}$, AUC, or a combination thereof of a compound of Formula (I) when comparing oral administration of the pharmaceutical composition in the fed and fasting states. In some embodiments, the ratio of the AUC of the compound of Formula (I) in the fed state to the AUC of the compound of Formula (I) in the fasted state is about 5 to about 10. In some embodiments, the ratio of the $C_{max}$ of the compound of Formula (I) in the fed state to the $C_{max}$ of the compound of Formula (I) in the fasted state is about 5 to about 10. In some embodiments, the ratio of the AUC of the compound of Formula (I) in the fed state to the AUC of the compound of Formula (I) in the fasted state is about 10 to about 20. In some embodiments, the ratio of the $C_{max}$ of the compound of Formula (I) in the fed state to the $C_{max}$ of the compound of Formula (I) in the fasted state is about 10 to about 20. In some embodiments, the ratio of the AUC of the compound of Formula (I) in the fed state to the AUC of the compound of Formula (I) in the fasted state is about 1 to about 4 or about 5 to about 10. In some embodiments, the ratio of the $C_{max}$ of the compound of Formula (I) in the fed state to the $C_{max}$ of the compound of Formula (I) in the fasted state is about 1 to about 4 or about 5 to about 10. In some embodiments, the ratio of the AUC of the compound of Formula (I) in the fed state to the AUC of the compound of Formula (I) in the fasted state is about 1 to about 4. In some embodiments, the ratio of the $C_{max}$ of the compound of Formula (I) in the fed state to the $C_{max}$ of the compound of Formula (I) in the fasted state is about 1 to about 4. In some embodiments, the ratio of the AUC of the compound of Formula (I) in the fed state to the AUC of the compound of Formula (I) in the fasted state is about 1.5 to about 3. In some embodiments, the ratio of the $C_{max}$ of the compound of Formula (I) in the fed state to the $C_{max}$ of the compound of Formula (I) in the fasted state is about 1.5 to about 3. In some embodiments, the ratio of the AUC of the compound of Formula (I) in the fed state to the AUC of the compound of Formula (I) in the fasted state is 1 to 4 or 5 to 10. In some embodiments, the ratio of the $C_{max}$ of the compound of Formula (I) in the fed state to the $C_{max}$ of the compound of Formula (I) in the fasted state is 1 to 4 or 5 to 10. In some embodiments, the ratio of the AUC of the compound of Formula (I) in the fed state to the AUC of the compound of Formula (I) in the fasted state is 1 to 4. In some embodiments, the ratio of the $C_{max}$ of the compound of Formula (I) in the fed state to the $C_{max}$ of the compound of Formula (I) in the fasted state is 1 to 4. In some embodiments, the ratio of the AUC of the compound of Formula (I) in the fed state to the AUC of the compound of Formula (I) in the fasted state is 1.5 to 3. In some embodiments, the ratio of the $C_{max}$ of the compound of Formula (I) in the fed state to the $C_{max}$ of the compound of Formula (I) in the fasted state is 1.5 to 3.

In some embodiments, the subject is a pediatric subject.

In some embodiments, the pharmaceutical composition is formulated for oral administration and exhibits a positive food effect when administered orally. In some embodiments, the compound of Formula (I) has a ratio of the AUC in the fed state to the AUC in the fasted state of about 5 to about 10. In some embodiments, the compound of Formula (I) has a ratio of the $C_{max}$ in the fed state to the $C_{max}$ in the fasted state of about 5 to about 10. In some embodiments, the compound of Formula (I) has a ratio of the AUC in the fed state to the AUC in the fasted state of about 10 to about 20. In some embodiments, the compound of Formula (I) has a ratio of the $C_{max}$ in the fed state to the $C_{max}$ in the fasted state of about 10 to about 20. In some embodiments, the compound of Formula (I) has a ratio of the AUC in the fed state to the AUC in the fasted state of about 1 to about 4 or about 5 to about 10. In some embodiments, the compound of Formula (I) has a ratio of the $C_{max}$ in the fed state to the $C_{max}$ in the fasted state of about 1 to about 4 or about 5 to about 10. In some embodiments, the compound of Formula (I) has a ratio of the AUC in the fed state to the AUC in the fasted state of about 1 to about 4. In some embodiments, the compound of Formula (I) has a ratio of the $C_{max}$ in the fed state to the $C_{max}$ in the fasted state of about 1 to about 4. In some embodiments, the compound of Formula (I) has a ratio of the AUC in the fed state to the AUC in the fasted state of about 1.5 to about 3. In some embodiments, the compound of Formula (I) has a ratio of the $C_{max}$ in the fed state to the $C_{max}$ in the fasted state of about 1.5 to about 3. In some embodiments, the compound of Formula (I) has a ratio of the AUC in the fed state to the AUC in the fasted state of 1 to 4 or 5 to 10. In some embodiments, the compound of Formula (I) has a ratio of the $C_{max}$ in the fed state to the $C_{max}$ in the fasted state of 1 to 4 or 5 to 10. In some embodiments, the compound of Formula (I) has a ratio of the AUC in the fed state to the AUC in the fasted state of 1 to 4. In some embodiments, the compound of Formula (I) has a ratio of the $C_{max}$ in the fed state to the $C_{max}$ in the fasted state of 1 to 4. In some embodiments, the compound of Formula (I) has a ratio of the AUC in the fed state to the AUC in the fasted state of 1.5 to 3. In some embodiments, the compound of Formula (I) has a ratio of the $C_{max}$ in the fed state to the $C_{max}$ in the fasted state of 1.5 to 3.

In some embodiments, the pharmaceutical composition is administered to the subject with a meal. In some embodiments, the meal is a high fat meal. In some embodiments, the meal is a low fat meal. In some embodiments, the pharmaceutical composition is administered within about 5 minutes after the start of the meal. In some embodiments, the meal is an evening meal. In some embodiments, the meal is a morning meal.

In some embodiments, administering the pharmaceutical composition exhibits a positive food effect. In some embodiments, the positive food effect is measured in terms of $C_{max}$, AUC, or combinations thereof of the compound of Formula (I) when comparing oral administration of the pharmaceutical composition in the fed and fasting states. In some embodiments, the ratio of the AUC of the compound of Formula (I) in the fed state to the AUC of the compound of Formula (I) in the fasted state is about 5 to about 10. In some embodiments, the ratio of the $C_{max}$ of the compound of Formula (I) in the fed state to the $C_{max}$ of the compound of Formula (I) in the fasted state is about 5 to about 10. In some embodiments, the ratio of the AUC of the compound of Formula (I) in the fed state to the AUC of the compound of Formula (I) in the fasted state is about 10 to about 20. In some embodiments, the ratio of the $C_{max}$ of the compound of Formula (I) in the fed state to the $C_{max}$ of the compound of Formula (I) in the fasted state is about 10 to about 20. In some embodiments, the ratio of the AUC of the compound of Formula (I) in the fed state to the AUC of the compound of Formula (I) in the fasted state is about 1 to about 4 or about 5 to about 10. In some embodiments, the ratio of the $C_{max}$ of the compound of Formula (I) in the fed state to the $C_{max}$ of the compound of Formula (I) in the fasted state is about 1 to about 4 or about 5 to about 10. In some embodiments, the ratio of the AUC of the compound of Formula (I) in the fed state to the AUC of the compound of Formula (I) in the fasted state is about 1 to about 4. In some embodiments, the ratio of the $C_{max}$ of the compound of Formula (I) in the fed state to the $C_{max}$ of the compound of Formula (I) in the fasted state is about 1 to about 4. In some embodiments, the ratio of the AUC of the compound of Formula (I) in the fed state to the AUC of the compound of Formula (I) in the fasted state is about 1.5 to about 3. In some embodiments, the ratio of the $C_{max}$ of the compound of Formula (I) in the fed state to the $C_{max}$ of the compound of Formula (I) in the fasted state is about 1.5 to about 3. In some embodiments, the ratio of the AUC of the compound of Formula (I) in the fed state to the AUC of the compound of Formula (I) in the fasted state is 1 to 4 or 5 to 10. In some embodiments, the ratio of the $C_{max}$ of the compound of Formula (I) in the fed state to the $C_{max}$ of the compound of Formula (I) in the fasted state is 1 to 4 or 5 to 10. In some embodiments, the ratio of the AUC of the compound of Formula (I) in the fed state to the AUC of the compound of Formula (I) in the fasted state is 1 to 4. In some embodiments, the ratio of the $C_{max}$ of the compound of Formula (I) in the fed state to the $C_{max}$ of the compound of Formula (I) in the fasted state is 1 to 4. In some embodiments, the ratio of the AUC of the compound of Formula (I) in the fed state to the AUC of the compound of Formula (I) in the fasted state is 1.5 to 3. In some embodiments, the ratio of the $C_{max}$ of the compound of Formula (I) in the fed state to the $C_{max}$ of the compound of Formula (I) in the fasted state is 1.5 to 3.

For the avoidance of doubt, also provided herein is the corresponding pharmaceutical composition of the invention, for use in the corresponding methods, as described herein.

**Reduction in glucocorticoid burden, adrenal androgens and precursors**

Glucocorticoids are a class of corticosteroids, which are a class of steroid hormones. Glucocorticoids are corticosteroids that bind to the glucocorticoid receptor that is present in almost every vertebrate animal cell. In some embodiments, the subject is concurrently receiving a dose of a glucocorticoid. In some embodiments, the glucocorticoid is selected from cortisol (hydrocortisone), cortisone, prednisone, prednisolone, methylprednisolone, dexamethasone, betamethasone, triamcinolone, fludrocortisone acetate, and deoxycorticosterone acetate. In some embodiments, the glucocorticoid is cortisol (hydrocortisone). In some embodiments, the glucocorticoid is cortisone. In some embodiments, the glucocorticoid is prednisone. In some embodiments, the glucocorticoid is dexamethasone.

In some embodiments, the glucocorticoid dose is measured in hydrocortisone equivalents. In some embodiments, the glucocorticoid dose is measured as a multiple of the upper limit of normal of physiologic dosing in hydrocortisone equivalents. Any glucocorticoid can be given in a dose that provides approximately the same glucocorticoid effects as normal cortisol production; this is referred to as physiologic, replacement, or maintenance dosing.

In some embodiments, the glucocorticoid dose is a physiologic dose as measured after a time period of administration of the compound of Formula (I), or a pharmaceutically acceptable salt thereof. In some embodiments, the glucocorticoid dose is a physiologic dose of about 4 to about 12 $mg/m^2$/day as measured after a time period of administration of the compound of Formula (I), or a pharmaceutically acceptable salt thereof. In some embodiments, the glucocorticoid dose is a physiologic dose of about 4 to about 9 $mg/m^2$/day as measured after a time period of administration of the compound of Formula (I), or a pharmaceutically acceptable salt thereof. In some embodiments, the glucocorticoid dose is a physiologic dose that is less than about 8 $mg/m^2$/day as measured after a time period of administration of the compound of Formula (I), or a pharmaceutically acceptable salt thereof.

In some embodiments, the glucocorticoid dose is a physiologic dose as measured after a time period of administration of the pharmaceutical composition comprising the compound of Formula (I), or a pharmaceutically acceptable salt thereof. In some embodiments, the glucocorticoid dose is a physiologic dose of about 4 to about 12 $mg/m^2$/day as measured after a time period of administration of the pharmaceutical composition comprising the compound

of Formula (I), or a pharmaceutically acceptable salt thereof. In some embodiments, the glucocorticoid dose is a physiologic dose of about 4 to about 9 $mg/m^2/day$ as measured after a time period of administration of the pharmaceutical composition comprising the compound of Formula (I), or a pharmaceutically acceptable salt thereof. In some embodiments, the glucocorticoid dose is a physiologic dose that is less than about 8 $mg/m^2/day$ as measured after a time period of administration of the pharmaceutical composition comprising the compound of Formula (I), or a pharmaceutically acceptable salt thereof.

In some embodiments, the glucocorticoid dose concurrently given to the subject is a normal physiological dose of hydrocortisone equivalents. In some embodiments, the glucocorticoid dose concurrently given to the subject is determined after a time period of administration of the compound of Formula (I), or a pharmaceutically acceptable salt thereof. In some embodiments, the glucocorticoid dose concurrently given to the subject is determined after a time period of administration of the pharmaceutical composition comprising the compound of Formula (I), or a pharmaceutically acceptable salt thereof. In some embodiments, a normal physiological dose of hydrocortisone equivalents is about 2 to about 16 $mg/m^2/day$. In some embodiments, a normal physiological dose of hydrocortisone equivalents is about 4 to about 12 $mg/m^2/day$. In some embodiments, a normal physiological dose of hydrocortisone equivalents is about 5 to about 11 $mg/m^2/day$. In some embodiments, a normal physiological dose of hydrocortisone equivalents is about 6 to about 10 $mg/m^2/day$. In some embodiments, a normal physiological dose of hydrocortisone equivalents is about 7 to about 9 $mg/m^2/day$. In some embodiments, a normal physiological dose of hydrocortisone equivalents is about 4 to about 9 $mg/m^2/day$. In some embodiments, a normal physiological dose of hydrocortisone equivalents is about 8 $mg/m^2/day$. In some embodiments, a normal physiological dose of hydrocortisone equivalents is about 12 $mg/m^2/day$. In some embodiments, a normal physiological dose of hydrocortisone equivalents is less than about 8 $mg/m^2/day$. In some embodiments, a normal physiological dose of hydrocortisone equivalents is about 2, about 3, about 4, about 5, about 6, about 7, about 8, about 9, about 10, about 11, about 12, about 13, about 14, about 15 or about 16 $mg/m^2/day$, or within a range defined by any of the preceding values.

In some embodiments, the glucocorticoid dose concurrently given to the subject is at the upper limit of normal of a normal physiological dose of hydrocortisone equivalents. In some embodiments, the glucocorticoid dose concurrently given to the subject is determined after a time period of administration of the compound of Formula (I), or a pharmaceutically acceptable salt thereof. In some embodiments, the glucocorticoid dose concurrently given to the subject is determined after a time period of administration of the pharmaceutical composition comprising the compound of Formula (I), or a pharmaceutically acceptable salt thereof. In some embodiments, the upper limit of normal is 1.5 times the normal physiological dose. In some embodiments, the upper limit of normal is about 1.5 times the normal physiological dose. In some embodiments, the upper limit of normal is about 1.5 times the normal physiological dose. In some embodiments, the upper limit of normal is about 2 times the normal physiological dose. In some embodiments, the upper limit of normal is about 2.5 times the normal physiological dose. In some embodiments, the upper limit of normal is about 1.0, about 1.1, about 1.2, about 1.3, about 1.4, about 1.5, about 1.6, about 1.7, about 1.8, about 1.9, about 2.0, about 2.1, about 2.2, about 2.3, about 2.4, about 2.5, about 2.6, about 2.7, about 2.8, about 2.9, or about 3.0 times the normal physiological dose, or within a range defined by any of the preceding values.

In some embodiments, the glucocorticoid dose of the subject is reduced by about 10% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 20% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 30% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 40% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 50% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 60% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 70% after a time period of administration of the compound of Formula (I), or a pharmaceutically acceptable salt thereof, wherein the reduction of the glucocorticoid dose is relative to the glucocorticoid

dose prior to administration of the compound of Formula (I), or a pharmaceutically acceptable salt thereof. In some embodiments, the glucocorticoid dose of the subject is reduced by less than about 20% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by about 20% to about 50% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof. In some embodiments, the glucocorticoid dose of the subject is reduced by greater than about 50% after a time period of administration of the compound of Formula (I), or a pharmaceutically acceptable salt thereof, wherein the reduction of the glucocorticoid dose is relative to the glucocorticoid dose prior to administration of the compound of Formula (I), or a pharmaceutically acceptable salt thereof.

In some embodiments, the glucocorticoid dose of the subject is reduced by about 10% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 20% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 30% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 40% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 50% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 60% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 70% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by less than about 20% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by about 20% to about 50% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof. In some embodiments, the glucocorticoid dose of the subject is reduced by greater than about 50% after a time period of administration of the pharmaceutical composition comprising the compound of Formula (I), or a pharmaceutically acceptable salt thereof, wherein the reduction of the glucocorticoid dose is relative to the glucocorticoid dose prior to administration of the pharmaceutical composition comprising the compound of Formula (I), or a pharmaceutically acceptable salt thereof.

In some embodiments, the glucocorticoid dose of the subject is reduced within a range defined by any of the preceding values.

In some embodiments, the level of 17-hydroxyprogesterone is reduced by at least 25% after a time period of administration of the compound of Formula (I), or a pharmaceutically acceptable salt thereof, wherein the reduction of the level of 17-hydroxyprogesterone is relative to the level of 17-hydroxyprogesterone prior to administration of the compound of Formula (I), or a pharmaceutically acceptable salt thereof. In some embodiments, the level of 17-hydroxyprogesterone

is reduced by at least 50% after a time period of administration of the compound of Formula (I), or a pharmaceutically acceptable salt thereof, wherein the reduction of the level of 17-hydroxyprogesterone is relative to the level of 17-hydroxyprogesterone prior to administration of the compound of Formula (I), or a pharmaceutically acceptable salt thereof. In some embodiments, the level of 17-hydroxyprogesterone is less than 1.5 times the upper limit of normal after a time period of administration of the compound of Formula (I), or a pharmaceutically acceptable salt thereof. In some embodiments, the level of 17-hydroxyprogesterone is within normal limits after a time period of administration of the compound of Formula (I), or a pharmaceutically acceptable salt thereof.

In some embodiments, the level of 17-hydroxyprogesterone is reduced by at least about 25% after a time period of administration of the pharmaceutical composition comprising the compound of Formula (I), or a pharmaceutically acceptable salt thereof, wherein the reduction of the level of 17-hydroxyprogesterone is relative to the level of 17-hydroxyprogesterone prior to administration of the pharmaceutical composition comprising the compound of Formula (I), or a pharmaceutically acceptable salt thereof. In some embodiments, the level of 17-hydroxyprogesterone is reduced by at least about 50% after a time period of administration of the pharmaceutical composition comprising the compound of Formula (I), or a pharmaceutically acceptable salt thereof, wherein the reduction of the level of 17-hydroxyprogesterone is relative to the level of 17-hydroxyprogesterone prior to administration of the pharmaceutical composition comprising the compound of Formula (I), or a pharmaceutically acceptable salt thereof. In some embodiments, the level of 17-hydroxyprogesterone is less than about 1.5 times the upper limit of normal after a time period of administration of the pharmaceutical composition comprising the compound of Formula (I), or a pharmaceutically acceptable salt thereof. In some embodiments, the level of 17-hydroxyprogesterone is within normal limits after a time period of administration of the pharmaceutical composition comprising the compound of Formula (I), or a pharmaceutically acceptable salt thereof.

In some embodiments, the level of 17-hydroxyprogesterone of the subject is reduced within a range defined by any of the preceding values.

In some embodiments, the level of adrenocorticotropic hormone is reduced by at least 25% after a time period of administration of the compound of Formula (I), or a pharmaceutically acceptable salt thereof, wherein the reduction of the level of adrenocorticotropic hormone is relative to the level of adrenocorticotropic hormone prior to administration of the compound of Formula (I), or a pharmaceutically acceptable salt thereof. In some embodiments, the level of adrenocorticotropic hormone is reduced by at least 40% after a time period of administration of the compound of Formula (I), or a pharmaceutically acceptable salt thereof, wherein the reduction of the level of adrenocorticotropic hormone is relative to the level of adrenocorticotropic hormone prior to administration of the compound of Formula (I), or a pharmaceutically acceptable salt thereof. In some embodiments, the level of adrenocorticotropic hormone is reduced by at least 50% after a time period of administration of the compound of Formula (I), or a pharmaceutically acceptable salt thereof, wherein the reduction of the level of adrenocorticotropic hormone is relative to the level of adrenocorticotropic hormone prior to administration of the compound of Formula (I), or a pharmaceutically acceptable salt thereof. In some embodiments, the level of adrenocorticotropic hormone is less than 1.5 times the upper limit of normal after a time period of administration of the compound of Formula (I), or a pharmaceutically acceptable salt thereof. In some embodiments, the level of adrenocorticotropic hormone is within normal limits after a time period of administration of the compound of Formula (I), or a pharmaceutically acceptable salt thereof.

In some embodiments, the level of adrenocorticotropic hormone is reduced by at least about 25% after a time period of administration of the pharmaceutical composition comprising the compound of Formula (I), or a pharmaceutically acceptable salt thereof, wherein the reduction of the level of adrenocorticotropic hormone is relative to the level of adrenocorticotropic hormone prior to administration of the pharmaceutical composition comprising the compound of Formula (I), or a pharmaceutically acceptable salt thereof. In some embodiments, the level of adrenocorticotropic hormone is reduced by at least about 40% after a time period of administration of the pharmaceutical composition comprising the compound of Formula (I), or a pharmaceutically acceptable salt thereof, wherein the reduction of the level of adrenocorticotropic hormone is relative to the level of adrenocorticotropic hormone prior to administration of the pharmaceutical composition comprising the compound of Formula (I), or a pharmaceutically acceptable salt thereof. In some embodiments, the level of adrenocorticotropic hormone is reduced by at least about 50% after a time period of administration of the pharmaceutical composition comprising the compound of Formula (I), or a pharmaceutically acceptable salt thereof, wherein the reduction of the level of adrenocorticotropic hormone is relative to the level of adrenocorticotropic hormone prior to administration of the pharmaceutical composition comprising the compound of Formula (I), or a pharmaceutically acceptable salt thereof. In some embodiments, the level of adrenocorticotropic hormone is less than about 1.5 times the upper limit of normal after a time period of administration of the pharmaceutical composition comprising the compound of Formula (I), or a pharmaceutically acceptable salt thereof. In some embodiments, the level of adrenocorticotropic hormone is within normal limits after a time period of administration of the pharmaceutical composition comprising the compound of Formula (I), or a pharmaceutically acceptable salt thereof.

In some embodiments, the level of adrenocorticotropic hormone of the subject is reduced within a range defined by any of the preceding values.

In some embodiments, the level of androstenedione is reduced by at least 25% after a time period of

administration of the compound of Formula (I), or a pharmaceutically acceptable salt thereof, wherein the reduction of the level of androstenedione is relative to the level of androstenedione prior to administration of the compound of Formula (I), or a pharmaceutically acceptable salt thereof. In some embodiments, the level of androstenedione is reduced by at least 30% after a time period of administration of the compound of Formula (I), or a pharmaceutically acceptable salt thereof, wherein the reduction of the level of androstenedione is relative to the level of androstenedione prior to administration of the compound of Formula (I), or a pharmaceutically acceptable salt thereof. In some embodiments, the level of androstenedione is reduced by at least 50% after a time period of administration of the compound of Formula (I), or a pharmaceutically acceptable salt thereof, wherein the reduction of the level of androstenedione is relative to the level of androstenedione prior to administration of the compound of Formula (I), or a pharmaceutically acceptable salt thereof. In some embodiments, the level of androstenedione is less than 1.5 times the upper limit of normal after a time period of administration of the compound of Formula (I), or a pharmaceutically acceptable salt thereof. In some embodiments, the level of androstenedione is within normal limits after a time period of administration of the compound of Formula (I), or a pharmaceutically acceptable salt thereof.

In some embodiments, the level of androstenedione is reduced by at least about 25% after a time period of administration of the pharmaceutical composition comprising the compound of Formula (I), or a pharmaceutically acceptable salt thereof, wherein the reduction of the level of androstenedione is relative to the level of androstenedione prior to administration of the pharmaceutical composition comprising the compound of Formula (I), or a pharmaceutically acceptable salt thereof. In some embodiments, the level of androstenedione is reduced by at least about 30% after a time period of administration of the pharmaceutical composition comprising the compound of Formula (I), or a pharmaceutically acceptable salt thereof, wherein the reduction of the level of androstenedione is relative to the level of androstenedione prior to administration of the pharmaceutical composition comprising the compound of Formula (I), or a pharmaceutically acceptable salt thereof. In some embodiments, the level of androstenedione is reduced by at least about 50% after a time period of administration of the pharmaceutical composition comprising the compound of Formula (I), or a pharmaceutically acceptable salt thereof, wherein the reduction of the level of androstenedione is relative to the level of androstenedione prior to administration of the pharmaceutical composition comprising the compound of Formula (I), or a pharmaceutically acceptable salt thereof. In some embodiments, the level of androstenedione is less than about 1.5 times the upper limit of normal after a time period of administration of the pharmaceutical composition comprising the compound of Formula (I), or a pharmaceutically acceptable salt thereof. In some embodiments, the level of androstenedione is within normal limits after a time period of administration of the pharmaceutical composition comprising the compound of Formula (I), or a pharmaceutically acceptable salt thereof.

In some embodiments, the level of androstenedione of the subject is reduced within a range defined by any of the preceding values.

In some embodiments, the level of testosterone is reduced by at least 25% after a time period of administration of the compound of Formula (I), or a pharmaceutically acceptable salt thereof, wherein the reduction of the level of testosterone is relative to the level of testosterone prior to administration of the compound of Formula (I), or a pharmaceutically acceptable salt thereof. In some embodiments, the level of testosterone is reduced by at least 30% after a time period of administration of the compound of Formula (I), or a pharmaceutically acceptable salt thereof, wherein the reduction of the level of testosterone is relative to the level of testosterone prior to administration of the compound of Formula (I), or a pharmaceutically acceptable salt thereof. In some embodiments, the level of testosterone is reduced by at least 50% after a time period of administration of the compound of Formula (I), or a pharmaceutically acceptable salt thereof, wherein the reduction of the level of testosterone is relative to the level of testosterone prior to administration of the compound of Formula (I), or a pharmaceutically acceptable salt thereof. In some embodiments, the level of testosterone is less than 1.5 times the upper limit of normal after a time period of administration of the compound of Formula (I), or a pharmaceutically acceptable salt thereof. In some embodiments, the level of testosterone is within normal limits after a time period of administration of the compound of Formula (I), or a pharmaceutically acceptable salt thereof.

In some embodiments, the level of testosterone is reduced by at least about 25% after a time period of administration of the pharmaceutical composition comprising the compound of Formula (I), or a pharmaceutically acceptable salt thereof, wherein the reduction of the level of testosterone is relative to the level of testosterone prior to administration of the pharmaceutical composition comprising the compound of Formula (I), or a pharmaceutically acceptable salt thereof. In some embodiments, the level of testosterone is reduced by at least about 30% after a time period of administration of the pharmaceutical composition comprising the compound of Formula (I), or a pharmaceutically acceptable salt thereof, wherein the reduction of the level of testosterone is relative to the level of testosterone prior to administration of the pharmaceutical composition comprising the compound of Formula (I), or a pharmaceutically acceptable salt thereof. In some embodiments, the level of testosterone is reduced by at least about 50% after a time period of administration of the pharmaceutical composition comprising the compound of Formula (I), or a pharmaceutically acceptable salt thereof, wherein the reduction of the level of testosterone is relative to the level of testosterone prior to administration of the pharmaceutical composition comprising the compound of Formula (I), or a pharmaceutically acceptable salt thereof. In some embodiments, the level of testosterone is less than about 1.5 times the upper limit of

normal after a time period of administration of the pharmaceutical composition comprising the compound of Formula (I), or a pharmaceutically acceptable salt thereof. In some embodiments, the level of testosterone is within normal limits after a time period of administration of the pharmaceutical composition comprising the compound of Formula (I), or a pharmaceutically acceptable salt thereof.

In some embodiments, the level of testosterone of the subject is reduced within a range defined by any of the preceding values.

In some embodiments, the level of 17-hydroxyprogesterone is reduced by at least 50% and the level of androstenedione is reduced by at least 50% after a time period of administration of the compound of Formula (I), or a pharmaceutically acceptable salt thereof, wherein the reduction of the level of 17-hydroxyprogesterone and the level of androstenedione is relative to the level of 17-hydroxyprogesterone and the level of androstenedione prior to administration of the compound of Formula (I), or a pharmaceutically acceptable salt thereof. In some embodiments, the level of 17-hydroxyprogesterone is less than 1.5 times the upper limit of normal and the level of androstenedione is less than 1.5 times the upper limit of normal after a time period of administration of the compound of Formula (I), or a pharmaceutically acceptable salt thereof. In some embodiments, the level of 17-hydroxyprogesterone is within normal limits and the level of androstenedione is within normal limits after a time period of administration of the compound of Formula (I), or a pharmaceutically acceptable salt thereof.

In some embodiments, the level of 17-hydroxyprogesterone is reduced by at least about 50% and the level of androstenedione is reduced by at least about 50% after a time period of administration of the pharmaceutical composition comprising the compound of Formula (I), or a pharmaceutically acceptable salt thereof, wherein the reduction of the level of 17-hydroxyprogesterone and the level of androstenedione is relative to the level of 17-hydroxyprogesterone and the level of androstenedione prior to administration of the pharmaceutical composition comprising the compound of Formula (I), or a pharmaceutically acceptable salt thereof. In some embodiments, the level of 17-hydroxyprogesterone is less than about 1.5 times the upper limit of normal and the level of androstenedione is less than about 1.5 times the upper limit of normal after a time period of administration of the pharmaceutical composition comprising the compound of Formula (I), or a pharmaceutically acceptable salt thereof. In some embodiments, the level of 17-hydroxyprogesterone is within normal limits and the level of androstenedione is within normal limits after a time period of administration of the pharmaceutical composition comprising the compound of Formula (I), or a pharmaceutically acceptable salt thereof.

In some embodiments, the level of 17-hydroxyprogesterone and androstenedione of the subject is reduced within a range defined by any of the preceding values.

In some embodiments, the subject exhibits a decrease in glucocorticoid burden after a time period of administration of the compound of Formula (I), or a pharmaceutically acceptable salt thereof, wherein the decrease in glucocorticoid burden is relative to the glucocorticoid burden prior to administration of the compound of Formula (I), or a pharmaceutically acceptable salt thereof. In some embodiments, one or more symptoms selected from quality of life, fatigue, sleep, insulin resistance, glucose tolerance, glucose control, dyslipidemia, hyperlipidemia, bone mineral density, bone turnover, fat mass, weight, central obesity, blood pressure, hirsutism severity, menstrual cyclicity, and fertility, is improved after a time period of administration of the compound of Formula (I), or a pharmaceutically acceptable salt thereof, wherein the improvement in the one or more symptoms is relative to the status of the one or more symptoms prior to administration of the compound of Formula (I), or a pharmaceutically acceptable salt thereof.

In some embodiments, the subject exhibits a decrease in glucocorticoid burden after a time period of administration of the pharmaceutical composition comprising the compound of Formula (I), or a pharmaceutically acceptable salt thereof, wherein the decrease in glucocorticoid burden is relative to the glucocorticoid burden prior to administration of the pharmaceutical composition comprising the compound of Formula (I), or a pharmaceutically acceptable salt thereof. In some embodiments, one or more symptoms of glucocorticoid burden selected from quality of life, fatigue, sleep, insulin resistance, glucose tolerance, glucose control, dyslipidemia, hyperlipidemia, bone mineral density, bone turnover, fat mass, weight, central obesity, blood pressure, hirsutism severity, menstrual cyclicity, and fertility, is improved after a time period of administration of the pharmaceutical composition comprising the compound of Formula (I), or a pharmaceutically acceptable salt thereof, wherein the improvement in the one or more symptoms is relative to the status of the one or more symptoms prior to administration of the pharmaceutical composition comprising the compound of Formula (I), or a pharmaceutically acceptable salt thereof.

In some embodiments, the quality of life as measured by the EuroQol 5 Dimensions 5 Levels (EQ-5D-5L) in the subject is improved after a time period of administration of the compound of Formula (I), or a pharmaceutically acceptable salt thereof, wherein the improvement in the EuroQol 5 Dimensions 5 Levels (EQ-5D-5L) is relative to the EuroQol 5 Dimensions 5 Levels (EQ-5D-5L) results prior to administration of the compound of Formula (I), or a pharmaceutically acceptable salt thereof.

In some embodiments, fatigue is reduced in the subject after a time period of administration of the compound of Formula (I), or a pharmaceutically acceptable salt thereof, wherein the reduction in fatigue is relative to the fatigue prior to administration of the compound of Formula (I), or a pharmaceutically acceptable salt thereof.

In some embodiments, sleep is increased in the subject after a time period of administration of the compound of

Formula (I), or a pharmaceutically acceptable salt thereof, wherein the increase in sleep is relative to the sleep prior to administration of the compound of Formula (I), or a pharmaceutically acceptable salt thereof.

In some embodiments, insulin resistance is reduced in the subject after a time period of administration of the compound of Formula (I), or a pharmaceutically acceptable salt thereof, wherein the reduction of insulin resistance is relative to the insulin resistance prior to administration of the compound of Formula (I), or a pharmaceutically acceptable salt thereof.

In some embodiments, glucose tolerance is reduced in the subject after a time period of administration of the compound of Formula (I), or a pharmaceutically acceptable salt thereof, wherein the reduction in glucose tolerance is relative to the glucose tolerance prior to administration of the compound of Formula (I), or a pharmaceutically acceptable salt thereof.

In some embodiments, glucose control is increased in the subject after a time period of administration of the compound of Formula (I), or a pharmaceutically acceptable salt thereof, wherein the increase in glucose control is relative to the glucose control prior to administration of the compound of Formula (I), or a pharmaceutically acceptable salt thereof.

In some embodiments, lipid levels reflecting dyslipidemia are reduced in the subject after a time period of administration of the compound of Formula (I), or a pharmaceutically acceptable salt thereof, wherein the reduction in lipid levels is relative to the lipid levels prior to administration of the compound of Formula (I), or a pharmaceutically acceptable salt thereof.

In some embodiments, lipid levels reflecting hyperlipidemia are reduced in the subject after a time period of administration of the compound of Formula (I), or a pharmaceutically acceptable salt thereof, wherein the reduction in lipid levels is relative to the lipid levels prior to administration of the compound of Formula (I), or a pharmaceutically acceptable salt thereof.

In some embodiments, bone mineral density is increased in the subject after a time period of administration of the compound of Formula (I), or a pharmaceutically acceptable salt thereof, wherein the increase in bone mineral density is relative to the bone mineral density prior to administration of the compound of Formula (I), or a pharmaceutically acceptable salt thereof.

In some embodiments, bone turnover is increased in the subject after a time period of administration of the compound of Formula (I), or a pharmaceutically acceptable salt thereof, wherein the increase in bone turnover is relative to the bone turnover prior to administration of the compound of Formula (I), or a pharmaceutically acceptable salt thereof.

In some embodiments, fat mass is decreased in the subject after a time period of administration of the compound of Formula (I), or a pharmaceutically acceptable salt thereof, wherein the decrease in fat mass is relative to the fat mass prior to administration of the compound of Formula (I), or a pharmaceutically acceptable salt thereof.

In some embodiments, body weight is decreased in the subject after a time period of administration of the compound of Formula (I), or a pharmaceutically acceptable salt thereof, wherein the decrease in body weight is relative to the body weight prior to administration of the compound of Formula (I), or a pharmaceutically acceptable salt thereof.

In some embodiments, central obesity is decreased in the subject after a time period of administration of the compound of Formula (I), or a pharmaceutically acceptable salt thereof, wherein the decrease in central obesity is relative to the central obesity prior to administration of the compound of Formula (I), or a pharmaceutically acceptable salt thereof.

In some embodiments, blood pressure is increased in the subject after a time period of administration of the compound of Formula (I), or a pharmaceutically acceptable salt thereof, wherein the increase in blood pressure is relative to the blood pressure prior to administration of the compound of Formula (I), or a pharmaceutically acceptable salt thereof.

In some embodiments, the severity of hirsutism is decreased in the subject after a time period of administration of the compound of Formula (I), or a pharmaceutically acceptable salt thereof, wherein the decrease in the severity of hirsutism is relative to the severity of hirsutism prior to administration of the compound of Formula (I), or a pharmaceutically acceptable salt thereof.

In some embodiments, menstrual cyclicity is increased in the subject after a time period of administration of the compound of Formula (I), or a pharmaceutically acceptable salt thereof, wherein the increase in menstrual cyclicity is relative to the menstrual cyclicity prior to administration of the compound of Formula (I), or a pharmaceutically acceptable salt thereof.

In some embodiments, fertility is increased in the subject after a time period of administration of the compound of Formula (I), or a pharmaceutically acceptable salt thereof, wherein the increase in fertility is relative to the fertility prior to administration of the compound of Formula (I), or a pharmaceutically acceptable salt thereof.

In some embodiments, gonadotropin levels are increased in the subject after a time period of administration of the compound of Formula (I), or a pharmaceutically acceptable salt thereof, wherein the increase in gonadotropin levels is relative to the gonadotropin levels prior to administration of the compound of Formula (I), or a pharmaceutically acceptable salt thereof.

In some embodiments, progesterone levels are increased in the subject after a time period of administration of the compound of Formula (I), or a pharmaceutically acceptable salt thereof, wherein the increase in progesterone levels is relative to the progesterone levels prior to administration of the compound of Formula (I), or a pharmaceutically acceptable

salt thereof.

In some embodiments, semen levels are increased in the subject after a time period of administration of the compound of Formula (I), or a pharmaceutically acceptable salt thereof, wherein the increase in semen levels is relative to the semen levels prior to administration of the compound of Formula (I), or a pharmaceutically acceptable salt thereof.

In some embodiments, LH (luteinizing hormone) levels are increased in the subject after a time period of administration of the compound of Formula (I), or a pharmaceutically acceptable salt thereof, wherein the increase in LH levels are relative to the LH levels prior to administration of the compound of Formula (I), or a pharmaceutically acceptable salt thereof.

In some embodiments, the time period of administration is at least about 4 weeks. In some embodiments, the time period of administration is at least about 24 weeks. In some embodiments, the time period of administration is at least about one year. In some embodiments, the time period of administration is at least 4 weeks. In some embodiments, the time period of administration is at least 24 weeks. In some embodiments, the time period of administration is at least one year. In some embodiments, the time period of administration is less than about 1 day. In some embodiments, the time period of administration is about 1, 2, 3, 4, 5, 6 or 7 days, or within a range of any of the preceding values. In some embodiments, the time period of administration is about 1, 2, 3, 4, 5, 6, 7, 8, 9, 10, 11, 12, 13, 14, 15, 16, 17, 18, 19, 20, 21, 22, 23 or 24 weeks, or within a range of any of the preceding values. In some embodiments, the time period of administration is about 1, 2, 3, 4, 5, 6, 7, 8, 9, 10, 11, or 12 months, or within a range of any of the preceding values. It is understood that comparative measurements occur preferably during the morning.

In some embodiments, the subject is a pediatric subject. In some embodiments, the pediatric subject is less than or equal to six years old. In some embodiments, the pediatric subject is greater than six years old and less than eleven years old. In some embodiments, the pediatric subject is greater than ten years old and less than fifteen years old. In some embodiments, the pediatric subject is greater than fourteen years old and less than nineteen years old.

In some embodiments, the subject is an adult subject. In some embodiments, the subject is over eighteen years old. In some embodiments, the subject is female. In some embodiments, the subject is male.

The compound of Formula (I), or a pharmaceutically acceptable salt thereof, is administered as a pharmaceutical composition of the invention. In some embodiments, the compound of Formula (I), or a pharmaceutically acceptable salt thereof, is administered as a pharmaceutical composition described in Example 12. In some embodiments, the compound of Formula (I), or a pharmaceutically acceptable salt thereof, is administered as a pharmaceutical composition described in Example 13. In some embodiments, the compound of Formula (I), or a pharmaceutically acceptable salt thereof, is administered as a hydrochloric acid salt or p-toluenesulfonic acid salt.

In some embodiments, the compound of Formula (I), or a pharmaceutically acceptable salt thereof, is administered as a p-toluenesulfonic acid salt described herein.

For the avoidance of doubt, the invention also provides the pharmaceutical composition of the invention, for use in the corresponding methods, as described herein.

**p-Toluenesulfonic Acid Salt**

In some embodiments, the compound of Formula (I) or a pharmaceutically acceptable salt thereof is 4-(2-chloro-4-methoxy -5-methylphenyl)-N-[(1S)-2-cyclopropyl-1-(3-fluoro-4-methylphenyl)ethyl]-5-methyl-N-prop-2-ynyl-1,3-thiazol-2-amine, p-toluenesulfonic acid salt.

In some embodiments, the 4-(2-chloro-4-methoxy -5-methylphenyl)-N-[(1S)-2-cyclopropyl-1-(3-fluoro-4-methylphenyl)ethyl]-5-methyl-N-prop-2-ynyl-1,3-thiazol-2-amine, p-toluenesulfonic acid salt is a crystalline salt. In some embodiments, the p-toluenesulfonic acid crystalline salt has Form 1.

In some embodiments, the p-toluenesulfonic acid crystalline salt has an X-ray powder diffraction pattern as substantially shown in Figure 27. In some embodiments, the p-toluenesulfonic acid crystalline salt has a DSC thermogram substantially as depicted in Figure 28. In some embodiments, the p-toluenesulfonic acid crystalline salt has a thermogravimetric analysis (TGA) thermogram substantially as depicted in Figure 28.

In some embodiments, the p-toluenesulfonic acid crystalline salt has at least one X-ray powder diffraction (XRPD) peak, in terms of 2-theta ($\pm$ 0.2 degrees), selected 9.1, 11.3, 13.2, 16.3 and 21.1 degrees. In some embodiments, the p-toluenesulfonic acid crystalline salt has at least two X-ray powder diffraction (XRPD) peaks, in terms of 2-theta ($\pm$ 0.2 degrees), selected from 9.1, 11.3, 13.2, 16.3 and 21.1 degrees. In some embodiments, the p-toluenesulfonic acid crystalline salt has at least three X-ray powder diffraction (XRPD) peaks, in terms of 2-theta ($\pm$ 0.2 degrees), selected from 9.1, 11.3, 13.2, 16.3 and 21.1 degrees. In some embodiments, the p-toluenesulfonic acid crystalline salt has at least four X-ray powder diffraction (XRPD) peaks, in terms of 2-theta ($\pm$ 0.2 degrees), selected from 9.1, 11.3, 13.2, 16.3 and 21.1 degrees. In some embodiments, the p-toluenesulfonic acid crystalline salt has characteristic X-ray powder diffraction (XRPD) peaks, in terms of 2-theta ($\pm$ 0.2 degrees), at 9.1, 11.3, 13.2, 16.3 and 21.1 degrees. In some embodiments, the p-toluenesulfonic acid crystalline salt has an endothermic peak having an onset of melt at about 156 °C (22.2 J/g) in a differential scanning calorimetry (DSC) thermogram.

**Liquid** Formulations

The invention provides a pharmaceutical composition in oral solution dosage form comprising:

(a) a compound of Formula (I):

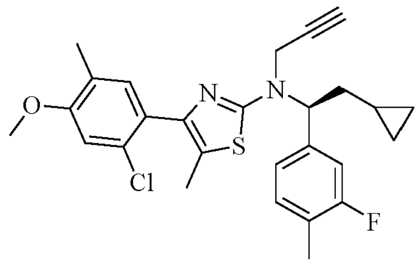

(I)

(4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine),
or a pharmaceutically acceptable salt thereof;
(b) one or more of a sweetener, an anti-oxidant, and a flavor; and
(c) a liquid vehicle, wherein the liquid vehicle is selected from medium-chain triglycerides, propylene glycol dicaprylate/dicaprate, glycerin, propylene glycol, polyethylene glycol, olive oil, soybean oil, corn oil, and diethylene glycol monoethyl ether.

In some embodiments, the pharmaceutical composition comprises about 1 w/v% to about 50 w/v% of the compound of Formula (I), or a pharmaceutically acceptable salt thereof, based on the weight of the free base. In some embodiments, the pharmaceutical composition comprises about 1 w/v% to about 10 w/v% of the compound of Formula (I), or a pharmaceutically acceptable salt thereof, based on the weight of the free base. In some embodiments, the pharmaceutical composition comprises about 5 w/v% of the compound of Formula (I), or a pharmaceutically acceptable salt thereof, based on the weight of the free base. In some embodiments, the pharmaceutical composition comprises about 1, 2, 3, 4, 5, 6, 7, 8, 9, or 10 w/v% of the compound of Formula (I), or a pharmaceutically acceptable salt thereof, based on the weight of the free base, or within a range of any of the preceding values.

In some embodiments, the pharmaceutical composition comprises a sweetener. A sweetener is a formulation component added to improve taste. In some embodiments, the pharmaceutical composition comprises about 0.01 w/v% to about 1.5 w/v% of the sweetener. In some embodiments, the pharmaceutical composition comprises about 0.1 w/v% to about 0.5 w/v% of the sweetener. In some embodiments, the pharmaceutical composition comprises about 0.15 w/v% of the sweetener. In some embodiments, the pharmaceutical composition comprises about 0.1, 0.2, 0.3, 0.4, or 0.5 w/v% of the sweetener, or within a range of any of the preceding values.

In some embodiments, the sweetener is selected from saccharin, sucrose, sucralose, aspartame, dextrose, fructose, maltitol, mannitol, sorbitol, and avantame. In some embodiments, the sweetener is saccharin.

In some embodiments, the pharmaceutical composition comprises an anti-oxidant. An anti-oxidant is a formulation component included to improve stability by preventing oxidation. In some embodiments, the pharmaceutical composition comprises about 0.01 w/v% to about 1.5 w/v% of the anti-oxidant. In some embodiments, the pharmaceutical composition comprises about 0.1 w/v% to about 0.5 w/v% of the anti-oxidant. In some embodiments, the pharmaceutical composition comprises about 0.17 w/v% of the anti-oxidant. In some embodiments, the pharmaceutical composition comprises about 0.1, 0.2, 0.3, 0.4, or 0.5 w/v% of the anti-oxidant, or within a range of any of the preceding values.

In some embodiments, the anti-oxidant is selected from butylated hydroxytoluene, vitamin E TPGS, butylated hydroxyanisole, ascorbic acid, lecithin, tert-butylhydroquinone, and citric acid. In some embodiments, the anti-oxidant is butylated hydroxytoluene.

In some embodiments, the pharmaceutical composition comprises a flavor. A flavor is a formulation component added to mask taste through aromatics. In some embodiments, the pharmaceutical composition comprises about 0.01 w/v% to about 0.5 w/v% of the flavor. In some embodiments, the pharmaceutical composition comprises about 0.05 w/v% to about 0.2 w/v% of the flavor. In some embodiments, the pharmaceutical composition comprises about 0.10 w/v% of the flavor. In some embodiments, the pharmaceutical composition comprises about 0.05, 0.06, 0.07, 0.08, 0.09, 0.10, 0.11, 0.12, 0.13, 0.14, 0.15, 0.16, 0.17, 0.18, 0.19 or 0.2 w/v% of the flavor, or within a range of any of the preceding values.

In some embodiments, the flavor is selected from FONA orange flavor, FONA Juicy Flavor, FONA Grape Flavor, Firmenich SA Lemon Flavor, Firmenich Tetrarome Orange Flavor, IFF Cherry Flavor, and IFF Grape Flavor. In some

embodiments, the flavor is FONA orange flavor.

A liquid vehicle is a solvent capable of dissolving or partially dissolving the compound of Formula (I), or a pharmaceutically acceptable salt thereof, for the purposes of delivery as an oral dosing solution. In some embodiments, the pharmaceutical composition comprises about 50 w/v% to about 99.9 w/v% of the liquid vehicle. In some embodiments, the pharmaceutical composition comprises about 90 w/v% to about 99 w/v% of the liquid vehicle. In some embodiments, the pharmaceutical composition comprises about 92 w/v% to about 97 w/v% of the liquid vehicle. In some embodiments, the pharmaceutical composition comprises about 94.6 w/v% of the liquid vehicle. In some embodiments, the pharmaceutical composition comprises about 90, 91, 92, 93, 94, 95, 96, 97, 98 or 99 w/v% of the liquid vehicle, or within a range of any of the preceding values.

According to the invention, the liquid vehicle is selected from medium-chain triglycerides, propylene glycol dicaprylate/dicaprate, glycerin, propylene glycol, polyethylene glycol, olive oil, soybean oil, corn oil, and transcutol. In some embodiments, the liquid vehicle is medium-chain triglycerides. In some embodiments, the medium-chain triglycerides is Labrafac Lipophile WL1349.

In some embodiments, the pharmaceutical composition further comprises a surfactant. A surfactant is a formulation component added to improve solubility or emulsion properties. In some embodiments, the pharmaceutical composition comprises about 1 w/v% to about 50 w/v% of the surfactant. In some embodiments, the pharmaceutical composition comprises about 10 w/v% to about 30 w/v% of the surfactant. In some embodiments, the pharmaceutical composition comprises about 20 w/v% of the surfactant. In some embodiments, the pharmaceutical composition comprises about 10, 11, 12, 13, 14, 15, 16, 17, 18, 19, 20, 21, 22, 23, 24, 25, 26, 27, 28, 29 or 30 w/v% of the surfactant, or within a range of any of the preceding values.

In some embodiments, the surfactant is selected from oleoyl polyoxyl-6 glycerides, linoleoyl polyoxyl-6 glycerides, Polysorbate 80, Polysorbate 20, vitamin E polyethylene glycol succinate, Gelucire, lauroyl polyoxyl-32 glycerides, sodium lauryl sulfate, Poloxamer, corn oil PEG-6 esters, and hydrogenated palm/palm kernel oil PEG-6 esters. In some embodiments, the surfactant is oleoyl polyoxyl-6 glycerides. In some embodiments, the oleoyl polyoxyl-6 glycerides is LABRAFIL M 1944 CS.

In some embodiments, the pharmaceutical composition comprises about 50 w/v% to about 90 w/v% of the liquid vehicle. In some embodiments, the pharmaceutical composition comprises about 70 w/v% to about 80 w/v% of the liquid vehicle. In some embodiments, the pharmaceutical composition comprises about 75 w/v% of the liquid vehicle. In some embodiments, the pharmaceutical composition comprises about 74.6 w/v% of the liquid vehicle. In some embodiments, the pharmaceutical composition comprises about 70, 71, 72, 73, 74, 75, 76, 77, 78, 79, or 80 w/v% of the liquid vehicle, or within a range of any of the preceding values.

In some embodiments, the liquid vehicle is selected from medium-chain triglycerides, propylene glycol dicaprylate/dicaprate, glycerin, propylene glycol, polyethylene glycol, olive oil, soybean oil, corn oil, and transcutol. In some embodiments, the liquid vehicle is medium-chain triglycerides. In some embodiments, the medium-chain triglycerides is Labrafac Lipophile WL1349.

In some embodiments, the pharmaceutical composition comprises:

(a) 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof;
(b) a sweetener;
(c) an anti-oxidant;
(d) a flavor; and
(e) the liquid vehicle.

In some embodiments, the pharmaceutical composition further comprises a surfactant.

In some embodiments, the pharmaceutical composition comprises:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of a sweetener;
(c) about 0.1 w/v% to about 0.2 w/v% of an anti-oxidant;
(d) about 0.05 w/v% to about 0.2 w/v% of a flavor; and
(e) about 92 w/v% to about 97 w/v% of the liquid vehicle.

In some embodiments, the pharmaceutical composition comprises:

(a) about 5 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.15 w/v% of a sweetener;
(c) about 0.17 w/v% of an anti-oxidant;
(d) about 0.1 w/v% of a flavor; and
(e) about 94.6 w/v% of the liquid vehicle.

In some embodiments, the pharmaceutical composition comprises:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of a sweetener;
(c) about 0.1 w/v% to about 0.2 w/v% of an anti-oxidant;
(d) about 0.05 w/v% to about 0.2 w/v% of a flavor;
(e) about 15 w/v% to about 25 w/v% of a surfactant; and
(f) about 70 w/v% to about 80 w/v% of the liquid vehicle.

In some embodiments, the pharmaceutical composition comprises:

(a) about 5 w/v% of 4-(2-chloro-4-methoxy-5-methylphenyl)-N-[(1S)-2-cyclopropyl-1-(3-fluoro-4-methylphenyl) ethyl]-5-methyl-N-prop-2-ynyl-1,3-thiazol-2-amine, or a pharmaceutically acceptable salt thereof, based on the weight of the free base;
(b) about 0.15 w/v% of a sweetener;
(c) about 0.17 w/v% of an anti-oxidant;
(d) about 0.1 w/v% of a flavor;
(e) about 20 w/v% of a surfactant; and
(f) about 75 w/v% of the liquid vehicle.

In some embodiments, the pharmaceutical composition comprises:

(a) 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof;
(b) saccharin;
(c) butylated hydroxytoluene;
(d) FONA orange flavor; and
(e) medium-chain triglycerides.

In some embodiments, the pharmaceutical composition further comprises oleoyl polyoxyl-6 glycerides.
In some embodiments, the pharmaceutical composition comprises:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of saccharin;
(c) about 0.1 w/v% to about 0.2 w/v% of butylated hydroxytoluene;
(d) about 0.05 w/v% to about 0.2 w/v% of FONA orange flavor; and
(e) about 92 w/v% to about 97 w/v% of medium-chain triglycerides.

In some embodiments, the pharmaceutical composition comprises:

(a) about 5 w/v% of 4-(2-chloro-4-methoxy-5-methylphenyl)-N-[(1S)-2-cyclopropyl-1-(3-fluoro-4-methylphenyl) ethyl]-5-methyl-N-prop-2-ynyl-1,3-thiazol-2-amine, or a pharmaceutically acceptable salt thereof, based on the weight of the free base;
(b) about 0.15 w/v% of saccharin;
(c) about 0.17 w/v% of butylated hydroxytoluene;
(d) about 0.1 w/v% of FONA orange flavor; and
(e) about 94.6 w/v% of medium-chain triglycerides.

In some embodiments, the pharmaceutical composition comprises:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of saccharin;
(c) about 0.1 w/v% to about 0.2 w/v% of butylated hydroxytoluene;
(d) about 0.05 w/v% to about 0.2 w/v% of FONA orange flavor;
(e) about 15 w/v% to about 25 w/v% of oleoyl polyoxyl-6 glycerides; and
(f) about 70 w/v% to about 80 w/v% of medium-chain triglycerides.

In some embodiments, the pharmaceutical composition comprises:

(a) about 5 w/v% of 4-(2-chloro-4-methoxy-5-methylphenyl)-N-[(1S)-2-cyclopropyl-1-(3-fluoro-4-methylphenyl) ethyl]-5-methyl-N-prop-2-ynyl-1,3-thiazol-2-amine, or a pharmaceutically acceptable salt thereof, based on the weight of the free base;
(b) about 0.15 w/v% of saccharin;
(c) about 0.17 w/v% of butylated hydroxytoluene;
(d) about 0.1 w/v% of FONA orange flavor;
(e) about 20 w/v% of oleoyl polyoxyl-6 glycerides; and
(f) about 75 w/v% of medium-chain triglycerides.

In some embodiments, the pharmaceutical composition comprises the compound of Formula (I) as a free base.
In some embodiments, the pharmaceutical composition is formulated in unit dosage form, wherein the compound of Formula (I), or a pharmaceutically acceptable salt thereof, is present in an amount of about 5 mg/mL to about 200 mg/mL, based on the weight of the free base. In some embodiments, the compound of Formula (I), or a pharmaceutically acceptable salt thereof, is present in the unit dosage form in an amount of about 75 mg/mL to about 150 mg/mL, based on the weight of the free base. In some embodiments, the compound of Formula (I), or a pharmaceutically acceptable salt thereof, is present in the unit dosage form in an amount of about 50 mg/mL, based on the weight of the free base. In some embodiments, the compound of Formula (I), or a pharmaceutically acceptable salt thereof, is present in the unit dosage form in an amount of about 100 mg/mL, based on the weight of the free base.
In some embodiments, the liquid pharmaceutical composition has a viscosity between about 1 to about 50 centipoise at about 25 °C.
Some embodiments provide a method for preparing a pharmaceutical composition comprising:

(a) mixing a liquid vehicle with a sweetener;
(b) mixing the mixture of step (a) with an anti-oxidant and a flavor;
(c) mixing the compound of Formula (I), or a pharmaceutically acceptable salt thereof, with the mixture of step (b); and
(d) mixing the mixture of step (c) with an additional portion of the liquid vehicle.

In some embodiments, step (a) of the method comprises mixing a liquid vehicle with a sweetener and a surfactant.

**Deuterated Compounds**

Also disclosed herein are compounds having the structure of the following formula (II):

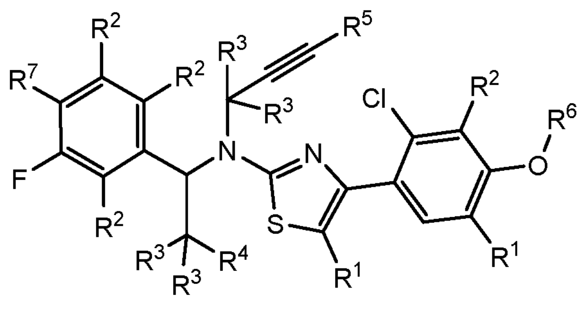

(II)

or a pharmaceutically acceptable salt thereof, wherein:

each $R^1$ is independently $C(R^A)_3$;
each $R^A$ is independently hydrogen or deuterium;
each $R^2$ is independently hydrogen or deuterium;
each $R^3$ is independently hydrogen or deuterium;
$R^4$ is

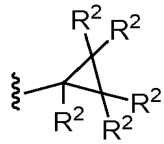
;

$R^5$ is hydrogen or deuterium;
$R^6$ is $C(R^A)_3$; and
$R^7$ is $C(R^B)_3$, wherein at least one of $R^A$, $R^B$, $R^2$, $R^3$ and $R^5$ is deuterium.

With regard to the compounds provided herein, when a particular atomic position is designated as having deuterium or "D" or "d", it is understood that the abundance of deuterium at that position is substantially greater than the natural abundance of deuterium, which is about 0.015%. A position designated as having deuterium typically has a minimum isotopic enrichment factor of, in certain embodiments, at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium position.

In some embodiments, the compound of Formula (II) may be one of the following, or a pharmaceutically acceptable salt thereof:

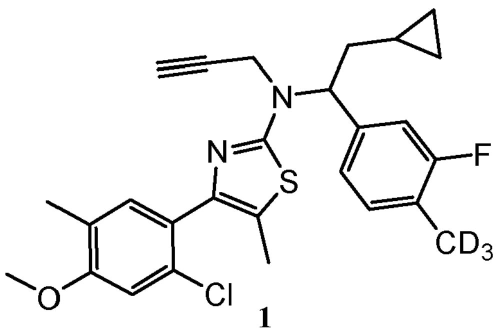
**1**

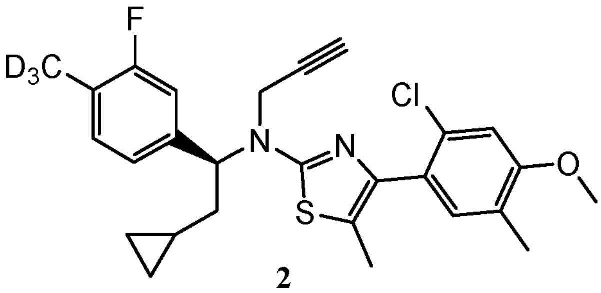
**2**

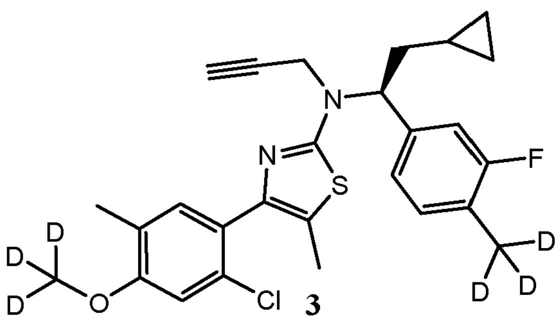
**3**

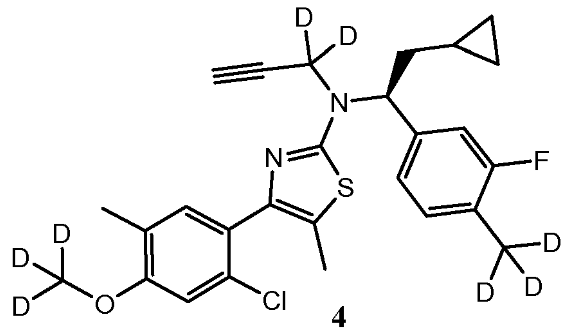

4

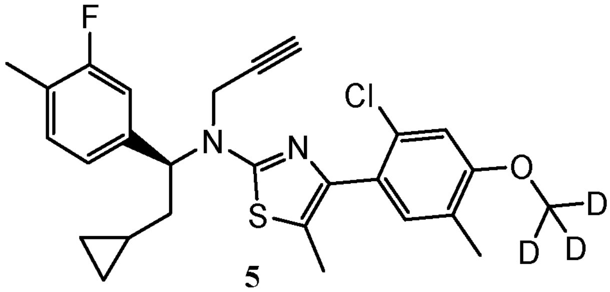

5

**Pharmaceutical compositions**

The methods and uses disclosed herein can comprise administering the compound of Formula (I) as a pharmaceutical composition of the invention.

In some embodiments of the methods described herein, the 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof is administered in a pharmaceutical composition further comprising one or more pharmaceutically acceptable excipients.

Also provided herein is a pharmaceutical composition of the invention, for use in any of the methods described herein.

In some embodiments described herein, the methods and uses described herein comprise administering a pharmaceutical composition that does not comprise a spray-dried dispersion of the compound of Formula (I), as specified, e.g., in Reference Example 1. Accordingly, in some embodiments, the pharmaceutical composition does not comprise any of the following polymers: hydroxypropylmethylcellulose acetate succinate-L (HPMCAS-L); polyvinyl pyrrolidone vinyl acetate 64 (PVP/VA 64); HPMCAS-M; and methyl methacrylate copolymer (1:1) (Eudragit® L100).

In some embodiments, the methods and uses described herein comprise administering a pharmaceutical composition that is not the reference formulation described in Reference Example 9. Accordingly, in some embodiments, the pharmaceutical composition does not comprise at least three of the excipients selected from caprylic/capric triglyceride (Labrafac® Lipophile, Gattefossé, France); propylene glycol dicrapolate/dicaprate (Labrafac® PG, Gattefossé, France); oleoyl polyoxyl-6 glycerides (Labrafil® M 1944 CS, Gattefossé, France); polysorbate 20; polyoxyl castor oil (Kolliphor® RH 40, BASF, Germany); polyoxyl 15 hydroxystearate (Kolliphor® HS 15, BASF, Germany); lauroyl polyoxyl-32 glycerides (Gelucire® 44/14, Gattefossé, France); d-$\alpha$-tocopheryl polyethylene glycol 1000 succinate (TPGS); and diethylene glycol monoethyl ether (Transcutol®, Gattefossé, France).In some embodiments, the methods and uses described herein comprise administering a pharmaceutical composition that is the formulation described in Example 12. In some embodiments, the methods and uses described herein comprise administering a pharmaceutical composition that is the formulation described in Example 13.

In some embodiments of the pharmaceutical compositions disclosed herein, the pharmaceutically acceptable excipient is selected from the group consisting of a filler, a lubricant, and combinations thereof. In some embodiments, the pharmaceutical excipients are selected from the group consisting of a glidant, a filler, a disintegrant, a lubricant, and a combination thereof.

In some embodiments, the pharmaceutical composition includes a filler. In some embodiments, the filler is selected from among binders, diluents, disintegrants, glidants, surfactants, and combinations thereof.

In some embodiments, the filler include saccharides (e.g., sugars, starch, and cellulose), gelatin, calcium carbonate, and synthetic polymers (e.g., polyvinylpyrrolidone, polyethylene glycol, and poloxamers (e.g., Poloxamer 188, a copolymer of polyoxyethylene and polyoxypropylene)). Exemplary fillers include, but are not limited to, glucose, sucrose,

lactose, a starch, including modified starches such as sodium starch glycolate (e.g., Explotab®), xylitol, dextrin, saccharose, sorbitol, mannitol (e.g., Parteck® M 200 (mannitol with an average particle size of about 50 μm to about 500 μm) or Parteck® M 100 (mannitol with an average particle size of less than 212 μm)), a cellulose, a polyvinylpyrrolidone, a polyethylene glycol, a polyvinyl alcohol, a polymethacrylate, dibasic calcium phosphate, magnesium stearate, calcium stearate, sodium stearate, stearic acid, hydrogenated vegetable oils, a mineral oil, sodium lauryl sulfate, magnesium lauryl sulfate, glyceryl palmitostearate, sodium benzoate, sodium stearyl fumarate, colloidal silicon dioxide, sodium benzoate, sodium oleate, sodium acetate, aliginic acid, alginates (e.g., sodium alginate), calcium silicate, and ion exchange resins. Exemplary cellulose fillers include microcrystalline cellulose (e.g., Avicel® PH-101 (microcrystalline cellulose with an average particle size of approximately 50 μm) or Avicel® PH 200 (microcrystalline cellulose with an average particle size of approximately 180 μm)), methyl cellulose, ethyl cellulose, hydroxypropyl cellulose, and hydroxypropylmethylcellulose. Exemplary fillers include cross-linked polyvinylpyrrolidone such as with an average particle size of 90 μm to 130 μm) or with an average particle size of 10 μm to 30 μm). Other fillers known to those of skill in the art are also contemplated as being useful when formulated in the pharmaceutical compositions described herein.

In some embodiments, the filler is a binder. Binders include agents that hold the active pharmaceutical ingredient and inactive ingredients together in a cohesive mix. Exemplary binders include, but are not limited to, glucose, sucrose, lactose, a starch, including modified starches such as sodium starch glycolate (Explotab®), xylitol, dextrin, saccharose, sorbitol, mannitol (e.g., Parteck® M 200 (mannitol with an average particle size of about 50 μm to about 500 μm), Parteck® M 100 (mannitol with an average particle size of less than 212 μm)), gelatin, gum tragacanth, acacia mucilage, a cellulose, a polyvinylpyrrolidone, a polyethylene glycol, a polyvinyl alcohol, a polymethacrylate, and sodium starch glycolate. Exemplary cellulose fillers include microcrystalline cellulose (e.g., Avicel® PH-101 (microcrystalline cellulose with an average particle size of approximately 50 μm) or Avicel® PH 200 (microcrystalline cellulose with an average particle size of approximately 180 μm)), cellulose ethers, methyl cellulose, ethyl cellulose, croscarmellose sodium, sodium carboxy methyl cellulose starches, hydroxypropyl cellulose, and hydroxypropyl methyl cellulose. Exemplary polyvinylpyrrolidone fillers include cross-linked polyvinylpyrrolidone such as Kollidon® CL (crospovidone with an average particle size of 90 μm to 130 μm) or Kollidon® CL-SF (crospovidone with an average particle size of 10 μm to 30 μm). Other binders known to those of skill in the art are also contemplated as being useful when formulated in the compositions described herein.

In some embodiments, the filler is a diluent. Suitable diluents include, but are not limited to, lactose, mannitol, isomalt, sucrose, dextrose, and sorbitol.

In some embodiments, the filler is a disintegrant. Disintegrants include any agent that promotes breakup of the formulation in an aqueous environment, for example, to promote more rapid release of the active pharmaceutical ingredient (e.g., the compound of Formula (I), or a pharmaceutically acceptable salt thereof). Exemplary disintegrants include, but are not limited to, starch and modified starches, such as corn starch, potato starch, sodium starch glycolate or croscarmellose sodium, alginic acid, alginates, such as sodium alginate, polyvinylpyrrolidone, bentonite, methylcellulose, agar, carboxymethylcellulose, crospovidone, acid-carbonate effervescent systems, such as citric acid with bicarbonate salts, and ion exchange resins. Other disintegrants known to those of skill in the art are also contemplated as being useful when formulated in the compositions described herein.

In some embodiments, the pharmaceutical composition comprises a disintegrant. In some embodiments, the pharmaceutical composition comprises about 1 w/w% to about 30 w/w% of the disintegrant. In some embodiments, the pharmaceutical composition comprises about 5 w/w% to about 15 w/w% of the disintegrant. In some embodiments, the pharmaceutical composition comprises about 10 w/w% of the disintegrant. In some embodiments, the disintegrant is selected from croscarmellose sodium, sodium starch glycolate, crospovidone, and sodium bicarbonate. In some embodiments, the disintegrant is croscarmellose sodium.

In some embodiments, the filler is a glidant. Glidants can be used to improve the flowability of a powder or granules or both. Glidants include, but are not limited to, silicone dioxide, such as colloidal silicon dioxide or hydrated silicon dioxide, magnesium silicate, magnesium aluminometasilicate, talc, starch, calcium silicate, light anhydrous silicic acid, and silicon dioxide aerogels.

In some embodiments, the pharmaceutical composition comprises a glidant. In some embodiments, the pharmaceutical composition comprises about 0.1 w/w% to about 5 w/w% of the glidant. In some embodiments, the pharmaceutical composition comprises about 0.1 w/w% to about 1 w/w% of the glidant. In some embodiments, the pharmaceutical composition comprises about 0.67 w/w% of the glidant. In some embodiments, the glidant is selected from calcium silicate, silicon dioxide, and talc. In some embodiments, the glidant is calcium silicate.

In some embodiments, the filler is a surfactant, wetting agent, solubilizer, or combination thereof. Examples include, but are not limited to, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., Tween®), polyoxyethylene stearates, sodium dodecylsulfate, tyloxapol (a nonionic liquid polymer of the alkyl aryl polyether alcohol type, also known as superinone or triton). Other examples include, but are not limited to, poloxamers such as Pluronic® F68, F127, and F108, which are block copolymers of ethylene oxide and propylene oxide, and polyxamines such as Tetronic® 908 (also known as Poloxamine® 908), which is a

tetrafunctional block copolymer derived from sequential addition of propylene oxide and ethylene oxide to ethylenediamine (available from BASF), dextran, lecithin, dialkylesters of sodium sulfosuccinic acid, such as Aerosol® OT, which is a dioctyl ester of sodium sulfosuccinic acid (available from American Cyanimid), Duponol® P, which is a sodium lauryl sulfate (available from DuPont), Triton® X-200, which is an alkyl aryl polyether sulfonate (available from Rohm and Haas), Tween® 20 and Tween® 80, which are polyoxyethylene sorbitan fatty acid esters (available from ICI Specialty Chemicals), Carbowax™ 3550 and 934, which are polyethylene glycols (available from Union Carbide), Crodesta™ F-110, which is a mixture of sucrose stearate and sucrose distearate, and Crodesta™ SL-40 (both available from Croda Inc.), and SA90HCO, which has the chemical formula $C_{18}H_{37}$-$CH_2(CON(CH_3)CH_2(CHOH)_4CH_2OH)_2$.

In some embodiments, the pharmaceutical composition comprises a filler. In some embodiments, the pharmaceutical composition comprises about 30 w/w% to about 99 w/w% of the filler. In some embodiments, the pharmaceutical composition comprises about 50 w/w% to about 90 w/w% of the filler. In some embodiments, the pharmaceutical composition comprises about 75.5 w/w% of the filler. In some embodiments, the filler is selected from mannitol, microcrystalline cellulose, lactose, starch, isomalt, silicified microcrystalline cellulose, Dicalcium Phosphate, maltodextrin, and a combination thereof. In some embodiments, the filler is a combination of mannitol and microcrystalline cellulose. In some embodiments, the pharmaceutical composition comprises about 30 w/w% to about 80 w/w% of mannitol. In some embodiments, the pharmaceutical composition comprises about 50 w/w% to about 60 w/w% of mannitol. In some embodiments, the pharmaceutical composition comprises about 56 w/w% of mannitol. In some embodiments, the pharmaceutical composition comprises about 1 w/w% to about 50 w/w% of microcrystalline cellulose. In some embodiments, the pharmaceutical composition comprises about 10 w/w% to about 30 w/w% of microcrystalline cellulose. In some embodiments, the pharmaceutical composition comprises about 20 w/w% of microcrystalline cellulose. In some embodiments, the pharmaceutical composition comprises about 56 w/w% of mannitol and about 20 w/w% of microcrystalline cellulose.

In some embodiments, the pharmaceutical composition includes a lubricant. Lubricants are agents added to pharmaceutical formulations to reduce friction during processing and prevent ingredients from clumping together. Exemplary lubricants include, but are not limited to, talc, starch, magnesium stearate, calcium stearate, sodium stearate, zinc stearate, stearic acid, vegetable stearin, adipic acid, waxy fatty acids, such as glyceryl behenate, a hydrogenated vegetable oil, a mineral oil, a polyethylene glycol, lycopodium, sodium lauryl sulfate, magnesium lauryl sulfate, glyceryl palmitostearate, sodium benzoate, sodium chloride, sterotex, glycerol monostearate, sodium stearyl fumarate, colloidal silicon dioxide, sodium benzoate, sodium oleate, and sodium acetate. Other lubricants known to those of skill in the art are also contemplated as being useful when formulated in the compositions described herein.

In some embodiments, the pharmaceutical composition comprises a lubricant. In some embodiments, the pharmaceutical composition comprises about 0.1 w/w% to about 10 w/w% of the lubricant. In some embodiments, the pharmaceutical composition comprises about 0.1 w/w% to about 1 w/w% of the lubricant. In some embodiments, the pharmaceutical composition comprises about 0.5 w/w% of the lubricant. In some embodiments, the pharmaceutical lubricant is selected from sodium stearyl fumarate, magnesium stearate, stearic acid sodium lauryl sulfate, sodium oleate, glyceryl behenate, and talc. In some embodiments, the lubricant is sodium stearyl fumarate.

Additional excipients can be included in the pharmaceutical formulations of the present invention. Further examples of excipients include, but are not limited to, pigments, colorants, flavoring agents, preservatives, and sweeteners. Flavors and colors can be added to improve the taste or appearance of a formulation. Examples of preservatives used in pharmaceutical compositions are aromatic alcohols, such as benzyl or phenol alcohol, antioxidants such as vitamin A, vitamin E, vitamin C, and selenium, amino acids such as cysteine and methionine, citric acid and sodium citrate, or synthetic preservatives such as methyl paraben and propyl paraben. Sweeteners can be added to make the ingredients more palatable, especially in liquids like syrups.

Also provided are methods for preparing a pharmaceutical composition, such as a pharmaceutical composition of the present invention.

The pharmaceutical compositions of the present invention are formulated for oral administration. In preparing the compositions in oral dosage form, any of the usual pharmaceutical media can be employed.

The oral pharmaceutical dosage forms of the invention are liquid.

The pharmaceutical compositions of the present disclosure can contain, per dosage unit, an amount of the active ingredient necessary to deliver an effective dose as described above.

In some embodiments, the pharmaceutical compositions of the present disclosure are formulated in unit dosage form. In some embodiments, the compound of Formula (I), or pharmaceutically acceptable salt thereof, is present in an amount of about 5 mg to about 200 mg in the unit dosage form. For example, about 5 mg to about 175 mg, about 5 mg to about 150 mg, about 5 mg to about 125 mg, about 5 mg to about 100 mg, about 5 mg to about 75 mg, about 5 mg to about 50 mg, about 5 mg to about 25 mg, about 25 mg to about 200 mg, about 25 mg to about 175 mg, about 25 mg to about 150 mg, about 25 mg to about 125 mg, about 25 mg to about 100 mg, about 25 mg to about 75 mg, about 25 mg to about 50 mg, about 50 mg to about 200 mg, about 50 mg to about 175 mg, about 50 mg to about 150 mg, about 50 mg to about 125 mg, about 50 mg to about 100 mg, about 50 mg to about 75 mg, about 75 mg to about 200 mg, about 75 mg to about 175 mg,

about 75 mg to about 150 mg, about 75 mg to about 125 mg, about 75 mg to about 100 mg, about 100 mg to about 200 mg, about 100 mg to about 175 mg, about 100 mg to about 150 mg, about 100 mg to about 125 mg, about 125 mg to about 200 mg, about 125 mg to about 175 mg, about 125 mg to about 150 mg, about 150 mg to about 200 mg, about 150 mg to about 175 mg, or about 175 mg to about 200 mg in the unit dosage form. In some embodiments, the compound of Formula (I), or pharmaceutically acceptable salt thereof, is present in an amount of about 25 mg to about 125 mg in the unit dosage form. In some embodiments, the compound of Formula (I), or pharmaceutically acceptable salt thereof, is present in an amount of about 75 mg to about 150 mg in the unit dosage form. In some embodiments, the compound of Formula (I), or pharmaceutically acceptable salt thereof, is present in an amount of about 5 mg, about 10 mg, about 25 mg, about 35 mg, about 50 mg, about 65 mg, about 75 mg, about 90 mg, about 100 mg, about 125 mg, about 150 mg, about 175 mg, or about 200 mg in the unit dosage form, or within a range defined by any of the preceding values. In some embodiments, the compound of Formula (I), or pharmaceutically acceptable salt thereof, is present in an amount of about 50 mg in the unit dosage form. In some embodiments, the compound of Formula (I), or pharmaceutically acceptable salt thereof, is present in an amount of about 100 mg in the unit dosage form.. In some embodiments, the compound of Formula (I), or pharmaceutically acceptable salt thereof, is present in an amount of about 25 mg in the unit dosage form. In some embodiments, the compound of Formula (I), or pharmaceutically acceptable salt thereof, is present in an amount of 5 mg to 250 mg in the unit dosage form. For example, 5 mg to 175 mg, 5 mg to 150 mg, 5 mg to 125 mg, 5 mg to 100 mg, 5 mg to 75 mg, 5 mg to 50 mg, 5 mg to 25 mg, 25 mg to 200 mg, 25 mg to 175 mg, 25 mg to 150 mg, 25 mg to 125 mg, 25 mg to 100 mg, 25 mg to 75 mg, 25 mg to 50 mg, 50 mg to 200 mg, 50 mg to 175 mg, 50 mg to 150 mg, 50 mg to 125 mg, 50 mg to 100 mg, 50 mg to 75 mg, 75 mg to 200 mg, 75 mg to 175 mg, 75 mg to 150 mg, 75 mg to 125 mg, 75 mg to 100 mg, 100 mg to 200 mg, 100 mg to 175 mg, 100 mg to 150 mg, 100 mg to 125 mg, 125 mg to 200 mg, 125 mg to 175 mg, 125 mg to 150 mg, 150 mg to 200 mg, 150 mg to 175 mg, or 175 mg to 200 mg in the unit dosage form. In some embodiments, the compound of Formula (I), or pharmaceutically acceptable salt thereof, is present in an amount of 25 mg to 125 mg in the unit dosage form. In some embodiments, the compound of Formula (I), or pharmaceutically acceptable salt thereof, is present in an amount of 75 mg to 150 mg in the unit dosage form. In some embodiments, the compound of Formula (I), or pharmaceutically acceptable salt thereof, is present in an amount of 5 mg, 10 mg, 25 mg, 35 mg, 50 mg, 65 mg, 75 mg, 90 mg, 100 mg, 125 mg, 150 mg, 175 mg, or 200 mg in the unit dosage form, or within a range defined by any of the preceding values. In some embodiments, the compound of Formula (I), or pharmaceutically acceptable salt thereof, is present in an amount of 50 mg in the unit dosage form. In some embodiments, the compound of Formula (I), or pharmaceutically acceptable salt thereof, is present in an amount of 100 mg in the unit dosage form.

In some embodiments, the compound of Formula (I), or a pharmaceutically acceptable salt thereof, is administered in a dose of about 25 mg, based on the weight of the free base. In some embodiments, the compound of Formula (I), or a pharmaceutically acceptable salt thereof, is administered in a dose of about 50 mg, based on the weight of the free base. In some embodiments, the compound of Formula (I), or a pharmaceutically acceptable salt thereof, is administered in a dose of about 75 mg, based on the weight of the free base. In some embodiments, the compound of Formula (I), or a pharmaceutically acceptable salt thereof, is administered in a dose of about 100 mg, based on the weight of the free base.

In some embodiments, the pharmaceutical composition is administered in a dose of about 25 mg of the compound of Formula (I), or a pharmaceutically acceptable salt thereof, based on the weight of the free base. In some embodiments, the pharmaceutical composition is administered in a dose of about 50 mg of the compound of Formula (I), or a pharmaceutically acceptable salt thereof, based on the weight of the free base. In some embodiments, the pharmaceutical composition is administered in a dose of about 75 mg of the compound of Formula (I), or a pharmaceutically acceptable salt thereof, based on the weight of the free base. In some embodiments, the pharmaceutical composition is administered in a dose of about 100 mg of the compound of Formula (I), or a pharmaceutically acceptable salt thereof, based on the weight of the free base.

In some embodiments, the pharmaceutical composition comprises about 25 mg of the compound of Formula (I), or a pharmaceutically acceptable salt thereof, based on the weight of the free base. In some embodiments, the pharmaceutical composition comprises about 50 mg of the compound of Formula (I), or a pharmaceutically acceptable salt thereof, based on the weight of the free base. In some embodiments, the pharmaceutical composition comprises about 75 mg of the compound of Formula (I), or a pharmaceutically acceptable salt thereof, based on the weight of the free base. In some embodiments, the pharmaceutical composition comprises about 100 mg of the compound of Formula (I), or a pharmaceutically acceptable salt thereof, based on the weight of the free base.

The daily dosage of the compound of Formula (I), or a pharmaceutically acceptable salt thereof, in a pharmaceutical composition as described in the present disclosure can be varied over a wide range from about 1.0 mg to about 10,000 mg per adult human per day, or higher, or any range therein. In some embodiments, an effective amount of the compound of Formula (I), or pharmaceutically acceptable salt thereof, can be supplied at a dosage level of from about 0.1 mg/kg to about 1000 mg/kg of body weight per day, or any range therein, for example, the range can be from about 0.5 mg/kg to about 500 mg/kg, about 1.0 mg/kg to about 250 mg/kg, about 0.1 mg/kg to about 100 mg/kg, about 0.1 mg/kg to about 50.0 mg/kg of body weight per day, about 0.1 mg/kg to about 15.0 mg/kg of body weight per day, about 0.5 mg/kg to about 7.5 mg/kg of body weight per day, or any amount to range therein. In some embodiments, an effective amount of the

compound of Formula (I), or pharmaceutically acceptable salt thereof, can be supplied at a dosage level of from 0.1 mg/kg to 1000 mg/kg of body weight per day, or any range therein, for example, the range can be from 0.5 mg/kg to 500 mg/kg, 1.0 mg/kg to 250 mg/kg, 0.1 mg/kg to 100 mg/kg, 0.1 mg/kg to 50.0 mg/kg of body weight per day, 0.1 mg/kg to 15.0 mg/kg of body weight per day, 0.5 mg/kg to 7.5 mg/kg of body weight per day, or any amount to range therein. A pharmaceutical composition as provided herein can be administered on a regimen of 1 to 4 times per day or in a single daily dose.

In some embodiments, the daily dose of the compound of Formula (I), or a pharmaceutically acceptable salt thereof, is about 25 mg, based on the weight of the free base. In some embodiments, the daily dose of the compound of Formula (I), or a pharmaceutically acceptable salt thereof, is about 50 mg, based on the weight of the free base. In some embodiments, the daily dose of the compound of Formula (I), or a pharmaceutically acceptable salt thereof, is about 75 mg, based on the weight of the free base. In some embodiments, the daily dose of the compound of Formula (I), or a pharmaceutically acceptable salt thereof, is about 100 mg, based on the weight of the free base. In some embodiments, the daily dose of the compound of Formula (I), or a pharmaceutically acceptable salt thereof, is about 150 mg, based on the weight of the free base. In some embodiments, the daily dose of the compound of Formula (I), or a pharmaceutically acceptable salt thereof, is about 200 mg, based on the weight of the free base.

In some embodiments, the compound of Formula (I), or a pharmaceutically acceptable salt thereof, is administered twice daily in a dose of about 25 mg, based on the weight of the free base. In some embodiments, the compound of Formula (I), or a pharmaceutically acceptable salt thereof, is administered twice daily in a dose of about 50 mg, based on the weight of the free base. In some embodiments, the compound of Formula (I), or a pharmaceutically acceptable salt thereof, is administered twice daily in a dose of about 75 mg, based on the weight of the free base. In some embodiments, the compound of Formula (I), or a pharmaceutically acceptable salt thereof, is administered twice daily in a dose of about 100 mg, based on the weight of the free base.

In some embodiments, the daily dose of the pharmaceutical composition is about 25 mg of the compound of Formula (I), or a pharmaceutically acceptable salt thereof, based on the weight of the free base. In some embodiments, the daily dose of the pharmaceutical composition is about 50 mg of the compound of Formula (I), or a pharmaceutically acceptable salt thereof, based on the weight of the free base. In some embodiments, the daily dose of the pharmaceutical composition is about 75 mg of the compound of Formula (I), or a pharmaceutically acceptable salt thereof, based on the weight of the free base. In some embodiments, the daily dose of the pharmaceutical composition is about 100 mg of the compound of Formula (I), or a pharmaceutically acceptable salt thereof, based on the weight of the free base. In some embodiments, the daily dose of the pharmaceutical composition is about 150 mg of the compound of Formula (I), or a pharmaceutically acceptable salt thereof, based on the weight of the free base. In some embodiments, the daily dose of the pharmaceutical composition is about 200 mg of the compound of Formula (I), or a pharmaceutically acceptable salt thereof, based on the weight of the free base.

In some embodiments, the pharmaceutical composition is administered twice daily in a dose of about 25 mg of the compound of Formula (I), or a pharmaceutically acceptable salt thereof, based on the weight of the free base. In some embodiments, the pharmaceutical composition is administered twice daily in a dose of about 50 mg of the compound of Formula (I), or a pharmaceutically acceptable salt thereof, based on the weight of the free base. In some embodiments, the compound of Formula (I), or a pharmaceutically acceptable salt thereof, is administered twice daily in a dose of about 75 mg of the compound of Formula (I), or a pharmaceutically acceptable salt thereof, based on the weight of the free base. In some embodiments, the pharmaceutical composition is administered twice daily in a dose of about 100 mg of the compound of Formula (I), or a pharmaceutically acceptable salt thereof, based on the weight of the free base.

In some embodiments, the method comprises administering a daily dose of the pharmaceutical composition comprising about 25 mg of the compound of Formula (I), or a pharmaceutically acceptable salt thereof, based on the weight of the free base. In some embodiments, the method comprises administering a daily dose of the pharmaceutical composition comprising about 50 mg of the compound of Formula (I), or a pharmaceutically acceptable salt thereof, based on the weight of the free base. In some embodiments, the method comprises administering a daily dose of the pharmaceutical composition comprising about 75 mg of the compound of Formula (I), or a pharmaceutically acceptable salt thereof, based on the weight of the free base. In some embodiments, the method comprises administering a daily dose of the pharmaceutical composition comprising about 100 mg of the compound of Formula (I), or a pharmaceutically acceptable salt thereof, based on the weight of the free base. In some embodiments, the method comprises administering a daily dose of the pharmaceutical composition comprising about 150 mg of the compound of Formula (I), or a pharmaceutically acceptable salt thereof, based on the weight of the free base. In some embodiments, the method comprises administering a daily dose of the pharmaceutical composition comprising about 200 mg of the compound of Formula (I), or a pharmaceutically acceptable salt thereof, based on the weight of the free base.

In some embodiments, the method comprises administering the pharmaceutical composition twice daily in a dose of about 25 mg of the compound of Formula (I), or a pharmaceutically acceptable salt thereof, based on the weight of the free base. In some embodiments, the method comprises administering the pharmaceutical composition twice daily in a dose of about 50 mg of the compound of Formula (I), or a pharmaceutically acceptable salt thereof, based on the weight of the free base. In some embodiments, the method comprises administering the compound of Formula (I), or a pharmaceutically acceptable salt thereof, twice daily in a dose of about 75 mg of the compound of Formula (I), or a pharmaceutically acceptable salt thereof, based on the weight of the free base. In some embodiments, the method comprises administering the pharmaceutical composition twice daily in a dose of about 100 mg of the compound of Formula (I), or a pharmaceutically acceptable salt thereof, based on the weight of the free base.

Factors associated with the particular subject being treated, including subject age, weight, diet, and time of administration, can result in the need to adjust dosages. In some embodiments, the subject is a human adult. In some embodiments, the subject is a pediatric subject.

One skilled in the art will recognize that both *in vivo* and *in vitro* trials using suitable, known, and generally accepted cell and/or animal models are predictive of the ability of a test compound to treat or prevent a given disorder. One skilled in the art will further recognize that human clinical trials including first-in-human, dose ranging and efficacy trials, in healthy subjects and/or those suffering from a given disorder, can be completed according to methods well known in the clinical and medical arts. For example, determining proper dosages for pediatric subjects can be determined using known methods, including weight, age, and models such as Simcyp® Pediatric Simulation modeling (CERTARA, Princeton, N.J.) which can be used to establish a pharmacokinetic approach for dosing that takes into account subject age, ontogeny of the clearance pathways that a compound of Formula (I), or a pharmaceutically acceptable salt thereof, and body surface area (BSA).

In some embodiments, the pharmaceutical compositions of the present invention are stable for at least 3 months. In some embodiments, the pharmaceutical compositions are stable for at least 6 months. In some embodiments, the pharmaceutical compositions are stable for at least 9 months. In some embodiments, the pharmaceutical compositions are stable for at least 12 months. For example, the compositions do not exhibit a change (e.g., greater than 5%) in appearance, pH, percent impurities, activity (as measured by in vitro assays), or osmolarity over time, e.g., at least 3 months, 6 months, 9 months, or at least 12 months as compared to the original composition after manufacturing. In some embodiments, the pharmaceutical compositions do not exhibit a significant change, as defined by the International Conference on Harmonisation of Technical Requirements for Registration of Pharmaceuticals for Human Use (ICH), in one or more of appearance, pH, percent impurities, activity (as measured by *in vitro* assays), or osmolarity over time, e.g., at least 12 months as compared to the original pharmaceutical composition after manufacturing.

**Kits**

Also provided are kits. Typically, a kit includes one or more pharmaceutical compositions of the invention. In certain embodiments, a kit can include one or more delivery systems, e.g., for delivering or administering the pharmaceutical composition of the invention, and directions for use of the kit (e.g., instructions for treating a subject). In some embodiments, the kit can include a pharmaceutical composition of the invention and a label that indicates that the contents are to be administered to a subject with congenital adrenal hyperplasia. The actual dose of the compound of Formula (I), or pharmaceutically acceptable salt thereof, provided herein depends on the specific formulation, the weight of the patient, and on the condition to be treated.

**EXAMPLES**

**Reference Example 1: Spray-dried dispersion formulations containing the compound of Formula (I) and various polymers**

***Spray-dried dispersion formulations***

A series of spray-dried dispersion (SDD) formulations containing the compound of Formula (I) and a polymer were prepared. The SDD formulations included: (1) 10% compound of Formula (I)/90% hydroxypropylmethylcellulose acetate succinate-L (HPMCAS-L); (2) 25% compound of Formula (I)/75% HPMCAS-L; (3) 40% compound of Formula (I)/60% HPMCAS-L; (4) 25% compound of Formula (I)/75% polyvinyl pyrrolidone vinyl acetate 64 (PVP/VA 64); (5) 25% compound of Formula (I)/60% Cabosil (fumed silica)/15% HPMCAS-L; (6) 25% compound of Formula (I)/75% HPMCAS-M; and (7) 25% compound of Formula (I)/75% methyl methacrylate copolymer (1:1) (Eudragit® L100).

The PVP/VA polymer was a copolymer of 1-vinyl-2-pyrrolidone and vinyl acetate with a ratio of 60:40 by weight 1-vinyl-2-pyrrolidone:vinyl acetate with an average molecular weight of 45,000-70,000 (copovidone, sold as Kollidon® VA 64, BASF, Florham Park, NJ). The HPMCAS was a mixture of acetic acid and monosuccinic acid esters of hydroxypropylmethyl cellulose that was either grade L (HPMCAS-L), with an acetyl content of 5-9%, a succinoyl content of 14-18%, a methoxyl content of 20-24%, and a hydroxypropoxy content of 5-9% (sold by Shin-Etsu, Japan); or grade M (HPMCAS-M), with an acetyl content of 7-11%, a succinoyl content of 10-14%, a methoxyl content of 21-25%, and a hydroxypropoxy content of 5-9% (sold by Shin-Etsu, Japan).

*Dissolution performance*

Dissolution performance of several of the SDD formulations described above was tested (see FIG. 1). 1000 μgA/mL of each SDD was tested in 0.5 wt% simulated intestinal fluid (SIF) in PBS, pH 6.5. Samples were tested at 5, 10, 20, 45, 90, and 1200 minutes. A lipid formulation containing 10% of the compound of Formula (I) was used as a control. The results are shown in Table 4, below.

**Table 4. Dissolution data of various SDDs**

| Sample | $C_{max90}$ (μg/mL) | $AUC_{90}$ (min*μg/mL) | $C_{max90}$ (μg/mL) | $Ultra_{90}$ (μg/mL) | $C_{1200}$ (μg/mL) | $Ultra_{1200}$ (μg/mL) |
|---|---|---|---|---|---|---|
| **2** | 762 | 66,080 | 743 | 210 | 671 | 166 |
| **4** | 322 | 27,330 | 306 | 109* | 268 | 199 |
| **6** | 718 | 62,240 | 708 | 202 | 632 | 217 |
| **7** | 742 | 60,600 | 742 | 113* | 674 | 194 |
| **Control** | 802 | 69,580 | 800 | 253 | 799 | 270 |
| *Large variability between replicates, high value discarded | | | | | | |

*Non-sink dissolution*

A membrane flux assay was performed (see, e.g., Stewart et al., Mol. Pharm. (2017) 14:2032-2046) and non-sink dissolution data was collected for several of the SDD formulations described above and compared to the compound of Formula (I) and several reference formulations, including a semi-solid lipidic formulation (Reference Formulation 1) and two self-emulsifying drug delivery system (SEDDS) formulations (Reference Formulations 2 and 3). The components of the Reference Formulations are shown in Table 5, below, and include, in addition to the compound of Formula (I), caprylic/capric triglyceride (Labrafac® Lipophile, Gattefossé, France); propylene glycol dicrapolate/dicaprate (Labrafac® PG, Gattefossé, France); oleoyl polyoxyl-6 glycerides (Labrafil® M 1944 CS, Gattefossé, France); polysorbate 20; polyoxyl castor oil (Kolliphor® RH 40, BASF, Germany); polyoxyl 15 hydroxystearate (Kolliphor® HS 15, BASF, Germany); lauroyl polyoxyl-32 glycerides (Gelucire® 44/14, Gattefossé, France); d-α-tocopheryl polyethylene glycol 1000 succinate (TPGS); and diethylene glycol monoethyl ether (Transcutol®, Gattefossé, France).

**Table 5. Reference formulations (capsules)**

| Formulation (mg/caps) | Ref. Formulation 1 | Ref. Formulation 2 | Ref. Formulation 3 |
|---|---|---|---|
| **Formula (I)** | 50.0 | 50.0 | 50.0 |
| **Labrafac® Lipophile** | 196.0 | 100.0 | 100.0 |
| **Labrafac® PG** | 102.0 | - | - |
| **Labrafil® M 1944 CS** | - | 135.0 | 46.0 |
| **Polysorbate 20** | - | - | 89.9 |
| **Kolliphor® RH 40** | - | - | 100.0 |
| **Kolliphor® HS 15** | - | 165.0 | - |
| **Gelucire® 44/14** | 95.0 | - | - |
| **TPGS** | 57.0 | - | 65.0 |
| **Transcutol®** | - | 50.0 | 50.0 |
| ***Total*** | 500.0 | 500.0 | 500.0 |

The assay measured the flux across simulated gastric and intestinal walls via UV spectroscopy (μDiss ProfilerTM, Pion Inc., Billerica, MA). Briefly, the assay was performed as follows. A vertical membrane flux cell consisting of a donor compartment and a receiver compartment, and separated by an Accurel PP 1E (55% porous, 100 μm thickness) polypropylene membrane (3M, Maplewood, MN) (FIG. 2), was impregnated with 50 μL of Pion GIT-0 lipid solution consisting of 20% w/w phospholipid dissolved into dodecane (Pion Inc., Billerica, MA) and attached to the receiver vessel.

Both the donor and receiver compartments were agitated by magnetic stirring. The receiver compartment contained a plastic spacer and grating to elevate the stir bar above the membrane. Samples were introduced to the donor vessel by pre-weighing directly into the donor vessel and subsequently adding dissolution medium. Once the dissolution medium was added to the donor vessel, the receiver vessel was inserted into the donor vessel and suspended vertically 5 mm above the donor compartment by a plastic sleeve. For this assay, the simulated gastric (feed) media was 0.1 N HCl, pH 2 and included 200 μgA/mL of each SDD, and the simulated intestinal (receiver) media was 0.5 wt% SIF in PBS, pH 6.5 and included 100 μgA/mL of each SDD. The temperature for the assay was maintained at 44.5°C. UV probes (10 mm path length) connected to a Rainbow UV spectrometer (Pion Inc.) system were used to determine the apparent drug concentration in the receiver vessels. Samples of the donor compartment were removed with a disposable pipet for centrifugation followed by HPLC and DLS analysis of the supernatant. The results are shown in FIG. 3 and Table 6, below.

**Table 6. Non-sink dissolution data**

| Sample | $C_{maxGB}$ (μg/mL) | $C_{max90\ IB}$ (μg/mL) | $AUC_{4\text{-}90IB}$ (min*μg/mL) | $C_{90}$ (μg/mL) | $Ultra_{90}$ (μg/mL) | $C_{1200}$ (μg/mL) |
|---|---|---|---|---|---|---|
| **Formula (I)** | 0 | 1 | 10 | 0 | 0 | 3 |
| **1** | 6 | 80 | 6,800 | 80 | 79 | 90 |
| **2** | 17 | 74 | 6,240 | 73 | 73 | 86 |
| **4** | 6 | 4 | 200 | 4 | 5 | 36 |
| **5** | 23 | 55 | 3,180 | 55 | 54 | 83 |
| **6** | 35 | 71 | 6,070 | 71 | 77 | 83 |
| **Ref. Formulation 1** | 205 | 109 | 9,050 | 109 | - | - |
| **Ref. Formulation 2** | 249 | 120 | 10,160 | 120 | - | - |
| **Ref. Formulation 3** | 218 | 107 | 9,100 | 107 | - | - |

The membrane flux of 1 mg/mL gastric barrier/intestinal barrier (GB/IB) 0.5 wt% SIF doses of the compound of Formula (I) and spray-dried dispersions (2) 25% compound of Formula (I)/75% HPMCAS-L and (4) 25% compound of Formula (I)/75% PVP/VA 64 were also determined. The results are shown in FIG. 4 as receiver concentration vs. time and flux vs. time (smoothed derivative of receiver concentration x volume/surface area).

**Reference Example 2: Characterization of a spray-dried dispersion containing 25% of the compound of Formula (I) and 75% of a polyvinyl pyrrolidone vinyl acetate (PVP/VA) polymer**

***SDD stability screening***

Several of the SDDs described in Reference Example 1 were tested for chemical and physical stability. Wet SDD stability studies were performed, with samples stored at both 5°C and 25°C. Measurements were taken after 1 week and 2 weeks of storage. The results are shown in Table 7 below. The column with a retention time of 32.36 min correlates with the compound of Formula (I).

**Table 7. Wet SDD stability data**

| **Retention time (min)** | | | 11.04 | 16.79 | 17.26 | 30.94 | 32.26 | | | |
|---|---|---|---|---|---|---|---|---|---|---|
| **Relative retention time** | | | 0.34 | 0.52 | 0.53 | 0.96 | 1.00 | | | |
| | **Storage temp** | **Timepoint** | | | | | | **Total impurities** | **Potency (mgA/g)** | **Std Dev** |
| **Ref. Std.** | | | | | | 0.37 | 99.63 | 0.37 | | |
| **Formula (I)** | | | | | | 0.26 | 99.74 | 0.26 | | |

(continued)

| | Storage temp | Timepoint | | | | | | Total impurities | Potency (mgA/g) | Std Dev |
|---|---|---|---|---|---|---|---|---|---|---|
| **Sample 1** | | initial | | 0.13 | 0.16 | 0.25 | 99.46 | 0.54 | 100 | 1.3 |
| | 5°C | 1 week | | 0.03 | 0.03 | 0.26 | 99.69 | 0.31 | 99 | 0.0 |
| | | 2 weeks | | 0.12 | 0.16 | 0.28 | 99.45 | 0.55 | 99 | 0.5 |
| | 25°C | 1 week | | 0.03 | 0.04 | 0.26 | 99.67 | 0.33 | 99 | 0.3 |
| | | 2 weeks | | 0.21 | 0.27 | 0.28 | 99.24 | 0.76 | 98 | 0.8 |
| **Sample 2** | | initial | <LOQ | 0.07 | 0.08 | 0.26 | 99.59 | 0.41 | 247 | 0.3 |
| | 5°C | 1 week | <LOQ | 0.02 | 0.03 | 0.26 | 99.70 | 0.30 | 248 | 1.3 |
| | | 2 weeks | <LOQ | 0.22 | 0.27 | 0.27 | 99.24 | 0.76 | 246 | 0.3 |
| | 25°C | 1 week | <LOQ | 0.02 | 0.03 | 0.26 | 99.69 | 0.31 | 248 | 0.9 |
| | | 2 weeks | <LOQ | 0.23 | 0.28 | 0.26 | 99.23 | 0.77 | 246 | 1.4 |
| LOQ = limit of quantification | | | | | | | | | | |

Solution stability studies were also performed, with samples stored at both 5°C and 25°C. Measurements were taken after 1 week and 2 weeks of storage. The results are shown in Table 8 below. The column with a retention time of 32.36 min correlates with the compound of Formula (I).

**Table 8. SDD solution stability data**

| **Retention time (min)** | | | 31.51 | 32.26 | |
|---|---|---|---|---|---|
| **Relative retention time** | | | 0.97 | 1.00 | |
| | **Storage temp** | **Timepoint** | | | **Total impurities** |
| **Ref. Std.** | | | 0.74 | 99.26 | 0.74 |
| **Formula (I)** | | | 0.26 | 99.74 | 0.26 |
| **Sample 1** | | initial | 0.33 | 99.67 | 0.33 |
| | 5°C | 2 weeks | 0.29 | 99.71 | 0.29 |
| | 25°C | 2 weeks | 0.38 | 99.62 | 0.38 |
| **Sample 2** | | initial | 0.25 | 99.75 | 0.25 |
| | 5°C | 2 weeks | 0.26 | 99.74 | 0.26 |
| | 25°C | 2 weeks | 0.33 | 99.67 | 0.33 |

Stability studies were also performed for the SDD containing 25% of the compound of Formula (I) and 75% PVP/VA 64, with samples stored at both 5°C (closed with desiccant), 25°C (60% RH, closed with desiccant), and 30°C (65% RH, closed with desiccant). Measurements were taken after storage for 1 month, 2 months, 3 months, 6 months, and 12 months. No change in purity was observed after 12 months of storage. The results are shown in Table 9 below. The column with a retention time of 30.2 min correlates with the compound of Formula (I).

**Table 9. SDD stability data**

| **Retention time (min)** | | | 28.7 | 30.2 | | |
|---|---|---|---|---|---|---|
| **Relative retention time** | | | 0.95 | 1.00 | | |
| | **Storage conditions** | **Timepoint** | | | **Total impurities** | **Potency (mgA/g)** |
| **Crystalline Formula (I)** | | | 0.26 | 99.74 | 0.26 | 1001 |

(continued)

| | Storage conditions | Timepoint | | | Total impurities | Potency (mgA/g) |
|---|---|---|---|---|---|---|
| **Sample 4 (25% Formula (I):75% PVP/VA 64)** | | initial | 0.26 | 99.74 | 0.26 | 245 |
| | 5°C (closed w/ desiccant) | 1 month | 0.25 | 99.75 | 0.25 | 247 |
| | | 2 months | 0.25 | 99.75 | 0.25 | 244 |
| | | 3 months | 0.26 | 99.74 | 0.26 | 246 |
| | | 6 months | 0.25 | 99.75 | 0.25 | 245 |
| | | 12 months | 0.25 | 99.75 | 0.25 | 248 |
| | 25°C/60% RH (closed with desiccant) | 1 month | 0.25 | 99.75 | 0.25 | 245 |
| | | 2 months | 0.25 | 99.75 | 0.25 | 247 |
| | | 3 months | 0.25 | 99.75 | 0.25 | 246 |
| | | 6 months | 0.25 | 99.75 | 0.25 | 242 |
| | | 12 months | 0.25 | 99.75 | 0.25 | 245 |
| | 30°C/65% RH (closed with desiccant) | 1 month | 0.25 | 99.75 | 0.25 | 249 |
| | | 2 months | 0.25 | 99.75 | 0.25 | 242 |
| | | 3 months | 0.25 | 99.75 | 0.25 | 246 |
| | | 6 months | 0.25 | 99.73 | 0.25 | 243 |
| | | 12 months | 0.25 | 99.75 | 0.25 | 242 |

While Samples 1 and 2 showed degradation after about 2 weeks of storage, the SDD containing 25% of the compound of Formula (I) and 75% PVP/VA 64 (Sample 4) was found to be both chemically and physically stable and was further screened and characterized as described below.

***25% Formula (I)/75% PVP/VA 64 SDD process parameter screening manufacture Round 1***

The 25% Formula (I)/75% PVP/VA 64 SDD was prepared on a Pharmaceutical Spray Dryer with 100 kg/hr drying gas capacity (PSD-1). The manufacturing summary is shown in Table 10, below.

**Table 10. Manufacturing summary of process parameters**

| | |
|---|---|
| **Formulation** | 25% Formula (I):75% PVP/VA 64 |
| **Solids Loading (wt%)** | 10 |
| **Batch Size (kg)** | 1.5 |
| **Solvent** | Acetone |
| **Atomizer (Pressure Swirl)** | SK 80-16 |
| **Solution Flow-rate (g/min)** | 160 |
| **Atomization Pressure (psig)** | 480 |
| **Inlet Temperature (°C)** | 94 |
| **Outlet Temperature (°C)** | 40 |
| **Calculated Outlet Acetone Saturation (% RS)** | 6.2 |
| **Dry Yield (%)** | 73 |

Based on the 73% yield observed in the first round of process screening, three sprays were performed to investigate the effect of reducing solution throughput and outlet temperature on product yield. All sprays were conducted at a reduced flow-rate of 110 g/min. The outlet temperature was varied at 40°C (Lot A), 35°C (Lot B), and 30°C (Lot C). The outlet temperature was decreased while maintaining a low outlet acetone saturation to increase the difference between the

chamber outlet temperature and the wet SDD $T_g$, thus improving product yields. The spray dryer chamber and outlet ductwork were cleaned between all manufactures. A manufacturing summary is shown in Table 11.

**Table 11. Manufacturing summary for process parameters (1.5 kg batch size)**

| Description | Low Flow-Rate | Low Flow-Rate/Low Outlet Temperature | Low Flow-Rate/Lower Outlet Temperature |
|---|---|---|---|
| Lot | A | B | C |
| Solids Loading (wt%) | 10 | 10 | 10 |
| Batch Size (kg) | 1.5 | 1.5 | 1.5 |
| Solvent | Acetone | Acetone | Acetone |
| **Atomizer (Pressure Swirl)** | Steinen A75 | Steinen A75 | Steinen A75 |
| **Solution Flow-Rate (g/min)** | 110 | 110 | 110 |
| **Atomization Pressure (psig)** | 275 | 285 | 285 |
| **Inlet Temperature (°C)** | 79 | 72 | 63 |
| **Outlet Temperature (°C)** | 40 | 35 | 30 |
| **Calculated Outlet Acetone Saturation (% RS)** | 4.3 | 5.2 | 6.4 |
| **Calculated wet SDD $T_g$ (°C)** | 72 | 71 | 69 |
| **Dry Yield (%)** | 55 | 80 | 43 |

The conditions used for Lot B were found to give the highest yield. One additional spray was then performed at the same processing conditions as Lot B while increasing the batch size from 1.5 kg to 3.5 kg to evaluate process consistency and to determine if product yield would continue to improve over time. The averaged process conditions for this lot are shown in Table 12.

**Table 12. Manufacturing summary of process parameters (1.5 kg and 3.5 kg batch sizes)**

| Description | Low Flow-Rate/Low Outlet Temperature | Low Flow-Rate/Low Outlet Temperature/Larger Batch Size |
|---|---|---|
| **Lot** | B | D |
| **Solids Loading (wt%)** | 10 | 10 |
| **Batch Size (kg)** | 1.5 | 3.5 |
| **Solvent** | Acetone | Acetone |
| **Atomizer (Pressure Swirl)** | Steinen A75 | Steinen A75 |
| **Solution Flow-Rate (g/min)** | 110 | 110 |
| **Atomization Pressure (psig)** | 285 | 285 |
| **Inlet Temperature (°C)** | 72 | 72 |
| **Outlet Temperature (°C)** | 35 | 35 |
| **Calculated Outlet Acetone Saturation (% RS)** | 5.2 | 5.2 |
| **Calculated wet SDD $T_g$ (°C)** | 71 | 71 |
| **Dry Yield (%)** | 80 | 84 |

The 1.5 kg batch size (Lot D) was sprayed with an 84% yield compared to the 80% yield of the 3.5 kg batch (Lot B).

***25% Formula (I)/75% PVP/VA 64 SDD process parameter screening characterization***

The 25% Formula (I)/75% PVP/VA 64 SDDs manufactured to evaluate processing parameters were characterized for powder properties, performance, and physical and chemical properties. Testing included particle size distribution by Malvern, determination of bulk and tapped density, microcentrifuge dissolution, modulated differential scanning calorimetry (mDSC), powder x-ray diffraction (PXRD), scanning electron microscope (SEM), and assay and related substances. The results did not show any significant differences between the lots.

The particle size distribution (PSD) and tabulated powder properties data of the 25% Formula (I)/75% PVP/VA 64 SDDs are shown in Table 13. All 25% Formula (I)/75% PVP/VA 64 SDDs were observed to have a very similar PSD with a $D_{50}$ of approximately 16 μm. All 25% Formula (I)/75% PVP/VA 64 SDDs were observed to have low bulk and tapped densities.

**Table 13. Powder properties of process parameter screening PVP/VA-64 SDDs**

| Sample | Lot | $D_{10}$ (μm) | $D_{50}$ (μm) | $D_{90}$ (μm) | $D_{(3,2)}$ (μm) | $D_{(4,3)}$ (μm) | Span | Bulk density (g/mL) | Tapped density (g/mL) |
|---|---|---|---|---|---|---|---|---|---|
| **40°C Outlet** | **A** | **5** | **15** | **34** | **8** | **17** | **1.93** | **0.12** | **0.25** |
| **35°C Outlet** | **B** | **5** | **16** | **36** | **9** | **19** | **1.97** | **0.11** | **0.23** |
| **30°C Outlet** | **C** | **5** | **15** | **32** | **7** | **17** | **1.86** | **0.12** | **0.27** |
| **35°C Outlet, 3.5 kg batch** | **D** | **5** | **16** | **38** | **9** | **19** | **1.98** | **0.12** | **0.24** |

The 3.5 kg batch size lot was analyzed and compared to process parameter Lot A. Dissolution performance was similar for each of these lots. Dissolution was rapid to $C_{max}$ and high free drug was sustained through 90 minutes. These data are shown in Table 14.

**Table 14. Dissolution performance of Lot A (1.5 kg batch size) vs. Lot D (3.5 kg batch size)**

| Sample | $C_{max90}$ (μg/mL) | $AUC_{90}$ (min*μg/mL) | $C_{90}$ (μg/mL) | $Ultra_{90}$ (μg/mL) |
|---|---|---|---|---|
| **Lot A** | 447 | 37,740 | 437 | 319 |
| **Lot D** | 437 | 37,120 | 433 | 301 |

The 25% Formula (I)/75% PVP/VA 64 SDDs were also evaluated by DSC, PXRD, and SEM. The DSC thermograms showed a single $T_g$ at 84°C, indicating homogeneous dispersions. PXRD diffractograms showed no evidence of crystals in the SDDs. SEM images showed inflated sphere morphology with some broken particles and some very small particles.

Additional testing on Lot B was carried out, which included assessing the chemical/physical stability of both spray solution and SDD prior to secondary drying (wet SDD) to establish maximum in-process hold times. Residual acetone concentration as a function of secondary drying time in a convection tray dryer was also evaluated to nominate tray drying conditions to ensure the SDD is dried below International Council for Harmonization of Technical Requirements for Pharmaceuticals for Human Use (ICH) guidelines for acetone.

Residual acetone content as a function of drying time was assessed by drying wet SDD in a tray dryer and collecting samples over a 24-hour period. Wet SDD was dried at 40°C/15% relative humidity (RH) and was observed to dry below ICH acetone guidelines (0.5 wt%, 5000 ppm) by four hours.

Spray solution hold time was determined by making up a representative solution that contained 2.5 wt% compound of Formula (I), 7.5 wt% PVP/VA 64, and 90 wt% acetone. These solutions were analyzed initially for related substances. and then aged at 5°C and 25°C. Aliquots were taken and analyzed for related substances periodically for 14 days. Results showed no change in impurity profile at either condition through 14 days.

Wet SDD was analyzed for impurities after storage at 5°C and 25°C for 1 and 2 weeks and compared to the impurity profiles of the ingoing compound of Formula (I) and the SDD that was secondarily dried immediately after spray drying. The impurity profiles were similar to that of the initial dried sample and the ingoing compound of Formula (I) through 2 weeks of storage.

The wet SDD stability samples were characterized for physical stability by DSC, PXRD, and SEM. DSC thermograms showed a single $T_g$ at 81°C, indicative of a homogeneous dispersion with no phase separation. The PXRD diffractograms did not show any evidence of crystals after storage at either condition. SEM images showed a typical morphology of mostly inflated spheres with some broken particles.

**Reference Example 3: Preparation of a 1000 g batch of a spray-dried dispersion containing 25% of the compound of Formula (I) and 75% PVP/VA 64**

A 1000 g batch of the spray-dried dispersion containing 25% of the compound of Formula (I) and 75% PVP/VA 64 was prepared as described in Reference Example 2 for the 1.5 kg and 3.5 kg batches. Briefly, acetone (90% (w/w) of the total mixture) was added to the mixing tank followed by the addition of 250.0 g of the compound of Formula (I) (2.5% (w/w) of the total mixture). The mixture was mixed for 30 minutes in the dark at a temperature range of 15°C to 27°C. At the end of the mixing period, the solution was clear and free of undissolved solids. The PVP/VA 64 (750.0 g, 7.5% (w/w) of the total mixture) was then added and the mixture was stirred for an additional 30 minutes in the dark at a temperature range of 15°C to 27°C. At the end of the mixing period, the solution was clear and free of undissolved solids.

The solution was pumped and atomized in a drying chamber. The spray-dried dispersions were prepared in a Pharmaceutical Spray Dryer with 100 kg/hr drying gas capacity (PSD-1). The inlet temperature was set at 75°C (varied between 60°C-90°C). The outlet temperature was set at 35°C (varied between 32°C-38°C). The feed pressure was set at 280 psig (varied between 230-330 psig). The feed rate was set at 110 g/min (varied between 90-130 g/min). The spray dried powder was then dried in a convection tray dryer with a bed depth of ≤ 2.5 cm at 40°C (±5°C) and 15% relative humidity (±10%) for 24 hours under amber light. The residual acetone after drying was < 0.5 wt% (5000 ppm). FIG. 5 is a flow diagram of the manufacturing process.

**Reference Example 4: Preparation of spray-dried dispersion formulations of the compound of Formula (I) for clinical use**

The spray-dried dispersion (SDD) containing 25% compound of Formula (I) and 75% PVP/VA 64, prepared as described above, was formulated as a suspension or a capsule for clinical use.

***Suspension preparation***

A suspension that contained 50 mg of the SDD was prepared as follows. A 30 mL amber dosing bottle was tared on a balance. 200.0 mg SDD (50 mgA) ± 5% was then weighed into the dosing bottle. Using a 10-mL syringe, 5.0 mL of water (purified, USP) was added to the dosing bottle and the bottle was capped and shaken moderately for 30 seconds. The SDD suspension was stored in an amber vial at 2-8°C prior to use, and dosed within 24 hours of preparation.

***Capsule preparation***

An empty hard gelatin capsule, size 0 (Capsugel, Morristown, NJ), was placed on a balance and the weight was recorded. 200.0 mg SDD (50 mgA) ± 5% was then weighed onto weigh paper or an equivalent. All contents were transferred to the capsule using a ProFunnel device for Size 0 capsules. The filled capsule was placed on the balance and the weight was recorded. The weight of the empty capsule was subtracted from the filled weight, ensuring that the weight of the SDD within the capsule was 200.0 mg SDD ± 5%, or from 190.0 mg to 210.0 mg. The capsule was securely closed with the head, assuring it clicked into place. The capsules were stored in an amber vial at 2-8°C prior to use, and were dosed within 24 hours of preparation.

**Reference Example 5: Dog relative bioavailability and food effect study**

Four spray-dried dispersions (SDDs), formulated in 0.25% methylcellulose as a suspension, were prepared: (1) 25% compound of Formula (I)/75% HPMCAS-L; (2) 10% compound of Formula (I)/90% HPMCAS-L; (3) 25% compound of Formula (I)/75% methyl methacrylate copolymer (1:1) (Eudragit® L100); and (4) 25% compound of Formula (I)/75% PVP/VA 64. A clinical capsule formulation was prepared as a reference formulation (Reference Formulation 1 from Table 5, above).

Dogs (two cohorts, six dogs in each) were dosed in six sessions, including fasted state sessions and fed sessions (high fat diet), with 50 mg dose of one of the SDDs or reference per dog in a 3-way crossover design. Each session had a 3-day washout in between. All formulations were well tolerated. The study design is shown in Table 15, below.

**Table 15. Study design**

| Cohort 1 | Session 1 (Fasted) | Session 2 (Fed) | Session 3 (Fasted) | Session 4 (Fed) | Session 5 (Fasted) | Session 6 (Fed) |
|---|---|---|---|---|---|---|
| **Dog 1001** | Reference | Reference | SDD 1 | SDD 1 | SDD 2 | SDD 2 |
| **Dog 1002** | Reference | Reference | SDD 1 | SDD 1 | SDD 2 | SDD 2 |

(continued)

| Cohort 1 | Session 1 (Fasted) | Session 2 (Fed) | Session 3 (Fasted) | Session 4 (Fed) | Session 5 (Fasted) | Session 6 (Fed) |
|---|---|---|---|---|---|---|
| **Dog 2001** | SDD 1 | SDD 1 | SDD 2 | SDD 2 | Reference | Reference |
| **Dog 2002** | SDD 1 | SDD 1 | SDD 2 | SDD 2 | Reference | Reference |
| **Dog 3001** | SDD 2 | SDD 2 | Reference | Reference | SDD 1 | SDD 1 |
| **Dog 3002** | SDD 2 | SDD 2 | Reference | Reference | SDD 1 | SDD 1 |
| | | | | | | |
| **Cohort 2** | **Session 1 (Fasted)** | **Session 2 (Fed)** | **Session 3 (Fasted)** | **Session 4 (Fed)** | **Session 5 (Fasted)** | **Session 6 (Fed)** |
| **Dog 4001** | Reference | Reference | SDD 3 | SDD 3 | SDD 4 | SDD 4 |
| **Dog 4002** | Reference | Reference | SDD 3 | SDD 3 | SDD 4 | SDD 4 |
| **Dog 5001** | SDD 3 | SDD 3 | SDD 4 | SDD 4 | Reference | Reference |
| **Dog 5002** | SDD 3 | SDD 3 | SDD 4 | SDD 4 | Reference | Reference |
| **Dog 6001** | SDD 4 | SDD 4 | Reference | Reference | SDD 3 | SDD 3 |
| **Dog 6002** | SDD 4 | SDD 4 | Reference | Reference | SDD 3 | SDD 3 |

The area under the plasma concentration versus time curve from 0 hours extrapolated to infinity ($AUC_{0-\infty}$), maximum plasma concentration ($C_{max}$), the apparent terminal half-life ($t_{½}$), and the time to achieve maximum plasma concentration ($t_{max}$) were calculated. Results are shown in Table 16, below, and in FIGS. 6A and 6B.

**Table 16. Pharmacokinetic results**

| Cohort | Form | Fasted [Mean (%CV)] | | Fed [Mean (%CV)] | | Ratio of Fed/Fasted [Mean (%CV)] | |
|---|---|---|---|---|---|---|---|
| | | $C_{max}$ (ng/mL) | $AUC_{inf}$ (hr*ng/mL) | $C_{max}$ (ng/mL) | $AUC_{inf}$ (hr*ng/mL) | $C_{max}$ | AUC |
| 1 (N=4) * | **Ref** | 652 (55.5) | 5170 (69.4) | 3650 (19.5) | 20100 (22) | 6.9 (48) | 5.5 (69.2) |
| | **SDD 1** | 524 (62.7) | 2870 (62.9) | 5760 (25.6) | 22800 (14.6) | 15.4 (66.9) | 8 (89.6) |
| | **SDD 2** | 487 (45.7) | 3950 (26.4) | 4220 (27.4) | 18100 (26.8) | 16.5 (40.7) | 6.3 (34.6) |
| 2 (N=6) | **Ref** | 854 (40.2) | 5520 (59.1) | 4220 (27.4) | 18100 (26.8) | 5.8 (46.5) | 4.1 (49.2) |
| | **SDD 3** | 453 (28.3) | 2830 (21.6) | 5320 (13.5) | 23800 (26.1) | 12.6 (30.3) | 8.6 (22.2) |
| | **SDD 4** | 353 (20.1) | 1840 (21.9) | 3060 (20.7) | 17200 (20.5) | 8.9 (29.3) | 8.4 (46.1) |
| *Animals 2001 and 2002 were excluded from summary statistics due to emesis in all 3 fed sessions, which resulted in notably lower exposures | | | | | | | |

The results showed that $t_{1/2}$ and $t_{max}$ were similar among the formulations, and comparable between the fed and fasted states. Under the fed state with a high fat meal, exposures increased and inter-animal variability decreased. The food effect was more notable with the spray-dried dispersion formulations, especially for peak exposure ($C_{max}$).

Compared to the reference form, SDD 4 (25% compound of Formula (I)/75% PVP/VA 64) appeared to have lower inter-animal variability, lower exposures under the fasted state, and slightly lower $C_{max}$ but relatively comparable AUC in the fed state.

**Reference Example 6: Phase 1 study to evaluate the pharmacokinetics, effect of food on pharmacokinetics, and safety of the compound of Formula (I) in healthy adult subjects**

The present study was designed to evaluate the pharmacokinetics (PK) of the compound of Formula (I) as well as to evaluate the effect of a fed condition on the PK of the compound of Formula (I). The 50 mg dose was chosen for this study

because it was within the tested dose range in completed Phase 1 and Phase 2 trials and was well tolerated in those studies. The objectives of the study were: to evaluate the PK of the compound of Formula (I) 50 mg in healthy adult subjects; to evaluate the effect of food on the PK of the compound of Formula (I) 50 mg; and to evaluate the safety and tolerability of the compound of Formula (I) 50 mg.

**Study Design**

This was a Phase 1, open-label, randomized, 2-period crossover study of the PK and the effect of food on the PK of the compound of Formula (I) in 16 healthy male and female adult subjects, 18 to 55 years of age.

After providing informed consent, subjects were screened for eligibility to participate in the study up to 28 days prior to Day 1 of treatment period 1. Eligible subjects were admitted to the clinical unit on Day -1 and randomized to 1 of the 2 treatment sequences (16 subjects [8 males and 8 females]; see Table 17, below). On Day 1 of each treatment period, subjects received a single dose of the compound of Formula (I) 50 mg under fasted or fed conditions. There were 21 days between doses.

**Table 17. Treatment sequences**

| Treatment sequence | Treatment period I | Treatment period 2 |
|---|---|---|
| 1 | Formula (I) - fasted | Formula (I) - fed |
| 2 | Formula (I) - fed | Formula (I) - fasted |

Subjects were required to fast for at least 4 hours before check-in on Day -1. In the fasted condition, subjects were required to fast overnight for at least 10 hours prior to dosing and continued to fast for an additional 4 hours after dosing. In the fed condition, subjects were required to fast overnight for at least 10 hours and then ingest a liquid dietary supplement with study drug (liquid dietary supplement was consumed within 30 minutes) and not consume any other food for 4 hours after dosing. During both treatment periods, water was not permitted for 1 hour before dosing until 2 hours after dosing except for the water/liquid dietary supplement provided for study drug dosing. Vanilla-flavored Ensure Plus® was used as the liquid dietary supplement.

On Day 1 of each treatment period, subjects were dosed with the compound of Formula (I) 50 mg. Blood samples were collected for PK analysis over a period of 36 hours during the in-house stay. Subjects remained in the unit on the day of dosing and were discharged on Day 2 of each treatment period, following completion of all required procedures. On the mornings of Days 8 and 15 of each treatment period, subjects returned to the clinical unit on an outpatient basis for PK blood sample collection and safety assessments. On Day 21 of treatment period 1, subjects arrived at the site and had Day 21 assessments completed in the evening, and they stayed overnight at the site and began Day 1 of treatment period 2 the following day. A final follow-up study visit was conducted on Day 22 of treatment period 2 (21 $\pm$ 2 days after treatment period 2 dosing) or upon early termination.

During each treatment period, blood samples for PK analysis were collected within 45 minutes before dosing, and at approximately 30 minutes, and 1, 2, 3, 4, 5, 6, 7, 8, 9, 10, 12, 16, 24, 36, 168, 336, and 504 hours after dosing.

Safety assessments (including clinical safety laboratory tests, vital sign measurements, physical examinations, and electrocardiograms (ECGs)) were conducted at scheduled times throughout the study. Adverse events (AEs) and the use of concomitant medications were monitored throughout the study. FIG. 7 illustrates the study design.

***Test product, dose and mode of administration***

The compound of Formula (I) was supplied as an encapsulated, lipidic semi-solid containing 50 mg of the compound of Formula (I) as free base equivalent for oral administration. Subjects swallowed a single capsule with approximately 240 mL of water in the fasted condition. Subjects swallowed a single capsule with a liquid dietary supplement (Ensure Plus® [237 mL container]) with up to an additional 120 mL of water in the fed condition.

***Duration of treatment***

The duration of study participation for each adult subject was approximately 10 weeks, including up to 28 days of screening, 2 days of dosing separated by 21 days, and a final follow-up study visit 21 days after receiving the last dose of study drug during treatment period 2.

**Criteria for Evaluation**

*Pharmacokinetics*

The following plasma PK parameters were calculated for the compound of Formula (I):

- Area under the plasma concentration versus time curve from 0 hours to last measurable concentration ($AUC_{0-tlast}$)
- Area under the plasma concentration versus time curve from 0 to 24 hours ($AUC_{0-24}$)
- Area under the plasma concentration versus time curve from 0 hours extrapolated to infinity ($AUC_{0-\infty}$)
- Maximum plasma concentration ($C_{max}$)
- Time to achieve maximum plasma concentration ($t_{max}$)
- Delay time between time of dosing and time of appearance of measurable test article ($T_{lag}$)
- Apparent terminal half-life ($t_{½}$)
- Apparent terminal rate constant ($\lambda z$)
- Apparent mean residence time (MRT)
- Molar AUC ratio of the hydroxylated metabolite of the compound of Formula (I) to the parent drug the compound of Formula (I)

The following plasma PK parameters were calculated only for the compound of Formula (I):

- Apparent systemic clearance after oral administration (CL/F)
- Apparent volume of distribution during terminal phase after oral administration (Vz/F)

*Safety*

Safety was monitored throughout the study and included the following assessments:

- Adverse events (AEs)
- Clinical laboratory tests (hematology, coagulation, clinical chemistry, and urinalysis)
- Vital sign measurements (including orthostatic blood pressure and pulse rate)
- Physical examinations
- 12-lead electrocardiograms (ECGs)

*Statistical Methods*

Pharmacokinetic parameters were calculated using noncompartmental methods and summarized by condition (fed or fasted) using descriptive statistics. Two-sided 90% confidence intervals were calculated for the ratio under the fed condition vs. under the fasted condition for $AUC_{0-\infty}$, $AUC_{0-tlast}$, and $C_{max}$ for the compound of Formula (I) and the hydroxylated metabolite of the compound of Formula (I).
Safety data were summarized with descriptive statistics.

**Pharmacokinetics**

*Pharmacokinetic assessments*

PK plasma samples for analyses of the compound of Formula (I) and the hydroxylated metabolite of the compound of Formula (I) were collected at the following times during each treatment period:

- Day 1: within 45 minutes before dosing, and at approximately 30 minutes, and 1, 2, 3, 4, 5,6, 7, 8, 9, 10, 12, and 16 hours after dosing.
- Day 2: approximately 24 and 36 hours after dosing.
- Day 8: approximately 168 hours after dosing.
- Day 15: approximately 336 hours after dosing.
- Approximately 504 hours after dosing (for treatment period 1, this sample was collected in the morning at least 30 minutes prior to the predose sample on Day 1 of treatment period 2).
- Final study visit for subjects who terminate early: 1 sample.

Blood samples on Day 1 were collected within 5 minutes of the scheduled sampling times (other than the predose sample). Blood samples on Days 2, 8, and 15 were collected within 2 hours of the scheduled sampling time. The 504 hour blood sample for treatment period 2 had a ± 2-day window. A PK sample was to be collected from subjects who terminated

early. The exact time of sampling in hour and minutes was recorded.

***Bioanalytical methods***

Plasma samples were analyzed for the compound of Formula (I) and for its hydroxylated metabolite by inVentiv Health, Princeton NJ, in compliance with Good Laboratory Practice (GLP) and relevant Standard Operating Procedures (SOPs).

The concentrations of the compound of Formula (I) and the hydroxylated metabolite of the compound of Formula (I) were quantified in plasma samples according to validated methods using tandem mass spectrometry in positive ion mode. This method was validated for the analysis of the compound of Formula (I) and the hydroxylated metabolite of the compound of Formula (I) in 25.0 μL dipotassium ethylenediaminetetraacetic acid ($K_2$-EDTA) human plasma samples over concentration ranges of 5.00 to 2500 ng/mL and 0.500 and 250 ng/mL, respectively. All analytical results were within acceptable limits. Incurred sample reanalysis (ISR) was successfully conducted in this study for both analytes.

**Results**

***Pharmacokinetic results***

Eight male and eight female subjects were enrolled. The mean age was 37.1 years (range, 21 to 55 years). The majority of subjects were White (93.8%) and of Hispanic ethnicity (81.3%). The mean weight at screening was 160.28 lbs (range, 102.0 to 222.2 lbs) and mean BMI was 25.50 kg/$m^2$ (range, 20.7 to 30.5 kg/$m^2$). The randomization was well balanced with respect to demographics and baseline characteristics.

All 16 subjects were included in the safety analysis set. No subjects were excluded from the safety analysis set and no subject had his or her PK data excluded from analysis.

The mean plasma concentration versus time profiles for the compound of Formula (I) under fasted and fed conditions are presented in FIGS. 8A and 8B, respectively. The compound of Formula (I) was slowly absorbed after oral administration in both fasted and fed conditions. Mean plasma concentrations were lower in the fasted than in the fed condition.

PK parameters for the compound of Formula (I) after treatment with the compound of Formula (I) under fasted and fed conditions are summarized in Table 18, below, where $AUC_{0-24}$=area under the plasma concentration vs. time curve from 0 to 24 hours, $AUC_{0-tlast}$=AUC from 0 hours to last measurable concentration, $AUC_{0-\infty}$=AUC from 0 hours extrapolated to infinity, CL/F=apparent systemic clearance after oral administration, CV=coefficient of variation, $C_{max}$=-maximum plasma concentration, CV(%)=coefficient of variation, max=maximum, min=minimum, MRT=apparent mean residence time, PK=pharmacokinetic, SD=standard deviation, $t_{½}$=apparent terminal half-life, $T_{lag}$=delay time between time of dosing and time of appearance of measurable test article, $t_{max}$=time to maximum plasma concentration, VZ/F=apparent volume of distribution during the terminal phase after oral administration.

The PK data for $t_{max}$, $T_{lag}$, $t_{½}$, MRT, and Vz/F were rounded to 2 significant figures and all other parameters ($AUC_{0-24}$, $AUC_{0-tlast}$, $AUC_{0-\infty}$, $C_{max}$, and CL/F) were rounded to 3 significant figures. The last significant figure was rounded up if the digit to the right of it was ≥5, and was rounded down if the digit to the right of it was ≤4.

**Table 18. Summary of Formula (I) PK parameters (safety analysis set)**

| Parameter Statistic | Fasted Formula (I) (50 mg) (N=16) | Fed Formula (I) (50 mg) (N=15) |
|---|---|---|
| **$AUC_{0-24}$ (ng×hr/mL)** | | |
| Mean (SD) | 5590 (2230) | 9950 (2540) |
| Geometric CV% | 32.7 | 26.2 |
| **$AUC_{0-tlast}$ (ng×hr/mL)** | | |
| Mean (SD) | 8020 (5110) | 16200 (5450) |
| Geometric CV% | 53.6 | 39.7 |
| **$AUC_{0-\infty}$ (ng×hr/mL)** | | |
| Mean (SD) | 9440 (2990) [n=7] | 17800 (4990) [n=12] |
| Geometric CV% | 39.7 | 28.1 |
| **$C_{max}$ (ng/mL)** | | |
| Mean (SD) | 731 (301) | 1550 (392) |
| Geometric CV% | 36.6 | 24.2 |
| **$t_{max}$ (hours)** | | |

(continued)

| Parameter Statistic | Fasted Formula (I) (50 mg) (N=16) | Fed Formula (I) (50 mg) (N=15) |
|---|---|---|
| Median (min, max) | 6.0 (3.0, 6.0) | 5.0 (3.0, 6.0) |
| **$T_{lag}$ (hours)**<br>Mean (SD) | <br>0.63 (0.22) | <br>0.94 (0.37) |
| **$t_{½}$ (hours)**<br>Mean (SD)<br>Geometric CV% | <br>33 (17) [n=7]<br>99 | <br>42 (6.8) [n=12]<br>15 |
| **MRT (hours)**<br>Mean (SD)<br>Geometric CV% | <br>28 (11) [n=7]<br>49 | <br>30 (5.0) [n=12]<br>16 |
| **CL/F (L/hr)**<br>Mean (SD)<br>Geometric CV% | <br>6.0 (2.7) [n=7]<br>40 | <br>3.0 (0.82) [n=12]<br>28 |
| **VZ/F (L)**<br>Mean (SD)<br>Geometric CV% | <br>240 (120) [n=7]<br>67 | <br>180 (57) [n=12]<br>30 |

As seen in Table 18, above, administration of the compound of Formula (I) 50 mg under fed compared with fasted conditions resulted in a higher mean $C_{max}$ of the compound of Formula (I) (approximately 2-fold higher; 1550 vs. 731 ng/mL), a longer $t_{½}$ (42 vs. 33 hours), a slightly shorter median $t_{max}$ (5.0 vs. 6.0 hours), and a higher mean $AUC_{0-\infty}$ (approximately 2-fold higher; 17800 vs. 9440 ng×hr/mL). The compound of Formula (I) geometric mean ratios for $C_{max}$ and $AUC_{0-tlast}$ for fed vs. fasted conditions were 218.6% and 215.2%, respectively, indicating that the compound of Formula (I) absorption was approximately 2-fold greater when administered with food. The upper and lower 90% confidence interval (CI) bounds for both $C_{max}$ (187.4% and 255.1%, respectively) and $AUC_{0-tlast}$ (182.9% and 253.1%, respectively) were outside of the "no-effect" range of 80.00% to 125.00%, indicating that there was a food effect on the compound of Formula (I) exposure.

Frequency distribution of $T_{lag}$ and $t_{max}$ values are presented in Table 19 and Table 20, respectively. Spaghetti plots for the compound of Formula (I) $AUC_{0-tlast}$, $AUC_{0-\infty}$ and $C_{max}$ are shown in FIGS. 9A, 9B, and 9C, respectively.

**Table 19. Frequency distribution of plasma Formula (I) $T_{lag}$ values by treatment (safety analysis set)**

| $T_{lag}$ (hr) | Statistic | Fasted (Formula (I) 50 mg) (N=16) | Fed (Formula (I) 50 mg) (N=15) |
|---|---|---|---|
| 0.50 | n (%) | 11 (68.8%) | 3 (20.0%) |
| 0.53 | n (%) | 1 (6.3%) | 0 (0.0%) |
| 0.55 | n (%) | 0 (0.0%) | 1 (6.7%) |
| 1.00 | n (%) | 4 (25.0%) | 8 (53.3%) |
| 1.02 | n (%) | 0 (0.0%) | 1 (6.7%) |
| 1.03 | n (%) | 0 (0.0%) | 1 (6.7%) |
| 2.00 | n (%) | 0 (0.0%) | 1 (6.7%) |

**Table 20. Frequency distribution of plasma Formula (I) $T_{max}$ values by treatment (safety analysis set)**

| $T_{max}$ (hr) | Statistic | Fasted (Formula (I) 50 mg) (N=16) | Fed (Formula (I) 50 mg) (N=15) |
|---|---|---|---|
| 3.00 | n (%) | 1 (6.3%) | 2 (13.3%) |
| 4.02 | n (%) | 1 (6.3%) | 0 (0.0%) |
| 5.00 | n (%) | 4 (25.0%) | 8 (53.3%) |
| 5.03 | n (%) | 0 (0.0%) | 1 (6.7%) |
| 5.05 | n (%) | 0 (0.0%) | 1 (6.7%) |

(continued)

| $T_{max}$ (hr) | Statistic | Fasted (Formula (I) 50 mg) (N=16) | Fed (Formula (I) 50 mg) (N=15) |
|---|---|---|---|
| 6.00 | n (%) | 10 (62.5%) | 2 (13.3%) |
| 6.02 | n (%) | 0 (0.0%) | 1 (6.7%) |

The compound of Formula (I) was slowly absorbed after oral administration in the fasted and fed conditions. In the fasted condition, the mean compound of Formula (I) $C_{max}$ was approximately 53% lower than in the fed condition (731 vs. 1550 ng/mL). Due to a prolonged elimination phase, $t_{½}$ values and therefore, $AUC_{0-\infty}$ values could not be determined for some the compound of Formula (I) concentration-time profiles. Mean $AUC_{0-\infty}$ was approximately 47% lower in the fasted condition than in the fed condition (9440 vs. 17800 ng×hr/mL) for those subjects for whom $AUC_{0-\infty}$ could be determined. Mean $AUC_{0-tlast}$ was approximately 50% lower in the fasted condition than in the fed condition (8020 vs. 16200 ng×hr/mL). Median $t_{max}$ was slightly longer in the fasted condition than in the fed condition (6.0 vs. 5.0 hours) and mean $t_{½}$ was shorter in the fasted condition than in the fed condition (33 vs. 42 hours) for those subjects for whom a $t_{½}$ could be determined. Variability in the compound of Formula (I) PK (geometric CV%) for AUC, $C_{max}$, $t_{½}$, and MRT was lower in the fed condition compared with the fasted condition.

The geometric mean ratios and associated 90% CIs for $AUC_{0-tlast}$ and $C_{max}$ for the compound of Formula (I) after treatment with the compound of Formula (I) for the fed vs. fasted condition are provided in Table 21, below, where $AUC_{0-tlast}$= AUC from 0 hours to last measurable concentration, $C_{max}$=maximum plasma concentration, and PK=pharmacokinetic.

**Table 21. Formula (I) geometric mean ratios for PK exposure parameters under fed vs. fasted conditions (safety analysis set)**

| Parameter | Ratio[a] (%) (Fed vs. Fasted Condition) | 90% Confidence Interval[b] |
|---|---|---|
| $AUC_{0-tlast}$ (ng×hr/mL) | 215.2 | 182.9, 253.1 |
| $C_{max}$ (ng/mL) | 218.6 | 187.4, 255.1 |
| [a] Ratio of geometric least-squares means was based on a mixed model using log-transformed (base 10) data.<br>[b] The 90% confidence interval for geometric mean ratio was based on least-squares means using log-transformed (base 10) data. | | |

The compound of Formula (I) geometric mean ratios for $C_{max}$ and $AUC_{0-tlast}$ for the fed vs. fasted conditions were 218.6% and 215.2%, respectively, indicating that the compound of Formula (I) absorption was approximately 2-fold greater when administered with food. The upper and lower 90% CI bounds for both $C_{max}$ (187.4%, 255.1%) and $AUC_{0-tlast}$ (182.9%, 253.1%) were outside of the "no-effect" range of 80.00% to 125.00%, indicating that there was a food effect on the compound of Formula (I) exposure. Due to the missing $AUC_{0-\infty}$ values, the food effect on overall exposure was not assessed using $AUC_{0-\infty}$ values.

***Conclusion***

Administration of the compound of Formula (I) 50 mg under fed compared with fasted conditions resulted in a higher mean $C_{max}$ of the compound of Formula (I) (approximately 2-fold higher; 1550 vs. 731 ng/mL), a longer $t_{½}$ (42 vs. 33 hours), a slightly shorter median $t_{max}$ (5.0 vs. 6.0 hours), and a higher mean $AUC_{0-\infty}$ (approximately 2-fold higher; 17800 vs. 9440 ng×hr/mL). The compound of Formula (I) geometric mean ratios for $C_{max}$ and $AUC_{0-tlast}$ for fed vs. fasted conditions were 218.6% and 215.2%, respectively, indicating that the compound of Formula (I) absorption was approximately 2-fold greater when administered with food. The upper and lower 90% CI bounds for both $C_{max}$ (187.4%, 255.1%) and $AUC_{0-tlast}$ (182.9%, 253.1%) were outside of the "no-effect" range of 80.00% to 125.00%, indicating that there was a food effect on the compound of Formula (I) exposure. Similar results were observed with the hydroxylated metabolite of the compound of Formula (I). Overall, these results indicate that the compound of Formula (I) 50 mg was well tolerated in healthy subjects when administered under fasted or fed conditions and that the total AUC and $C_{max}$ were increased when the compound of Formula (I) was taken with food.

**Reference Example 7: Phase 1 study to evaluate the relative bioavailability, effect of food on pharmacokinetics, and safety of formulations of the compound of Formula (I) in healthy adult subjects**

A Phase 1 study to compare the relative bioavailability of a 50 mg dose of different formulations of the compound of Formula (I) as well as to evaluate the effect of fasting and fed conditions on the pharmacokinetics (PK) of the compound of Formula (I) was designed. The 50 mg dose was chosen for this study because it is within the tested dose range in completed Phase 1 and Phase 2 trials and was well tolerated in those studies. The objectives of the study are: to evaluate the PK and compare the relative bioavailability of the compound of Formula (I) 50 mg formulations in healthy adult subjects; to evaluate the effect of food on the PK of the compound of Formula (I) 50 mg formulations; and to evaluate the safety and tolerability of the compound of Formula (I) 50 mg formulations.

**Study Design**

This is a Phase 1, open-label, randomized, three-period crossover study of the relative bioavailability and the effect of food on the PK of the compound of Formula (I) 50 mg in healthy adult subjects. During treatment period 1 and treatment period 2, subjects will receive a single dose of the compound of Formula (I) 50 mg administered as an encapsulated, lipidic semi-solid (reference) and 1 of 2 different spray-dried dispersion (SDD) test formulations (suspension or capsule) under fed conditions, and during treatment period, three subjects will receive the same SDD test formulation under fasted conditions.

A total of 36 healthy adult subjects will be randomized to 1 of 4 treatment sequences (9 subjects per sequence; approximately equal distribution of males and females per sequence; see Table 22, below). There will be 21 days between each dose.

**Table 22. Treatment sequences**

| Treatment sequence | Treatment period 1 (Fed) | Treatment period 2 (Fed) | Treatment period 3 (Fasted) |
|---|---|---|---|
| 1 | Reference | SDD suspension | SDD suspension |
| 2 | SDD suspension | Reference | SDD suspension |
| 3 | Reference | SDD capsule | SDD capsule |
| 4 | SDD capsule | Reference | SDD capsule |

After providing informed consent, subjects will be screened for eligibility to participate in the study. Screening will begin up to 28 days prior to Day 1 of treatment period 1. Eligible subjects will be admitted to the clinical unit on Day -1, and randomized to 1 of the 4 treatment sequences on Day 1 of treatment period 1. During treatment periods 1 and 2, subjects will fast overnight for at least 10 hours and then ingest a liquid dietary supplement (vanilla-flavored Ensure Plus®, 237 mL container) with the study drug and not consume any other food for 4 hours after dosing. During treatment period 3, subjects will fast overnight for at least 10 hours prior to dosing and continue to fast for an additional 4 hours after dosing. During all treatment periods, water will not be permitted for 1 hour before dosing until 2 hours after dosing except for the water/liquid dietary supplement provided for study drug dosing.

On Day 1 of each treatment period, subjects will be dosed with the compound of Formula (I) 50 mg and have blood samples collected for PK analysis. Subjects will complete a taste satisfaction questionnaire after study drug ingestion on Day 1 of treatment period 3 (only for subjects who receive the SDD suspension under the fasted condition). Subjects will remain in the unit on the day of dosing and will be discharged on Day 2 of each treatment period, following completion of all required procedures, including collection of the 36-hour PK sample. On the mornings of Days 8 and 15 of each treatment period, subjects will return to the clinical unit on an outpatient basis for PK blood sample collection and safety assessments. On Day 21 of treatment period 1 and treatment period 2, subjects will arrive at the site and have Day 21 assessments completed, and they will stay overnight at the site and begin Day 1 of treatment period 2 or treatment period 3 the following day. A final follow-up study visit will be conducted on Day 22 of treatment period 3 (21 $\pm$ 2 days after treatment period 3 dosing) or upon early termination.

Blood samples for PK analysis and safety assessments will be collected/conducted at scheduled times throughout the study. The study design schematic is shown in FIG. 10.

***Duration of treatment***

The expected duration of study participation for each healthy adult subject will be approximately 13 weeks, including up to 28 days of screening, 3 doses each separated by 21 days, and a final follow-up study visit 21 days after

receiving the last dose of study drug during treatment period 3.

***Test product, dose, and mode of administration***

The compound of Formula (I) will be supplied as two different test formulations for oral administration: as powder in bottles for constitution into a suspension (20 mL) and as powder-filled capsules. The compound of Formula (I) test formulations will contain 50 mg of the compound of Formula (I) as free base equivalent. Subjects must swallow the study drug with a liquid dietary supplement (Ensure Plus® [237 mL container]) with an additional 100 mL of water (SDD capsule formulation) or with an additional 80 mL of water (SDD suspension formulation) during treatment period 1 or 2. Subjects must swallow the study drug with 330 mL of water (SDD capsule formulation) or 310 mL of water (SDD suspension formulation) during treatment period 3.

***Reference therapy, dose, and mode of administration***

The compound of Formula (I) reference formulation (encapsulated, lipidic semi-solid formulation) will be supplied as capsules for oral administration. The compound of Formula (I) reference capsules will contain 50 mg of the compound of Formula (I) as free base equivalent. Subjects must swallow a single capsule with a liquid dietary supplement (Ensure Plus [237 mL container]) with an additional 100 mL of water during treatment period 1 or 2.

**Criteria for Evaluation**

***Pharmacokinetics***

Blood samples for assessment of plasma concentrations of the compound of Formula (I) and metabolites will be collected within 45 minutes before dosing, and at approximately 30 minutes, and 1, 2, 3, 4, 5, 6, 7, 8, 9, 10, 12, 16, 24, 36, 168, 336, and 504 hours after dosing.

The following plasma PK parameters will be calculated for the compound of Formula (I) and metabolites:

- Area under the plasma concentration versus time curve from 0 hours to the time of the last measurable concentration ($AUC_{0\text{-tlast}}$)
- Area under the plasma concentration curve extrapolated from 0 hours to infinity ($AUC_{0\text{-}\infty}$)
- Maximum plasma concentration ($C_{max}$)
- Time to achieve maximum plasma concentration ($t_{max}$)
- Delay time between time of dosing and time of appearance of measurable test article ($T_{lag}$)
- Apparent terminal half-life ($t_{½}$)
- Apparent terminal rate constant ($\lambda z$)
- Apparent mean residence time (MRT)
- Molar AUC ratio of primary metabolite(s) to the parent drug the compound of Formula (I)

The following plasma PK parameters will be calculated only for the compound of Formula (I):

- Apparent systemic clearance after oral administration (CL/F)
- Apparent volume of distribution during terminal phase after oral administration (Vz/F)

***Other assessment***

A taste satisfaction questionnaire will be administered.

***Safety assessments***

Safety will be monitored throughout the study and will include the following assessments:

- AEs

- Clinical laboratory tests (hematology, coagulation, clinical chemistry, and urinalysis)

- Vital sign measurements (including orthostatic blood pressure and pulse rate)

- Physical examinations
- 12-lead electrocardiogram (ECG)

***Statistical methods***

Pharmacokinetic parameters will be calculated using noncompartmental methods and summarized by formulation using descriptive statistics. Two-sided 90% confidence intervals will be calculated for the ratio of each test formulation (SDD suspension and SDD capsule) vs. the reference formulation for $AUC_{0-\infty}$, $AUC_{0-tlast}$, and $C_{max}$ for the compound of Formula (I) and metabolites under fed conditions. Further, two-sided 90% confidence intervals will be calculated for the ratio of each test formulation under fasted conditions vs. under fed conditions for $AUC_{0-\infty}$, $AUC_{0-tlast}$, and $C_{max}$ for the compound of Formula (I) and metabolites.

Safety and taste satisfaction questionnaire data will be summarized with descriptive statistics.

**Results**

***Pharmacokinetic results***

Pharmacokinetic results are shown in Tables 23-26, below.

**Table 23: Summary of Plasma Pharmacokinetic Parameters (Safety Analysis Set - SDD Suspension Group)**

| | | Compound of Formula (I) Plasma Concentration | |
|---|---|---|---|
| **Parameter (units) Statistic** | **Reference Capsule (Fed) (N=18)** | **SDD Suspension (Fed) (N=18)** | **SDD Suspension (Fasted) (N=18)** |
| **$AUC_{0-tlast}$ (ng×hr/mL)** | | | |
| Mean (SD) | 16600 (7880) | 5980 (3960) | 737 (417) |
| Geom CV(%) | 46.8 | 72.8 | 68.7 |
| **$AUC_{0-\infty}$ (ng×hr/mL)** | | | |
| Mean | 17400 (8010) | 6500 (4180) | 893 (480) |
| Geom CV(%) | 45.4 | 71.7 | 60.1 |
| **$C_{max}$ (ng/mL)** | | | |
| Mean (SD) | 1480 (506) | 672 (323) | 72.3 (44.9) |
| Geom CV(%) | 32.1 | 57.9 | 57.5 |
| **$t_{max}$ (hr)** | | | |
| Median (min,max) | 5.0 (3.0, 6.0) | 5.0 (5.0, 10) | 7.0 (5.0, 12) |
| **$T_{lag}$ (hr)** | | | |
| Mean (SD) | 1.0 (0.31) | 0.85 (0.29) | 1.2 (0.65) |
| **$t_{½}$ (hr)** | | | |
| Mean (SD) | 50 (20) | 19 (6.1) | 9.0 (0.46) |
| Geom CV(%) | 140 | 93 | 23 |
| **MRT (hr)** | | | |
| Mean (SD) | 36 (43) | 23 (24) | 17 (2.9) |
| Geom CV(%) | 84 | 62 | 18 |
| **CL/F (L/hr)** | | | |
| Mean (SD) | 3.4 (1.4) | 11 (8.0) | 80 (54) |
| Geom CV(%) | 45 | 72 | 63 |

(continued)

| | | | |
|---|---|---|---|
| **Vz/F (L)** | | | |
| Mean (SD) | 180 (260) | 210 (140) | 970 (560) |
| Geom CV(%) | 93 | 77 | 53 |
| **$T_{last}$ (hr)** | | | |
| Mean (SD) | 170 (140) | 77 (110) | 31 (7.6) |
| Geom CV(%) | 120 | 92 | 30 |

**Table 24: Geometric Mean Ratios for Pharmacokinetic Exposure Parameters** by **formulation and fed vs fasted (Safety Analysis Set - SDD Suspension Group)**

| **Analyte Parameter (units)** | **Treatment Comparison** | **Ratio (%)** | **90% CI** |
|---|---|---|---|
| **Compound of Formula (I)** | | | |
| $AUC_{0-tlast}$ (ng×hr/mL) | SDD Suspension (Fed) vs. Reference Capsule (Fed) | 32.2 | (26.4%, 39.4%) |
| $AUC_{0-\infty}$ (ng×hr/mL) | SDD Suspension (Fed) vs. Reference Capsule (Fed) | 33.5 | (27.7%, 40.6%) |
| $C_{max}$ (ng/mL) | SDD Suspension (Fed) vs. Reference Capsule (Fed) | 42.2 | (34.8%, 51.2%) |
| $AUC_{0-tlast}$ (ng×hr/mL) | SDD Suspension (Fasted) vs. Reference Capsule (Fed) | 4.4 | (3.4%, 5.7%) |
| $AUC_{0-\infty}$ (ng×hr/mL) | SDD Suspension (Fasted) vs. Reference Capsule (Fed) | 5.0 | (3.8%, 6.5%) |
| $C_{max}$ (ng/mL) | SDD Suspension (Fasted) vs. Reference Capsule (Fed) | 4.5 | (3.8%, 5.4%) |

**Table 25: Summary of Plasma Pharmacokinetic Parameters (Safety Analysis Set - SDD Capsule Group)**

| | | **Compound of Formula (I) Plasma Concentration** | |
|---|---|---|---|
| **Parameter (units) Statistic** | **Reference Capsule (Fed) (N=18)** | **SDD Capsule (Fed) (N=18)** | **SDD Capsule (Fasted) (N=17)** |
| **$AUC_{0-tlast}$ (ng×hr/mL)** | | | |
| Mean (SD) | 19500 (7960) | 9470 (4670) | 1840 (1650) |
| Geom CV(%) | 50.4 | 48.0 | 78.7 |
| **$AUC_{0-\infty}$ (ng×hr/mL)** | | | |
| Mean | 20100 (7850) | 10000 (4610) | 2120 (1830) |
| Geom CV(%) | 47.1 | 44.9 | 73.7 |
| **$C_{max}$ (ng/mL)** | | | |
| Mean (SD) | 1770 (494) | 1110 (449) | 143 (110) |
| Geom CV(%) | 25.7 | 44.5 | 74.1 |
| **$t_{max}$ (hr)** | | | |
| Median (min,max) | 5.0 (5.0, 8.0) | 5.0 (3.0, 6.0) | 7.0 (6.0, 12) |
| **$T_{lag}$ (hr)** | | | |
| Mean (SD) | 1.0 (0.38) | 1.3 (0.49) | 1.2 (0.88) |
| **$t_{½}$ (hr)** | | | |
| Mean (SD) | 35 (18) | 26 (20) | 14 (11) |
| Geom CV(%) | 69 | 98 | 49 |
| **MRT (hr)** | | | |
| Mean (SD) | 28 (12) | 25 (14) | 21 (9.2) |
| Geom CV(%) | 45 | 55 | 31 |

(continued)

| CL/F (L/hr) | | | |
|---|---|---|---|
| Mean (SD) | 3.0 (1.5) | 5.9 (2.4) | 36 (18) |
| Geom CV(%) | 47 | 45 | 74 |
| **Vz/F (L)** | | | |
| Mean (SD) | 130 (61) | 190 (140) | 630 (380) |
| Geom CV(%) | 47 | 75 | 72 |
| **$T_{last}$ (hr)** | | | |
| Mean (SD) | 170 (96) | 100 (89) | 44 (32) |
| Geom CV(%) | 91 | 100 | 39 |

**Table 26: Geometric Mean Ratios for Pharmacokinetic Exposure Parameters by formulation and fed vs fasted (Safety Analysis Set - SDD Suspension Group)**

| Analyte Parameter (units) | Treatment Comparison | Ratio (%) | 90% CI |
|---|---|---|---|
| **Compound of Formula (I)** | | | |
| $AUC_{0\text{-tlast}}$ (ng×hr/mL) | SDD Capsule (Fed) vs. Reference Capsule (Fed) | 48.2 | (41.4%, 56.1%) |
| $AUC_{0\text{-}\infty}$ (ng×hr/mL) | SDD Capsule (Fed) vs. Reference Capsule (Fed) | 49.7 | (43.1%, 57.2%) |
| $C_{max}$ (ng/mL) | SDD Capsule (Fed) vs. Reference Capsule (Fed) | 59.8 | (51.7%, 69.2%) |
| $AUC_{0\text{-tlast}}$ (ng×hr/mL) | SDD Capsule (Fasted) vs. Reference Capsule (Fed) | 8.0 | (5.7%, 11.2%) |
| $AUC_{0\text{-}\infty}$ (ng×hr/mL) | SDD Capsule (Fasted) vs. Reference Capsule (Fed) | 9.0 | (6.6%, 12.4%) |
| $C_{max}$ (ng/mL) | SDD Capsule (Fasted) vs. Reference Capsule (Fed) | 6.7 | (4.9%, 9.2%) |

**Reference Example 8: Phase 2 study of the compound of Formula (I) in adult subjects with congenital adrenal hyperplasia**

A Phase 2 study to assess the safety, tolerability, pharmacokinetics (PK), and pharmacodynamics (PD) of the compound of Formula (I) in adult subjects with classic congenital adrenal hyperplasia (CAH) was designed. The objectives of the study are: to assess the safety and tolerability of two ascending doses of the compound of Formula (I) in adult subjects with CAH; to evaluate the effect of repeated doses of the compound of Formula (I) on endogenous levels of PD biomarkers in adult subjects with CAH; and to evaluate plasma exposures following repeated doses of the compound of Formula (I) administered nightly.

The lower dose strength selected for this study, the compound of Formula (I) 50 mg, was well tolerated in both single and repeat-dose safety and PK studies in healthy volunteers. Doses up to 100 mg were also well tolerated in both single-dose and repeat-dose Phase 1 studies in healthy volunteers, and importantly, in a large Phase 2 study in non-elderly female and male subjects with major depressive disorder receiving the compound of Formula (I) during an 8-week, double-blind treatment period. Furthermore, the anticipated steady state exposures with the selected the compound of Formula (I) doses, using the predicted $C_{max}$ and AUC, are within the acceptable safety margins defined by the nonclinical toxicology studies that have been conducted to date.

**Study Design**

A Phase 2, open-label, multiple-dose, dose-escalation study to assess the safety, tolerability, PK, and PD of the compound of Formula (I) in approximately 30 adult female and male subjects (18 to 50 years of age) with a documented medical diagnosis of classic 21-hydroxylase deficiency CAH was designed. The study will include a sequential-cohort design with four compound of Formula (I) dose cohorts: 50 mg and 100 mg, with each dose administered for 14 consecutive days:

- Cohort 1: compound of Formula (I) 50 mg once daily with a bottle of vanilla-flavored Ensure Plus® (~237 mL) at approximately 2200 hours.

- Cohort 2: compound of Formula (I) 100 mg once daily with a bottle of vanilla-flavored Ensure Plus® (~237 mL) at approximately 2200 hours.
- Cohort 3: compound of Formula (I) 100 mg once daily with the evening meal at approximately 1900 hours.
- Cohort 4: compound of Formula (I) 100 mg twice daily with breakfast at approximately 0700 hours and with the evening meal at approximately 1900 hours.

There will be an approximate 2-week period to evaluate safety and tolerability data before proceeding from Cohort 1 to Cohort 2. Subjects who previously completed the current study in Cohort 1 or Cohort 2 may reenroll to participate in Cohorts 3 or 4 (in addition to new subjects). Table 27 below depicts the dose cohorts, doses, and number of subjects per cohort.

**Table 27. Dose cohorts, doses, and number of subjects**

| Cohort | Compound of Formula (I) Dose | Approximate Dosing Time(s) | Number of Subjects |
|---|---|---|---|
| 1 | 50 mg | 2200 hours | 8-10 |
| 2 | 100 mg | 2200 hours | 8-10 |
| 3 | 100 mg | 1900 hours | 8-10 |
| 4 | 100 mg | 0700 and 1900 hours | Up to 8 |

Subjects will be screened for eligibility to participate in the study for up to approximately 3 weeks (Days -28 to -8). Subjects who reenroll and have had a stable medication regimen for CAH since their last visit in this study do not have to undergo screening; those who reenroll and have had a change to their medication regimen for CAH must undergo a second screening visit. During screening, subjects will provide a single blood sample in the morning between 0700 and 1000 hours (prior to first morning dose of hydrocortisone) to determine their 17-hydroxyprogesterone (17-OHP) levels for study entry.

Eligible subjects who have a screening 17-OHP level ≥1,000 ng/dL will be admitted to the study center for 1 night and have baseline serial PD samples collected over a 24-hour period beginning in the evening of Day -7. Baseline serial PD samples will be collected at approximately 2145, 2300, 2400, 0100, 0200, 0400, 0600, 0800, 1000, 1200, 1400, 1600, and 2200 hours. The subjects' usual morning dose of steroidal treatments will be administered after the 1000 hours PD sample is collected on Day -6. Subjects will be discharged on Day -6 after the last PD sample is collected.

Subjects within each dose cohort will be admitted to the study center on Days 1 and 14 (first and last day of dosing). Subjects will have a blood sample collected on Day 1 for CYP21A2 genotyping. Baseline safety assessments will be collected on Day 1 prior to the first dose of study drug. Study drug (the compound of Formula (I) 50 or 100 mg) will be administered at approximately 2200 hours for Cohorts 1 and 2 and at approximately 1845, 2000, 2100, 2200, 2300, 2400, 0100, 0200, 0400, 0600, 0800, 1000, 1200, 1400, 1800, 1900, and 2200 hours for Cohorts 3 and 4. The subjects' usual morning dose of steroidal treatments will be administered after the 1000 hours PD sample is collected on Day -6. Subjects will be discharged on Day -6 after the last PD sample is collected.

Subjects in Cohorts 1 and 2 will be admitted to the study center on Days 1 and 14 (first and last day of dosing). Subjects will have a blood sample collected on Day 1 for cytochrome P450 (CYP) 21A2 genotyping. Baseline safety assessments will be collected on Day 1 prior to the first dose of study drug. Study drug (compound of Formula(I) 50 mg or 100 mg) will be administered at approximately 2200 hours. The subjects' usual morning dose of concurrent steroidal treatments will be administered after the 12-hour post-dose PK/PD samples are collected (at approximately 1000 hours) on Day 2 and after the 16-hour post-dose PK/PD samples are collected (at approximately 1400 hours) on Day 15. Subjects will be discharged from the study center the evening on Days 2 and 15 following completion of all study-related procedures for those days. Prior to this discharge on Day 2, study drug will be administered at the study center at approximately 2200 hours. Study drug will then be self-administered nightly at home at approximately 2200 hours on Days 3 to 13. Subjects will take their usual morning dose of concurrent steroidal treatments at approximately 1000 hours on Days 3 to 14. On Day 7 during the treatment period, PK, PD, and safety assessments will be conducted in an outpatient setting at the study center.

Subjects in Cohorts 3 and 4 will have a blood sample collected on Day 1 for CYP21A2 genotyping (only for subjects who have not previously participated in Cohorts 1 or 2). Baseline safety assessments will be collected on Day 1 prior to the first dose of study drug. For Cohort 3, study drug (compound of Formula(I) 100 mg) will be administered at home on Days 1 to 13 at approximately 1900 hours with each subject's evening meal. For Cohort 4, study drug compound of Formula(I) 100 mg) will be administered at home on Days 2 to 14 at approximately 0700 hours with each subject's breakfast and on Days 1 to 13 at approximately 1900 hours with each subjects' evening meal. For both cohorts, the Day 14 evening dose will be administered at the study site. Subjects will take their usual morning dose of concurrent steroidal treatments at approximately 1000 hours on Days 1 to 14. On Day 7 during the treatment period, PK, PD, and safety

assessments will be conducted in an outpatient setting at the study center. Subjects will be admitted to the study center on Day 14 (last day of dosing). On Day 14, subjects will receive study drug in the study center at approximately 1900 hours with a standard (moderate fat/moderate calorie) evening meal. The subjects' usual morning dose of concurrent steroidal treatments will be administered after PK/PD samples are collected at approximately 1400 hours on Day 15. Subjects will be discharged from the study center the evening on Day 15 following completion of all study-related procedures.

For all cohorts, follow-up visits on Days 21, 28, and 35 will be conducted at the study center or the subject's home by a qualified home healthcare provider (based on the subject's preference). A final study visit will be conducted at the study site approximately 5 weeks after the last dose of study drug (on Day 49 or early termination). There will be a visit window of - 8 hours for Day 7, -8 hours/+3 days for Days 21, 28, and 35, and +7 days for the final study visit. Safety, tolerability, PK, and PD will be assessed at scheduled times throughout the study. The study design schematic is shown in FIG. 11.

***Dose escalation procedure***

Cohort 1 will consist of approximately 8 to 10 subjects who will receive a daily dose of the compound of Formula (I) 50 mg at approximately 2200 hours for 14 days (subjects will receive study drug at the site on Days 1, 2, and 14, and self-administer study drug at home on Days 3 to 13). Following the completion of Day 15 assessments for all subjects in the Cohort 1, a medical monitor will review the accumulated safety and tolerability results to ensure there are no safety concerns with proceeding to the 100 mg dose (Cohorts 2 and 3), and to determine if a maximum tolerated dose (MTD) has been reached. If the MTD is reached, no dose escalation will occur. There will be an approximate 2-week period between Cohorts 1 and 2 to accommodate this safety review. A similar procedure will be used prior to proceeding to the 100 mg twice daily dose (Cohort 4).

If the medical monitor determines that it is safe to proceed to the compound of Formula (I) 100 mg, subjects in Cohort 2 will be administered the compound of Formula (I) 100 mg daily for 14 days. Dosing for Cohorts 3 and 4 may begin simultaneously with Cohort 2.

During the 14-day dosing period for any cohort, dosing may be postponed or halted if one or more subjects experience a severe or serious adverse event (AE), or if the type, frequency, or severity of AEs becomes unacceptable. If dosing is postponed, the medical monitor will review all available safety, tolerability, and PK data before allowing any further subjects to receive study drug.

***Study Population***

Approximately 30 adult female and male subjects (18 to 50 years of age) with a documented medical diagnosis of classic 21-hydroxylase deficiency CAH, who meet all protocol eligibility criteria, will be enrolled. Subjects who previously completed the current study in Cohort 1 or Cohort 2 may reenroll to participate in Cohorts 3 or 4 (in addition to new subjects).

***Duration of treatment***

The expected duration of study participation for each subject is approximately 11 weeks, including up to approximately 3 weeks for screening, a 24-hour PD baseline period (approximately 7 days prior to the first day of dosing), 14 days of dosing, and a follow-up period of approximately 5 weeks. The total duration of the study will be an additional 8 to 11 weeks for subjects who reenroll.

***Test product, dose, and mode of administration***

The compound of Formula (I) will be supplied as capsules containing 50 mg of the compound of Formula (I) free base for oral administration (see, e.g., Reference 1 formulation as described in Reference Example 9). Doses of the compound of Formula (I) are 50 mg and 100 mg, administered in oral capsule form. Each dose of study drug for Cohorts 1 and 2 is to be administered with a bottle of vanilla-flavored Ensure Plus® (~237 mL). Each dose of study drug for Cohort 3 is to be administered with each subject's evening meal at approximately 1900 hours. Each dose of study drug for Cohort 4 is to be administered with each subject's breakfast at approximately 1900 hours (i.e., a total daily dose of 200 mg).

**Criteria for Evaluation**

*Cohorts 1 and 2*

Blood samples to evaluate 24-hour PD baseline will be collected on Days -7 to -6 at approximately 2145, 2300,

2400, 0100, 0200, 0400, 0600, 0800, 1000, 1200, 1400, 1600, and 2200 hours. Blood samples to evaluate PK and PD parameters of the compound of Formula (I) will be collected on Days 1 to 2 and Days 14 to 15 at: 15 minutes pre-dose and at 1, 2, 3, 4, 6, 8, 10, 12, 14, 16, 20, and 24 hours post-dose; Day 7 (at approximately 24 hours post-dose); Days 21, 28, and 35 (at approximately 168, 336, and 504 hours post-dose); and at the final study visit (Day 49 or early termination).

*Cohorts 3 and 4*

Blood samples to evaluate 24-hour PD baseline will be collected on Days -7 to -6 at approximately 1845, 2000, 2100, 2200, 2300, 2400, 0100, 0200, 0400, 0600, 0800, 1000, 1200, 1400, 1800, 1900, and 2200 hours. Blood samples to evaluate PK and PD parameters of the compound of Formula (I) will be collected on Days 14 to 15 at the following times (for Cohort 4, all times are relative to evening dosing unless otherwise indicated): 15 minutes predose and at 1, 2, 3, 4, 5, 6, 7, 9, 11, 13, 15, 17, 19, 23, 24, and 27 hours postdose; Day 7 (at 24 hours postdose for Cohort 3 or at 12 hours post morning dose but prior to the evening dose for Cohort 4); Days 21, 28, and 35 (at approximately 168, 336, and 504 hours postdose); and at the final study visit (Day 49 or early termination).

***Pharmacokinetics***

The following plasma PK parameters will be calculated for the compound of Formula (I) and metabolites:

- Area under the plasma concentration versus time curve from 0 hours to last measurable concentration ($AUC_{0\text{-tlast}}$)
- Area under the plasma concentration versus time curve from 0 to 24 hours ($AUC_{0\text{-}24}$)
- Maximum plasma concentration ($C_{max}$)
- Time to maximum plasma concentration ($t_{max}$)
- Delay time between time of dosing and time of appearance of measurable test article ($T_{lag}$)
- Terminal half-life (t½)
- Apparent terminal rate constant ($\lambda z$)
- Apparent mean residence time (MRT)

Additional PK parameters for Day 14 only:

- Average plasma concentration at steady state ($C_{avg}$)
- Percent fluctuation at steady state (% fluctuation)
- Accumulation index at steady state
- Apparent systemic clearance after oral administration (CL/F) (the compound of Formula (I) only)

***Pharmacodynamics***

Primary: Morning 17-OHP (serum; ng/dL) from the 0600, 0800, and 1000 hour samples (8-, 10-, and 12-hour post-dose samples from Cohorts 1 and 2 and 11-, 13-, and 15-hour postdose samples from Cohorts 3 and 4.

Secondary: 17-OHP at all other times, androstenedione (serum; ng/dL), testosterone (serum; ng/dL), cortisol (serum; $\mu$g/dL), and adrenocorticotropic hormone (plasma ACTH; pg/mL).

***Safety***

Safety and tolerability will be monitored throughout the study and will include the following assessments:

- Adverse events
- Clinical laboratory tests - clinical chemistry (including creatine kinase, myoglobin, total and conjugated bilirubin), hematology, coagulation (prothrombin time, aPTT, d-dimer, fibrinogen), and urinalysis (including quantitative myoglobin, casts and crystals)
- Vital signs
- Physical examinations (including musculoskeletal exam)
- 12-lead electrocardiograms (ECGs)
- Columbia-Suicide Severity Rating Scale (C-SSRS)
- Brief Psychiatric Rating Scale (BPRS)

***Statistical methods***

Safety, PK, and PD variables will be summarized within each dose cohort (the compound of Formula (I) 50 mg and 100 mg) using descriptive statistics. Summaries of PD measures will include both observed values and changes from pre-dose.

**Results**

***Pharmacokinetic results***

Eight subjects (four female and four male) were enrolled in this study and completed Cohort 1. Age, sex, and BMI information for the study participants is shown in Table 28, below.

**Table 28. Cohort 1 Subjects**

| **Subject ID** | **Age (years)** | **Sex (Male/Female)** | **BMI (kg/m$^2$)** |
|---|---|---|---|
| 001 | 37 | Female | 24.9 |
| 002 | 25 | Female | 32.0 |
| 003 | 49 | Male | 37.2 |
| 004 | 36 | Male | 25.0 |
| 005 | 27 | Female | 25.5 |
| 006 | 19 | Male | 21.7 |
| 007 | 25 | Male | 27.5 |
| 008 | 31 | Female | 34.4 |

The subjects received a daily dose of the compound of Formula (I) at 50 mg at approximately 2200 hours (~10 p.m. or bedtime) for 14 days. Arithmetic mean values for ACTH (FIG. 12A) and 17-OHP (FIG. 12B) for all 8 Cohort 1 subjects were plotted at each timepoint for Pre-treatment Baseline, Day 1, and Day 14. Both ACTH and 17OHP concentration profiles at day 1 and day 14 show clear reductions from the baseline mean profiles. Cohort 1 mean PK parameters of $T_{max}$, $C_{max}$, and $AUC_{24}$ for the compound of Formula (I) are shown in Table 29 below. These PK parameters are consistent with observations from Phase 1 studies in healthy volunteers.

**Table 29. Cohort 1 Mean PK parameters of $T_{max}$, $C_{max}$, and $AUC_{24}$**

| | **Day 1** | | | **Day 14** | | |
|---|---|---|---|---|---|---|
| | **$T_{max}$** | **$C_{max}$** | **$AUC_{24}$** | **$T_{max}$** | **$C_{max}$** | **$AUC_{24}$** |
| **Mean** | **5.4** | **1305** | **10,292** | **4.4** | **1349** | **14,297** |
| **CV%** | 30 | 25 | 24 | 32 | 15 | 24 |

Additional measurements of cohort 1 and cohort 2 mean PK parameters of $T_{max}$, $C_{max}$, and $AUC_{24}$ for the compound of Formula (I) on Day 1 of dosing are shown in Table 30 below.

**Table 30: Mean PK parameters of $T_{max}$, $C_{max}$, and $AUC_{24}$ on Day 1 of dosing**

| | **Cohort 1 50 mg- Ensure** | | | **Cohort 2 100 mg-Ensure** | | |
|---|---|---|---|---|---|---|
| **Day 1** | **$T_{max}$* (h)** | **$C_{max}$ (ng/ml)** | **$AUC_{24}$ (h*ng/ml)** | **$T_{max}$ (h)** | **$C_{max}$ (ng/ml)** | **$AUC_{24}$ (h*ng/ml)** |
| **G.Mean** | 6 | 1,270 | 10,411 | 4 | 2,370 | 24,725 |
| **CV%** | - | 25 | 24 | - | 21 | 42 |
| **N** | 8 | 8 | 8 | 8 | 4 | 4 |
| *Median | | | | | | |

Additional measurements of cohort 1, cohort 2 and cohort 3 mean PK parameters of $T_{max}$, $C_{max}$, and $AUC_{24}$ for the compound of Formula (I) on Day 14 of dosing are shown in Table 31 below.

**Table 31: Mean PK parameters of $T_{max}$, $C_{max}$, and $AUC_{24}$ on Day 14 of dosing**

| | Cohort 1 50 mg- Ensure | | | Cohort 2 100 mg-Ensure | | | 3 100 mg w/evening Meal | | |
|---|---|---|---|---|---|---|---|---|---|
| Day 14 | Tmax* (h) | Cmax (ng/ml) | AUC24 (h*ng/ml) | Tmax (h) | Cmax (ng/ml) | AUC24 (h*ng/ml) | Tmax (h) | Cmax (ng/ml) | AUC24 (h*ng/ml) |
| G. Mean | 4 | 1,335 | 14,070 | 4 | 3,379 | 35,416 | 3 | 3,650 | 34,706 |
| CV% | | 15 | 24 | | 31 | 37 | | 32 | 15 |
| N | 8 | 8 | 8 | 6 | 6 | 6 | 8 | 8 | 8 |
| *Median | | | | | | | | | |

Arithmetic mean values for androstenedione (FIG. 13A) and testosterone (FIG. 13B) for all 8 Cohort 1 subjects were plotted at each timepoint for Pre-treatment Baseline, day 1, and day 14. The androstenedione concentration profile on Day 1 and Day 14 shows a clear reduction from the baseline mean profile.

When focusing exclusively on the critical morning window period (timepoints at 8-, 10-, and 12-hours postdose) from 6:00 a.m. to 10:00 a.m., the levels of ACTH show marked reductions from baseline at each of the 3 timepoints on Days 1 and 14 (FIG. 14A). Arithmetic mean values across all three timepoints show >=50% reductions from baseline at Day 1 and Day 14 (FIG. 14B).

When focusing exclusively on the critical morning window period (timepoints at 8-, 10-, and 12-hours postdose) from 6:00 a.m. to 10:00 a.m., the levels of 17-OHP show marked reductions from baseline at each of the 3 timepoints on Days 1 and 14 (FIG. 15A). Arithmetic mean values across all three timepoints show >=40% reductions from baseline at Day 1 and Day 14 (FIG 15B).

When focusing exclusively on the critical morning window period (timepoints at 8-, 10-, and 12-hours postdose) from 6:00 a.m. to 10:00 a.m., the levels of androstenedione show marked reductions from baseline at each of the 3 timepoints on Days 1 and 14 (FIG. 16A). Arithmetic mean values across all three timepoints show >=30% reductions from baseline at Day 1 and Day 14 (FIG. 16B).

A summary of reduction in 17-OHP and androstenedione in cohort 1 is shown in Table 32. Further, the androstenedione levels of three subjects was normalized by the treatment for the three subjects with Subject ID Nos. 001, 002, and 006).

**Table 32. Cohort 1 Summary of Reduction in 17-OHP and Androstenedione at each timepoint in the morning window (6 a.m. to 10 a.m.)**

| Subject ID | Sex/ Age | Dosing Day | 17-OHP % change from baseline | | | Androstenedione % change from baseline | | |
|---|---|---|---|---|---|---|---|---|
| | | | 6 AM | 8 AM | 10 AM | 6 AM | 8 AM | 10 AM |
| **001** | F/37 | D1<br>D14 | -61.4<br>-90.3 | 14.7<br>-67.2 | -24.1<br>-37.2 | -42.0<br>-84.6 | 2.0<br>-58.6 | -19.5<br>-55.1 |
| **002** | F/25 | D1<br>D14 | -96.0<br>-37.7 | -95.5<br>-58.6 | 45.4<br>-24.9 | -68.0<br>-34.2 | -66.7<br>-47.8 | -21.7<br>-38.7 |
| **003** | M/49 | D1<br>D14 | -11.8<br>-24.5 | -46.6<br>-5.2 | -34.0<br>-22.9 | -13.4<br>-7.5 | -47.1<br>-10.8 | -22.7<br>-16.0 |
| **004** | M/36 | D1<br>D14 | -13.7<br>-53.6 | 17.1<br>-46.8 | 10.1<br>-35.8 | 4.5<br>-13.8 | 15.9<br>-15.2 | 3.0<br>-21.2 |
| **005** | F/27 | D1<br>D14 | -24.4<br>-78.8 | -83.6<br>-95.1 | -52.2<br>-86.3 | -35.6<br>26.7 | -50.3<br>-13.7 | -33.5<br>-10.9 |
| **006** | M/19 | D1<br>D14 | -5.1<br>-25.4 | -1.1<br>-20.4 | -97.8<br>-98.3 | -10.0<br>-67.8 | -18.5<br>-62.7 | -78.9<br>-92.1 |
| **007** | M/25 | D1<br>D14 | -50.0<br>14.9 | -28.0<br>4.0 | -27.4<br>-7.1 | -24.8<br>-4.8 | -30.0<br>-36.8 | -15.8<br>-30.2 |

(continued)

| Subject ID | Sex/ Age | Dosing Day | 17-OHP % change from baseline | | | Androstenedione % change from baseline | | |
|---|---|---|---|---|---|---|---|---|
| | | | 6 AM | 8 AM | 10 AM | 6 AM | 8 AM | 10 AM |
| **008** | F/31 | D1<br>D14 | -64.5<br>-59.0 | -80.7<br>-65.2 | -92.8<br>-89.1 | -12.2<br>74.5 | 7.0<br>99.4 | -9.3<br>59.2 |

**Table 33. Cohort 1 Summary of PK parameters of $T_{max}$, $C_{max}$, and $AUC_{24}$ for each subject at days 1 and 14**

| | Day 1 | | | Day 14 | | |
|---|---|---|---|---|---|---|
| **Subject ID** | **$T_{max}$ (h)** | **$C_{max}$ (ng/ml)** | **$AUC_{24}$ (h*ng/ml)** | **$T_{max}$ (h)** | **$C_{max}$ (ng/ml)** | **$AUC_{24}$ (h*ng/ml)** |
| **001** | 6 | 1830 | 14,487 | 6 | 1570 | 20,863 |
| **002** | 6 | 1210 | 11,829 | 4 | 1610 | 15,494 |
| **003** | 6 | 1060 | 11,068 | 3 | 1310 | 15,599 |
| **004** | 4 | 1670 | 9,669 | 3 | 1160 | 10,180 |
| **005** | 8 | 961 | 9,266 | 6 | 1550 | 15,130 |
| **006** | 4 | 1030 | 6,574 | 3 | 1090 | 10,288 |
| **007** | 6 | 1550 | 8,239 | 6 | 1170 | 12,880 |
| **008** | 3 | 1130 | 11,209 | 4 | 1330 | 13,944 |
| **Mean** | **5.4** | **1305** | **10,292** | **4.4** | **1349** | **14,297** |
| **CV%** | 30 | 25 | 24 | 32 | 15 | 24 |
| **N** | 8 | 8 | 8 | 8 | 8 | 8 |

After 14 days of once-daily compound of Formula (I) administration, a majority of participants in Cohorts 1-3 showed reduced serum concentrations of adrenal androgens and precursors. Mean changes from baseline (±standard deviation) in Cohort 1 were as follows: 17-OHP, -2341.0±1535.0 ng/dL; androstenedione, -98.4±98.7 ng/dL; and ACTH, -157.0±194.9 pg/mL. Mean reductions were larger in Cohort 2 (17-OHP, -4406.0±5516.1; androstenedione, -362.8 ±354.0; ACTH, -180.9±155.2) and Cohort 3 (17-OHP, -4760.1±4018.2; androstenedione, -210.9±188.6; ACTH, -358.9 ±177.6), suggesting a possible dose response. FIGs. 17A-17C, 18A-18C, and 19A-19C depict results from Cohorts 1, 2, and 3, respectively.

***Summary***

The results from this ongoing Phase II open-label study demonstrated a reduction of at least 50 percent from baseline in 17-hydroxyprogesterone (17-OHP) and adrenocorticotropic hormone (ACTH) levels in more than 50 percent of CAH patients in cohort 1 treated with the compound of Formula (I) for 14 days (i.e., 6 of 8 patients in cohort 1 had a reduction of ≥50% from baseline levels of 17-OHP during at least one morning window timepoint, see, e.g., Table 32). Meaningful reductions were also observed in other biomarkers, including androstenedione (i.e., 4 of these patients also had a reduction of ≥50% from baseline levels of androstenedione during at least one morning window timepoint, see, e.g., Table 32). The greater reductions in biomarkers in cohorts 2 and 3, treated with double the dose of the compound of Formula (I) compared with cohort 1, suggest a possible dose response. Further, the compound of Formula (I) was well-tolerated with a relatively small number of mild adverse events (AEs) reported (e.g., headache, ovulation pain, fatigue, localized infection (toe), dizziness, nausea, URI, contusion with the most common being headache). No clinically significant findings from routine labs, vital signs, or electrocardiograms were found.

**Reference Example 9: Reference Formulation 1 of the compound of Formula (I)**

Tables 34A and 34B show Reference formulation 1 of the compound of Formula (I) as used in the clinical studies described in Reference Examples 6 and 8, above. An example manufacturing process is shown in FIG. 20. Another example manufacturing process is shown in FIG. 21.

**Table 34A:**

| Component | Quality Standard | Function | 50 mg Capsule | |
|---|---|---|---|---|
| | | | Weight (mg/un it) | % (w/w) |
| Compound of Formula (I), free base | In-house | Active Ingredient | 50.0 | 10.0 |
| Medium-Chain Triglycerides (Labrafac TM Lipophile WL1349) | NF | Oily Phase Vehicle | 196.0 | 39.2 |
| Propylene Glycol Dicaprylate/Dicaprate, (LabrafacTM PG) | NF | Emulsifying Agent | 102.0 | 20.4 |
| Lauroyl Polyoxyl-32 Glycerides (Gelucire® 44/14) | NF | Nonionic Surfactant & Solubilizing Agent | 95.0 | 19.0 |
| Vitamin E Polyethylene Glycol Succinate (Kolliphor® TPGS) | USP/NF | Solubilizing Agent | 57.0 | 11.4 |
| **Total Emulsion Weight** | | | **500.0** | **100.0** |
| Gelatin Capsule Shell. Size #00, Swedish Orange cap/body; (Coni-Snap®) | Non Pharmacopo eial | Capsule Shell | -- | -- |
| Gelatin Powder, 220 Bloom | USP | Capsule shell banding agent | -- | -- |
| Purified Water | USP | Capsule shell banding solvent | -- | -- |

**Table 34B:**

| Component | Quality Standard | Function | 50 mg Capsule | |
|---|---|---|---|---|
| | | | Weight (mg/un it) | % (w/w) |
| Compound of Formula (I), free base | In-house | Active Ingredient | 50.0 | 10.0 |
| Medium-Chain Triglycerides (caprylic:capric acid 60:40; Miglyol 812N) | Ph. Eur./NF | Vehicle | 195.85 | 39.2 |
| Propylene Glycol Dicaprylocaprate, (LabrafacTM PG) | Ph. Eur. | Vehicle | 102.15 | 20.4 |
| Lauroyl macrogolglycerides type 1500-Lauroyl polyoxylglycerides type 1500 (Gelucire® 44/14) | Ph. Eur./NF | Surfactant | 95.0 | 19.0 |
| Vitamin E Polyethylene Glycol Succinate, 260 mg/g d-alpha tocopherol (Vitamin E/TPGS 260) | NF | Surfactant | 57.0 | 11.4 |
| **Total Emulsion Weight** | | | **500.0** | **100.0** |
| Orange opaque hard capsule, size 0, composed of gelatin, titanium dioxide and red ferric oxide (Swedish Orange ®) | Non Pharmacopo eial | Capsule Shell | -- | -- |
| Ethanol (96%) and Purified Water | USP | Capsule shell banding solvent | -- | -- |

**Reference Example 10: Study Evaluating Effect of Ensure Plus, Ensure Pudding, Milk and High Fat Meal on Reference Capsule**

**Study design**

This is a Phase 1, open-label, randomized, four-period crossover study to evaluate the effect of food with different

levels of fat and caloric content on the PK, safety, and tolerability of the compound of Formula (I) in healthy adult subjects.

A total of 16 healthy adult subjects (8 males and 8 females) will be randomly assigned to 1 of 4 treatment sequences (4 subjects per sequence [2 males and 2 females per sequence]; see Table 35 below). During each treatment period, subjects will receive a single dose of the compound of Formula (I) 100 mg administered with the appropriate meal, according to the randomization scheme. There will be a washout of at least 21 days between each dose.

**Table 35:**

| Treatment Sequence | Treatment Period 1 | Treatment Period 2 | Treatment Period 3 | Treatment Period 4 |
|---|---|---|---|---|
| 1 | Reference meal | Test meal 1 | Test meal 2 | Test meal 3 |
| 2 | Test meal 1 | Test meal 3 | Reference meal | Test meal 2 |
| 3 | Test meal 2 | Reference meal | Test meal 3 | Test meal 1 |
| 4 | Test meal 3 | Test meal 2 | Test meal 1 | Reference meal |
| Reference meal: vanilla-flavored Ensure Plus® Test meal 1: Low fat, low caloric content meal<br>1 Test meal 2: Low fat, low caloric content meal 2<br>Test meal 3: standard high fat, high caloric content meal | | | | |

After providing informed consent, subjects will be screened for eligibility to participate in the study. Screening will begin up to 28 days before Day 1 of treatment period 1. Eligible subjects will be admitted to the clinical unit on Day -1 and randomized to 1 of the 4 treatment sequences on Day 1 of treatment period 1. During each treatment period, subjects will fast overnight for at least 10 hours until the start of the assigned meal, according to the randomization scheme, and ingest the study drug at approximately 0800 hours. Subjects must complete the entire meal within the specified time period and should not consume any other food for 4 hours after dosing. For all treatment periods, water will not be permitted for 1 hour before dosing until 2 hours after dosing except for the water provided with study drug dosing and planned meals.

On Day 1 of each treatment period, subjects will be dosed with the compound of Formula (I) 100 mg. Blood samples will be collected for PK analysis over a period of 36 hours during the in-house stay. During each treatment period, subjects will remain in the clinic on the day of dosing and will be discharged on Day 2 after completing all required procedures. On the mornings of Days 8 and 15 of each treatment period, subjects will return for an outpatient visit to the clinic for PK blood sample collection and safety assessments. On Day 21 of treatment periods 1 to 3, subjects will arrive at the site and have Day 21 assessments completed and they will stay overnight at the site and begin Day 1 of the subsequent treatment period the following day. A final follow-up study visit will be conducted on Day 22 of treatment period 4 (21 $\pm$ 2 days after treatment period 4 dosing) or upon early termination.

Blood samples for PK d/conducted at scheduled times throughout the study.

**Study population**

Sixteen healthy adult subjects (8 males and 8 females) between 18 and 55 years of age, inclusive, who meet all protocol eligibility criteria, will be enrolled.

**Duration of treatment**

The expected duration of study participation for each healthy adult subject will be approximately 16 weeks, including up to 28 days of screening, 4 days of dosing with at least 21 days between consecutive doses, and a final follow-up study visit 21 days ($\pm$ 2 days) after receiving the last dose of study drug during treatment period 4.

**Test product, dose, and mode of administration**

The compound of Formula (I) will be supplied as capsules for oral administration (encapsulated, lipidic semi-solid formulation, e.g., Reference Example 9). The compound of Formula (I) capsules will contain 50 mg of the compound of Formula (I) as free base equivalent. During each treatment period, subjects will receive two 50 mg capsules (100 mg) of the study drug along with a meal and water as defined by the randomization scheme. The food, water, and study drug administration are as follows:

<u>Reference meal:</u> Two capsules of study drug will be administered approximately 5 minutes after the start of a liquid dietary supplement (i.e., Ensure Plus® [237 mL container]) and an additional 120 mL of water for study drug dosing.
<u>Test</u> meal 1: Two capsules of study drug will be administered approximately 5 minutes after the start of a low fat, low

caloric meal 1 with 120 mL of water for study drug dosing.
Test meal 2: Two capsules of study drug will be administered approximately 5 minutes after the start of a low fat, low caloric meal 2 with 120 mL of water for study drug dosing.
Test meal 3: Two capsules of study drug will be administered approximately 30 minutes after the start of a high fat, high caloric meal with 120 mL of water for study drug dosing.

**CRITERIA FOR EVALUATION**

**Pharmacokinetics**

Blood samples for assessment of plasma concentrations of the compound of Formula (I) and metabolites will be collected within 45 minutes before dosing, and at approximately 30 minutes, and 1, 2, 3, 4, 5, 6, 7, 8, 9, 10, 12, 16, 24, 36, 168, 336, and 504 hours after dosing.
The following plasma PK parameters will be calculated for the compound of Formula (I) and metabolites:

- Area under the plasma concentration versus time curve from 0 hours to last measurable concentration ($AUC_{0-last}$)
- Area under the plasma concentration versus time curve from 0 hours extrapolated to infinity ($AUC_{0-\infty}$)
- Maximum plasma concentration ($C_{max}$)
- Time to achieve maximum plasma concentration ($t_{max}$)
- Delay time between time of dosing and time of appearance of measurable test article ($T_{lag}$)
- Apparent terminal half-life (t½)
- Apparent terminal rate constant ($\lambda z$)
- Apparent mean residence time (MRT)
- Molar AUC ratio of primary metabolite(s) to the parent drug compound of Formula (I). The following plasma PK parameters will be calculated only for the compound of Formula (I):
- Apparent systemic clearance after oral administration (CL/F)
- Apparent volume of distribution during terminal phase after oral administration (Vz/F)

Safety Assessments

Safety will be monitored throughout the study and will include the following assessments:

- Adverse events (AEs)
- Clinical laboratory tests (hematology, coagulation, clinical chemistry, and urinalysis)
- Vital sign measurements (including orthostatic blood pressure and pulse rate)
- Physical examinations
- 12-lead electrocardiograms (ECGs)

**Statistical methods**

Pharmacokinetic parameters will be calculated using noncompartmental methods and summarized by meal type (test meal/reference meal) using descriptive statistics. Two-sided 90% confidence intervals will be calculated for the ratio of each test meal versus the reference meal for $AUC_{0-\infty}$, $AUC_{0-tlast}$, and $C_{max}$ for the compound of Formula (I) and metabolites.
Safety data will be summarized with descriptive statistics.

**Results**

***Pharmacokinetic results***

Pharmacokinetic results are shown in Table 36 below.

**Table 36: Summary of Plasma Pharmacokinetic Parameters**

| | | Compound of Formula (I) Plasma Concentration | | |
|---|---|---|---|---|
| **Parameter (units) Statistic** | **Ensure Plus (Fed) (N=18)** | **Ensure Pudding (Fed) (N=18)** | **Whole Milk (Fed) (N=17)** | **High Fat Meal (Fed) (N=17)** |
| **$AUC_{0-tlast}$ (ng×hr/mL)** | | | | |
| Mean (SD) | 36703 (17400) | 34077 (11597) | 35561 (15866) | 55487 (23242) |
| Geom CV(%) | 58 | 39.7 | 44.6 | 41.9 |
| **$AUC_{0-\infty}$ (ng×hr/mL)** | | | | |
| Mean | 45386 (19648) | 40037 (9515) | 46737 (17395) | 63755 (19139) |
| Geom CV(%) | 43.3 | 23.8 | 38.8 | 31 |
| **$C_{max}$ (ng/mL)** | | | | |
| Mean (SD) | 3090 (1070) | 3038 (984) | 2835 (1005) | 4336 (1938) |
| Geom CV(%) | 39.7 | 34.9 | 34.5 | 53.6 |
| **$t_{max}$ (hr)** | | | | |
| Median (min,max) | 5.0 (2.0, 10.0) | 5.0 (4.0, 6.0) | 5.0 (4.0, 7.0) | 5.0 (4.0, 7.0) |
| **$T_{lag}$ (hr)** | | | | |
| Mean (SD) | 0.38 (0.46) | 0.20 (0.25) | 0.16 (0.31) | 0.29 (0.37) |
| **$t_{½}$ (hr)** | | | | |
| Mean (SD) | 361 (263) | 373 (196) | 373 (143) | 326 (120) |
| Geom CV(%) | 61.1 | 48.6 | 37.1 | 33.8 |
| **CL/F (L/hr)** | | | | |
| Mean (SD) | 2.49 (0.956) | 2.636 (0.659) | 2.427 (0.975) | 1.706 (0.526) |
| Geom CV(%) | 41.2 | 24.8 | 38.8 | 31.1 |
| **Vz/F (L)** | | | | |
| Mean (SD) | 1254 (906) | 1414 (720) | 1308 (627) | 816 (378) |
| Geom CV(%) | 72.7 | 58.4 | 61.3 | 54.9 |

**Reference Example 11: Spray-dried Dispersion Granule Formulation of the Compound of Formula (I) (SDD-G)**

Table 37 shows a granule formulation of the compound of Formula (I) using a SDD prepared according to Reference Example 3, above. An example manufacturing process is shown in FIG. 22A and FIG. 22B.

**Table 37:**

| Component | Quality Standard | Function | 50 mg Sachet | | Batch Weight (g) |
|---|---|---|---|---|---|
| | | | Weight (mg/unit) | % (w/w) | |
| Reference Example 3, SDD | In-house | Drug Substance | 200.0 | 13.33 | 40 |
| Calcium Silicate (ZEOPHARM 250) | NF | Glidant | 10.0 | 0.67 | 2 |
| Mannitol (Pearlitol 200SD) | NF / EP / JP | Filler | 832.5 | 55.5 | 166.5 |
| Microcrystalline Cellulose (Avicel PH-102) | NF / EP / JP | Filler | 300.0 | 20.0 | 60 |
| Croscarmellose Sodium (Ac-Di-Sol®) SD711 | NF / EP / JP | Disintegrant | 150.0 | 10.0 | 30 |
| Sodium Stearyl Fumarate | NF / EP / JP | Lubricant | 7.5 | 0.5 | 1.5 |

(continued)

| Component | Quality Standard | Function | 50 mg Sachet | | Batch Weight (g) |
|---|---|---|---|---|---|
| | | | Weight (mg/unit) | % (w/w) | |
| Total | | | 1500 | 100.0 | 300 |

**Example 12: Liquid Formulation 1 of the Compound of Formula (I)**

Table 38 shows liquid formulation 1 of the Compound of Formula (I) free base. An example manufacturing process is shown in FIG. 23.

**Table 38:**

| Component | Quality Standard | Function | 50 mg/mL Oral Solution | | Batch Weight (g) |
|---|---|---|---|---|---|
| | | | Weight (mg/mL) | % (w/v) | |
| Compound of Formula (I) FB | In-house | Drug Substance | 50.0 | 5 | 20.03 |
| Saccharin | NF / EP | Sweetener | 1.5 | 0.15 | 0.61 |
| Butylated hydroxytoluene | NF / EP | Anti-oxidant | 1.7 | 0.17 | 0.69 |
| FONA orange flavor | NF | Flavor | 1.0 | 0.1 | 0.41 |
| Labrafac Lipophile WL1349 | NF / EP | Liquid Vehicle | to 1 mL | 94.58 | 358.87 |
| Total | | | 1 mL | 100.0 | 380.6 |

**Example 13: Liquid Formulation 2 of the Compound of Formula (I)**

Table 39 shows liquid formulation 2 of the Compound of Formula (I) free base. An example manufacturing process is shown in FIG. 24.

**Table 39:**

| Component | Quality Standard | Function | 50 mg/mL Oral Solution | | Batch Weight (g) |
|---|---|---|---|---|---|
| | | | Weight (mg/mL) | % (w/v) | |
| Compound of Formula (I) FB | In-house | Drug Substance | 50.0 | 5 | 20.17 |
| Saccharin | NF / EP | Sweetener | 1.5 | 0.15 | 0.61 |
| Butylated hydroxytoluene | NF / EP | Anti-oxidant | 1.7 | 0.17 | 0.68 |
| LABRAFIL M 1944 CS | NF / EP | Surfactant | 200.0 | 20 | 80.16 |
| FONA orange flavor | NF | Flavor | 1.0 | 0.1 | 0.40 |
| Labrafac Lipophile WL1349 | NF / EP | Liquid Vehicle | to 1 mL | 74.58 | 278.68 |
| Total | | | 1 mL | 100.0 | 380.7 |

**Example 14: A Phase I, Open-Label Study to Evaluate the Pharmacokinetics, Relative Bioavailability, Effect of Food, Safety and Tolerability of Different Compound of Formula (I) Prototype Formulations in Healthy Adult Subjects.**

**Methodology**

This is a single center, open-label, randomized, single dose 4-period crossover study in healthy adult subjects designed to investigate the pharmacokinetic (PK) and safety of up to 4 compound of Formula (I) liquid lipidic prototype formulations (compound of Formula (I) Oral Solution, Prototype Formulations, 50 mg/mL), a compound of Formula (I)

spray dried dispersion formulation (compound of Formula (I) Granule for Sprinkle, 25 - 50 mg) and compound of Formula (I), 50 mg Capsules (Reference). It is planned to enroll 36 subjects to be allocated as 3 cohorts of 12 subjects per cohort, with 6 sub-cohorts of 6 subjects per sub-cohort. In each of these 6 sub- cohorts, 6 subjects will be assigned to one of 3 sub-cohorts where a single oral dose of Investigational Medicinal Product (IMP) is administered in 4 dosing periods (Periods 1 to 4) in multiple fed or fasted states or to one of 3 sub-cohorts where a single oral dose of IMP is administered in 2 dosing periods (Periods 1 and 2) only in the fed state. Within each sub-cohort, subjects will also be randomized before administration of the first dose of IMP in Period 1 to one of the following treatment sequences (Table 40):

**Table 40:**

| Sub-Cohort | Sequence | Number of Subjects | Total Number of Periods Dosed | Regimen | | | |
|---|---|---|---|---|---|---|---|
| | | | | Period 1 | Period 2 | Period 3 | Period 4 |
| 1A | ABEF | 3 | 4 | Regimen A | Regimen B | Regimen E | Regimen F |
| | BAEF | 3 | 4 | Regimen B | Regimen A | Regimen E | Regimen F |
| 1B | AB | 3 | 2 | Regimen A | Regimen B | N/A | N/A |
| | BA | 3 | 2 | Regimen B | Regimen A | N/A | N/A |
| 2A | ADEF | 3 | 4 | Regimen A | Regimen D | Regimen E | Regimen F |
| | DAEF | 3 | 4 | Regimen D | Regimen A | Regimen E | Regimen F |
| 2B | AD | 3 | 2 | Regimen A | Regimen D | N/A | N/A |
| | DA | 3 | 2 | Regimen D | Regimen A | N/A | N/A |
| 3A | ACEF | 3 | 4 | Regimen A | Regimen C | Regimen E | Regimen F |
| | CAEF | 3 | 4 | Regimen C | Regimen A | Regimen E | Regimen F |
| 3B | AC | 3 | 2 | Regimen A | Regimen C | N/A | N/A |
| | CA | 3 | 2 | Regimen C | Regimen A | N/A | N/A |

At informed consent, subjects will agree either to participate in 2 study periods or 4 study periods. Once placed into a sub-cohort, the order in which subjects receive the study treatments will be randomized based on the schedule above.

Subjects will receive up to 4 regimens in up to 4 periods in an order according to the randomization schedule within each sub-cohort.

The effect of different prandial states on the PK of the compound of Formula (I) may be explored in Periods 3 and 4 by administering in the fasted state or after an alternative meal (e.g. high fat, standard or light breakfast, etc.).

The proposed regimens are presented in Table 41 below:

**Table 41:**

| Regimen | Investigational Medicinal Product | Dose | Route of Administration | Prandial State |
|---|---|---|---|---|
| A | Compound of Formula (I), 50 mg Capsules (Reference) | 50 mg | Oral | Fed (Ensure Plus) |
| B | Compound of Formula (I) Oral Solution, Prototype Formulation 1, 50 mg/mL | 50 mg | Oral | Fed (Ensure Plus) |
| C | Compound of Formula (I) Oral Solution, Prototype Formulation 2, 50 mg/mL | 50 mg | Oral | Fed (Ensure Plus) |
| D | Compound of Formula (I) Granule for Sprinkle, 25 - 50 mg | 50 mg | Oral | Fed (Ensure Plus) |
| E | Compound of Formula (I) Oral Solution, Prototype Formulation 1 | 100 mg | Oral | Fed (Ensure Plus) |

(continued)

| Regimen | Investigational Medicinal Product | Dose | Route of Administration | Prandial State |
|---|---|---|---|---|
| F | Compound of Formula (I) Oral Solution, Granule for Sprinkle | 50 mg | Oral | Fed (alternative meal) |

**Study Design:**

Subjects will be screened for eligibility to participate in the study up to 28 days before the first dose of IMP in Period 1. Each study period will follow the same study design. Subjects will be admitted to the clinical unit on the evening prior to IMP administration (Day -1). For Periods 1 and 2 (Regimen A and one of either Regimens B, C or D), all subjects will receive the compound of Formula (I) formulations in the morning according to the randomization schedule in the fed state with a liquid dietary supplement (Ensure Plus). For Periods 3 and 4 (Regimens E and F), subjects will receive the compound of Formula (I) formulations in the morning according to the randomization schedule in the fed state with a liquid dietary supplement or an alternate prandial state (fasted or alternative meal). IMP administration will be performed on Day 1 with an appropriate interval between subjects based on logistical requirements (approximately 10 min). Meals will be standardized for each treatment regimen across periods.

Subjects will remain in the clinical unit until 36 h post-dose when they will be discharged. Subjects will return to the clinical unit at 168 h (7 days) and 336 h (14 days) post-dose for a PK blood sample and safety assessments. The minimum washout between IMP dosing occasions will be 14 days between Periods 1 and 2 and 21 days or more to accommodate interim data reviews between Periods 2 and 3 and between Periods 3 and 4.

There will be a follow-up phone call 18 to 24 days post-final visit to ensure the ongoing wellbeing of subjects.

Following the completion of Period 2 for all cohorts, there will be an interim data review during which the PK and safety data will be reviewed, plus any relevant emerging Chemistry, Manufacturing and Control (CMC) stability study information, to determine the formulation, dose level and prandial state in which to administer the IMP in Period 3 (Regimen E). There will be a similar interim review following completion of Period 3 (administration of Regimen E) to determine the formulation, dose level and prandial state in which to administer the IMP in Period 4 (Regimen F). The criteria for the interim decisions will be based on available compound of Formula (I) PK data: e.g., $C_{max}$, $T_{max}$, AUC(0-36), $F_{rel}$ and safety data.

**Number of Subjects Planned:**

It is planned to enroll 36 healthy male and female (non-pregnant, non-lactating) subjects in 6 sub-cohorts of 6 subjects per sub-cohort. These sub-cohorts will be combined in sets of 2 to create 3 cohorts of n = 12 for Periods 1 and 2 to target data in 10 evaluable subjects in each cohort for the primary objectives per formulation variant. A total of 18 subjects, 6 from each of Sub-Cohorts 1A, 2A and 3A participating in Periods 1 and 2, will additionally participate in Periods 3 and 4 with a target of a minimum of 6 evaluable subjects. A subject will be considered evaluable for a particular regimen if they have received an IMP and has completed sufficient planned PK assessments up to 336 h (14 days) after dosing for that regimen to allow for the assessment of study endpoints. A subject will be considered evaluable for a particular comparison (e.g., food effect, relative bioavailability) if they have received both IMPs under comparison and have sufficient PK data up to 14 days after each regimen to allow for assessment of study endpoints.

Subjects withdrawn due to an IMP-related adverse event (AE) will not be replaced. Subjects who are withdrawn for other reasons may be replaced as required by agreement between the principal investigator (PI) and sponsor to ensure sufficient numbers of evaluable subjects at the end of each study period. Replacement subjects may be required to be dosed with specific formulations from the previous regimens in order to obtain the minimum number of evaluable subjects required for interim decisions and to obtain data in any other regimen that is required to fulfil the study objective comparisons, with the exception that any previously dosed IMP that has been considered sub-optimal will not be dosed. Up to 8 replacement subjects in total may be enrolled into the study. The maximum number of subjects that may be dosed is 44 in total.

If a subject withdraws from Sub-Cohort 1A, 2A or 3A after Period 2, it is acceptable to replace them with a subject from Sub-Cohort 1B, 2B or 3B provided the subject signs an updated consent form agreeing to participate in four treatment periods. At the discretion of the investigator, such a subject may not be required to undergo repeat screening procedures.

**Duration of Study:**

For subjects enrolled to receive single dose administration on 4 separate occasions in Periods 1 to 4 (Sub-Cohorts 1A, 2A and 3A), the estimated time from screening until the follow-up phone call is approximately 15 to 16 weeks.

For subjects enrolled to receive single dose administration on 2 separate occasions in Periods 1 to 2 only (Sub-Cohorts 1B, 2B and 3B), the estimated time from screening until the follow-up phone call is approximately 8 to 9 weeks.

**Pharmacokinetic Assessments:**

The plasma concentration data for a compound of Formula (I) will be analyzed for final reporting by Quotient Sciences and for interim reviews by Neurocrine Biosciences, Inc. (NBI), using Phoenix WinNonlin v8.0 or a more recent version (Certara USA, Inc., USA). NBI will be responsible for PK analysis for interim review.

PK analysis of the concentration time data obtained will be performed using appropriate non-compartmental techniques to obtain estimates of the following PK parameters (Table 42) where possible and appropriate:

**Table 42:**

| | |
|---|---|
| $T_{lag}$ | Time prior to the first measurable (non-zero) concentration |
| $T_{max}$ | Time to maximum plasma concentration |
| $C_{max}$ | Maximum plasma concentration |
| $AUC_{0\text{-tlast}}$ | Area under the plasma concentration versus time curve (AUC) from 0 h to last measurable concentration |
| $AUC_{0\text{-inf}}$ | AUC from 0 h extrapolated to infinity |
| $AUC_{extrap}$ | Percentage of $AUC_{0\text{-inf}}$ extrapolated beyond the last measurable concentration |
| Lambda-z | Slope of the apparent terminal phase |
| $T_{½}$ | Apparent terminal half-life |
| CL/F[a] | Apparent systemic clearance after oral administration |
| Vd/F[a] | Apparent volume of distribution based on the area after a single oral administration |
| MRT | Mean residence time |
| MPR $AUC_{0\text{-tlast}}$ | Metabolite to parent ratio based on $AUC_{0\text{-tlast}}$ |
| MPR $AUC_{0\text{-inf}}$ | Metabolite to parent ratio based on $AUC_{0\text{-inf}}$ |

**Taste Assessments:**

Taste will be assessed for each IMP formulation and vehicle (e.g., Ensure Plus, soft food) using a questionnaire designed for this purpose and adapted for this specific study as required.

The questionnaire will ask subjects to rate the acceptability of smell, sweetness, bitterness, flavor, mouth feel, and aftertaste on a 6-point scale, and overall experience on a 5-point scale for each IMP formulation independently of any previous formulations.

**Statistical Methodology:**

Descriptive summaries for all safety data, PK assessments and taste questionnaire data will be provided. No hypothesis testing will be performed for the safety or taste questionnaire data.

**Periods 1 and 2 - for Each Cohort Separately**

***Relative Bioavailability***

Statistical modelling will be performed on the natural log-transformed compound of Formula (I) PK parameters (AUC(0-tlast), AUC(0-inf) and Cmax) to assess relative bioavailability (Frel) using a mixed effects model with terms for regimen, period and sequence as fixed effects and subject within sequence as a random effect. Ratios of geometric means (GMRs) and 90% confidence interval (CI) for the relevant comparison of interest, i.e., between each of the prototype formulations (compound of Formula (I) Oral Solution, Prototype Formulation 1, 50 mg/mL, e.g., Example 12; compound of Formula (I) Oral Solution, Prototype Formulation 2, 50 mg/mL, e.g., Example 13; and compound of Formula (I) Granule for Sprinkle, 25 - 50 mg, e.g. Example 11 [Regimens B, C and D, respectively]) and compound of Formula (I), 50 mg Capsules, e.g., Reference Example 9 (Reference; Regimen A) will be presented.

**All Periods (Periods 1 to 4)**

***Food Effect***

Statistical modelling will be performed on the compound of Formula (I) PK parameters AUC(0-tlast), AUC(0-inf) and Cmax to assess for the effects of food, if relevant. The natural log-transformed PK parameters will be analyzed for bioavailability using a mixed effects model with terms for prandial state (and meal type if applicable) as a fixed effect and subject as a random effect. Ratios of geometric means and 90% CI for the relevant comparisons of interest will be presented where the ratio is defined as fasted/fed or test meal/reference meal (if applicable).

***Relative Bioavailability***

Statistical modelling will be performed on the natural log-transformed compound of Formula (I) PK parameters (AUC(0-tlast), AUC(0-inf) and Cmax) to assess relative bioavailability using a mixed effects model with terms for regimen as a fixed effect and subject as a random effect. Ratios of geometric means and 90% CI for the relevant comparison of interest i.e., between each of the prototype formulations (Regimens E and F, IMPs to be determined by interim reviews following completion of Periods 2 and 3) and compound of Formula (I), 50 mg Capsules (Reference; Regimen A) will be presented.

**Results**

Preliminary PK data is summarized in Table 43 below:

**Table 43: Preliminary Data from Periods 1 and 2**

| | A- Reference | B-Oral Solution 1 | C-Oral Solution 2 | D- SDD Granule |
|---|---|---|---|---|
| N | 35 | 12 | 12 | 12 |
| Tmax * (h) | 5 (2,6) | 6 (5,7) | 5 (5,7) | 5 (5,7) |
| Cmax (ng/ml) | 1361 (33) | 790 (31) | 1082 (46) | 1075 (35) |
| AUC36 (h*ng/ml) | 9452 (30) | 5556 (34) | 7582 (37) | 6691 (41) |
| *Geometric Mean /CV% for AUC and Cmax; Median for Tmax | | | | |

**Example 15. Compound of Formula (I) Crystalline Free Base Form I**

**Example 15A**

**Scheme 1: Preparation of 4-(2-chloro-4-methoxy-5-methylphenyl)-N-[(1S)-2-cyclopropyl-1-(3-fluoro-4-methylphenyl)ethyl]-5-methyl-N-(2-propyn-1-yl)-2-thiazolamine (Compound of Formula (I), Form I)**

**Scheme 1**

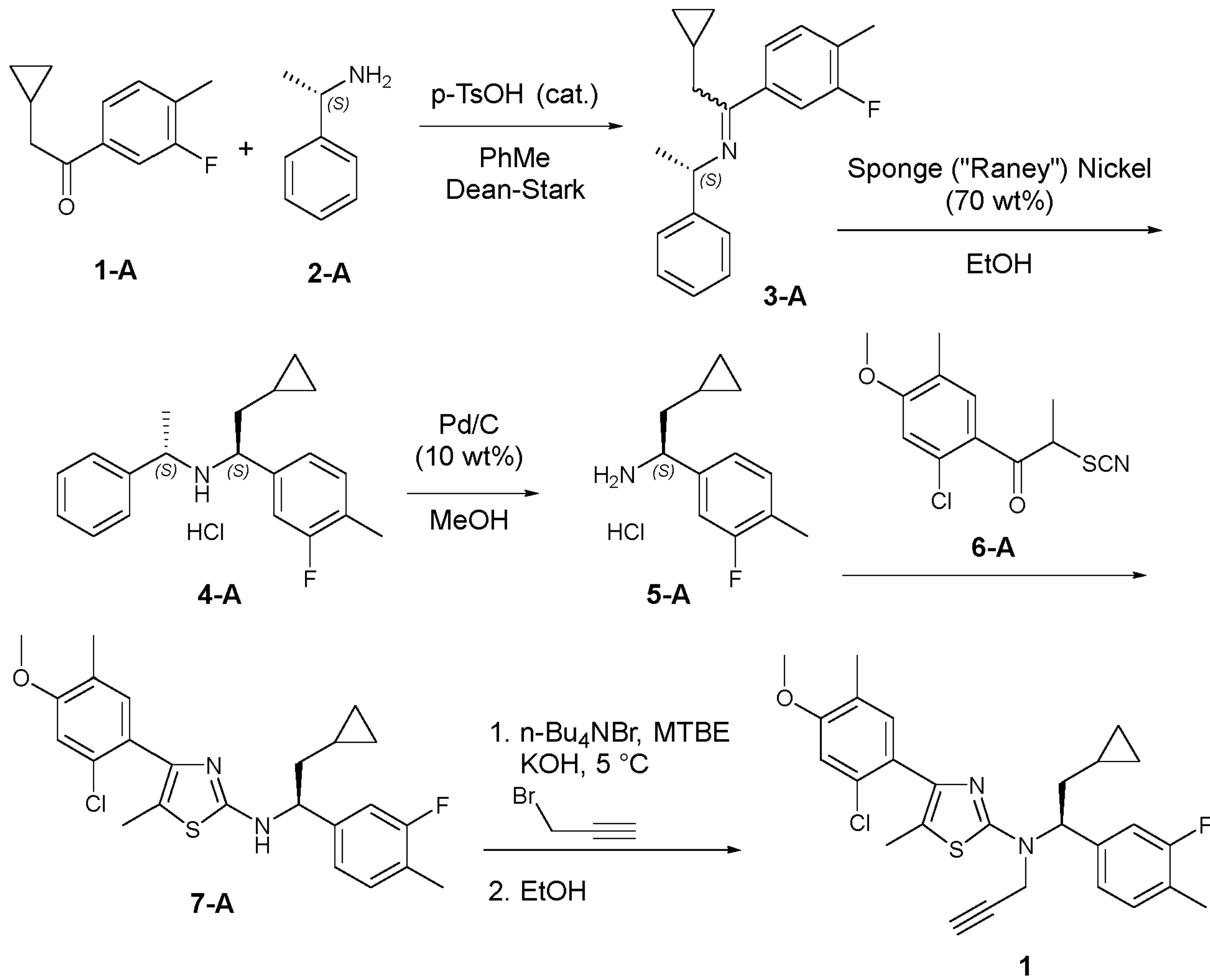


1-A 2-A 3-A 4-A 5-A 6-A 7-A 1

Step 1: Preparation of (5)-2-cyclopropyl-1-(3-fluoro-4-methylphenyl)-N-(1-phenylethyl)ethan-1-imine (Compound 3-A)

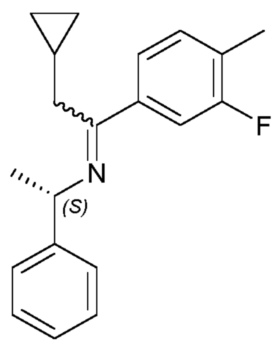

Compound 3-A

A mixture of 2-cyclopropyl-1-(3-fluoro-4-methylphenyl)ethan-1-one (1-A, 150.7 kg, 1 eq., as a 27.6% w/w solution in toluene, Example 15C), (S)-(-)-1-Phenylethylamine (2-A, 112.9 kg, 1.19 eq.), and p-toluenesulfonic acid (7.4 kg, 0.05 eq.) is refluxed at 110 - 120 °C for 23 - 25 h in a reactor set up in a Dean-Stark configuration. The solvent is then removed at 125 - 135 °C under atmospheric pressure until distillation halts and a portion of toluene (275 kg, 2.24 w/w) is added to afford a suspension. The suspension is refluxed at 110 - 120 °C for 23 - 25 h. The mixture is cooled to 22 °C and

washed twice with aqueous $NH_4Cl$ (10%, 301.2 kg, 0.72 eq.) and once with aqueous $NaHCO_3$ (5%, 301.2 kg, 0.23 eq., check pH 8 - 9). The solvent is removed at 125 - 135 °C and atmospheric pressure to a target volume of 256 L, the mixture is filtered over celite, the cake is washed with toluene (25 kg). The resulting mixture containing compound 3-A is used directly in the next step without isolation. The yield is determined by correcting for the LOD and GC-FID purity of the sample (208.4 kg, 90.0% corrected, 0.89% Compound 2-A). EI-MS: 294.1 $[M-H]^+$, 190.1 $[M-C_6H_5CH(CH_3)]^+$, 105.1 $[C_6H_5CH(CH_3)]^+$.

Step 2: Preparation of (*S*)-2-cyclopropyl-1-(3-fluoro-4-methylphenyl)-N-((*S*)-1-phenylethyl)ethan-1-amine hydrochloride (Compound 4-A)

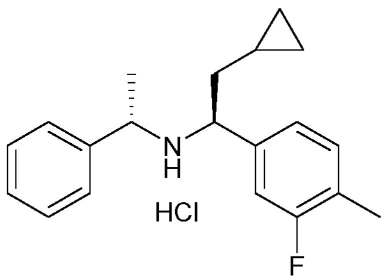

Compound 4-A

Sponge nickel catalyst (144 kg, 0.70 w/w, shipped as a 50% w/w suspension in water) is added to a hydrogenation reactor, equipped with a dip tube capable of removing material from the top of the mass inside, minimizing the amount of water introduced. The supernatant is discarded, ethanol (329.3 kg, 1.58 w/w, anhydrous) is added, the suspension is stirred and then allowed to settle. This process is repeated four more times and the Karl Fisher (KF) of the supernatant is checked (≤ 1% $H_2O$ w/w). Compound 3-A (208.4 kg, 1 eq., as a 62.6% solution in toluene) is added to the mixture in the hydrogenation reactor and ethanol (387.6 kg, 1.86 w/w) is used to rinse the addition flask into the hydrogenation reactor. The hydrogenation reactor is pressurized/depressurized twice with nitrogen (2 bar) and twice with hydrogen (5 bar) then pressurized with hydrogen (9.8 - 10.2 bar) and heated to 33 - 37 °C and stirred for 17 - 19 h. The system is depressurized/pressurized three times with nitrogen (1 bar) and the suspension is filtered and washed with three times with ethanol (493.8 kg, 2.37 w/w). HCl (concentrated, 83.4 kg, 1.07 eq.) is added and the mixture stirred 25 - 35 min at 20 - 24 °C. The mixture is concentrated by distillation at 78 - 80 °C and atmospheric pressure to remove water with a distillate target volume of 1167 L (5.6 L/kg, Compound 3-A) and the KF of the solution is checked (≤ 1.5% $H_2O$ w/w). The mixture is stirred at 48 - 52 °C for 55 - 65 min, then 68 - 72 °C for 55 - 65 min, then cooled to 20 - 24 °C at a rate of 12 °C/h and stirred for 25 - 35 min, then cooled to 0 - 4 °C at a rate of 10 °C/h and stirred for 55 - 65 min. The suspension is filtered, the cake is washed twice with precooled ethanol (329.2 kg, 1.58 w/w, 0 °C), and the collected solid is dried at 40 °C to afford compound 4-A (156.5 kg, 66.4% uncorrected). $^1H$ NMR (400 MHz, DMSO-d6) δ ppm -0.33 - -0.06 (m, 2 H) 0.11 - 0.31 (m, 3 H) 1.57 (d, *J*=6.57 Hz, 3 H) 1.95 (br t, *J*=7.07 Hz, 2 H) 2.26 (d, *J*=1.26 Hz, 3 H) 3.68 (br d, *J*=7.83 Hz, 1 H) 3.92 (br t, *J*=6.44 Hz, 1 H) 6.98 (dd, *J*=7.71, 1.14 Hz, 1 H) 7.28 - 7.36 (m, 2 H) 7.37 - 7.50 (m, 5 H). ESI-MS: 298.2 m/z $[M+H]^+$.

Step 3: Preparation of (*S*)-2-cyclopropyl-1-(3-fluoro-4-methylphenyl)ethan-1-amine hydrochloride (Compound 5-A)

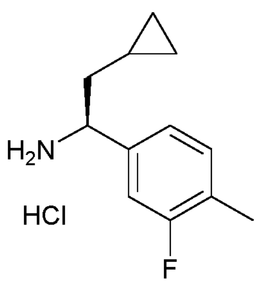

Compound 5-A

Compound 4-A (156.5 kg, 1.00 eq.) and Pd/C (7.8 kg, 10% Pd basis) are added to an inerted hydrogenation reactor. The reactor is then pressurized/depressurized twice with nitrogen (2 bar) and then methanol (494.5 kg, 3.16 w/w) is added. The reactor is depressurized/pressurized three times with nitrogen (2 bar) then three times with hydrogen (5 bar), pressurized with hydrogen (9.8 - 10.2 bar), heated to 58 - 62 °C and stirred for 7 - 9 h. The reaction mixture is cooled to 20 - 24 °C. The reactor is depressurized/pressurized three times with nitrogen (1 bar) and the suspension is filtered and washed three times with methanol (492.9 kg, 3.15 w/w). The solution is concentrated at 63 - 67 °C and atmospheric pressure to a distillate target volume of 1408 L (9.0 L/kg Compound 4-A). n-Heptane (1173.8 kg, 7.5 w/w) is added and the mixture is refluxed at 65 - 80 °C and atmospheric pressure in Dean-Stark configuration to remove methanol. The suspension is cooled to 31 - 35 °C and filtered, the cake washed with n-heptane (147.1 kg, 0.94 w/w), and the solid dried at 40 °C (101.0 kg, 93.8% uncorrected, 99.2% *ee*). $^1$H NMR (400 MHz, DMSO-d6) δ ppm -0.12 - 0.14 (m, 2 H) 0.26 - 0.42 (m, 2 H) 0.44 - 0.55 (m, 1 H) 1.70 - 1.83 (m, 2 H) 2.23 (d, *J*=1.52 Hz, 3 H) 4.24 (t, *J*=7.33 Hz, 1 H) 7.22 - 7.29 (m, 1 H) 7.29 - 7.36 (m, 1 H) 7.40 (dd, *J*=10.99, 1.39 Hz, 1 H). ESI-MS: 194.2 $[M+H]^+$, 177.0 $[M-NH_2]^+$.

Step 4: Preparation of (*S*)-4-(2-chloro-4-methoxy-5-methylphenyl)-N-(2-cyclopropyl-1-(3-fluoro-4-methylphenyl) ethyl)-5-methylthiazol-2-amine (Compound 7-A)

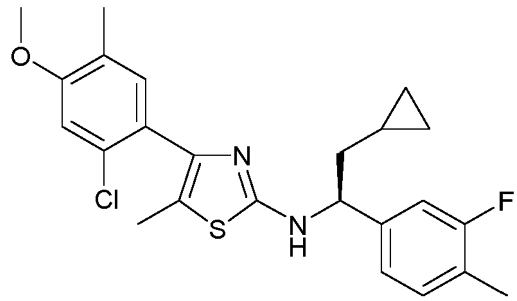

Compound 7-A

A mixture of n-heptane (146 kg), water (142 kg), Compound 5-A (57.4 kg), and aqueous sodium hydroxide (30% w/w, 41.0 kg) was stirred together. The layers were partitioned, and the aqueous layer removed. The organic layer was washed with water (170 kg) and the layers partitioned. The organic layer was set aside using n-heptane (40 kg) to rinse and n-heptane (145 kg) and 1-(2-chloro-4-methoxy-5-methylphenyl)-2-thiocyanatopropan-1-one (6-A, 66.1 kg) were added to the reactor and heated to 85 °C. The previously set aside organic layer containing the free base of Compound 5-A was added at 84 - 85 °C to the reactor and rinsed with n-heptane (20 kg). The resulting mixture was stirred for 2 h at 83 °C. Subsequently, the solvent was switched to methanol by four put-and-take additions/vacuum distillations of methanol (180 kg) at 55 °C with the target volume being 287 L remaining in the reactor. The suspension was cooled to 5 °C and water (570 kg) was added over 4 h at 5 - 10 °C, with the first 60 kg added very slowly. The suspension was aged 2 h at °C and then isolated by filtration and washed with a mixture of methanol/water (91/115 kg) and then a mixture of methanol/water (134/57 kg). The yellow solid was dried at 25 °C and 1 mbar for 17 h then 40 °C and 1 mbar for 22 h to afford Compound 7-A (97.4 kg, 87.5% yield). $^1$H NMR (400 MHz, DMSO-d6) δ ppm -0.01 - 0.14 (m, 2 H) 0.29 - 0.42 (m, 2 H) 0.61 - 0.73 (m, 1 H) 1.47 (dt, *J*=13.83, 6.85 Hz, 1 H) 1.76 (dt, *J*=13.89, 7.20 Hz, 1 H) 2.00 (s, 3 H) 2.11 (s, 3 H) 2.19 (d, *J*=1.01 Hz, 3 H) 3.82 (s, 3 H) 4.54 (q, *J*=7.58 Hz, 1 H) 7.00 (s, 1 H) 7.06 (d, *J*=0.76 Hz, 1H) 7.08 - 7.14 (m, 2 H) 7.18 - 7.23 (m, 1 H) 7.89 (d, *J*=8.08 Hz, 1 H). ESI-MS: 445.3 m/z $[M+H]^+$.

Step 5: Preparation of 4-(2-chloro-4-methoxy-5-methylphenyl)-N-[(1*S*)-2-cyclopropyl-1-(3-fluoro-4-methylphenyl) ethyl]-5-methyl-N-(2-propyn-1-yl)-2-thiazolamine (Compound of Formula (I))

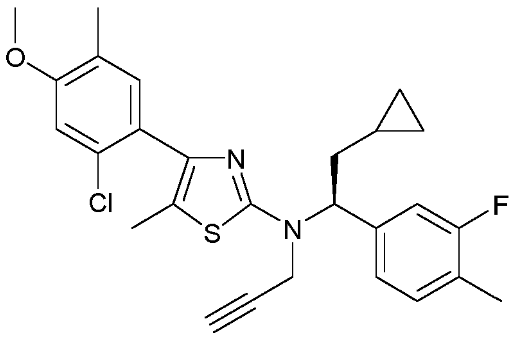

Compound of Formula (I)

A mixture of MTBE (279 kg), tetra-n-butylammonium bromide (10.5 kg), and Compound 7-A (95.4 kg) were heated at 60 °C external temperature for 30 min and then cooled to 0 °C. Aqueous potassium hydroxide (52.4% w/w, 364 kg) and propargyl bromide (39.4 kg as an 80% w/w solution in toluene, 1.19 eq.) were added at 0 - 5 °C and the biphasic mixture aged 14.5 h at 4 - 6 °C with vigorous stirring. Subsequently, water (191 kg) was added and the aqueous layer was discharged. The organic layer was washed twice with water (382 kg) and once with aqueous acetic acid (5.26% w/w, 190 kg) at 20 °C. The mixture is polish filtered, rinsed with ethanol (11 kg) and then the solvent switched to ethanol by 3 put-and-take additions/vacuum distillations of ethanol (300 kg) at 25 - 30 °C for the first cycle and then 35 - 40 °C with the target volume of each cycle being 250 L remaining in the reactor. Ethanol (164 kg) was added and the mixture heated at 60 °C external for 0.5 h before it was cooled to 25 °C in 1 h and seeded with authentic compound of Formula (I) (0.340 kg) which may be prepared as described below in Example 15B. The suspension was aged for 5 h, cooled to 0 °C in 2 h, aged 12 h, filtered, and washed twice with ethanol (24 kg each) pre-cooled to 0 °C. The white solid was dried at 40 °C and 1 mbar for 19 h to yield 80.15 kg of the compound of Formula (I), Form I (77.2% yield). $^{1}$H NMR (400 MHz, DMSO-d6) δ ppm 0.14 (qt, *J*=8.59, 4.42 Hz, 2 H) 0.29 - 0.48 (m, 2 H) 0.61 - 0.82 (m, 1 H) 1.89 (dt, *J*=14.08, 6.98 Hz, 1 H), 2.07 (br d, *J*=7.83 Hz, 1 H) 2.10 (s, 3 H) 2.14 (s, 3 H) 2.20 (d, *J*=1.01 Hz, 3 H) 3.11 (t, *J*=2.27 Hz, 1 H) 3.83 (s, 3 H) 3.94 - 4.22 (m, 2 H) 5.26 (t, *J*=7.58 Hz, 1 H) 7.05 (s, 1 H) 7.10 - 7.36 (m, 4 H). ESI-MS: 483.2 m/z $[M+H]^+$.

The crystallinity of the crystalline free base Compound of Formula (I), Form I was confirmed by XRPD (Figure 25, Table 44) and further supported by DSC (Figure 26), indicating the crystalline compound having an onset of melt at about 84.4 °C (71.9 J/g). TGA of the crystalline free base exhibited about 0.6% of weight loss due to solvent/$H_2O$.

**Table 44. XRPD Peak Data for the Compound of Formula (I) Crystalline Free Base Form I**

| 2-Theta (°) | Height (cps) |
|---|---|
| 5.901(15) | 1221(101) |
| 10.367(11) | 1280(103) |
| 11.762(13) | 1377(107) |
| 12.582(11) | 1591(115) |
| 13.802(2) | 7326(247) |
| 14.1541(16) | 20179(410) |
| 15.173(14) | 449(61) |
| 15.854(7) | 2906(156) |
| 16.746(5) | 5113(206) |
| 18.366(7) | 1171(99) |
| 19.586(2) | 27789(481) |
| 20.100(4) | 10759(299) |
| 20.794(4) | 5441(213) |
| 21.730(5) | 7125(244) |
| 22.239(7) | 10370(294) |
| 23.056(11) | 3482(170) |

(continued)

| 2-Theta (°) | Height (cps) |
|---|---|
| 23.714(7) | 2300(138) |
| 24.115(7) | 8402(265) |
| 25.666(4) | 26173(467) |
| 26.296(6) | 1505(112) |
| 26.752(4) | 9919(288) |
| 27.264(7) | 1016(92) |
| 27.874(7) | 2092(132) |
| 28.623(3) | 10560(297) |
| 29.546(6) | 5811(220) |
| 30.025(3) | 2248(137) |
| 30.737(10) | 1333(105) |
| 31.017(19) | 1406(108) |
| 31.588(10) | 2292(138) |
| 31.809(8) | 2212(136) |
| 32.126(13) | 593(70) |
| 33.200(16) | 839(84) |
| 33.613(13) | 2996(158) |
| 33.914(13) | 1156(98) |
| 34.276(16) | 1008(92) |
| 34.564(12) | 1056(94) |
| 35.397(18) | 816(82) |
| 36.073(10) | 1928(127) |
| 36.67(3) | 562(68) |
| 37.347(9) | 1553(114) |
| 37.776(12) | 1573(114) |
| 39.070(7) | 1890(125) |
| 39.743(15) | 1042(93) |
| 40.643(9) | 2808(153) |
| 41.106(8) | 1107(96) |
| 41.984(11) | 2686(150) |
| 42.376(16) | 986(91) |
| 42.901(16) | 492(64) |
| 43.543(10) | 4744(199) |
| 44.419(16) | 2810(153) |

**Example 15B**

**Preparation of 4-(2-chloro-4-methoxy-5-methylphenyl)-N-[(1S)-2-cyclopropyl-1-(3-fluoro-4-methylphenyl) ethyl]-5-methyl-N-(2-propyn-1-yl)-2-thiazolamine (Compound of Formula (I), seed batch)**

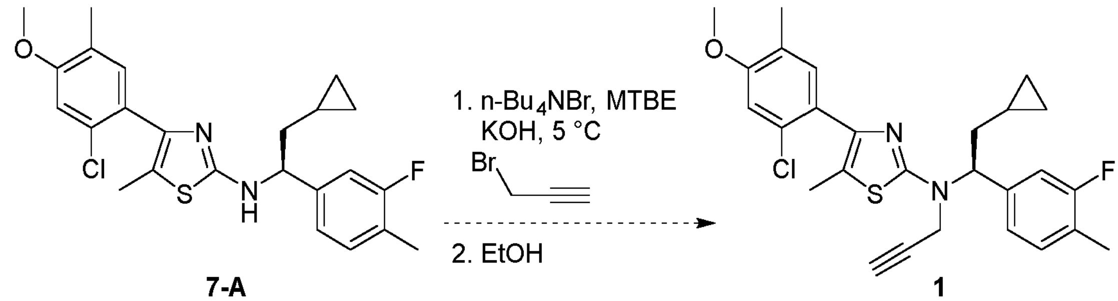


7-A 1

A mixture of MTBE, *tetra*-n-butylammonium bromide, and Compound 7-A cooled to 0 °C is treated with aqueous potassium hydroxide and propargyl bromide maintaining the temperature at 0 - 5 °C. The resulting biphasic mixture is aged 23 h at 4 - 6 °C. Subsequently, water and MTBE are added and the aqueous layer is discharged. The organic layer is washed twice with water and once with aqueous acetic acid at 20 °C. Ethanol is added and then the solvent switched to ethanol by 3 put-and-take additions/vacuum distillations of ethanol at 35 - 40 °C with a target volume of each cycle remaining in the vessel, except for the third cycle where the mixture is concentrated to dryness. Ethanol is added to the vessel and the mixture heated at 60 °C external for 0.5 h before it is cooled to 20 °C in 1 h and aged 18 h affording a suspension. The suspension is cooled to 0 °C, aged 6 h, filtered, and washed twice with ethanol pre-cooled to 0 °C to afford a solid. The solid is dried at 40 °C under vacuum to afford the compound of Formula (I).

**Example 15C**

**Scheme 2: Preparation of 2-cyclopropyl-1-(3-fluoro-4-methylphenyl)ethan-1-one**

**(Compound 1-A)**

**Scheme 2**

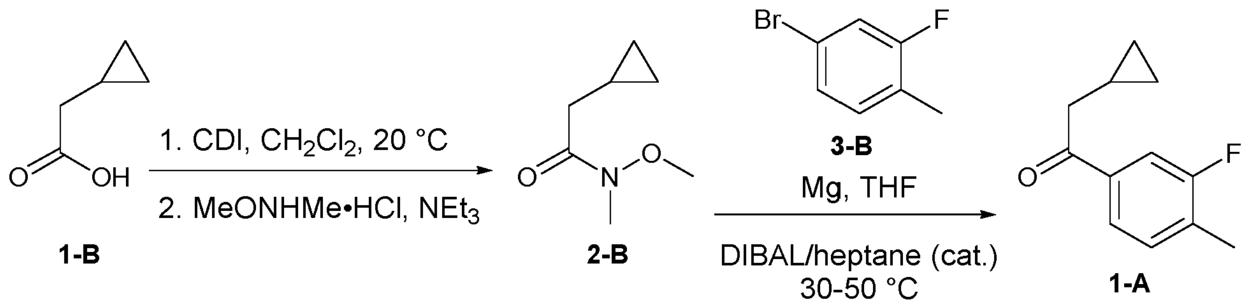


1-B 2-B 3-B 1-A

Step 1: Preparation of 2-cyclopropyl-*N*-methoxy-*N*-methylacetamide (Compound 2-B)

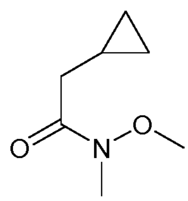

Compound 2-B

A suspension of 1,1'-Carbonyldiimidazole (152.6 kg, 1.01 eq.) in DCM (682 kg, 513 L, 7.3 w/w relative to 2-cyclopropylacetic acid) was treated with a solution of 2-cyclopropylacetic acid (1-B, 93.6 kg, 1 eq.) in DCM (248 kg, 186 L, 2.65 w/w) over at least 1 h, keeping the temperature ≤ 25 °C and compensating for significant effervescence. The resulting mixture is stirred for 15 min at 22 °C and then *N*, *O*-dimethylhydroxylamine•HCl (93.3 kg, 1.03 eq.) is added in portions,

keeping the temperature ≤30 °C. Subsequently, triethylamine (46.4 kg, 0.49 eq.) is added to the stirring mixture at 20 - 25 °C. The resulting mixture is stirred at 22 °C at least 1 h. The mixture is washed once with $KHSO_4$ solution (0.24 M, 357.1 kg, 0.09 eq.), once with $KHSO_4$ solution (0.40 M, 365.4 kg, 0.15 eq.), once with $KHSO_4$ solution (0.80 M, 384.5 kg, 0.30 eq.), and once with $NaHCO_3$ solution (0.60 M, 393.1 kg, 0.24 eq.). Residual DCM is removed by three put-and-takes of THF (166.6 kg, 1.78 w/w) and vacuum distillation (50 - 60 °C, to minimum volume/until distillation stops). THF (333.2 kg. 3.56 w/w) is added and the yield is determined by correcting for the LOD and GC-FID purity of the sample (131.5 kg, 98.2% corrected). $^1$H-NMR (400 MHz, DMSO-d6) δ: - 0.01 - 0.03 (m, 2H) 0.32 - 0.36 (m, 2H) 0.81 - 0.90 (br m, 1H) 2.18 (d, J=6.80 Hz, 2H) 2.97 (s, 3 H) 3.53 (s, 3H). ESI-MS: 144.0 $[M+H]^+$.

Step 2: Preparation of 2-cyclopropyl-1-(3-fluoro-4-methylphenyl)ethan-1-one (Compound 1-A)

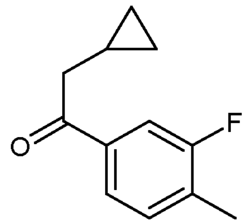

Compound 1-A

Mg (turnings, 28.6 kg, 1.37 eq.) are suspended in THF (244.7 kg, 2.0 w/w) and DIBAL-H (1 M in n-heptane, 18.9 kg, 0.03 eq.) is added dropwise at 30 °C, The resulting mixture is stirred at 30 °C for at least 10 min and then 4-bromo-2-fluoro-1-methylbenzene (3-B, neat, 21.1 kg, 0.13 eq.) is added over at least 30 min at 30 - 50 °C. Subsequently, the mixture is treated with a solution of 4-bromo-2-fluoro-1-methylbenzene (3-B, 191.6 kg, 1.18 eq.) in THF (414.5 kg, 3.37 w/w) at 30 - 50 °C over 3 h or less. The mixture is stirred at 30 °C for at least 1 h. Subsequently, the mixture is treated with 2-cyclopropyl-N-methoxy-N-methylacetamide (2-B, 123.0 kg, 1 eq., 25.9% w/w solution in THF) over at least 1 h at 15 - 25 °C. The resulting mixture is stirred at 20 - 25 °C for at least 1 h. The stirring mixture is then treated with aqueous HCl (3 M, 10.3% w/w, 668.9 kg, 2.24 eq.) at 10 - 25 °C and the resulting mixture is stirred at least 2 h (check pH 3.0 - 3.5). The layers are separated, and the aqueous layer is combined with heptane (290.3 kg, 2.36 w/w). The layers are separated, and the organic layer is washed once with $NaHCO_3$ solution (0.63 M, 211.6 kg, 0.15 eq.) and once with NaCl solution (2.57 M, 213.0 kg, 0.55 eq.). The residual solvents are removed by vacuum distillation at 58 - 62 °C until distillation stops and then one put-and-take of toluene (275.5 kg, 2.24 w/w) at 107 - 117 °C until distillation stops. Toluene (275.5 kg, 2.24 w/w) is added and the yield is determined by correcting for the LOD and GC-FID purity of the sample (150.7 kg, 91.3% corrected). $^1$H NMR (400 MHz, DMSO-d6) δ ppm 0.07 - 0.21 (m, 2 H) 0.40 - 0.54 (m, 2 H) 1.02 (ttt, *J*=8.16, 8.16, 6.68, 6.68, 4.86, 4.86 Hz, 1 H) 2.30 (d, *J*=1.77 Hz, 3 H) 2.91 (d, *J*=6.57 Hz, 2 H) 7.44 (t, *J*=7.83 Hz, 1 H) 7.57 - 7.78 (m, 2 H). ESI-MS: 193.1 $[M+H]^+$.

**Example 16: Compound of Formula (I) Crystalline Tosylate Salt Form 1**

Approximately 20 mg of the Compound of Formula (I) was weighed into a vial. Using a positive displacement pipette, 250 μL of solvent (IPA) was added to the vial along with a stir bar. The vial was placed in an aluminum block on a Reacti-Therm mixer and heated to 60 °C for ~1 hour. A molar equivalent of para-toluenesulfonic acid was added to the vial (20 μL of a 2M solution in water) and allowed to stir. The sample was slow cooled back to room temperature along with mild Nitrogen gas for evaporation. Precipitate was collected, left to dry overnight, and then analyzed by XRPD, DSC, and TGA.

The crystallinity of the crystalline tosylate form 1 was confirmed by XRPD (Figure 27, Table 45) and further supported by DSC (Figure 28), indicating the crystalline compound having an onset of melt at about 156 °C (22.2 J/g). TGA of the crystalline compound is provided in Figure 28, and exhibited about 0.5% of weight loss due to solvent/$H_2O$.

**Table 45. XRPD Peak Data for the Compound of Formula (I) Crystalline Tosylate Form 1**

| 2-Theta (°) | Height (cps) |
|---|---|
| 8.112(9) | 957(89) |
| 9.124(2) | 12296(320) |
| 9.471(2) | 4519(194) |
| 10.525(3) | 8507(266) |

(continued)

| 2-Theta (°) | Height (cps) |
|---|---|
| 11.316(3) | 10211(292) |
| 13.182(5) | 6158(227) |
| 13.494(6) | 1598(115) |
| 14.249(9) | 2197(135) |
| 15.208(9) | 1746(121) |
| 15.711(7) | 1437(109) |
| 16.252(5) | 5723(218) |
| 16.692(3) | 3848(179) |
| 17.540(4) | 1578(115) |
| 19.031(6) | 6774(238) |
| 19.265(4) | 6491(233) |
| 19.499(10) | 3152(162) |
| 20.401(3) | 8581(267) |
| 20.656(4) | 3040(159) |
| 21.142(3) | 11498(310) |
| 21.703(6) | 4979(204) |
| 21.870(11) | 5331(211) |
| 22.277(5) | 3701(176) |
| 22.769(3) | 10159(291) |
| 23.297(3) | 14954(353) |
| 23.532(4) | 3597(173) |
| 23.810(3) | 9590(283) |
| 24.73(2) | 2325(139) |
| 25.47(4) | 1704(119) |
| 26.087(5) | 2413(142) |
| 26.71(3) | 422(59) |
| 27.210(6) | 1648(117) |
| 27.593(5) | 2825(153) |
| 28.472(4) | 6417(231) |
| 29.51(3) | 1423(109) |
| 29.98(3) | 849(84) |
| 30.63(6) | 1262(103) |
| 30.77(3) | 1121(97) |
| 31.577(3) | 5563(215) |
| 32.74(4) | 874(85) |
| 33.73(4) | 1111(96) |
| 34.415(13) | 2054(131) |
| 34.799(11) | 706(77) |
| 35.139(15) | 1320(105) |

(continued)

| 2-Theta (°) | Height (cps) |
|---|---|
| 35.682(16) | 1028(93) |
| 38.39(4) | 751(79) |
| 39.743(17) | 1790(122) |
| 40.219(16) | 762(80) |
| 41.23(3) | 925(88) |
| 43.16(3) | 1540(113) |
| 44.288(12) | 1142(98) |

## Claims

1. A pharmaceutical composition in oral solution dosage form comprising:

    (a) a compound of Formula (I):

    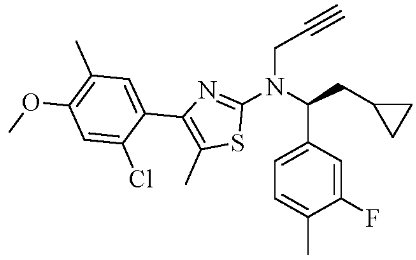

    (I)

    (4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine),
    or a pharmaceutically acceptable salt thereof;
    (b) one or more of a sweetener, an anti-oxidant, and a flavor; and
    (c) a liquid vehicle, wherein the liquid vehicle is selected from medium-chain triglycerides, propylene glycol dicaprylate/dicaprate, glycerin, propylene glycol, polyethylene glycol, olive oil, soybean oil, corn oil, and diethylene glycol monoethyl ether.

2. The pharmaceutical composition of claim 1, comprising about 1 w/v% to about 50 w/v% of the compound of Formula (I), or a pharmaceutically acceptable salt thereof, based on the weight of the free base.

3. The pharmaceutical composition of claim 1, comprising about 1 w/v% to about 10 w/v% of the compound of Formula (I), or a pharmaceutically acceptable salt thereof, based on the weight of the free base.

4. The pharmaceutical composition of claim 1, comprising about 5 w/v% of the compound of Formula (I), or a pharmaceutically acceptable salt thereof, based on the weight of the free base.

5. The pharmaceutical composition of any one of claims 1-4, comprising a sweetener.

6. The pharmaceutical composition of claim 5, comprising about 0.01 w/v% to about 1.5 w/v% of the sweetener.

7. The pharmaceutical composition of claim 5, comprising about 0.1 w/v% to about 0.5 w/v% of the sweetener.

8. The pharmaceutical composition of claim 5, comprising about 0.15 w/v% of the sweetener.

9. The pharmaceutical composition of any one of claims 5-8, wherein the sweetener is selected from saccharin, sucrose,

sucralose, aspartame, dextrose, fructose, maltitol, mannitol, sorbitol, and avantame.

**10.** The pharmaceutical composition of any one of claims 5-8, wherein the sweetener is saccharin.

**11.** The pharmaceutical composition of any one of claims 1-10, comprising an anti-oxidant.

**12.** The pharmaceutical composition of claim 11, comprising about 0.01 w/v% to about 1.5 w/v% of the anti-oxidant.

**13.** The pharmaceutical composition of claim 11, comprising about 0.1 w/v% to about 0.5 w/v% of the anti-oxidant.

**14.** The pharmaceutical composition of claim 11, comprising about 0.17 w/v% of the anti-oxidant.

**15.** The pharmaceutical composition of any one of claims 11-14, wherein the anti-oxidant is selected from butylated hydroxytoluene, vitamin E TPGS, butylated hydroxyanisole, ascorbic acid, lecithin, tert-butylhydroquinone, and citric acid.

**16.** The pharmaceutical composition of any one of claims 11-15, wherein the anti-oxidant is butylated hydroxytoluene.

**17.** The pharmaceutical composition of any one of claims 1-16, comprising a flavor.

**18.** The pharmaceutical composition of claim 17, comprising about 0.01 w/v% to about 0.5 w/v% of the flavor.

**19.** The pharmaceutical composition of claim 17, comprising about 0.05 w/v% to about 0.2 w/v% of the flavor.

**20.** The pharmaceutical composition of claim 17, comprising about 0.10 w/v% of the flavor.

**21.** The pharmaceutical composition of any one of claims 1-20, comprising about 50 w/v% to about 99.9 w/v% of the liquid vehicle.

**22.** The pharmaceutical composition of any one of claims 1-20, comprising about 90 w/v% to about 99 w/v% of the liquid vehicle.

**23.** The pharmaceutical composition of any one of claims 1-20, comprising about 92 w/v% to about 97 w/v% of the liquid vehicle.

**24.** The pharmaceutical composition of any one of claims 1-20, comprising about 94.6 w/v% of the liquid vehicle.

**25.** The pharmaceutical composition of any one of claims 1-20, comprising about 70 w/v% to about 80 w/v% of the liquid vehicle.

**26.** The pharmaceutical composition of any one of claims 1-20, comprising about 75 w/v% of the liquid vehicle.

**27.** The pharmaceutical composition of any one of claims 1-26, wherein the liquid vehicle is medium-chain triglycerides.

**28.** The pharmaceutical composition of claim 27, wherein the medium-chain triglycerides is caprylic/capric triglycerides.

**29.** The pharmaceutical composition of any one of claims 1-28, further comprising a surfactant.

**30.** The pharmaceutical composition of claim 29, comprising about 1 w/v% to about 50 w/v% of the surfactant.

**31.** The pharmaceutical composition of claim 29, comprising about 10 w/v% to about 30 w/v% of the surfactant.

**32.** The pharmaceutical composition of claim 29, comprising about 20 w/v% of the surfactant.

**33.** The pharmaceutical composition of any one of claims 29-32, wherein the surfactant is selected from oleoyl polyoxyl-6 glycerides, linoleoyl polyoxyl-6 glycerides, Polysorbate 80, Polysorbate 20, vitamin E polyethylene glycol succinate, lauroyl polyoxyl-32 glycerides, sodium lauryl sulfate, Poloxamer, corn oil PEG-6 esters, and hydrogenated palm/palm kernel oil PEG-6 esters.

**34.** The pharmaceutical composition of any one of claims 29-33, wherein the surfactant is oleoyl polyoxyl-6 glycerides.

**35.** The pharmaceutical composition of claim 1, comprising:

(a) 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof;
(b) a sweetener;
(c) an anti-oxidant;
(d) a flavor; and
(e) the liquid vehicle.

**36.** The pharmaceutical composition of claim 35, further comprising a surfactant.

**37.** The pharmaceutical composition of claim 1, comprising:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of a sweetener;
(c) about 0.1 w/v% to about 0.2 w/v% of an anti-oxidant;
(d) about 0.05 w/v% to about 0.2 w/v% of a flavor; and
(e) about 92 w/v% to about 97 w/v% of the liquid vehicle.

**38.** The pharmaceutical composition of claim 1, comprising:

(a) about 5 w/v% of 4-(2-chloro-4-methoxy-5-methylphenyl)-N-[(1S)-2-cyclopropyl-1-(3-fluoro-4-methylphenyl) ethyl]-5-methyl-N-prop-2-ynyl-1,3-thiazol-2-amine, or a pharmaceutically acceptable salt thereof, based on the weight of the free base;
(b) about 0.15 w/v% of a sweetener;
(c) about 0.17 w/v% of an anti-oxidant;
(d) about 0.1 w/v% of a flavor; and
(e) about 94.6 w/v% of the liquid vehicle.

**39.** The pharmaceutical composition of claim 1, comprising:

(a) about 4 w/v% to about 6 w/v% of 4-(2-chloro-4-methoxy-5-methylphenyl)-N-[(1S)-2-cyclopropyl-1-(3-fluoro-4-methylphenyl)ethyl]-5-methyl-N-prop-2-ynyl-1,3-thiazol-2-amine, or a pharmaceutically acceptable salt thereof, based on the weight of the free base;
(b) about 0.1 w/v% to about 0.2 w/v% of a sweetener;
(c) about 0.1 w/v% to about 0.2 w/v% of an anti-oxidant;
(d) about 0.05 w/v% to about 0.2 w/v% of a flavor;
(e) about 15 w/v% to about 25 w/v% of a surfactant; and
(f) about 70 w/v% to about 80 w/v% of the liquid vehicle.

**40.** The pharmaceutical composition of claim 1, comprising:

(a) about 5 w/v% of 4-(2-chloro-4-methoxy-5-methylphenyl)-N-[(1S)-2-cyclopropyl-1-(3-fluoro-4-methylphenyl) ethyl]-5-methyl-N-prop-2-ynyl-1,3-thiazol-2-amine, or a pharmaceutically acceptable salt thereof, based on the weight of the free base;
(b) about 0.15 w/v% of a sweetener;
(c) about 0.17 w/v% of an anti-oxidant;
(d) about 0.1 w/v% of a flavor;
(e) about 20 w/v% of a surfactant; and
(f) about 75 w/v% of the liquid vehicle.

**41.** The pharmaceutical composition of claim 40, wherein

(a) the compound of Formula **(I)** is the free base;

(b) the sweetener is saccharin;
(c) the anti-oxidant is butylated hydroxytoluene;
(d) the surfactant is oleoyl polyoxyl-6-glycerides; and
(e) the liquid vehicle is medium-chain triglycerides.

**42.** The pharmaceutical composition of claim 40, wherein the medium-chain triglycerides is caprylic/capric triglycerides

**43.** The pharmaceutical composition of any one of claims 1-40, comprising the compound of Formula **(I)** as a free base.

**44.** The pharmaceutical composition of any one of claims 1-43, formulated in unit dosage form, wherein the compound of Formula (I), or a pharmaceutically acceptable salt thereof, is present in an amount of about 5 mg/mL to about 200 mg/mL, based on the weight of the free base.

**45.** The pharmaceutical composition of claim 44, wherein the compound of Formula (I), or a pharmaceutically acceptable salt thereof, is present in an amount of about 75 mg/mL to about 150 mg/mL, based on the weight of the free base.

**46.** The pharmaceutical composition of claim 44, wherein the compound of Formula (I), or a pharmaceutically acceptable salt thereof, is present in an amount of about 50 mg/mL, based on the weight of the free base.

**47.** The pharmaceutical composition of claim 44, wherein the compound of Formula (I), or a pharmaceutically acceptable salt thereof, is present in an amount of about 100 mg/mL, based on the weight of the free base.

**48.** A method for preparing the pharmaceutical composition of any one of claims 1-47, comprising:

(a) mixing the liquid vehicle with a sweetener;
(b) mixing the mixture of step (a) with an anti-oxidant and a flavor;
(c) mixing the compound of Formula (I), or a pharmaceutically acceptable salt thereof, with the mixture of step (b); and
(d) mixing the mixture of step (c) with an additional portion of the liquid vehicle.

**49.** The method of claim 48, wherein step (a) comprises mixing the liquid vehicle with a sweetener and a surfactant.

**50.** A pharmaceutical composition of any one of claims 1-47 for use in a method of treating congenital adrenal hyperplasia (CAH) in a subject.

**51.** A pharmaceutical composition for use according to claim 50, wherein the congenital adrenal hyperplasia (CAH) is classic CAH.

**52.** The pharmaceutical composition for use according to claim 50 or 51, wherein the subject is in a fed state.

**53.** The pharmaceutical composition for use according to any one of claims 50-52, wherein the subject is administered the pharmaceutical composition with a nutritional composition.

**54.** The pharmaceutical composition for use according to claim 53, wherein the nutritional composition is a liquid dietary supplement comprising about 1000 to about 2000 calories per liter with a fat content greater than about 30%.

**55.** The pharmaceutical composition for use according to claim 53, wherein the nutritional composition is a liquid dietary supplement comprising 1500 calories per liter with a caloric distribution of 14.7% protein, 32% fat and 53.3% carbohydrate.

**56.** The pharmaceutical composition for use according to any one of claims 53-55, wherein the nutritional composition is administered in an amount of about 6 to about 12 fluid ounces.

**57.** The pharmaceutical composition for use according to any one of claims 53-55, wherein the nutritional composition is administered in an amount of about 8 fluid ounces.

**58.** The pharmaceutical composition for use according to any one of claims 53-57, wherein the nutritional composition is administered within 30 minutes of administration of the pharmaceutical composition.

**59.** The pharmaceutical composition for use according to any one of claims 52-58, wherein administering the pharmaceutical composition exhibits a positive food effect.

**60.** The pharmaceutical composition for use according to any one of claims 50-59, wherein the subject is a human subject.

**61.** The pharmaceutical composition for use according to any one of claims 52-58, wherein the pharmaceutical composition exhibits a positive food effect when administered orally.

**62.** The pharmaceutical composition for use according to any one of claims 50-61, wherein the pharmaceutical composition is administered to the subject with a meal.

**63.** The pharmaceutical composition for use according to claim 62, wherein the meal is a high fat meal.

**64.** The pharmaceutical composition for use according to claim 62, wherein the meal is a low fat meal.

**65.** The pharmaceutical composition for use according to any one of claims 62-64, wherein the pharmaceutical composition is administered within about 5 minutes after the start of the meal.

**66.** The pharmaceutical composition for use according to any one of claims 62-64, wherein the meal is an evening meal.

**67.** The pharmaceutical composition for use according to any one of claims 62-64, wherein the meal is a morning meal.

**68.** The pharmaceutical composition for use according to any one of claims 62-67, wherein administering the pharmaceutical composition exhibits a positive food effect.

**69.** The pharmaceutical composition for use according to any one of claims 50-68, wherein the pharmaceutical composition comprises about 25 mg of the compound of Formula (I), or a pharmaceutically acceptable salt thereof, based on the weight of the free base.

**70.** The pharmaceutical composition for use according to any one of claims 50-68, wherein the pharmaceutical composition comprises about 50 mg of the compound of Formula (I), or a pharmaceutically acceptable salt thereof, based on the weight of the free base.

**71.** The pharmaceutical composition for use according to any one of claims 50-68, wherein the pharmaceutical composition comprises about 75 mg of the compound of Formula (I), or a pharmaceutically acceptable salt thereof, based on the weight of the free base.

**72.** The pharmaceutical composition for use according to any one of claims 50-68, wherein the pharmaceutical composition comprises about 100 mg of the compound of Formula (I), or a pharmaceutically acceptable salt thereof, based on the weight of the free base.

**73.** The pharmaceutical composition for use according to any one of claims 50-68, wherein the method comprises administering a daily dose of the pharmaceutical composition comprising about 25 mg of the compound of Formula (I), or a pharmaceutically acceptable salt thereof, based on the weight of the free base.

**74.** The pharmaceutical composition for use according to any one of claims 50-68, wherein the method comprises administering a daily dose of the pharmaceutical composition comprising about 50 mg of the compound of Formula (I), or a pharmaceutically acceptable salt thereof, based on the weight of the free base.

**75.** The pharmaceutical composition for use according to any one of claims 50-68, wherein the method comprises administering a daily dose of the pharmaceutical composition comprising about 75 mg of the compound of Formula (I), or a pharmaceutically acceptable salt thereof, based on the weight of the free base.

**76.** The pharmaceutical composition for use according to any one of claims 50-68, wherein the method comprises administering a daily dose of the pharmaceutical composition comprising about 100 mg of the compound of Formula (I), or a pharmaceutically acceptable salt thereof, based on the weight of the free base.

**77.** The pharmaceutical composition for use according to any one of claims 50-68, wherein the method comprises

administering a daily dose of the pharmaceutical composition comprising about 150 mg of the compound of Formula (I), or a pharmaceutically acceptable salt thereof, based on the weight of the free base.

**78.** The pharmaceutical composition for use according to any one of claims 50-68, wherein the method comprises administering a daily dose of the pharmaceutical composition comprising about 200 mg of the compound of Formula (I), or a pharmaceutically acceptable salt thereof, based on the weight of the free base.

**79.** The pharmaceutical composition for use according to any one of claims 50-68, wherein the method comprises administering the pharmaceutical composition twice daily in a dose of about 25 mg of the compound of Formula (I), or a pharmaceutically acceptable salt thereof, based on the weight of the free base.

**80.** The pharmaceutical composition for use according to any one of claims 50-68, wherein the method comprises administering the pharmaceutical composition twice daily in a dose of about 50 mg of the compound of Formula (I), or a pharmaceutically acceptable salt thereof, based on the weight of the free base.

**81.** The pharmaceutical composition for use according to any one of claims 50-68, wherein the method comprises administering the pharmaceutical composition twice daily in a dose of about 75 mg of the compound of Formula (I), or a pharmaceutically acceptable salt thereof, based on the weight of the free base.

**82.** The pharmaceutical composition for use according to any one of claims 50-68, wherein the method comprises administering the pharmaceutical composition twice daily in a dose of about 100 mg of the compound of Formula (I), or a pharmaceutically acceptable salt thereof, based on the weight of the free base.

**83.** The pharmaceutical composition for use according to any one of claims 50-82, wherein the subject is concurrently receiving a dose of a glucocorticoid.

**84.** The pharmaceutical composition for use according to claim 83, wherein the glucocorticoid is selected from cortisol (hydrocortisone), cortisone, prednisone, prednisolone, methylprednisolone, dexamethasone, betamethasone, triamcinolone, fludrocortisone acetate, and deoxycorticosterone acetate.

**85.** The pharmaceutical composition for use according to claim 83 or 84, wherein the glucocorticoid is cortisol (hydrocortisone).

**86.** The pharmaceutical composition for use according to claim 83 or 84, wherein the glucocorticoid is cortisone.

**87.** The pharmaceutical composition for use according to claim 83 or 84, wherein the glucocorticoid is prednisone.

**88.** The pharmaceutical composition for use according to any one of claims 83-87, wherein the glucocorticoid dose is measured in hydrocortisone equivalents.

**89.** The pharmaceutical composition for use according to any one of claims 83-88, wherein the glucocorticoid dose is measured as a multiple of the upper limit of normal of physiologic dosing in hydrocortisone equivalents.

**90.** The pharmaceutical composition for use according to any one of claims 83-89, wherein the glucocorticoid dose is a physiologic dose as measured after a time period of administration of the pharmaceutical composition.

**91.** The pharmaceutical composition for use according to any one of claims 83-89, wherein the glucocorticoid dose is a physiologic dose of about 4 to about 12 $mg/m^2/day$ as measured after a time period of administration of the pharmaceutical composition.

**92.** The pharmaceutical composition for use according to any one of claims 83-89, wherein the glucocorticoid dose is a physiologic dose of about 4 to about 9 $mg/m^2/day$ as measured after a time period of administration of the pharmaceutical composition.

**93.** The pharmaceutical composition for use according to any one of claims 83-89, wherein the glucocorticoid dose is a physiologic dose that is less than about 8 $mg/m^2/day$ as measured after a time period of administration of the pharmaceutical composition.

**94.** The pharmaceutical composition for use according to any one of claims 83-93, wherein the glucocorticoid dose of the subject is reduced by about 10% after a time period of administration of the pharmaceutical composition, wherein the reduction of the glucocorticoid dose is relative to the glucocorticoid dose prior to administration of the pharmaceutical composition

**95.** The pharmaceutical composition for use according to any one of claims 83-93, wherein the glucocorticoid dose of the subject is reduced by about 20% after a time period of administration of the pharmaceutical composition, wherein the reduction of the glucocorticoid dose is relative to the glucocorticoid dose prior to administration of the pharmaceutical composition.

**96.** The pharmaceutical composition for use according to any one of claims 83-93, wherein the glucocorticoid dose of the subject is reduced by about 30% after a time period of administration of the pharmaceutical composition, wherein the reduction of the glucocorticoid dose is relative to the glucocorticoid dose prior to administration of the pharmaceutical composition.

**97.** The pharmaceutical composition for use according to any one of claims 83-93, wherein the glucocorticoid dose of the subject is reduced by about 40% after a time period of administration of the pharmaceutical composition, wherein the reduction of the glucocorticoid dose is relative to the glucocorticoid dose prior to administration of the pharmaceutical composition.

**98.** The pharmaceutical composition for use according to any one of claims 83-93, wherein the glucocorticoid dose of the subject is reduced by about 50% after a time period of administration of the pharmaceutical composition, wherein the reduction of the glucocorticoid dose is relative to the glucocorticoid dose prior to administration of the pharmaceutical composition.

**99.** The pharmaceutical composition for use according to any one of claims 83-93, wherein the glucocorticoid dose of the subject is reduced by about 60% after a time period of administration of the pharmaceutical composition, wherein the reduction of the glucocorticoid dose is relative to the glucocorticoid dose prior to administration of the pharmaceutical composition.

**100.**

The pharmaceutical composition for use according to any one of claims 83-93, wherein the glucocorticoid dose of the subject is reduced by about 70% after a time period of administration of the pharmaceutical composition, wherein the reduction of the glucocorticoid dose is relative to the glucocorticoid dose prior to administration of the pharmaceutical composition.

**101.**

The pharmaceutical composition for use according to any one of claims 83-93, wherein the glucocorticoid dose of the subject is reduced by less than about 20% after a time period of administration of the pharmaceutical composition, wherein the reduction of the glucocorticoid dose is relative to the glucocorticoid dose prior to administration of the pharmaceutical composition.

**102.**

The pharmaceutical composition for use according to any one of claims 83-93, wherein the glucocorticoid dose of the subject is reduced by about 20% to about 50% after a time period of administration of the pharmaceutical composition, wherein the reduction of the glucocorticoid dose is relative to the glucocorticoid dose prior to administration of the pharmaceutical composition.

**103.**

The pharmaceutical composition for use according to any one of claims 83-93, wherein the glucocorticoid dose of the subject is reduced by greater than about 50% after a time period of administration of the pharmaceutical composition, wherein the reduction of the glucocorticoid dose is relative to the glucocorticoid dose prior to administration of the pharmaceutical composition.

**104.**

The pharmaceutical composition for use according to any one of claims 50-103, wherein the level of 17-hydroxyprogesterone is reduced by at least about 25% after a time period of administration of the pharmaceutical composition, wherein the reduction of the level of 17-hydroxyprogesterone is relative to the level of 17-hydroxyprogesterone prior to

administration of the pharmaceutical composition.

**105.**
The pharmaceutical composition for use according to any one of claims 50-103, wherein the level of 17-hydroxyprogesterone is reduced by at least about 50% after a time period of administration of the pharmaceutical composition, wherein the reduction of the level of 17-hydroxyprogesterone is relative to the level of 17-hydroxyprogesterone prior to administration of the pharmaceutical composition.

**106.**
The pharmaceutical composition for use according to any one of claims 50-103, wherein the level of 17-hydroxyprogesterone is less than about 1.5 times the upper limit of normal after a time period of administration of the pharmaceutical composition.

**107.**
The pharmaceutical composition for use according to any one of claims 50-103, wherein the level of 17-hydroxyprogesterone is within normal limits after a time period of administration of the pharmaceutical composition.

**108.**
The pharmaceutical composition for use according to any one of claims 50-107, wherein the level of adrenocorticotropic hormone is reduced by at least about 25% after a time period of administration of the pharmaceutical composition, wherein the reduction of the level of adrenocorticotropic hormone is relative to the level of adrenocorticotropic hormone prior to administration of the pharmaceutical composition.

**109.**
The pharmaceutical composition for use according to any one of claims 50-107, wherein the level of adrenocorticotropic hormone is reduced by at least about 40% after a time period of administration of the pharmaceutical composition, wherein the reduction of the level of adrenocorticotropic hormone is relative to the level of adrenocorticotropic hormone prior to administration of the pharmaceutical composition.

**110.**
The pharmaceutical composition for use according to any one of claims 50-107, wherein the level of adrenocorticotropic hormone is reduced by at least about 50% after a time period of administration of the pharmaceutical composition, wherein the reduction of the level of adrenocorticotropic hormone is relative to the level of adrenocorticotropic hormone prior to administration of the pharmaceutical composition.

**111.** The pharmaceutical composition for use according to any one of claims 50-107, wherein the level of adrenocorticotropic hormone is less than about 1.5 times the upper limit of normal after a time period of administration of the pharmaceutical composition.

**112.**
The pharmaceutical composition for use according to any one of claims 50-103, wherein the level of adrenocorticotropic hormone is within normal limits after a time period of administration of the pharmaceutical composition.

**113.**
The pharmaceutical composition for use according to any one of claims 50-112, wherein the level of androstenedione is reduced by at least about 25% after a time period of administration of the pharmaceutical composition, wherein the reduction of the level of androstenedione is relative to the level of androstenedione prior to administration of the pharmaceutical composition.

**114.**
The pharmaceutical composition for use according to any one of claims 50-112, wherein the level of androstenedione is reduced by at least about 30% after a time period of administration of the pharmaceutical composition, wherein the reduction of the level of androstenedione is relative to the level of androstenedione prior to administration of the pharmaceutical composition.

**115.**
The pharmaceutical composition for use according to any one of claims 50-112, wherein the level of androstenedione is reduced by at least about 50% after a time period of administration of the pharmaceutical composition, wherein the

reduction of the level of androstenedione is relative to the level of androstenedione prior to administration of the pharmaceutical composition.

**116.**
The pharmaceutical composition for use according to any one of claims 50-112, wherein the level of androstenedione is less than about 1.5 times the upper limit of normal after a time period of administration of the pharmaceutical composition.

**117.**
The pharmaceutical composition for use according to any one of claims 50-112, wherein the level of androstenedione is within normal limits after a time period of administration of the pharmaceutical composition.

**118.**
The pharmaceutical composition for use according to any one of claims 50-117, wherein the level of testosterone is reduced by at least about 25% after a time period of administration of the pharmaceutical composition, wherein the reduction of the level of testosterone is relative to the level of testosterone prior to administration of the pharmaceutical composition.

**119.**
The pharmaceutical composition for use according to any one of claims 50-117, wherein the level of testosterone is reduced by at least about 30% after a time period of administration of the pharmaceutical composition, wherein the reduction of the level of testosterone is relative to the level of testosterone prior to administration of the pharmaceutical composition.

**120.**
The pharmaceutical composition for use according to any one of claims 50-117, wherein the level of testosterone is reduced by at least about 50% after a time period of administration of the pharmaceutical composition, wherein the reduction of the level of testosterone is relative to the level of testosterone prior to administration of the pharmaceutical composition.

**121.**
The pharmaceutical composition for use according to any one of claims 50-117, wherein the level of testosterone is less than about 1.5 times the upper limit of normal after a time period of administration of the pharmaceutical composition.

**122.**
The pharmaceutical composition for use according to any one of claims 50-117, wherein the level of testosterone is within normal limits after a time period of administration of the pharmaceutical composition comprising the compound of Formula (I), or a pharmaceutically acceptable salt thereof.

**123.**
The pharmaceutical composition for use according to any one of claims 50-103, wherein the level of 17-hydroxyprogesterone is reduced by at least about 50% and the level of androstenedione is reduced by at least about 50% after a time period of administration of the pharmaceutical composition, wherein the reduction of the level of 17-hydroxyprogesterone and the level of androstenedione is relative to the level of 17-hydroxyprogesterone and the level of androstenedione prior to administration of the pharmaceutical composition.

**124.**
The pharmaceutical composition for use according to any one of claims 50-103, wherein the level of 17-hydroxyprogesterone is less than about 1.5 times the upper limit of normal and the level of androstenedione is less than about 1.5 times the upper limit of normal after a time period of administration of the pharmaceutical composition.

**125.**
The pharmaceutical composition for use according to any one of claims 50-103, wherein the level of 17-hydroxyprogesterone is within normal limits and the level of androstenedione is within normal limits after a time period of administration of the pharmaceutical composition.

**126.**
The pharmaceutical composition for use according to any one of claims 83-125, wherein the subject exhibits a decrease in glucocorticoid burden after a time period of administration of the pharmaceutical composition, wherein the decrease in

glucocorticoid burden is relative to the glucocorticoid burden prior to administration of the pharmaceutical composition.

**127.**
The pharmaceutical composition for use according to claim 126, wherein one or more symptoms of glucocorticoid burden selected from quality of life, fatigue, sleep, insulin resistance, glucose tolerance, glucose control, dyslipidemia, hyperlipidemia, bone mineral density, bone turnover, fat mass, weight, central obesity, blood pressure, hirsutism severity, menstrual cyclicity, and fertility, is improved after a time period of administration of the pharmaceutical composition, wherein the improvement in the one or more symptoms is relative to the status of the one or more symptoms prior to administration of the pharmaceutical composition.

**128.**
The pharmaceutical composition for use according to any one of claims 90-127, wherein the time period of administration is at least about 4 weeks.

**129.**
The pharmaceutical composition for use according to any one of claims 90-127, wherein the time period of administration is at least about 24 weeks.

**130.**
The pharmaceutical composition for use according to any one of claims 90-127, wherein the time period of administration is at least about one year.

**131.**
The pharmaceutical composition for use according to any one of claims 50-130, wherein the subject is a pediatric subject.

**132.**
The pharmaceutical composition for use according to claim 131, wherein the pediatric subject is less than or equal to six years old.

**133.**
The pharmaceutical composition for use according to claim 131, wherein the pediatric subject is greater than six years old and less than eleven years old.

**134.**
The pharmaceutical composition for use according to claim 131, wherein the pediatric subject is greater than ten years old and less than fifteen years old.

**135.**
The pharmaceutical composition for use according to claim 131, wherein the pediatric subject is greater than fourteen years old and less than nineteen years old.

**136.**
The pharmaceutical composition for use according to any one of claims 50-130, wherein the subject is an adult subject.

**137.**
The pharmaceutical composition for use according to claim 136, wherein the adult subject is over eighteen years old.

**138.**
The pharmaceutical composition for use according to any one of claims 50-137, wherein the subject is female.

**139.**
The pharmaceutical composition for use according to any one of claims 50-137, wherein the subject is male.

**140.**
The pharmaceutical composition for use according to any one of claims 50-139, wherein the compound of Formula (I), or a pharmaceutically acceptable salt thereof, is administered as a hydrochloric acid salt or p-toluenesulfonic acid salt.

**141.**

The pharmaceutical composition for use according to any one of claims 50-139, wherein the compound of Formula (I), or a pharmaceutically acceptable salt thereof, is administered as a p-toluenesulfonic acid crystalline salt.

## Patentansprüche

1. Pharmazeutische Zusammensetzung in Lösung als orale Dosierungsform, umfassend:

   (a) eine Verbindung der Formel (I):

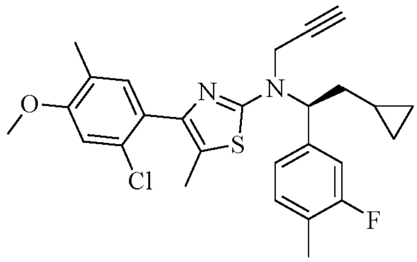

(I)

   (4-(2-Chlor-4-methoxy-5-methylphenyl)-N-[(1S)-2-cyclopropyl-1-(3-fluor-4-methylphenyl)ethyl]-5-methyl-N-prop-2-inyl-1,3-thiazol-2-amin) oder ein pharmazeutisch unbedenkliches Salz davon;
   (b) einen oder mehrere Süßstoffe, ein Antioxidans und einen Geschmacksstoff; und
   (c) ein flüssiges Vehikel, wobei das flüssige Vehikel aus mittelkettigen Triglyceriden, Propylenglykoldicaprylat/-dicaprat, Glycerin, Propylenglykol, Polyethylenglykol, Olivenöl, Sojaöl, Maisöl und Diethylenglykolmonoethylether ausgewählt ist.

2. Pharmazeutische Zusammensetzung nach Anspruch 1, umfassend etwa 1 % w/v bis etwa 50 % w/v der Verbindung der Formel (I) oder eines pharmazeutisch unbedenklichen Salzes davon, bezogen auf das Gewicht der freien Base.

3. Pharmazeutische Zusammensetzung nach Anspruch 1, umfassend etwa 1 % w/v bis etwa 10 % w/v der Verbindung der Formel (I) oder eines pharmazeutisch unbedenklichen Salzes davon, bezogen auf das Gewicht der freien Base.

4. Pharmazeutische Zusammensetzung nach Anspruch 1, umfassend etwa 5 % w/v der Verbindung der Formel (I) oder eines pharmazeutisch unbedenklichen Salzes davon, bezogen auf das Gewicht der freien Base.

5. Pharmazeutische Zusammensetzung nach einem der Ansprüche 1-4, umfassend einen Süßstoff.

6. Pharmazeutische Zusammensetzung nach Anspruch 5, umfassend etwa 0,01 % w/v bis etwa 1,5 % w/v des Süßstoffs.

7. Pharmazeutische Zusammensetzung nach Anspruch 5, umfassend etwa 0,1 % w/v bis etwa 0,5 % w/v des Süßstoffs.

8. Pharmazeutische Zusammensetzung nach Anspruch 5, umfassend etwa 0,15 % w/v des Süßstoffs.

9. Pharmazeutische Zusammensetzung nach einem der Ansprüche 5-8, wobei der Süßstoff aus Saccharin, Saccharose, Sucralose, Aspartam, Dextrose, Fructose, Maltitol, Mannit, Sorbit und Avantam ausgewählt ist.

10. Pharmazeutische Zusammensetzung nach einem der Ansprüche 5-8, wobei es sich bei dem Süßstoff um Saccharin handelt.

11. Pharmazeutische Zusammensetzung nach einem der Ansprüche 1-10, umfassend ein Antioxidans.

12. Pharmazeutische Zusammensetzung nach Anspruch 11, umfassend etwa 0,01 % w/v bis etwa 1,5 % w/v des Antioxidans.

13. Pharmazeutische Zusammensetzung nach Anspruch 11, umfassend etwa 0,1 % w/v bis etwa 0,5 % w/v des

Antioxidans.

**14.** Pharmazeutische Zusammensetzung nach Anspruch 11, umfassend etwa 0,17 % w/v des Antioxidans.

**15.** Pharmazeutische Zusammensetzung nach einem der Ansprüche 11-14, wobei das Antioxidans aus Butylhydroxytoluol, Vitamin E TPGS, Butylhydroxyanisol, Ascorbinsäure, Lecithin, tert.-Butylhydrochinon und Citronensäure ausgewählt ist.

**16.** Pharmazeutische Zusammensetzung nach einem der Ansprüche 11-15, wobei es sich bei dem Antioxidans um Butylhydroxytoluol handelt.

**17.** Pharmazeutische Zusammensetzung nach einem der Ansprüche 1-16, umfassend einen Geschmacksstoff.

**18.** Pharmazeutische Zusammensetzung nach Anspruch 17, umfassend etwa 0,01 % w/v bis etwa 0,5 % w/v des Geschmacksstoffes.

**19.** Pharmazeutische Zusammensetzung nach Anspruch 17, umfassend etwa 0,05 % w/v bis etwa 0,2 % w/v des Geschmacksstoffes.

**20.** Pharmazeutische Zusammensetzung nach Anspruch 17, umfassend etwa 0,10 % w/v des Geschmacksstoffes.

**21.** Pharmazeutische Zusammensetzung nach einem der Ansprüche 1-20, umfassend etwa 50 % w/v bis etwa 99,9 % w/v des flüssigen Vehikels.

**22.** Pharmazeutische Zusammensetzung nach einem der Ansprüche 1-20, umfassend etwa 90 % w/v bis etwa 99 % w/v des flüssigen Vehikels.

**23.** Pharmazeutische Zusammensetzung nach einem der Ansprüche 1-20, umfassend etwa 92 % w/v bis etwa 97 % w/v des flüssigen Vehikels.

**24.** Pharmazeutische Zusammensetzung nach einem der Ansprüche 1-20, umfassend etwa 94,6 % w/v des flüssigen Vehikels.

**25.** Pharmazeutische Zusammensetzung nach einem der Ansprüche 1-20, umfassend etwa 70 % w/v bis etwa 80 % w/v des flüssigen Vehikels.

**26.** Pharmazeutische Zusammensetzung nach einem der Ansprüche 1-20, umfassend etwa 75 % w/v des flüssigen Vehikels.

**27.** Pharmazeutische Zusammensetzung nach einem der Ansprüche 1-26, wobei es sich bei dem flüssigen Vehikel um mittelkettige Triglyceride handelt.

**28.** Pharmazeutische Zusammensetzung nach Anspruch 27, wobei es sich bei den mittelkettigen Triglyceriden um Capryl-/Caprintriglyceride handelt.

**29.** Pharmazeutische Zusammensetzung nach einem der Ansprüche 1-28, ferner umfassend ein Tensid.

**30.** Pharmazeutische Zusammensetzung nach Anspruch 29, umfassend etwa 1 % w/v bis etwa 50 % w/v des Tensids.

**31.** Pharmazeutische Zusammensetzung nach Anspruch 29, umfassend etwa 10 % w/v bis etwa 30 % w/v des Tensids.

**32.** Pharmazeutische Zusammensetzung nach Anspruch 29, umfassend etwa 20 % w/v des Tensids.

**33.** Pharmazeutische Zusammensetzung nach einem der Ansprüche 29-32, wobei das Tensid aus Oleoylpolyoxyl-6-glyceriden, Linoleoylpolyoxyl-6-glyceriden, Polysorbat 80, Polysorbat 20, Vitamin-E-polyethylenglykolsuccinat, Lauroylpolyoxyl-32-glyceriden, Natriumlaurylsulfat, Poloxamer, Maisöl-PEG-6-estern und hydrierten Palm-/Palmkernöl-PEG-6-estern ausgewählt ist.

**34.** Pharmazeutische Zusammensetzung nach einem der Ansprüche 29-33, wobei es sich bei dem Tensid um Oleoylpolyoxyl-6-glyceride handelt.

**35.** Pharmazeutische Zusammensetzung nach Anspruch 1, umfassend:

(a) 4-(2-Chlor-4-methoxy-5-methylphenyl)-N-[(1S)-2-cyclopropyl-1-(3-fluor-4-methylphenyl)ethyl]-5-methyl-N-prop-2-inyl-1,3-thiazol-2-amin oder ein pharmazeutisch unbedenkliches Salz davon;
(b) einen Süßstoff;
(c) ein Antioxidans;
(d) einen Geschmacksstoff; und
(e) das flüssige Vehikel.

**36.** Pharmazeutische Zusammensetzung nach Anspruch 35, ferner umfassend ein Tensid.

**37.** Pharmazeutische Zusammensetzung nach Anspruch 1, umfassend:

(a) etwa 4 % w/v bis etwa 6 % w/v 4-(2-Chlor-4-methoxy-5-methylphenyl)-N-[(1S)-2-cyclopropyl-1-(3-fluor-4-methylphenyl)ethyl]-5-methyl-N-prop-2-inyl-1,3-thiazol-2-amin oder eines pharmazeutisch unbedenklichen Salzes davon, bezogen auf das Gewicht der freien Base;
(b) etwa 0,1 % w/v bis etwa 0,2 % w/v eines Süßstoffs;
(c) etwa 0,1 % w/v bis etwa 0,2 % w/v eines Antioxidans;
(d) etwa 0,05 % w/v bis etwa 0,2 % w/v eines Geschmacksstoffs; und
(e) etwa 92 % w/v bis etwa 97 % w/v des flüssigen Vehikels.

**38.** Pharmazeutische Zusammensetzung nach Anspruch 1, umfassend:

(a) etwa 5 % w/v 4-(2-Chlor-4-methoxy-5-methylphenyl)-N-[(1S)-2-cyclopropyl-1-(3-fluor-4-methylphenyl) ethyl]-5-methyl-N-prop-2-inyl-1,3-thiazol-2-amin oder eines pharmazeutisch unbedenklichen Salzes davon, bezogen auf das Gewicht der freien Base;
(b) etwa 0,15 % w/v eines Süßstoffs;
(c) etwa 0,17 % w/v eines Antioxidans;
(d) etwa 0,1 % w/v eines Geschmacksstoffs; und
(e) etwa 94,6 % w/v des flüssigen Vehikels.

**39.** Pharmazeutische Zusammensetzung nach Anspruch 1, umfassend:

(a) etwa 4 % w/v bis etwa 6 % w/v 4-(2-Chlor-4-methoxy-5-methylphenyl)-N-[(1S)-2-cyclopropyl-1-(3-fluor-4-methylphenyl)ethyl]-5-methyl-N-prop-2-inyl-1,3-thiazol-2-amin oder eines pharmazeutisch unbedenklichen Salzes davon, bezogen auf das Gewicht der freien Base;
(b) etwa 0,1 % w/v bis etwa 0,2 % w/v eines Süßstoffs;
(c) etwa 0,1 % w/v bis etwa 0,2 % w/v eines Antioxidans;
(d) etwa 0,05 % w/v bis etwa 0,2 % w/v eines Geschmacksstoffs;
(e) etwa 15 % w/v bis etwa 25 % w/v eines Tensids; und
(f) etwa 70 % w/v bis etwa 80 % w/v des flüssigen Vehikels.

**40.** Pharmazeutische Zusammensetzung nach Anspruch 1, umfassend:

(a) etwa 5 % w/v 4-(2-Chlor-4-methoxy-5-methylphenyl)-N-[(1S)-2-cyclopropyl-1-(3-fluor-4-methylphenyl) ethyl]-5-methyl-N-prop-2-inyl-1,3-thiazol-2-amin oder eines pharmazeutisch unbedenklichen Salzes davon, bezogen auf das Gewicht der freien Base;
(b) etwa 0,15 % w/v eines Süßstoffs;
(c) etwa 0,17 % w/v eines Antioxidans;
(d) etwa 0,1 % w/v eines Geschmacksstoffs;
(e) etwa 20 % w/v eines Tensids; und
(f) etwa 75 % w/v des flüssigen Vehikels.

**41.** Pharmazeutische Zusammensetzung nach Anspruch 40, wobei

(a) es sich bei der Verbindung der Formel (I) um die freie Base handelt;
(b) es sich bei dem Süßstoff um Saccharin handelt;
(c) es sich bei dem Antioxidans um Butylhydroxytoluol handelt;
(d) es sich bei dem Tensid um Oleoylpolyoxyl-6-glyceride handelt; und
(e) es sich bei dem flüssigen Vehikel um mittelkettige Triglyceride handelt.

**42.** Pharmazeutische Zusammensetzung nach Anspruch 40, wobei es sich bei den mittelkettigen Triglyceriden um Capryl-/Caprintriglyceride handelt.

**43.** Pharmazeutische Zusammensetzung nach einem der Ansprüche 1-40, umfassend die Verbindung der Formel (I) als freie Base.

**44.** Pharmazeutische Zusammensetzung nach einem der Ansprüche 1-43, formuliert als Einheitsdosisform, wobei die Verbindung der Formel (I) oder ein pharmazeutisch unbedenkliches Salz davon in einer Menge von 5 mg/ml bis etwa 200 mg/ml, bezogen auf das Gewicht der freien Base, vorhanden ist.

**45.** Pharmazeutische Zusammensetzung nach Anspruch 44, wobei die Verbindung der Formel (I) oder ein pharmazeutisch unbedenkliches Salz davon in einer Menge von etwa 75 mg/ml bis etwa 150 mg/ml, bezogen auf das Gewicht der freien Base, vorhanden ist.

**46.** Pharmazeutische Zusammensetzung nach Anspruch 44, wobei die Verbindung der Formel (I) oder ein pharmazeutisch unbedenkliches Salz davon in einer Menge von etwa 50 mg/ml, bezogen auf das Gewicht der freien Base, vorhanden ist.

**47.** Pharmazeutische Zusammensetzung nach Anspruch 44, wobei die Verbindung der Formel (I) oder ein pharmazeutisch unbedenkliches Salz davon in einer Menge von etwa 100 mg/ml, bezogen auf das Gewicht der freien Base, vorhanden ist.

**48.** Verfahren zum Herstellen der pharmazeutischen Zusammensetzung nach einem der Ansprüche 1-47, umfassend:

(a) Mischen des flüssigen Vehikels mit einem Süßstoff;
(b) Mischen der Mischung aus Schritt (a) mit einem Antioxidans und einem Geschmacksstoff;
(c) Mischen der Verbindung der Formel (I) oder eines pharmazeutisch unbedenklichen Salzes davon mit der Mischung aus Schritt (b); und
(d) Mischen der Mischung aus Schritt (c) mit einem zusätzlichen Anteil des flüssigen Vehikels.

**49.** Verfahren nach Anspruch 48, wobei Schritt (a) Mischen des flüssigen Vehikels mit einem Süßstoff und einem Tensid umfasst.

**50.** Pharmazeutische Zusammensetzung nach einem der Ansprüche 1-47 zur Verwendung bei einem Verfahren zum Behandeln von kongenitaler Nebennierenhyperplasie (CAH = congenital adrenal hyperplasia) bei einem Individuum.

**51.** Pharmazeutische Zusammensetzung nach Anspruch 50, wobei es sich bei der kongenitalen Nebennierenhyperplasie (CAH = congenital adrenal hyperplasia) um klassische CAH handelt.

**52.** Pharmazeutische Zusammensetzung zur Verwendung nach Anspruch 50 oder 51, wobei sich das Individuum in einem genährten Zustand befindet.

**53.** Pharmazeutische Zusammensetzung zur Verwendung nach einem der Ansprüche 50-52, wobei die pharmazeutische Zusammensetzung mit einer Nährstoffzusammensetzung an das Individuum verabreicht wird.

**54.** Pharmazeutische Zusammensetzung zur Verwendung nach Anspruch 53, wobei es sich bei der Nährstoffzusammensetzung um ein flüssiges Nahrungsergänzungsmittel handelt, das etwa 1000 bis etwa 2000 Kalorien pro Liter mit einem Fettgehalt von mehr als 30 % umfasst.

**55.** Pharmazeutische Zusammensetzung zur Verwendung nach Anspruch 53, wobei es sich bei der Nährstoffzusammensetzung um ein flüssiges Nahrungsergänzungsmittel handelt, das etwa 1500 Kalorien pro Liter mit einer kalorischen Verteilung von 14,7 % Protein, 32 % Fett und 53,3 % Kohlenhydrat umfasst.

**56.** Pharmazeutische Zusammensetzung zur Verwendung nach einem der Ansprüche 53-55, wobei die Nährstoffzusammensetzung in einer Menge von etwa 6 bis etwa 12 Fluid Ounces verabreicht wird.

**57.** Pharmazeutische Zusammensetzung zur Verwendung nach einem der Ansprüche 53-55, wobei die Nährstoffzusammensetzung in einer Menge von etwa 8 Fluid Ounces verabreicht wird.

**58.** Pharmazeutische Zusammensetzung zur Verwendung nach einem der Ansprüche 53-57, wobei die Nährstoffzusammensetzung innerhalb von 30 Minuten nach der Verabreichung der pharmazeutischen Zusammensetzung verabreicht wird.

**59.** Pharmazeutische Zusammensetzung zur Verwendung nach einem der Ansprüche 52-58, wobei Verabreichen der pharmazeutischen Zusammensetzung einen positiven Nahrungseffekt bewirkt.

**60.** Pharmazeutische Zusammensetzung zur Verwendung nach einem der Ansprüche 50-59, wobei es sich bei dem Individuum um einen Menschen handelt.

**61.** Pharmazeutische Zusammensetzung zur Verwendung nach einem der Ansprüche 52-58, wobei die pharmazeutische Zusammensetzung einen positiven Nahrungseffekt bewirkt, wenn sie oral verabreicht wird.

**62.** Pharmazeutische Zusammensetzung zur Verwendung nach einem der Ansprüche 50-61, wobei die pharmazeutische Zusammensetzung mit einer Mahlzeit an das Individuum verabreicht wird.

**63.** Pharmazeutische Zusammensetzung zur Verwendung nach Anspruch 62, wobei es sich bei der Mahlzeit um eine fettreiche Mahlzeit handelt.

**64.** Pharmazeutische Zusammensetzung zur Verwendung nach Anspruch 62, wobei es sich bei der Mahlzeit um eine fettarme Mahlzeit handelt.

**65.** Pharmazeutische Zusammensetzung zur Verwendung nach einem der Ansprüche 62-64, wobei die pharmazeutische Zusammensetzung innerhalb von 5 Minuten nach dem Beginn der Mahlzeit verabreicht wird.

**66.** Pharmazeutische Zusammensetzung zur Verwendung nach einem der Ansprüche 62-64, wobei es sich bei der Mahlzeit um ein Abendessen handelt.

**67.** Pharmazeutische Zusammensetzung zur Verwendung nach einem der Ansprüche 62-64, wobei es sich bei der Mahlzeit um ein Frühstück handelt.

**68.** Pharmazeutische Zusammensetzung zur Verwendung nach einem der Ansprüche 62-67, wobei Verabreichen der pharmazeutischen Zusammensetzung einen positiven Nahrungseffekt bewirkt.

**69.** Pharmazeutische Zusammensetzung zur Verwendung nach einem der Ansprüche 50-68, wobei die pharmazeutische Zusammensetzung etwa 25 mg der Verbindung der Formel (I) oder eines pharmazeutisch unbedenklichen Salzes davon, bezogen auf das Gewicht der freien Base, umfasst.

**70.** Pharmazeutische Zusammensetzung zur Verwendung nach einem der Ansprüche 50-68, wobei die pharmazeutische Zusammensetzung etwa 50 mg der Verbindung der Formel (I) oder eines pharmazeutisch unbedenklichen Salzes davon, bezogen auf das Gewicht der freien Base, umfasst.

**71.** Pharmazeutische Zusammensetzung zur Verwendung nach einem der Ansprüche 50-68, wobei die pharmazeutische Zusammensetzung etwa 75 mg der Verbindung der Formel (I) oder eines pharmazeutisch unbedenklichen Salzes davon, bezogen auf das Gewicht der freien Base, umfasst.

**72.** Pharmazeutische Zusammensetzung zur Verwendung nach einem der Ansprüche 50-68, wobei die pharmazeutische Zusammensetzung etwa 100 mg der Verbindung der Formel (I) oder eines pharmazeutisch unbedenklichen Salzes davon, bezogen auf das Gewicht der freien Base, umfasst.

**73.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 25 mg der Verbindung

der Formel (I) oder eines pharmazeutisch unbedenklichen Salzes davon, bezogen auf das Gewicht der freien Base, umfasst.

**74.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 50 mg der Verbindung der Formel (I) oder eines pharmazeutisch unbedenklichen Salzes davon, bezogen auf das Gewicht der freien Base, umfasst.

**75.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 75 mg der Verbindung der Formel (I) oder eines pharmazeutisch unbedenklichen Salzes davon, bezogen auf das Gewicht der freien Base, umfasst.

**76.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 100 mg der Verbindung der Formel (I) oder eines pharmazeutisch unbedenklichen Salzes davon, bezogen auf das Gewicht der freien Base, umfasst.

**77.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 150 mg der Verbindung der Formel (I) oder eines pharmazeutisch unbedenklichen Salzes davon, bezogen auf das Gewicht der freien Base, umfasst.

**78.** Pharmazeutische Zusammensetzung zur Verwendung nach einem der Ansprüche 50-68, wobei das Verfahren Verabreichen einer Tagesdosis der pharmazeutischen Zusammensetzung umfasst, die etwa 200 mg der Verbindung der Formel (I) oder eines pharmazeutisch unbedenklichen Salzes davon, bezogen auf das Gewicht der freien Base, umfasst.

**79.** Pharmazeutische Zusammensetzung zur Verwendung nach einem der Ansprüche 50-68, wobei das Verfahren Verabreichen der pharmazeutischen Zusammensetzung zweimal täglich in einer Dosis von etwa 25 mg der Verbindung der Formel (I) oder eines pharmazeutisch unbedenklichen Salzes davon, bezogen auf das Gewicht der freien Base, umfasst.

**80.** Pharmazeutische Zusammensetzung zur Verwendung nach einem der Ansprüche 50-68, wobei das Verfahren Verabreichen der pharmazeutischen Zusammensetzung zweimal täglich in einer Dosis von etwa 50 mg der Verbindung der Formel (I) oder eines pharmazeutisch unbedenklichen Salzes davon, bezogen auf das Gewicht der freien Base, umfasst.

**81.** Pharmazeutische Zusammensetzung zur Verwendung nach einem der Ansprüche 50-68, wobei das Verfahren Verabreichen der pharmazeutischen Zusammensetzung zweimal täglich in einer Dosis von etwa 75 mg der Verbindung der Formel (I) oder eines pharmazeutisch unbedenklichen Salzes davon, bezogen auf das Gewicht der freien Base, umfasst.

**82.** Pharmazeutische Zusammensetzung zur Verwendung nach einem der Ansprüche 50-68, wobei das Verfahren Verabreichen der pharmazeutischen Zusammensetzung zweimal täglich in einer Dosis von etwa 100 mg der Verbindung der Formel (I) oder eines pharmazeutisch unbedenklichen Salzes davon, bezogen auf das Gewicht der freien Base, umfasst.

**83.** Pharmazeutische Zusammensetzung zur Verwendung nach einem der Ansprüche 50-82, wobei das Individuum gleichzeitig eine Dosis eines Glucocorticoids erhält.

**84.** Pharmazeutische Zusammensetzung zur Verwendung nach Anspruch 83, wobei das Glucocorticoid aus Cortisol (Hydrocortison), Cortison, Prednison, Prednisolon, Methylprednisolon, Dexamethason, Betamethason, Triamcinolon, Fludrocortisonacetat und Desoxycorticosteronacetat ausgewählt ist.

**85.** Pharmazeutische Zusammensetzung zur Verwendung nach Anspruch 83 oder 84, wobei es sich bei dem Glucocorticoid um Cortisol (Hydrocortison) handelt.

**86.** Pharmazeutische Zusammensetzung zur Verwendung nach Anspruch 83 oder 84, wobei es sich bei dem Glucocorticoid um Cortison handelt.

**87.** Pharmazeutische Zusammensetzung zur Verwendung nach Anspruch 83 oder 84, wobei es sich bei dem Glucocorticoid um Prednison handelt.

**88.** Pharmazeutische Zusammensetzung zur Verwendung nach einem der Ansprüche 83-87, wobei die Glucocorticoiddosis in Hydrocortisonäquivalenten gemessen wird.

**89.** Pharmazeutische Zusammensetzung zur Verwendung nach einem der Ansprüche 83-88, wobei die Glucocorticoiddosis als ein Vielfaches der Obergrenze der normalen physiologischen Dosierung in Hydrocortisonäquivalenten gemessen wird.

**90.** Pharmazeutische Zusammensetzung zur Verwendung nach einem der Ansprüche 83-89, wobei es sich bei der Glucocorticoiddosis um eine nach einem Verabreichungszeitraum der pharmazeutischen Zusammensetzung gemessene physiologische Dosis handelt.

**91.** Pharmazeutische Zusammensetzung zur Verwendung nach einem der Ansprüche 83-89, wobei es sich bei der Glucocorticoiddosis um eine nach einem Verabreichungszeitraum der pharmazeutischen Zusammensetzung gemessene physiologische Dosis von etwa 4 bis etwa 12 $mg/m^2$/Tag handelt.

**92.** Pharmazeutische Zusammensetzung zur Verwendung nach einem der Ansprüche 83-89, wobei es sich bei der Glucocorticoiddosis um eine nach einem Verabreichungszeitraum der pharmazeutischen Zusammensetzung gemessene physiologische Dosis von etwa 4 bis etwa 9 $mg/m^2$/Tag handelt.

**93.** Pharmazeutische Zusammensetzung zur Verwendung nach einem der Ansprüche 83-89, wobei es sich bei der Glucocorticoiddosis um eine nach einem Verabreichungszeitraum der pharmazeutischen Zusammensetzung gemessene physiologische Dosis von weniger als etwa 8 $mg/m^2$/Tag handelt.

**94.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 10 % reduziert wird, wobei sich die Reduzierung der Glucocorticoiddosis auf die Glucocorticoiddosis vor Verabreichung der pharmazeutischen Zusammensetzung bezieht.

**95.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 20 % reduziert wird, wobei sich die Reduzierung der Glucocorticoiddosis auf die Glucocorticoiddosis vor Verabreichung der pharmazeutischen Zusammensetzung bezieht.

**96.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 30 % reduziert wird, wobei sich die Reduzierung der Glucocorticoiddosis auf die Glucocorticoiddosis vor Verabreichung der pharmazeutischen Zusammensetzung bezieht.

**97.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 40 % reduziert wird, wobei sich die Reduzierung der Glucocorticoiddosis auf die Glucocorticoiddosis vor Verabreichung der pharmazeutischen Zusammensetzung bezieht.

**98.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 50 % reduziert wird, wobei sich die Reduzierung der Glucocorticoiddosis auf die Glucocorticoiddosis vor Verabreichung der pharmazeutischen Zusammensetzung bezieht.

**99.** Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 60 % reduziert wird, wobei sich die Reduzierung der Glucocorticoiddosis auf die Glucocorticoiddosis vor Verabreichung der pharmazeutischen Zusammensetzung bezieht.

**100.**

Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 70 % reduziert wird, wobei sich die Reduzierung der Glucocorticoiddosis auf die Glucocorticoiddosis vor Verabreichung der pharmazeutischen Zusammensetzung bezieht.

**101.**

Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um weniger als etwa 20 % reduziert wird, wobei sich die Reduzierung der Glucocorticoiddosis auf die Glucocorticoiddosis vor Verabreichung der pharmazeutischen Zusammensetzung bezieht.

**102.**

Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um etwa 20 % bis etwa 50 % reduziert wird, wobei sich die Reduzierung der Glucocorticoiddosis auf die Glucocorticoiddosis vor Verabreichung der pharmazeutischen Zusammensetzung bezieht.

**103.**

Pharmazeutische Zusammensetzung zur Verwendung nach einem der Ansprüche 83-93, wobei die Glucocorticoiddosis des Individuums nach einem Verabreichungszeitraum der pharmazeutischen Zusammensetzung um mehr als etwa 50 % reduziert wird, wobei sich die Reduzierung der Glucocorticoiddosis auf die Glucocorticoiddosis vor Verabreichung der pharmazeutischen Zusammensetzung bezieht.

**104.**

Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung um weniger als etwa 25 % reduziert wird, wobei sich die Reduzierung des 17-Hydroxyprogesteronniveaus auf das 17-Hydroxyprogesteronniveau vor Verabreichung der pharmazeutischen Zusammensetzung bezieht.

**105.**

Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung um weniger als etwa 50 % reduziert wird, wobei sich die Reduzierung des 17-Hydroxyprogesteronniveaus auf das 17-Hydroxyprogesteronniveau vor Verabreichung der pharmazeutischen Zusammensetzung bezieht.

**106.**

Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung weniger als etwa das 1,5-Fache der normalen Obergrenze beträgt.

**107.**

Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung im Normalbereich liegt.

**108.**

Pharmazeutische Zusammensetzung zur Verwendung nach einem der Ansprüche 50-107, wobei das Adrenocorticotropinniveau nach einem Verabreichungszeitraum der pharmazeutischen Zusammensetzung um mindestens etwa 25 % reduziert wird, wobei sich die Reduzierung des Adrenocorticotropinniveaus auf das Adrenocorticotropinniveau vor Verabreichung der pharmazeutischen Zusammensetzung bezieht.

**109.**

Pharmazeutische Zusammensetzung zur Verwendung nach einem der Ansprüche 50-107, wobei das Adrenocorticotropinniveau nach einem Verabreichungszeitraum der pharmazeutischen Zusammensetzung um mindestens etwa 40 % reduziert wird, wobei sich die Reduzierung des Adrenocorticotropinniveaus auf das Adrenocorticotropinniveau vor Verabreichung der pharmazeutischen Zusammensetzung bezieht.

**110.**

Pharmazeutische Zusammensetzung zur Verwendung nach einem der Ansprüche 50-107, wobei das Adrenocorticotropinniveau nach einem Verabreichungszeitraum der pharmazeutischen Zusammensetzung um mindestens etwa 50 % reduziert wird, wobei sich die Reduzierung des Adrenocorticotropinniveaus auf das Adrenocorticotropinniveau vor Verabreichung der pharmazeutischen Zusammensetzung bezieht.

**111.** Pharmazeutische Zusammensetzung zur Verwendung nach einem der Ansprüche 50-107, wobei das Adrenocorticotropinniveau nach einem Verabreichungszeitraum der pharmazeutischen Zusammensetzung weniger als das 1,5-Fache der normalen Obergrenze beträgt.

**112.**
Pharmazeutische Zusammensetzung zur Verwendung nach einem der Ansprüche 50-103, wobei das Adrenocorticotropinniveau nach einem Verabreichungszeitraum der pharmazeutischen Zusammensetzung im Normalbereich liegt.

**113.**
Pharmazeutische Zusammensetzung zur Verwendung nach einem der Ansprüche 50-112, wobei das Androstendionniveau nach einem Verabreichungszeitraum der pharmazeutischen Zusammensetzung um mindestens etwa 25 % reduziert wird, wobei sich die Reduzierung des Androstendionniveaus auf das Androstendionniveau vor Verabreichung der pharmazeutischen Zusammensetzung bezieht.

**114.**
Pharmazeutische Zusammensetzung zur Verwendung nach einem der Ansprüche 50-112, wobei das Androstendionniveau nach einem Verabreichungszeitraum der pharmazeutischen Zusammensetzung um mindestens etwa 30 % reduziert wird, wobei sich die Reduzierung des Androstendionniveaus auf das Androstendionniveau vor Verabreichung der pharmazeutischen Zusammensetzung bezieht.

**115.**
Pharmazeutische Zusammensetzung zur Verwendung nach einem der Ansprüche 50-112, wobei das Androstendionniveau nach einem Verabreichungszeitraum der pharmazeutischen Zusammensetzung um mindestens etwa 50 % reduziert wird, wobei sich die Reduzierung des Androstendionniveaus auf das Androstendionniveau vor Verabreichung der pharmazeutischen Zusammensetzung bezieht.

**116.**
Pharmazeutische Zusammensetzung zur Verwendung nach einem der Ansprüche 50-112, wobei das Androstendionniveau nach einem Verabreichungszeitraum der pharmazeutischen Zusammensetzung weniger als das 1,5-Fache der normalen Obergrenze beträgt.

**117.**
Pharmazeutische Zusammensetzung zur Verwendung nach einem der Ansprüche 50-112, wobei das Androstendionniveau nach einem Verabreichungszeitraum der pharmazeutischen Zusammensetzung im Normalbereich liegt.

**118.**
Pharmazeutische Zusammensetzung zur Verwendung nach einem der Ansprüche 50-117, wobei das Testosteronniveau nach einem Verabreichungszeitraum der pharmazeutischen Zusammensetzung um mindestens etwa 25 % reduziert wird, wobei sich die Reduzierung des Testosteronniveaus auf das Testosteronniveau vor Verabreichung der pharmazeutischen Zusammensetzung bezieht.

**119.**
Pharmazeutische Zusammensetzung zur Verwendung nach einem der Ansprüche 50-117, wobei das Testosteronniveau nach einem Verabreichungszeitraum der pharmazeutischen Zusammensetzung um mindestens etwa 30 % reduziert wird, wobei sich die Reduzierung des Testosteronniveaus auf das Testosteronniveau vor Verabreichung der pharmazeutischen Zusammensetzung bezieht.

**120.**
Pharmazeutische Zusammensetzung zur Verwendung nach einem der Ansprüche 50-117, wobei das Testosteronniveau nach einem Verabreichungszeitraum der pharmazeutischen Zusammensetzung um mindestens etwa 50 % reduziert wird, wobei sich die Reduzierung des Testosteronniveaus auf das Testosteronniveau vor Verabreichung der pharmazeutischen Zusammensetzung bezieht.

**121.**
Pharmazeutische Zusammensetzung zur Verwendung nach einem der Ansprüche 50-117, wobei das Testosteronniveau nach einem Verabreichungszeitraum der pharmazeutischen Zusammensetzung weniger als das 1,5-Fache der normalen Obergrenze beträgt.

**122.**
Pharmazeutische Zusammensetzung zur Verwendung nach einem der Ansprüche 50-117, wobei das Testosteronniveau nach einem Verabreichungszeitraum der pharmazeutischen Zusammensetzung, die die Verbindung der Formel (I) umfasst, oder eines pharmazeutisch unbedenklichen Salzes davon im Normalbereich liegt.

**123.**
Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung um mindestens etwa 50 % reduziert ist und das Androstendionniveau um mindestens etwa 50 % reduziert wird, wobei sich die Reduzierung des 17-Hydroxyprogesteronniveaus und des Androstendionniveaus auf das 17-Hydroxyprogesteronniveau und das Androstendionniveau vor Verabreichung der pharmazeutischen Zusammensetzung bezieht.

**124.**
Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung weniger als das 1,5-Fache der normalen Obergrenze beträgt und das Androstendionniveau nach einem Verabreichungszeitraum der pharmazeutischen Zusammensetzung weniger als das 1,5-Fache der normalen Obergrenze beträgt.

**125.**
Pharmazeutische Zusammensetzung zur Verwendung nach einem der Ansprüche 50-103, wobei das 17-Hydroxyprogesteronniveau nach einem Verabreichungszeitraum der pharmazeutischen Zusammensetzung im Normalbereich liegt und das Androstendionniveau nach einem Verabreichungszeitraum der pharmazeutischen Zusammensetzung im Normalbereich liegt.

**126.**
Pharmazeutische Zusammensetzung zur Verwendung nach einem der Ansprüche 83-125, wobei das Individuum eine Verkleinerung der Glucocorticoidbelastung nach einem Verabreichungszeitraum der pharmazeutischen Zusammensetzung aufzeigt, wobei sich die Verkleinerung der Glucocorticoidbelastung auf die Glucocorticoidbelastung vor Verabreichung der pharmazeutischen Zusammensetzung bezieht.

**127.**
Pharmazeutische Zusammensetzung zur Verwendung nach Anspruch 126, wobei eines oder mehrere Symptome von Glucocorticoidbelastung, die aus Lebensqualität, Ermüdung, Schlaf, Insulinresistenz, Glucosetoleranz, Glucoseregulierung, Dyslipidämie, Hyperlipidämie, Knochenmineraldichte, Knochenumsatz, Fettmasse, Gewicht, zentralem Übergewicht, Blutdruck, Hirsutismusschweregrad, Menstruationszyklizität und Fruchtbarkeit ausgewählt sind, nach einem Verabreichungszeitraum der pharmazeutischen Zusammensetzung verbessert ist/sind, wobei die Verbesserung des einen oder der mehreren Symptome auf das Status des einen oder der mehreren Symptome vor Verabreichung der pharmazeutischen Zusammensetzung bezieht.

**128.**
Pharmazeutische Zusammensetzung zur Verwendung nach einem der Ansprüche 90-127, wobei der Verabreichungszeitraum mindestens etwa 4 Wochen beträgt.

**129.**
Pharmazeutische Zusammensetzung zur Verwendung nach einem der Ansprüche 90-127, wobei der Verabreichungszeitraum mindestens etwa 24 Wochen beträgt.

**130.**
Pharmazeutische Zusammensetzung zur Verwendung nach einem der Ansprüche 90-127, wobei der Verabreichungszeitraum mindestens etwa ein Jahr beträgt.

**131.**
Pharmazeutische Zusammensetzung zur Verwendung nach einem der Ansprüche 50-130, wobei es sich bei dem

Individuum um ein Kind handelt.

**132.**
Pharmazeutische Zusammensetzung zur Verwendung nach Anspruch 131, wobei das Kind sechs Jahre alt oder jünger ist.

**133.**
Pharmazeutische Zusammensetzung zur Verwendung nach Anspruch 131, wobei das Kind älter als sechs Jahre und jünger als elf Jahre ist.

**134.**
Pharmazeutische Zusammensetzung zur Verwendung nach Anspruch 131, wobei das Kind älter als zehn Jahre und jünger als fünfzehn Jahre ist.

**135.**
Pharmazeutische Zusammensetzung zur Verwendung nach Anspruch 131, wobei das Kind älter als vierzehn Jahre und jünger als neunzehn Jahre ist.

**136.**
Pharmazeutische Zusammensetzung zur Verwendung nach einem der Ansprüche 50-130, wobei es sich bei dem Individuum um ein erwachsenes Individuum handelt.

**137.**
Pharmazeutische Zusammensetzung zur Verwendung nach Anspruch 136, wobei das erwachsene Individuum über achtzehn Jahre alt ist.

**138.**
Pharmazeutische Zusammensetzung zur Verwendung nach einem der Ansprüche 50-137, wobei das Individuum weiblich ist.

**139.**
Pharmazeutische Zusammensetzung zur Verwendung nach einem der Ansprüche 50-137, wobei das Individuum männlich ist.

**140.**
Pharmazeutische Zusammensetzung zur Verwendung nach einem der Ansprüche 50-139, wobei die Verbindung der Formel (I) oder ein pharmazeutisch unbedenkliches Salz davon als Salzsäuresalz oder p-Toluolsulfonsäuresalz verabreicht wird.

**141.**
Pharmazeutische Zusammensetzung zur Verwendung nach einem der Ansprüche 50-139, wobei die Verbindung der Formel (I) oder ein pharmazeutisch unbedenkliches Salz davon als kristallines p-Toluolsulfonsäuresalz verabreicht wird.

## Revendications

**1.** Composition pharmaceutique sous forme posologique de solution orale comprenant :

(a) un composé de formule (I) :

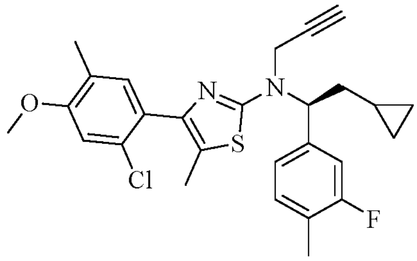

(I)

(4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine), ou un sel pharmaceutiquement acceptable de celle-ci ;
(b) un ou plusieurs parmi un édulcorant, un antioxydant et un arôme ; et
(c) un véhicule liquide, dans laquelle le véhicule liquide est choisi parmi les triglycérides à chaîne moyenne, le dicaprylate/dicaprate de propylène glycol, la glycérine, le propylène glycol, le polyéthylène glycol, l'huile d'olive, l'huile de soja, l'huile de maïs et l'éther monoéthylique de diéthylène glycol.

2. Composition pharmaceutique selon la revendication 1, comprenant environ 1 % p/v à environ 50 % p/v du composé de formule (I), ou d'un sel pharmaceutiquement acceptable de celui-ci, sur la base du poids de la base libre.

3. Composition pharmaceutique selon la revendication 1, comprenant environ 1 % p/v à environ 10 % p/v du composé de formule (I), ou d'un sel pharmaceutiquement acceptable de celui-ci, sur la base du poids de la base libre.

4. Composition pharmaceutique selon la revendication 1, comprenant environ 5 % p/v du composé de formule (I), ou d'un sel pharmaceutiquement acceptable de celui-ci, sur la base du poids de la base libre.

5. Composition pharmaceutique selon l'une quelconque des revendications 1 à 4, comprenant un édulcorant.

6. Composition pharmaceutique selon la revendication 5, comprenant environ 0,01 % p/v à environ 1,5 % p/v de l'édulcorant.

7. Composition pharmaceutique selon la revendication 5, comprenant environ 0,1 % p/v à environ 0,5 % p/v de l'édulcorant.

8. Composition pharmaceutique selon la revendication 5, comprenant environ 0,15 % p/v de l'édulcorant.

9. Composition pharmaceutique selon l'une quelconque des revendications 5 à 8, dans laquelle l'édulcorant est choisi parmi la saccharine, le saccharose, le sucralose, l'aspartame, le dextrose, le fructose, le maltitol, le mannitol, le sorbitol et l'avantame.

10. Composition pharmaceutique selon l'une quelconque des revendications 5 à 8, dans laquelle l'édulcorant est la saccharine.

11. Composition pharmaceutique selon l'une quelconque des revendications 1 à 10, comprenant un antioxydant.

12. Composition pharmaceutique selon la revendication 11, comprenant environ 0,01 % p/v à environ 1,5 % p/v de l'antioxydant.

13. Composition pharmaceutique selon la revendication 11, comprenant environ 0,1 % p/v à environ 0,5 % p/v de l'antioxydant.

14. Composition pharmaceutique selon la revendication 11, comprenant environ 0,17 % p/v de l'antioxydant.

15. Composition pharmaceutique selon l'une quelconque des revendications 11 à 14, dans laquelle l'antioxydant est choisi parmi l'hydroxytoluène butylé, la vitamine E TPGS, l'hydroxyanisole butylé, l'acide ascorbique, la lécithine, la tert-butylhydroquinone et l'acide citrique.

16. Composition pharmaceutique selon l'une quelconque des revendications 11 à 15, dans laquelle l'antioxydant est l'hydroxytoluène butylé.

17. Composition pharmaceutique selon l'une quelconque des revendications 1 à 16, comprenant un arôme.

18. Composition pharmaceutique selon la revendication 17, comprenant environ 0,01 % p/v à environ 0,5 % p/v de l'arôme.

19. Composition pharmaceutique selon la revendication 17, comprenant environ 0,05 % p/v à environ 0,2 % p/v de l'arôme.

20. Composition pharmaceutique selon la revendication 17, comprenant environ 0,10 % p/v de l'arôme.

21. Composition pharmaceutique selon l'une quelconque des revendications 1 à 20, comprenant environ 50 % p/v à environ 99,9 % p/v du véhicule liquide.

22. Composition pharmaceutique selon l'une quelconque des revendications 1 à 20, comprenant environ 90 % p/v à environ 99 % p/v du véhicule liquide.

23. Composition pharmaceutique selon l'une quelconque des revendications 1 à 20, comprenant environ 92 % p/v à environ 97 % p/v du véhicule liquide.

24. Composition pharmaceutique selon l'une quelconque des revendications 1 à 20, comprenant environ 94,6 % p/v du véhicule liquide.

25. Composition pharmaceutique selon l'une quelconque des revendications 1 à 20, comprenant environ 70 % p/v à environ 80 % p/v du véhicule liquide.

26. Composition pharmaceutique selon l'une quelconque des revendications 1 à 20, comprenant environ 75 % p/v du véhicule liquide.

27. Composition pharmaceutique selon l'une quelconque des revendications 1 à 26, dans laquelle le véhicule liquide est des triglycérides à chaîne moyenne.

28. Composition pharmaceutique selon la revendication 27, dans laquelle les triglycérides à chaîne moyenne sont des triglycérides capryliques/capriques.

29. Composition pharmaceutique selon l'une quelconque des revendications 1 à 28, comprenant en outre un tensioactif.

30. Composition pharmaceutique selon la revendication 29, comprenant environ 1 % p/v à environ 50 % p/v du tensioactif.

31. Composition pharmaceutique selon la revendication 29, comprenant environ 10 % p/v à environ 30 % p/v du tensioactif.

32. Composition pharmaceutique selon la revendication 29, comprenant environ 20 % p/v du tensioactif.

33. Composition pharmaceutique selon l'une quelconque des revendications 29 à 32, dans laquelle le tensioactif est choisi parmi les polyoxyl-6 glycérides d'oléyle, les polyoxyl-6 glycérides de linoléoyle, le polysorbate 80, le polysorbate 20, le succinate de polyéthylène glycol vitamine E, les polyoxyl-32 glycérides de lauroyle, le laurylsulfate de sodium, un poloxamère, les esters de PEG-6 d'huile de maïs et les esters de PEG-6 d'huile de palme/palmiste hydrogénée.

34. Composition pharmaceutique selon l'une quelconque des revendications 29 à 33, dans laquelle le tensioactif est des polyoxyl-6 glycérides d'oléyle.

35. Composition pharmaceutique selon la revendication 1, comprenant :

(a) 4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine, ou un sel pharmaceutiquement acceptable de celle-ci ;
(b) un édulcorant ;
(c) un antioxydant ;
(d) un arôme ; et
(e) le véhicule liquide.

**36.** Composition pharmaceutique selon la revendication 35, comprenant en outre un tensioactif.

**37.** Composition pharmaceutique selon la revendication 1, comprenant :

(a) environ 4 % p/v à environ 6 % p/v de 4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine, ou d'un sel pharmaceutiquement acceptable de celle-ci, sur la base du poids de la base libre ;
(b) environ 0,1 % p/v à environ 0,2 % p/v d'un édulcorant ;
(c) environ 0,1 % p/v à environ 0,2 % p/v d'un antioxydant ;
(d) environ 0,05 % p/v à environ 0,2 % p/v d'un arôme ; et
(e) environ 92 % p/v à environ 97 % p/v du véhicule liquide.

**38.** Composition pharmaceutique selon la revendication 1, comprenant :

(a) environ 5 % p/v de 4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine, ou d'un sel pharmaceutiquement acceptable de celle-ci, sur la base du poids de la base libre ;
(b) environ 0,15 % p/v d'un édulcorant ;
(c) environ 0,17 % p/v d'un antioxydant;
(d) environ 0,1 % p/v d'un arôme ; et
(e) environ 94,6 % p/v du véhicule liquide.

**39.** Composition pharmaceutique selon la revendication 1, comprenant :

(a) environ 4 % p/v à environ 6 % p/v de 4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine, ou d'un sel pharmaceutiquement acceptable de celle-ci, sur la base du poids de la base libre ;
(b) environ 0,1 % p/v à environ 0,2 % p/v d'un édulcorant ;
(c) environ 0,1 % p/v à environ 0,2 % p/v d'un antioxydant ;
(d) environ 0,05 % p/v à environ 0,2 % p/v d'un arôme;
(e) environ 15 % p/v à environ 25 % p/v d'un tensioactif ; et
(f) environ 70 % p/v à environ 80 % p/v du véhicule liquide.

**40.** Composition pharmaceutique selon la revendication 1, comprenant :

(a) environ 5 % p/v de 4-(2-chloro-4-méthoxy-5-méthylphényl)-N-[(1S)-2-cyclopropyl-1-(3-fluoro-4-méthylphényl)éthyl]-5-méthyl-N-prop-2-ynyl-1,3-thiazole-2-amine, ou d'un sel pharmaceutiquement acceptable de celle-ci, sur la base du poids de la base libre ;
(b) environ 0,15 % p/v d'un édulcorant ;
(c) environ 0,17 % p/v d'un antioxydant ;
(d) environ 0,1 % p/v d'un arôme ;
(e) environ 20 % p/v d'un tensioactif ; et
(f) environ 75 % p/v du véhicule liquide.

**41.** Composition pharmaceutique selon la revendication 40, dans laquelle

(a) le composé de formule (I) est la base libre ;
(b) l'édulcorant est la saccharine ;
(c) l'antioxydant est l'hydroxytoluène butylé ;
(d) le tensioactif est des polyoxyl-6-glycérides d'oléyle ; et
(e) le véhicule liquide est des triglycérides à chaîne moyenne.

**42.** Composition pharmaceutique selon la revendication 40, dans laquelle les triglycérides à chaîne moyenne sont des triglycérides capryliques/capriques

**43.** Composition pharmaceutique selon l'une quelconque des revendications 1 à 40, comprenant le composé de formule (I) sous forme de base libre.

**44.** Composition pharmaceutique selon l'une quelconque des revendications 1 à 43, formulée sous forme de dosage unitaire, dans laquelle le composé de formule (I), ou un sel pharmaceutiquement acceptable de celui-ci, est présent en une quantité d'environ 5 mg/mL à environ 200 mg/mL, sur la base du poids de la base libre.

**45.** Composition pharmaceutique selon la revendication 44, dans laquelle le composé de formule (I), ou un sel pharmaceutiquement acceptable de celui-ci, est présent en une quantité d'environ 75 mg/mL à environ 150 mg/mL, sur la base du poids de la base libre.

**46.** Composition pharmaceutique selon la revendication 44, dans laquelle le composé de formule (I), ou un sel pharmaceutiquement acceptable de celui-ci, est présent en une quantité d'environ 50 mg/mL, sur la base du poids de la base libre.

**47.** Composition pharmaceutique selon la revendication 44, dans laquelle le composé de formule (I), ou un sel pharmaceutiquement acceptable de celui-ci, est présent en une quantité d'environ 100 mg/mL, sur la base du poids de la base libre.

**48.** Procédé de préparation de la composition pharmaceutique selon l'une quelconque des revendications 1 à 47, comprenant :

(a) mélanger le véhicule liquide avec un édulcorant ;
(b) mélanger le mélange de l'étape (a) avec un antioxydant et un arôme ;
(c) mélanger le composé de formule (I), ou un sel pharmaceutiquement acceptable de celui-ci, avec le mélange de l'étape (b) ; et
(d) mélanger le mélange de l'étape (c) avec une partie supplémentaire du véhicule liquide.

**49.** Procédé selon la revendication 48, dans laquelle l'étape (a) comprend le mélange du véhicule liquide avec un édulcorant et un tensioactif.

**50.** Composition pharmaceutique selon l'une quelconque des revendications 1-47 pour une utilisation dans un procédé de traitement de l'hyperplasie surrénale congénitale (CAH) chez un sujet.

**51.** Composition pharmaceutique destinée à être utilisée selon la revendication 50, dans laquelle l'hyperplasie surrénale congénitale (CAH) est une CAH classique.

**52.** Composition pharmaceutique destinée à être utilisée selon la revendication 50 ou 51, dans laquelle le sujet est dans un état nourri.

**53.** Composition pharmaceutique destinée à être utilisée selon l'une quelconque des revendications 50 à 52, dans laquelle on administre au sujet la composition pharmaceutique avec une composition nutritionnelle.

**54.** Composition pharmaceutique destinée à être utilisée selon la revendication 53, dans laquelle la composition nutritionnelle est un complément alimentaire liquide comprenant environ 1 000 à environ 2 000 calories par litre avec une teneur en graisse supérieure à environ 30 %.

**55.** Composition pharmaceutique destinée à être utilisée selon la revendication 53, dans laquelle la composition nutritionnelle est un complément alimentaire liquide comprenant 1)500 calories par litre avec une distribution calorique de 14,7 % de protéines, 32 % de graisses et 53 % de glucides.

**56.** Composition pharmaceutique destinée à être utilisée selon l'une quelconque des revendications 53 à 55, dans laquelle la composition nutritionnelle est administrée en une quantité d'environ 6 à environ 12 onces liquides.

**57.** Composition pharmaceutique destinée à être utilisée selon l'une quelconque des revendications 53 à 55, dans

laquelle la composition nutritionnelle est administrée en une quantité d'environ 8 onces liquides.

**58.** Composition pharmaceutique destinée à être utilisée selon l'une quelconque des revendications 53 à 57, dans laquelle la composition nutritionnelle est administrée dans les 30 minutes suivant l'administration de la composition pharmaceutique.

**59.** Composition pharmaceutique destinée à être utilisée selon l'une quelconque des revendications 52 à 58, dans laquelle l'administration de la composition pharmaceutique présente un effet alimentaire positif.

**60.** Composition pharmaceutique destinée à être utilisée selon l'une quelconque des revendications 50 à 59, dans laquelle le sujet est un sujet humain.

**61.** Composition pharmaceutique destinée à être utilisée selon l'une quelconque des revendications 52 à 58, dans laquelle la composition pharmaceutique présente un effet alimentaire positif lorsqu'elle est administrée par voie orale.

**62.** Composition pharmaceutique destinée à être utilisée selon l'une quelconque des revendications 50 à 61, dans laquelle la composition pharmaceutique est administrée au sujet avec un repas.

**63.** Composition pharmaceutique destinée à être utilisée selon la revendication 62, dans laquelle le repas est un repas riche en graisses.

**64.** Composition pharmaceutique destinée à être utilisée selon la revendication 62, dans laquelle le repas est un repas à faible teneur en graisses.

**65.** Composition pharmaceutique destinée à être utilisée selon l'une quelconque des revendications 62 à 64, dans laquelle la composition pharmaceutique est administrée dans les 5 minutes environ après le début du repas.

**66.** Composition pharmaceutique destinée à être utilisée selon l'une quelconque des revendications 62 à 64, dans laquelle le repas est un repas du soir.

**67.** Composition pharmaceutique destinée à être utilisée selon l'une quelconque des revendications 62 à 64, dans laquelle le repas est un repas du matin.

**68.** Composition pharmaceutique destinée à être utilisée selon l'une quelconque des revendications 62 à 67, dans laquelle l'administration de la composition pharmaceutique présente un effet alimentaire positif.

**69.** Composition pharmaceutique destinée à être utilisée selon l'une quelconque des revendications 50 à 68, dans laquelle la composition pharmaceutique comprend environ 25 mg du composé de formule (I), ou d'un sel pharmaceutiquement acceptable de celui-ci, sur la base du poids de la base libre.

**70.** Composition pharmaceutique destinée à être utilisée selon l'une quelconque des revendications 50 à 68, dans laquelle la composition pharmaceutique comprend environ 50 mg du composé de formule (I), ou d'un sel pharmaceutiquement acceptable de celui-ci, sur la base du poids de la base libre.

**71.** Composition pharmaceutique destinée à être utilisée selon l'une quelconque des revendications 50 à 68, dans laquelle la composition pharmaceutique comprend environ 75 mg du composé de formule (I), ou d'un sel pharmaceutiquement acceptable de celui-ci, sur la base du poids de la base libre.

**72.** Composition pharmaceutique destinée à être utilisée selon l'une quelconque des revendications 50 à 68, dans laquelle la composition pharmaceutique comprend environ 100 mg du composé de formule (I), ou d'un sel pharmaceutiquement acceptable de celui-ci, sur la base du poids de la base libre.

**73.** Composition pharmaceutique destinée à être utilisée selon l'une quelconque des revendications 50 à 68, dans laquelle le procédé comprend l'administration d'une dose quotidienne de la composition pharmaceutique comprenant environ 25 mg du composé de formule (I), ou d'un sel pharmaceutiquement acceptable de celui-ci, sur la base du poids de la base libre.

**74.** Composition pharmaceutique destinée à être utilisée selon l'une quelconque des revendications 50 à 68, dans

laquelle le procédé comprend l'administration d'une dose quotidienne de la composition pharmaceutique comprenant environ 50 mg du composé de formule (I), ou d'un sel pharmaceutiquement acceptable de celui-ci, sur la base du poids de la base libre.

**75.** Composition pharmaceutique destinée à être utilisée selon l'une quelconque des revendications 50 à 68, dans laquelle le procédé comprend l'administration d'une dose quotidienne de la composition pharmaceutique comprenant environ 75 mg du composé de formule (I), ou d'un sel pharmaceutiquement acceptable de celui-ci, sur la base du poids de la base libre.

**76.** Composition pharmaceutique destinée à être utilisée selon l'une quelconque des revendications 50 à 68, dans laquelle le procédé comprend l'administration d'une dose quotidienne de la composition pharmaceutique comprenant environ 100 mg du composé de formule (I), ou d'un sel pharmaceutiquement acceptable de celui-ci, sur la base du poids de la base libre.

**77.** Composition pharmaceutique destinée à être utilisée selon l'une quelconque des revendications 50 à 68, dans laquelle le procédé comprend l'administration d'une dose quotidienne de la composition pharmaceutique comprenant environ 150 mg du composé de formule (I), ou d'un sel pharmaceutiquement acceptable de celui-ci, sur la base du poids de la base libre.

**78.** Composition pharmaceutique destinée à être utilisée selon l'une quelconque des revendications 50 à 68, dans laquelle le procédé comprend l'administration d'une dose quotidienne de la composition pharmaceutique comprenant environ 200 mg du composé de formule (I), ou d'un sel pharmaceutiquement acceptable de celui-ci, sur la base du poids de la base libre.

**79.** Composition pharmaceutique destinée à être utilisée selon l'une quelconque des revendications 50 à 68, dans laquelle le procédé comprend l'administration de la composition pharmaceutique deux fois par jour à une dose d'environ 25 mg du composé de formule (I), ou d'un sel pharmaceutiquement acceptable de celui-ci, sur la base du poids de la base libre.

**80.** Composition pharmaceutique destinée à être utilisée selon l'une quelconque des revendications 50 à 68, dans laquelle le procédé comprend l'administration de la composition pharmaceutique deux fois par jour à une dose d'environ 50 mg du composé de formule (I), ou d'un sel pharmaceutiquement acceptable de celui-ci, sur la base du poids de la base libre.

**81.** Composition pharmaceutique destinée à être utilisée selon l'une quelconque des revendications 50 à 68, dans laquelle le procédé comprend l'administration de la composition pharmaceutique deux fois par jour à une dose d'environ 75 mg du composé de formule (I), ou d'un sel pharmaceutiquement acceptable de celui-ci, sur la base du poids de la base libre.

**82.** Composition pharmaceutique destinée à être utilisée selon l'une quelconque des revendications 50 à 68, dans laquelle le procédé comprend l'administration de la composition pharmaceutique deux fois par jour à une dose d'environ 100 mg du composé de formule (I), ou d'un sel pharmaceutiquement acceptable de celui-ci, sur la base du poids de la base libre.

**83.** Composition pharmaceutique destinée à être utilisée selon l'une quelconque des revendications 50 à 82, dans laquelle le sujet reçoit simultanément une dose d'un glucocorticoïde.

**84.** Composition pharmaceutique destinée à être utilisée selon la revendication 83, dans laquelle le glucocorticoïde est choisi parmi le cortisol (hydrocortisone), la cortisone, la prednisone, la prednisolone, la méthylprednisolone, la dexaméthasone, la bétaméthasone, la triamcinolone, l'acétate de fludrocortisone et l'acétate de désoxycorticostérone.

**85.** Composition pharmaceutique destinée à être utilisée selon la revendication 83 ou 84, dans laquelle le glucocorticoïde est le cortisol (hydrocortisone).

**86.** Composition pharmaceutique destinée à être utilisée selon la revendication 83 ou 84, dans laquelle le glucocorticoïde est la cortisone.

**87.** Composition pharmaceutique destinée à être utilisée selon la revendication 83 ou 84, dans laquelle le glucocorticoïde est la prednisone.

**88.** Composition pharmaceutique destinée à être utilisée selon l'une quelconque des revendications 83 à 87, dans laquelle la dose de glucocorticoïdes est mesurée en équivalents d'hydrocortisone.

**89.** Composition pharmaceutique destinée à être utilisée selon l'une quelconque des revendications 83 à 88, dans laquelle la dose de glucocorticoïdes est mesurée comme un multiple de la limite supérieure de la normale du dosage physiologique en équivalents d'hydrocortisone.

**90.** Composition pharmaceutique destinée à être utilisée selon l'une quelconque des revendications 83 à 89, dans laquelle la dose de glucocorticoïdes est une dose physiologique telle que mesurée après une période de temps d'administration de la composition pharmaceutique.

**91.** Composition pharmaceutique destinée à être utilisée selon l'une quelconque des revendications 83 à 89, dans laquelle la dose de glucocorticoïdes est une dose physiologique d'environ 4 à environ 12 $mg/m^2$/jour telle que mesurée après une période de temps d'administration de la composition pharmaceutique.

**92.** Composition pharmaceutique destinée à être utilisée selon l'une quelconque des revendications 83 à 89, dans laquelle la dose de glucocorticoïdes est une dose physiologique d'environ 4 à environ 9 $mg/m^2$/jour telle que mesurée après une période de temps d'administration de la composition pharmaceutique.

**93.** Composition pharmaceutique destinée à être utilisée selon l'une quelconque des revendications 83 à 89, dans laquelle la dose de glucocorticoïdes est une dose physiologique qui est inférieure à environ 8 $mg/m^2$/jour telle que mesurée après une période de temps d'administration de la composition pharmaceutique.

**94.** Composition pharmaceutique destinée à être utilisée selon l'une quelconque des revendications 83 à 93, dans laquelle la dose de glucocorticoïdes du sujet est réduite d'environ 10 % après une période de temps d'administration de la composition pharmaceutique, dans laquelle la réduction de la dose de glucocorticoïdes est relative à la dose de glucocorticoïdes avant l'administration de la composition pharmaceutique.

**95.** Composition pharmaceutique destinée à être utilisée selon l'une quelconque des revendications 83 à 93, dans laquelle la dose de glucocorticoïdes du sujet est réduite d'environ 20 % après une période de temps d'administration de la composition pharmaceutique, dans laquelle la réduction de la dose de glucocorticoïdes est relative à la dose de glucocorticoïdes avant l'administration de la composition pharmaceutique.

**96.** Composition pharmaceutique destinée à être utilisée selon l'une quelconque des revendications 83 à 93, dans laquelle la dose de glucocorticoïdes du sujet est réduite d'environ 30 % après une période de temps d'administration de la composition pharmaceutique, dans laquelle la réduction de la dose de glucocorticoïdes est relative à la dose de glucocorticoïdes avant l'administration de la composition pharmaceutique.

**97.** Composition pharmaceutique destinée à être utilisée selon l'une quelconque des revendications 83 à 93, dans laquelle la dose de glucocorticoïdes du sujet est réduite d'environ 40 % après une période de temps d'administration de la composition pharmaceutique, dans laquelle la réduction de la dose de glucocorticoïdes est relative à la dose de glucocorticoïdes avant l'administration de la composition pharmaceutique.

**98.** Composition pharmaceutique destinée à être utilisée selon l'une quelconque des revendications 83 à 93, dans laquelle la dose de glucocorticoïdes du sujet est réduite d'environ 50 % après une période de temps d'administration de la composition pharmaceutique, dans laquelle la réduction de la dose de glucocorticoïdes est relative à la dose de glucocorticoïdes avant l'administration de la composition pharmaceutique.

**99.** Composition pharmaceutique destinée à être utilisée selon l'une quelconque des revendications 83 à 93, dans laquelle la dose de glucocorticoïdes du sujet est réduite d'environ 60 % après une période de temps d'administration de la composition pharmaceutique, dans laquelle la réduction de la dose de glucocorticoïdes est relative à la dose de glucocorticoïdes avant l'administration de la composition pharmaceutique.

**100.**
Composition pharmaceutique destinée à être utilisée selon l'une quelconque des revendications 83 à 93, dans laquelle la

dose de glucocorticoïdes du sujet est réduite d'environ 70 % après une période de temps d'administration de la composition pharmaceutique, dans laquelle la réduction de la dose de glucocorticoïdes est relative à la dose de glucocorticoïdes avant l'administration de la composition pharmaceutique.

**101.**
Composition pharmaceutique destinée à être utilisée selon l'une quelconque des revendications 83 à 93, dans laquelle la dose de glucocorticoïdes du sujet est réduite de moins d'environ 20 % après une période de temps d'administration de la composition pharmaceutique, dans laquelle la réduction de la dose de glucocorticoïdes est relative à la dose de glucocorticoïdes avant l'administration de la composition pharmaceutique.

**102.**
Composition pharmaceutique destinée à être utilisée selon l'une quelconque des revendications 83 à 93, dans laquelle la dose de glucocorticoïdes du sujet est réduite d'environ 20 % à environ 50 % après une période de temps d'administration de la composition pharmaceutique, dans laquelle la réduction de la dose de glucocorticoïdes est relative à la dose de glucocorticoïdes avant l'administration de la composition pharmaceutique.

**103.**
Composition pharmaceutique destinée à être utilisée selon l'une quelconque des revendications 83 à 93, dans laquelle la dose de glucocorticoïdes du sujet est réduite de plus d'environ 50 % après une période de temps d'administration de la composition pharmaceutique, dans laquelle la réduction de la dose de glucocorticoïdes est relative à la dose de glucocorticoïdes avant l'administration de la composition pharmaceutique.

**104.**
Composition pharmaceutique destinée à être utilisée selon l'une quelconque des revendications 50 à 103, dans laquelle le taux d'hydroxyprogestérone est réduit d'au moins environ 25 % après une période de temps d'administration de la composition pharmaceutique, dans laquelle la réduction du taux de 17-hydroxyprogestérone est relative au taux de 17-hydroxyprogestérone avant l'administration de la composition pharmaceutique.

**105.**
Composition pharmaceutique destinée à être utilisée selon l'une quelconque des revendications 50 à 103, dans laquelle le taux de 17-hydroxyprogestérone est réduit d'au moins environ 50 % après une période de temps d'administration de la composition pharmaceutique, dans laquelle la réduction du taux de 17-hydroxyprogestérone est relative au taux de 17-hydroxyprogestérone avant l'administration de la composition pharmaceutique.

**106.**
Composition pharmaceutique destinée à être utilisée selon l'une quelconque des revendications 50 à 103, dans laquelle le taux de 17-hydroxyprogestérone est inférieur à environ 1,5 fois la limite supérieure de la normale après une période de temps d'administration de la composition pharmaceutique.

**107.**
Composition pharmaceutique destinée à être utilisée selon l'une quelconque des revendications 50 à 103, dans laquelle le taux de 17-hydroxyprogestérone est dans les limites normales après une période de temps d'administration de la composition pharmaceutique.

**108.**
Composition pharmaceutique destinée à être utilisée selon l'une quelconque des revendications 50 à 107, dans laquelle le taux d'hormone adrénocorticotrope est réduit d'au moins environ 25 % après une période de temps d'administration de la composition pharmaceutique, dans laquelle la réduction du taux d'hormone adrénocorticotrope est relative au taux d'hormone adrénocorticotrope avant l'administration de la composition pharmaceutique.

**109.**
Composition pharmaceutique destinée à être utilisée selon l'une quelconque des revendications 50 à 107, dans laquelle le taux d'hormone adrénocorticotrope est réduit d'au moins environ 40 % après une période de temps d'administration de la composition pharmaceutique, dans laquelle la réduction du taux d'hormone adrénocorticotrope est relative au taux d'hormone adrénocorticotrope avant l'administration de la composition pharmaceutique.

**110.**
Composition pharmaceutique destinée à être utilisée selon l'une quelconque des revendications 50 à 107, dans laquelle le

taux d'hormone adrénocorticotrope est réduit d'au moins environ 50 % après une période de temps d'administration de la composition pharmaceutique, dans laquelle la réduction du taux d'hormone adrénocorticotrope est relative au taux d'hormone adrénocorticotrope avant l'administration de la composition pharmaceutique.

**111.** Composition pharmaceutique destinée à être utilisée selon l'une quelconque des revendications 50 à 107, dans laquelle le taux d'hormone adrénocorticotrope est inférieur à environ 1,5 fois la limite supérieure de la normale après une période de temps d'administration de la composition pharmaceutique.

**112.**
Composition pharmaceutique destinée à être utilisée selon l'une quelconque des revendications 50 à 103, dans laquelle le taux d'hormone adrénocorticotrope est dans les limites normales après une période de temps d'administration de la composition pharmaceutique.

**113.**
Composition pharmaceutique destinée à être utilisée selon l'une quelconque des revendications 50 à 112, dans laquelle le taux d'androstènedione est réduit d'au moins environ 25 % après une période de temps d'administration de la composition pharmaceutique, dans laquelle la réduction du taux d'androstènedione est relative au taux d'androstènedione avant l'administration de la composition pharmaceutique.

**114.**
Composition pharmaceutique destinée à être utilisée selon l'une quelconque des revendications 50 à 112, dans laquelle le taux d'androstènedione est réduit d'au moins environ 30 % après une période de temps d'administration de la composition pharmaceutique, dans laquelle la réduction du taux d'androstènedione est relative au taux d'androstènedione avant l'administration de la composition pharmaceutique.

**115.**
Composition pharmaceutique destinée à être utilisée selon l'une quelconque des revendications 50 à 112, dans laquelle le taux d'androstènedione est réduit d'au moins environ 50 % après une période de temps d'administration de la composition pharmaceutique, dans laquelle la réduction du taux d'androstènedione est relative au taux d'androstènedione avant l'administration de la composition pharmaceutique.

**116.**
Composition pharmaceutique destinée à être utilisée selon l'une quelconque des revendications 50 à 112, dans laquelle le taux d'androstènedione est inférieur à environ 1,5 fois la limite supérieure de la normale après une période de temps d'administration de la composition pharmaceutique.

**117.**
Composition pharmaceutique destinée à être utilisée selon l'une quelconque des revendications 50 à 112, dans laquelle le taux d'androstènedione est dans les limites normales après une période de temps d'administration de la composition pharmaceutique.

**118.**
Composition pharmaceutique destinée à être utilisée selon l'une quelconque des revendications 50 à 117, dans laquelle le taux de testostérone est réduit d'au moins environ 25 % après une période de temps d'administration de la composition pharmaceutique, dans laquelle la réduction du taux de testostérone est relative au taux de testostérone avant l'administration de la composition pharmaceutique.

**119.**
Composition pharmaceutique destinée à être utilisée selon l'une quelconque des revendications 50 à 117, dans laquelle le taux de testostérone est réduit d'au moins environ 30 % après une période de temps d'administration de la composition pharmaceutique, dans laquelle la réduction du taux de testostérone est relative au taux de testostérone avant l'administration de la composition pharmaceutique.

**120.**
Composition pharmaceutique destinée à être utilisée selon l'une quelconque des revendications 50 à 117, dans laquelle le taux de testostérone est réduit d'au moins environ 50 % après une période de temps d'administration de la composition pharmaceutique, dans laquelle la réduction du taux de testostérone est relative au taux de testostérone avant l'administration de la composition pharmaceutique.

**121.**
Composition pharmaceutique destinée à être utilisée selon l'une quelconque des revendications 50 à 117, dans laquelle le taux de testostérone est inférieur à environ 1,5 fois la limite supérieure de la normale après une période de temps d'administration de la composition pharmaceutique.

**122.**
Composition pharmaceutique destinée à être utilisée selon l'une quelconque des revendications 50 à 117, dans laquelle le taux de testostérone est dans les limites normales après une période de temps d'administration de la composition pharmaceutique comprenant le composé de formule (I), ou un sel pharmaceutiquement acceptable de celui-ci.

**123.**
Composition pharmaceutique destinée à être utilisée selon l'une quelconque des revendications 50 à 103, dans laquelle le taux de 17-hydroxyprogestérone est réduit d'au moins environ 50 % et le taux d'androstènedione est réduit d'au moins environ 50 % après une période de temps d'administration de la composition pharmaceutique, dans laquelle la réduction du taux de 17-hydroxyprogestérone et du taux d'androstènedione est relative au taux de 17-hydroxyprogestérone et au taux d'androstènedione avant l'administration de la composition pharmaceutique.

**124.**
Composition pharmaceutique destinée à être utilisée selon l'une quelconque des revendications 50 à 103, dans laquelle le taux de 17-hydroxyprogestérone est inférieur à environ 1,5 fois la limite supérieure de la normale et le taux d'androstènedione est inférieur à environ 1,5 fois la limite supérieure de la normale après une période de temps d'administration de la composition pharmaceutique.

**125.**
Composition pharmaceutique destinée à être utilisée selon l'une quelconque des revendications 50 à 103, dans laquelle le taux de 17-hydroxyprogestérone est dans les limites normales et le taux d'androstènedione est dans les limites normales après une période de temps d'administration de la composition pharmaceutique.

**126.**
Composition pharmaceutique destinée à être utilisée selon l'une quelconque des revendications 83 à 125, dans laquelle le sujet présente une diminution de la charge de glucocorticoïdes après une période de temps d'administration de la composition pharmaceutique, dans laquelle la diminution de la charge de glucocorticoïdes est relative à la charge de glucocorticoïdes avant l'administration de la composition pharmaceutique.

**127.**
Composition pharmaceutique destinée à être utilisée selon la revendication 126, dans laquelle un ou plusieurs symptômes de la charge de glucocorticoïdes choisis parmi la qualité de vie, la fatigue, le sommeil, la résistance à l'insuline, la tolérance au glucose, le contrôle de la glycémie, la dyslipidémie, l'hyperlipidémie, la densité minérale osseuse, le renouvellement osseux, la masse grasse, le poids, l'obésité centrale, la pression artérielle, la gravité de l'hirsutisme, le cycle menstruel et la fertilité, sont améliorés après une période d'administration de la composition pharmaceutique, l'amélioration du ou des symptômes étant relative à l'état du ou des symptômes avant l'administration de la composition pharmaceutique.

**128.**
Composition pharmaceutique destinée à être utilisée selon l'une quelconque des revendications 90 à 127, dans laquelle la période d'administration est d'au moins environ 4 semaines.

**129.**
Composition pharmaceutique destinée à être utilisée selon l'une quelconque des revendications 90 à 127, dans laquelle la période d'administration est d'au moins environ 24 semaines.

**130.**
Composition pharmaceutique destinée à être utilisée selon l'une quelconque des revendications 90 à 127, dans laquelle la période d'administration est d'au moins environ un an.

**131.**
Composition pharmaceutique destinée à être utilisée selon l'une quelconque des revendications 50 à 130, dans laquelle le sujet est un sujet pédiatrique.

**132.**
Composition pharmaceutique destinée à être utilisée selon la revendication 131, dans laquelle le sujet pédiatrique est d'un âge de moins de ou égal à six ans.

**133.**
Composition pharmaceutique destinée à être utilisée selon la revendication 131, dans laquelle le sujet pédiatrique est d'un âge de plus de six ans et de moins de onze ans.

**134.**
Composition pharmaceutique destinée à être utilisée selon la revendication 131, dans laquelle le sujet pédiatrique est d'un âge de plus de dix ans et de moins de quinze ans.

**135.**
Composition pharmaceutique destinée à être utilisée selon la revendication 131, dans laquelle le sujet pédiatrique est d'un âge de plus de quatorze ans et de moins de dix-neuf ans.

**136.**
Composition pharmaceutique destinée à être utilisée selon l'une quelconque des revendications 50 à 130, dans laquelle le sujet est un sujet adulte.

**137.**
Composition pharmaceutique destinée à être utilisée selon la revendication 136, dans laquelle le sujet adulte est d'un âge de plus de dix-huit ans.

**138.**
Composition pharmaceutique destinée à être utilisée selon l'une quelconque des revendications 50 à 137, dans laquelle le sujet est une femme.

**139.**
Composition pharmaceutique destinée à être utilisée selon l'une quelconque des revendications 50 à 137, dans laquelle le sujet est un homme.

**140.**
Composition pharmaceutique destinée à être utilisée selon l'une quelconque des revendications 50 à 139, dans laquelle le composé de formule (I), ou un sel pharmaceutiquement acceptable de celui-ci, est administré sous la forme d'un sel d'acide chlorhydrique ou d'un sel d'acide p-toluènesulfonique.

**141.**
Composition pharmaceutique destinée à être utilisée selon l'une quelconque des revendications 50 à 139, dans laquelle le composé de formule (I), ou un sel pharmaceutiquement acceptable de celui-ci, est administré sous la forme d'un sel cristallin d'acide p-toluènesulfonique.

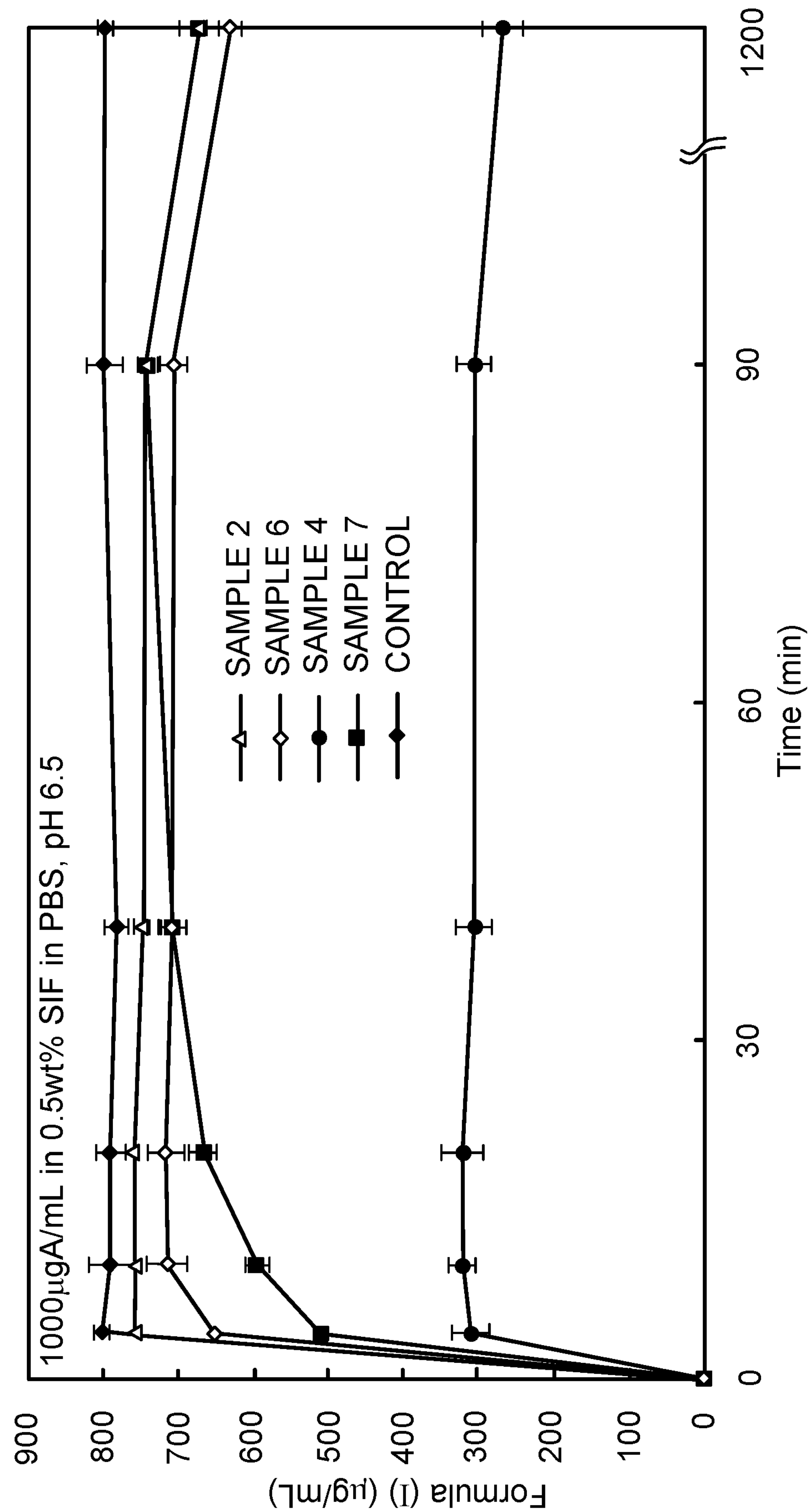
1000µgA/mL in 0.5wt% SIF in PBS, pH 6.5
SAMPLE 2
SAMPLE 6
SAMPLE 4
SAMPLE 7
CONTROL
Formula (I) (µg/mL)
0
100
200
300
400
500
600
700
800
900
0
30
60
90
1200
Time (min)

FIG. 1

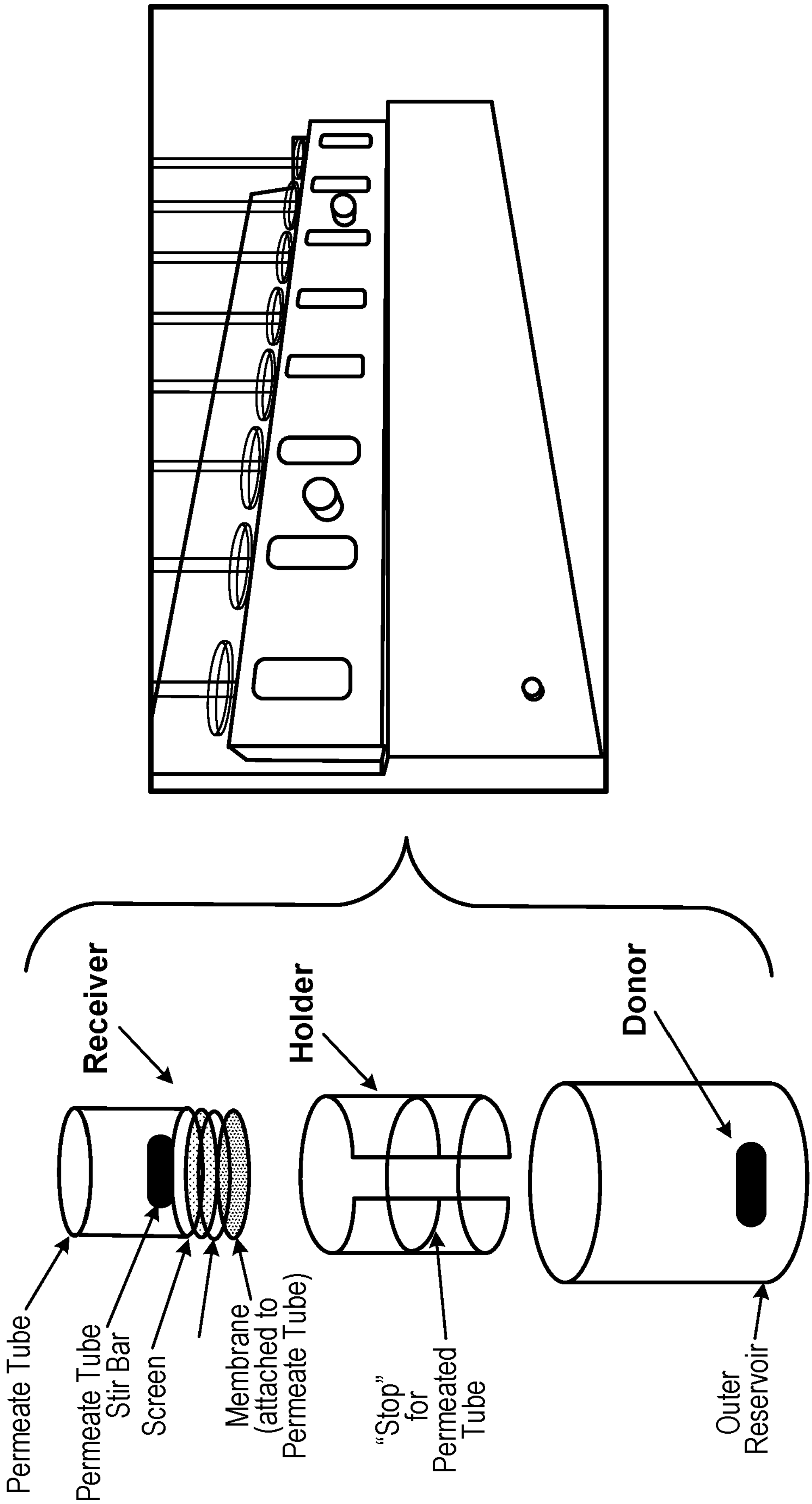
Permeate Tube
Permeate Tube Stir Bar
Screen
Membrane (attached to Permeate Tube)
Receiver
Holder
"Stop" for Permeated Tube
Donor
Outer Reservoir

FIG. 2

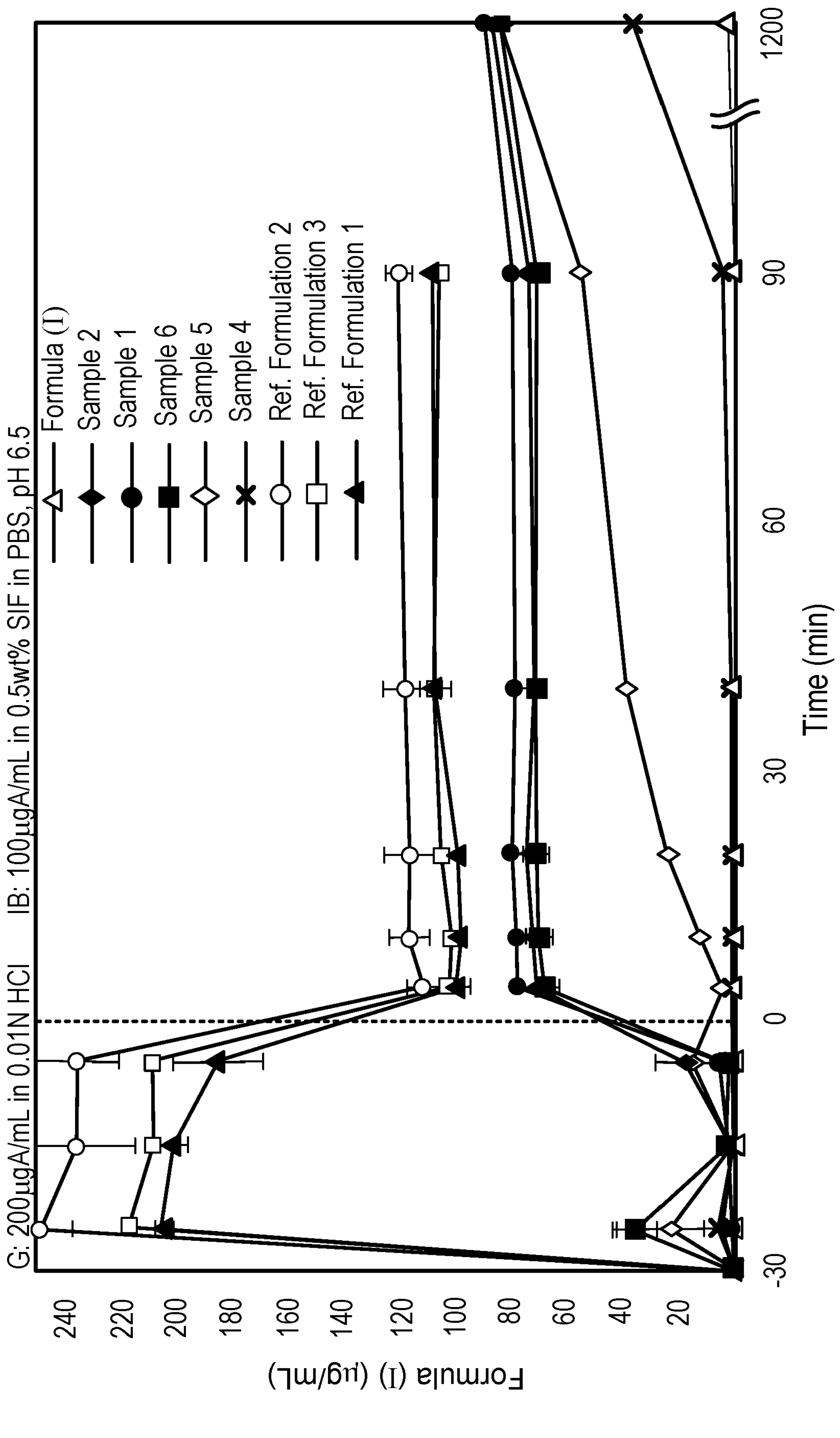
G: 200μgA/mL in 0.01N HCl
IB: 100μgA/mL in 0.5wt% SIF in PBS, pH 6.5
Formula (I)
Sample 2
Sample 1
Sample 6
Sample 5
Sample 4
Ref. Formulation 2
Ref. Formulation 3
Ref. Formulation 1
Formula (I) (μg/mL)
240
220
200
180
160
140
120
100
80
60
40
20
-30
0
30
60
90
1200
Time (min)

FIG. 3

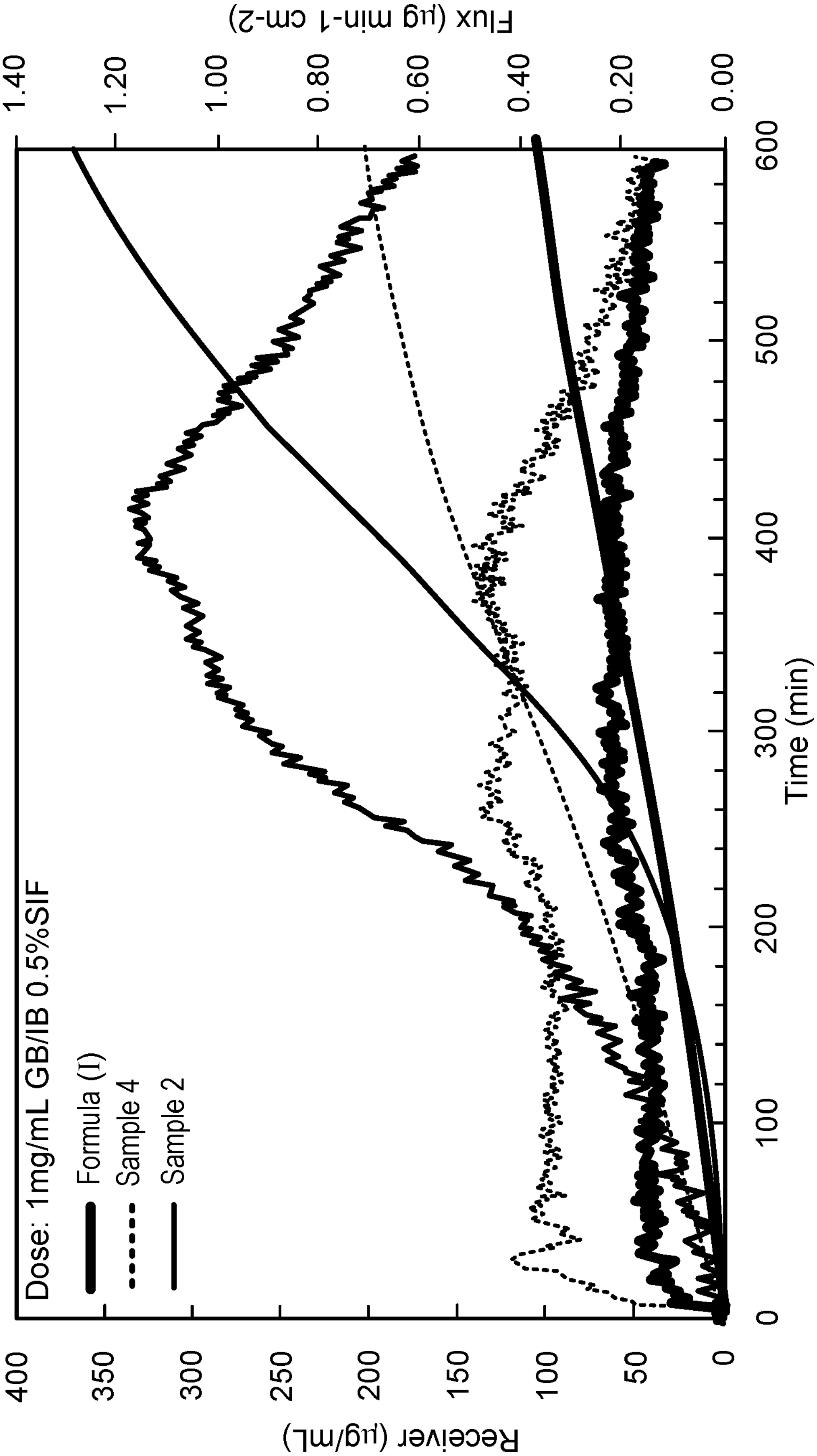
Dose: 1mg/mL GB/IB 0.5%SIF
Formula (I)
Sample 4
Sample 2
Receiver (µg/mL)
0
50
100
150
200
250
300
350
400
Flux (µg min-1 cm-2)
0.00
0.20
0.40
0.60
0.80
1.00
1.20
1.40
Time (min)
0
100
200
300
400
500
600

FIG. 4

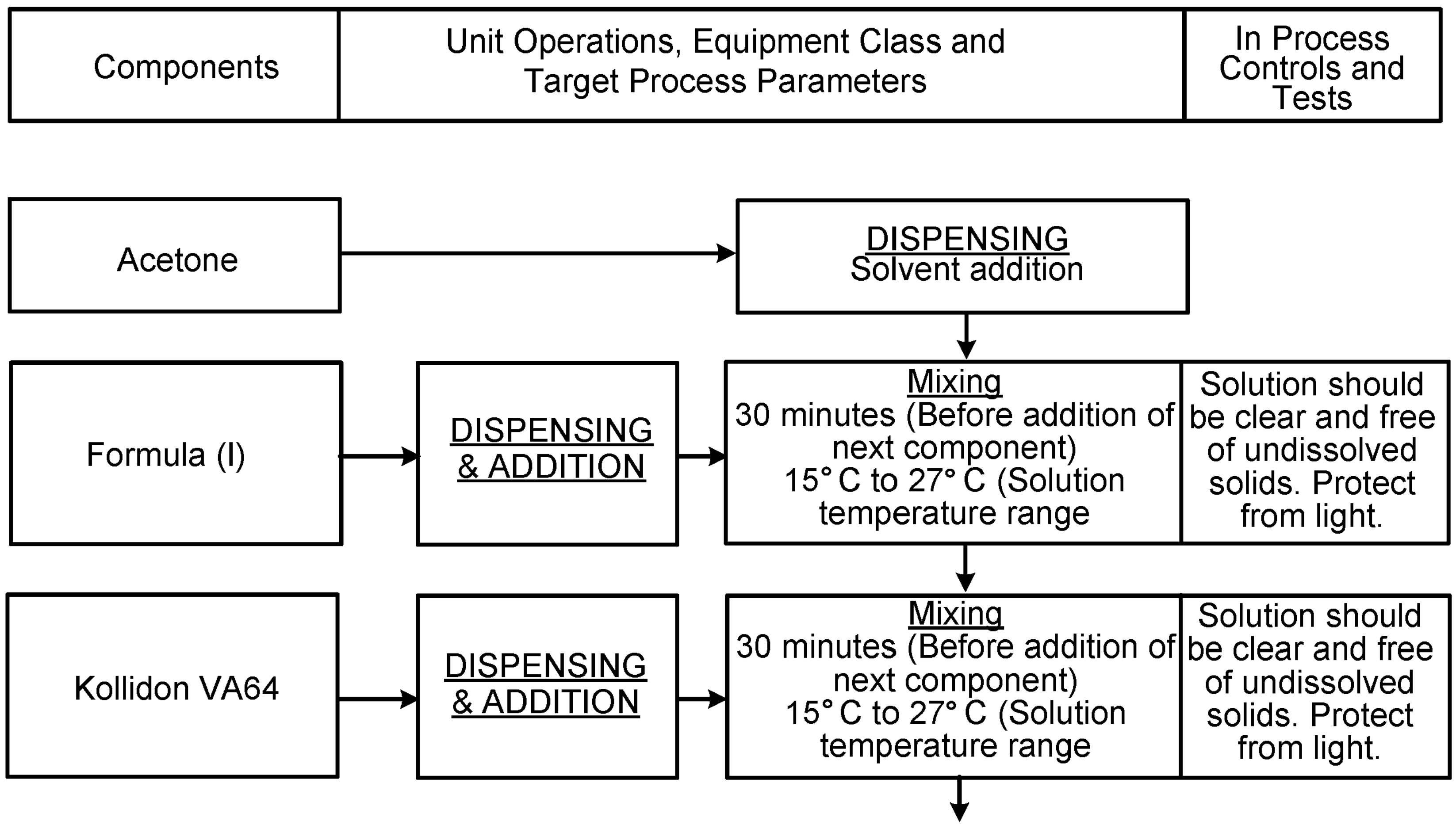
Components
Unit Operations, Equipment Class and Target Process Parameters
In Process Controls and Tests
Acetone
DISPENSING
Solvent addition
Formula (I)
DISPENSING & ADDITION
Mixing
30 minutes (Before addition of next component)
15° C to 27° C (Solution temperature range
Solution should be clear and free of undissolved solids. Protect from light.
Kollidon VA64
DISPENSING & ADDITION
Mixing
30 minutes (Before addition of next component)
15° C to 27° C (Solution temperature range
Solution should be clear and free of undissolved solids. Protect from light.

FIG. 5

FIG. 5 (cont)

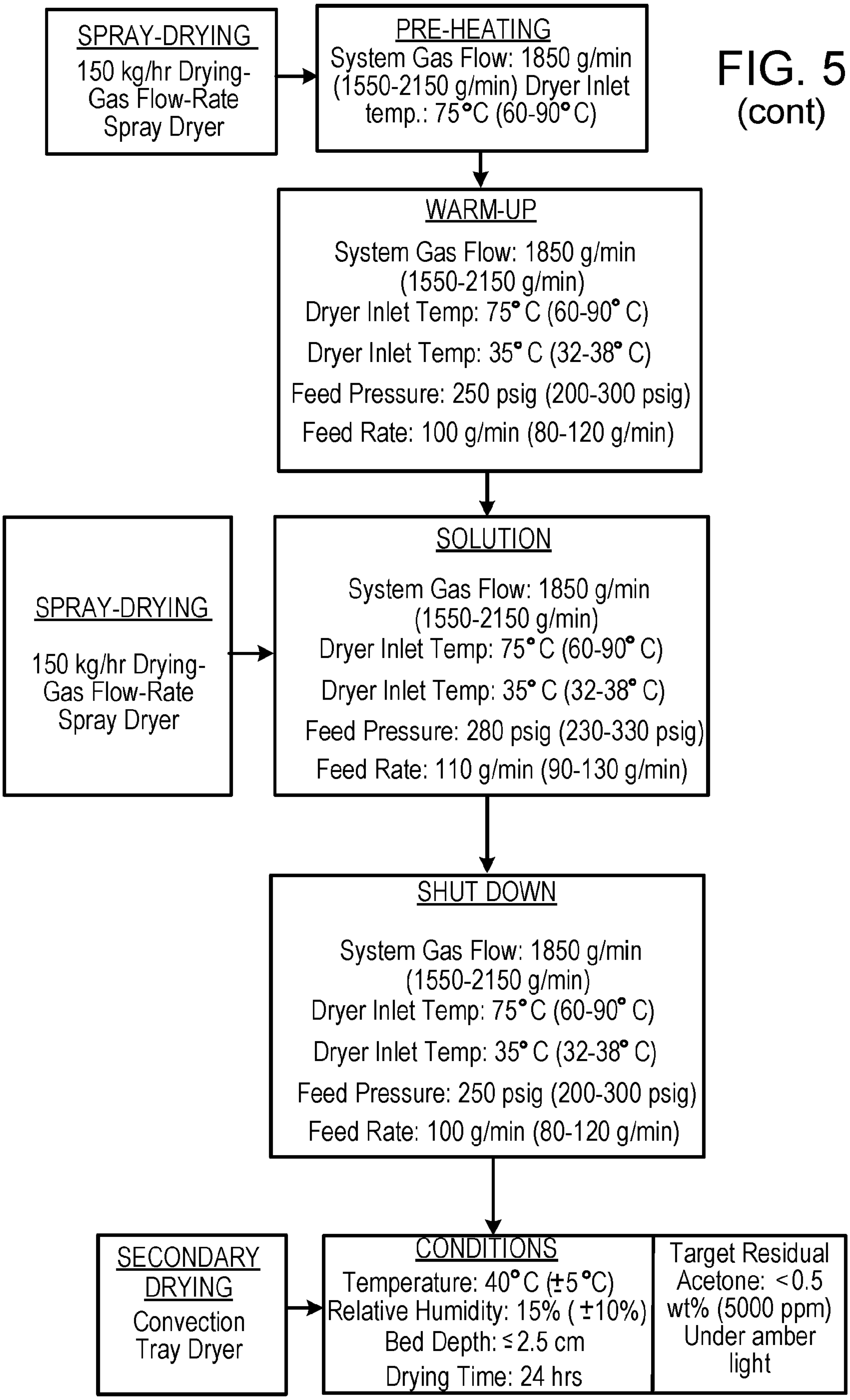
SPRAY-DRYING
150 kg/hr Drying-Gas Flow-Rate Spray Dryer
PRE-HEATING
System Gas Flow: 1850 g/min (1550-2150 g/min) Dryer Inlet temp.: 75°C (60-90°C)
WARM-UP
System Gas Flow: 1850 g/min (1550-2150 g/min)
Dryer Inlet Temp: 75° C (60-90° C)
Dryer Inlet Temp: 35° C (32-38° C)
Feed Pressure: 250 psig (200-300 psig)
Feed Rate: 100 g/min (80-120 g/min)
SPRAY-DRYING
150 kg/hr Drying-Gas Flow-Rate Spray Dryer
SOLUTION
System Gas Flow: 1850 g/min (1550-2150 g/min)
Dryer Inlet Temp: 75° C (60-90° C)
Dryer Inlet Temp: 35° C (32-38° C)
Feed Pressure: 280 psig (230-330 psig)
Feed Rate: 110 g/min (90-130 g/min)
SHUT DOWN
System Gas Flow: 1850 g/min (1550-2150 g/min)
Dryer Inlet Temp: 75° C (60-90° C)
Dryer Inlet Temp: 35° C (32-38° C)
Feed Pressure: 250 psig (200-300 psig)
Feed Rate: 100 g/min (80-120 g/min)
SECONDARY DRYING
Convection Tray Dryer
CONDITIONS
Temperature: 40°C (±5°C)
Relative Humidity: 15% (±10%)
Bed Depth: ≤2.5 cm
Drying Time: 24 hrs
Target Residual Acetone: <0.5 wt% (5000 ppm) Under amber light

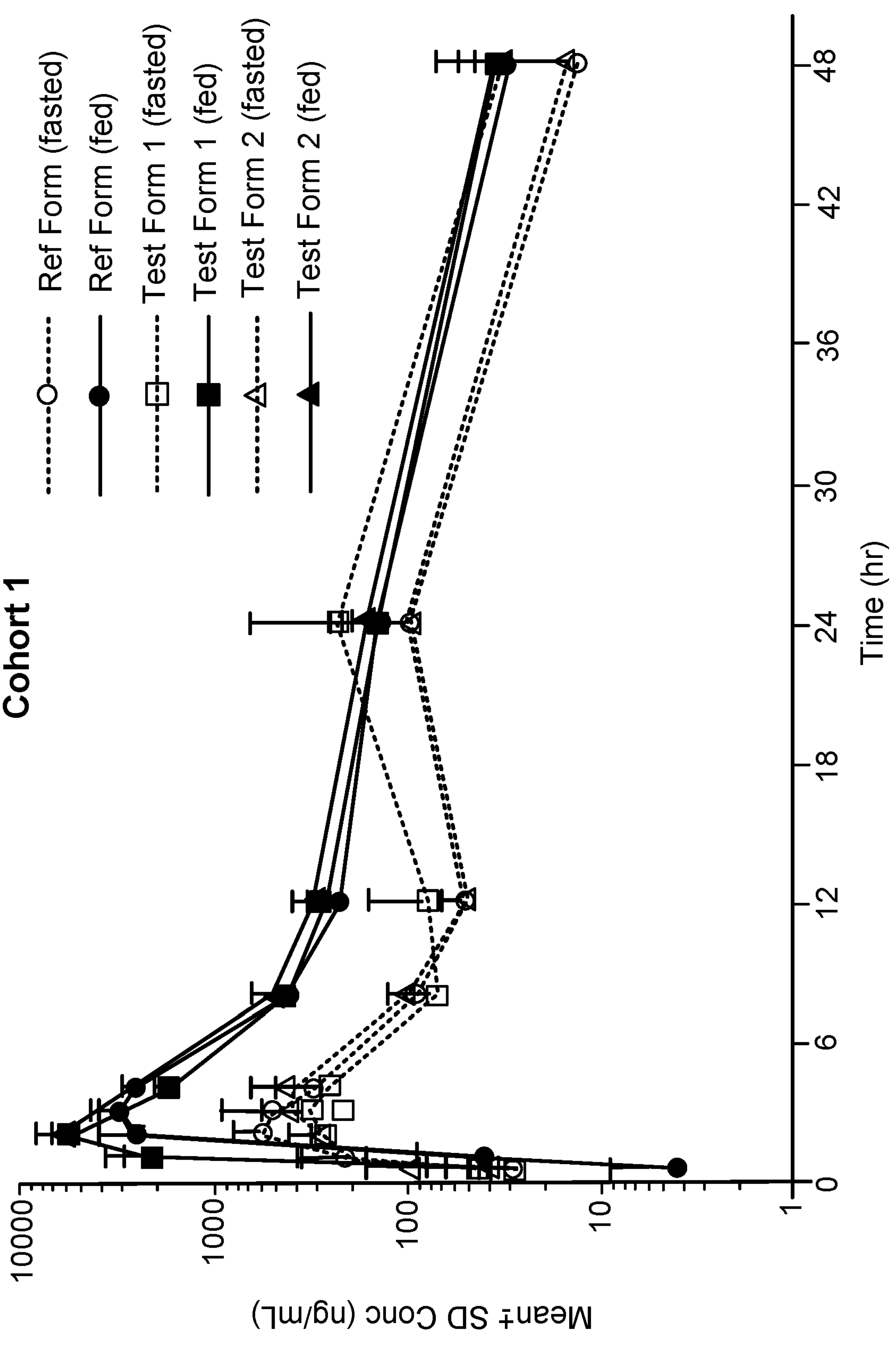
Cohort 1
Ref Form (fasted)
Ref Form (fed)
Test Form 1 (fasted)
Test Form 1 (fed)
Test Form 2 (fasted)
Test Form 2 (fed)
Mean± SD Conc (ng/mL)
10000
1000
100
10
1
Time (hr)
0
6
12
18
24
30
36
42
48

FIG. 6A

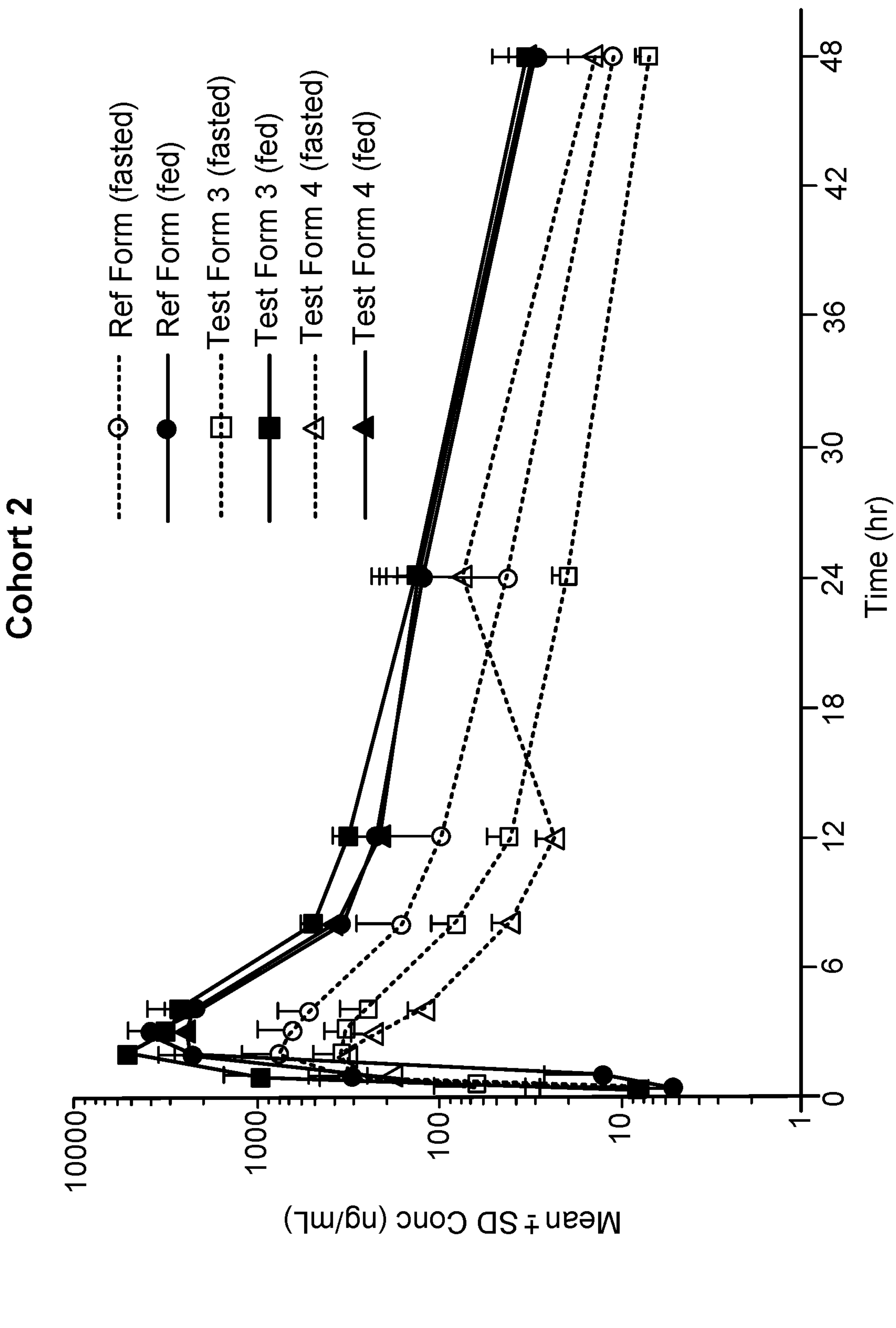
Cohort 2
Ref Form (fasted)
Ref Form (fed)
Test Form 3 (fasted)
Test Form 3 (fed)
Test Form 4 (fasted)
Test Form 4 (fed)
Mean ± SD Conc (ng/mL)
10000
1000
100
10
1
0
6
12
18
24
30
36
42
48
Time (hr)

FIG. 6B

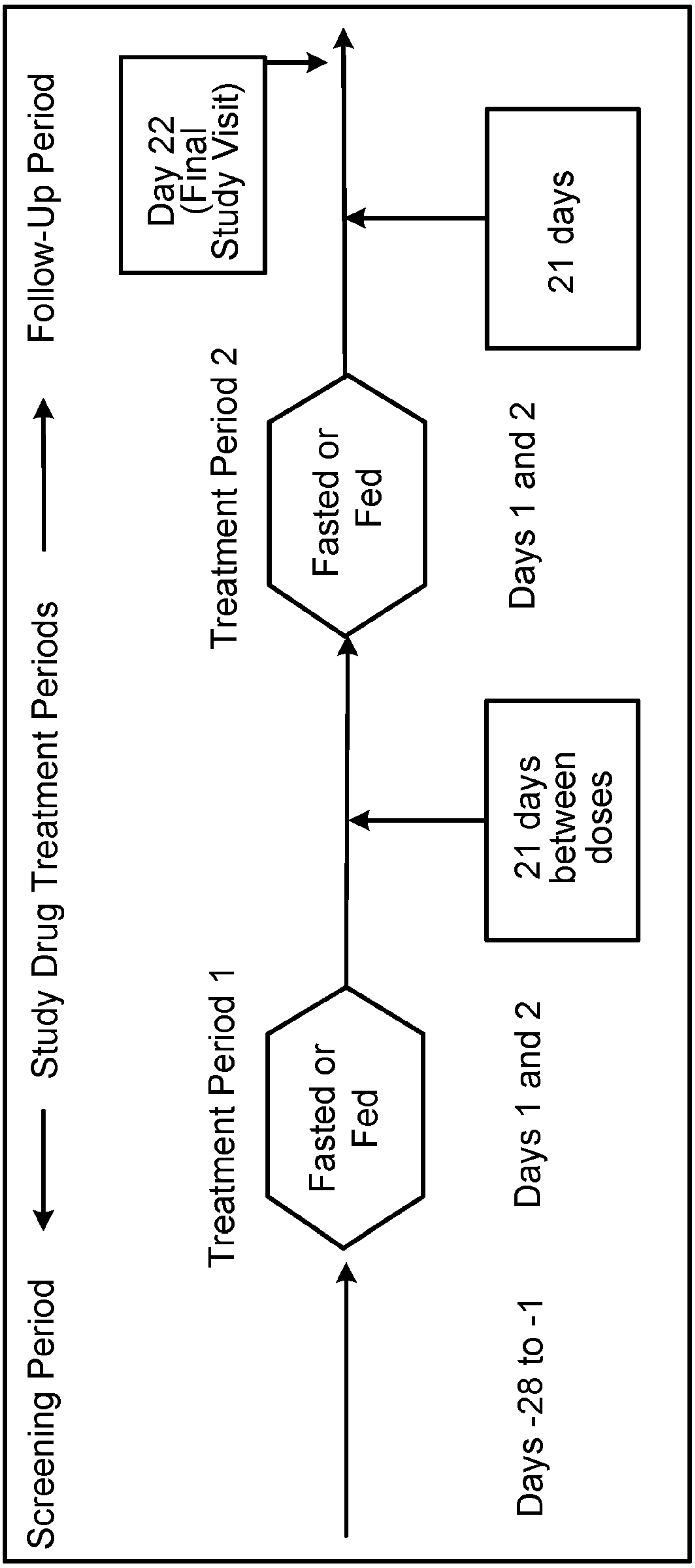
Screening Period
Study Drug Treatment Periods
Follow-Up Period
Days -28 to -1
Treatment Period 1
Fasted or Fed
Days 1 and 2
21 days between doses
Treatment Period 2
Fasted or Fed
Days 1 and 2
21 days
Day 22 (Final Study Visit)

FIG. 7

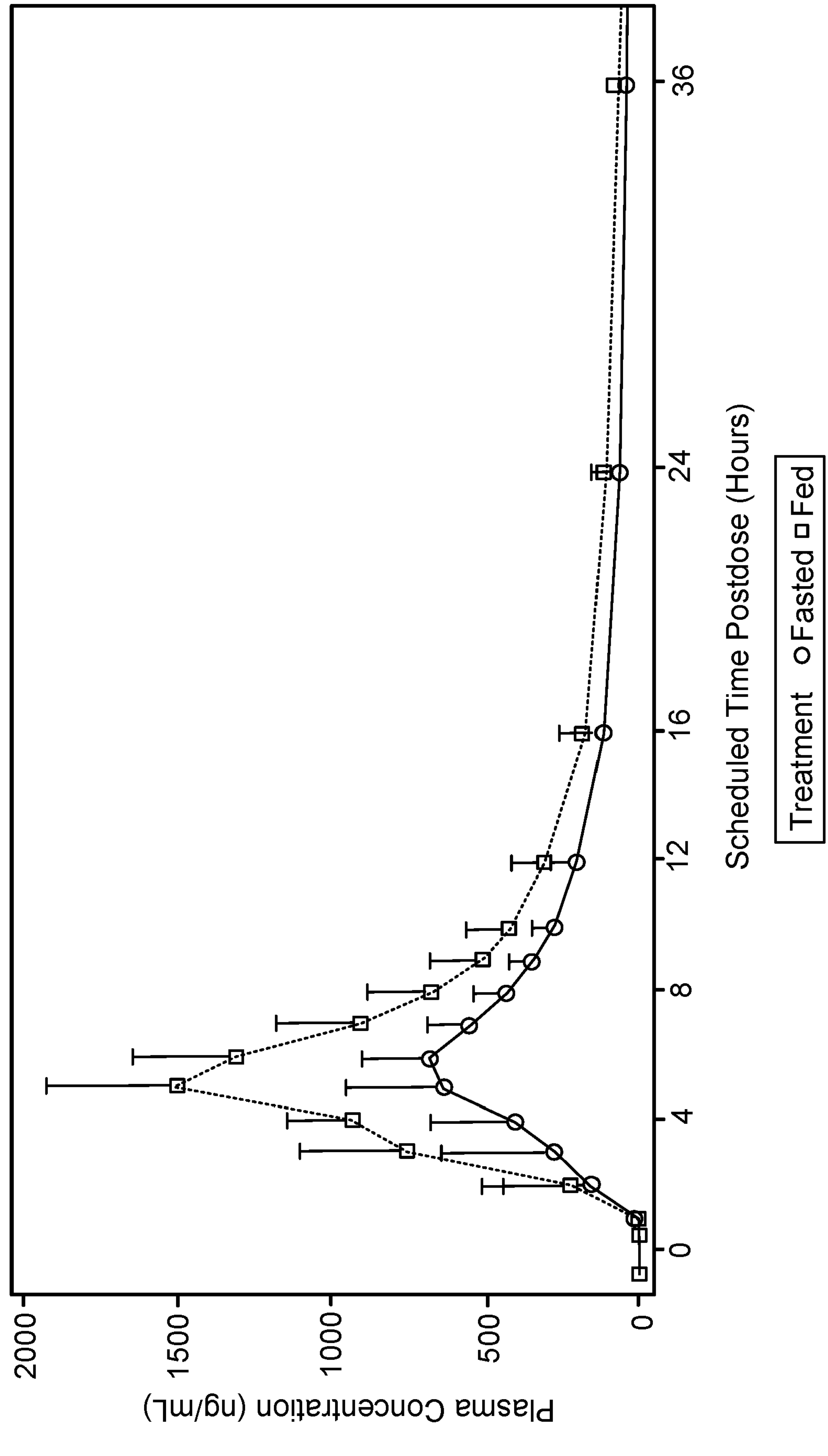
Plasma Concentration (ng/mL)
0
500
1000
1500
2000
0
4
8
12
16
24
36
Scheduled Time Postdose (Hours)
Treatment
Fasted
Fed

FIG. 8A

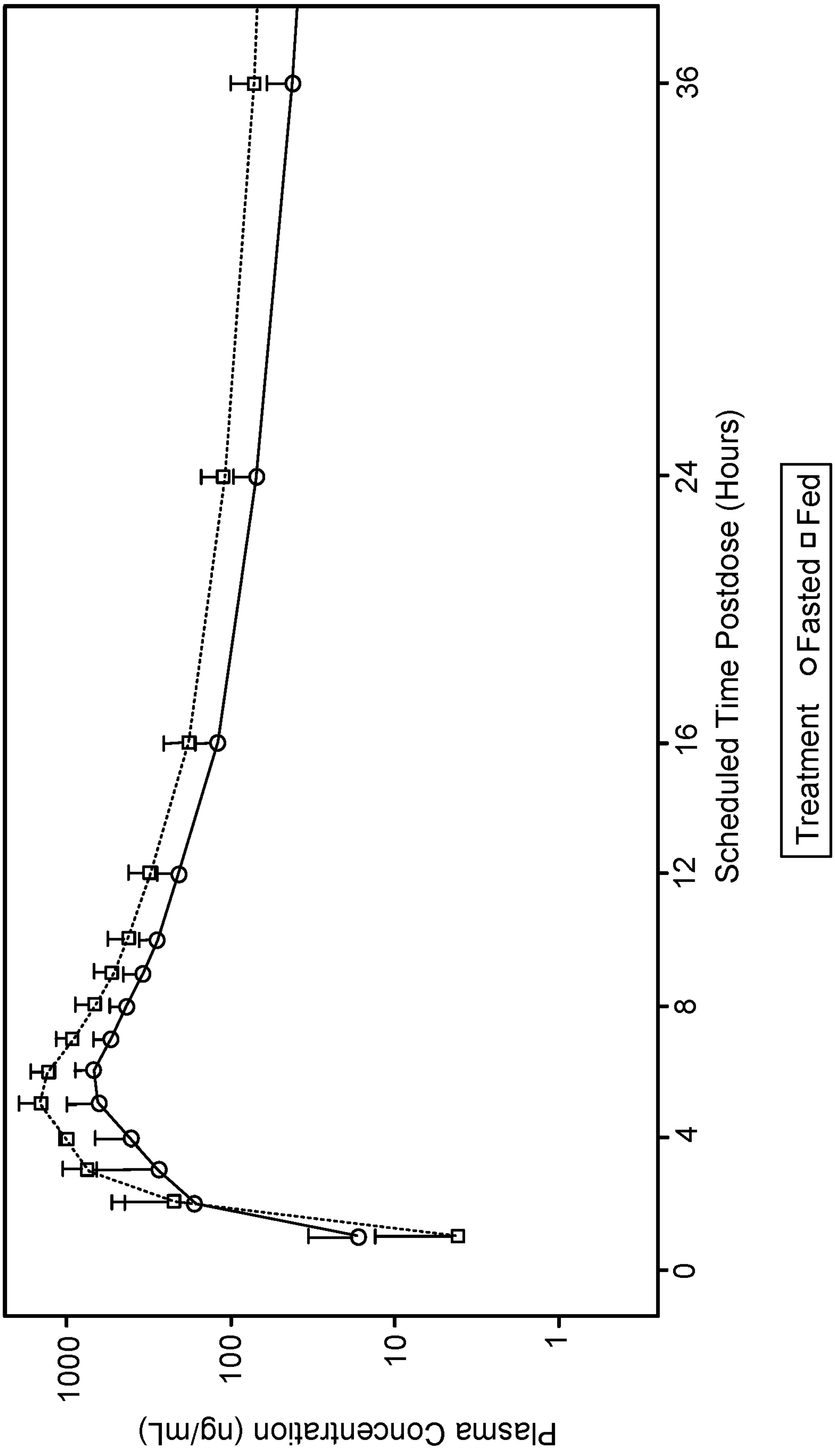
Plasma Concentration (ng/mL)
1000
100
10
1
0
4
8
12
16
24
36
Scheduled Time Postdose (Hours)
Treatment
Fasted
Fed

FIG. 8B

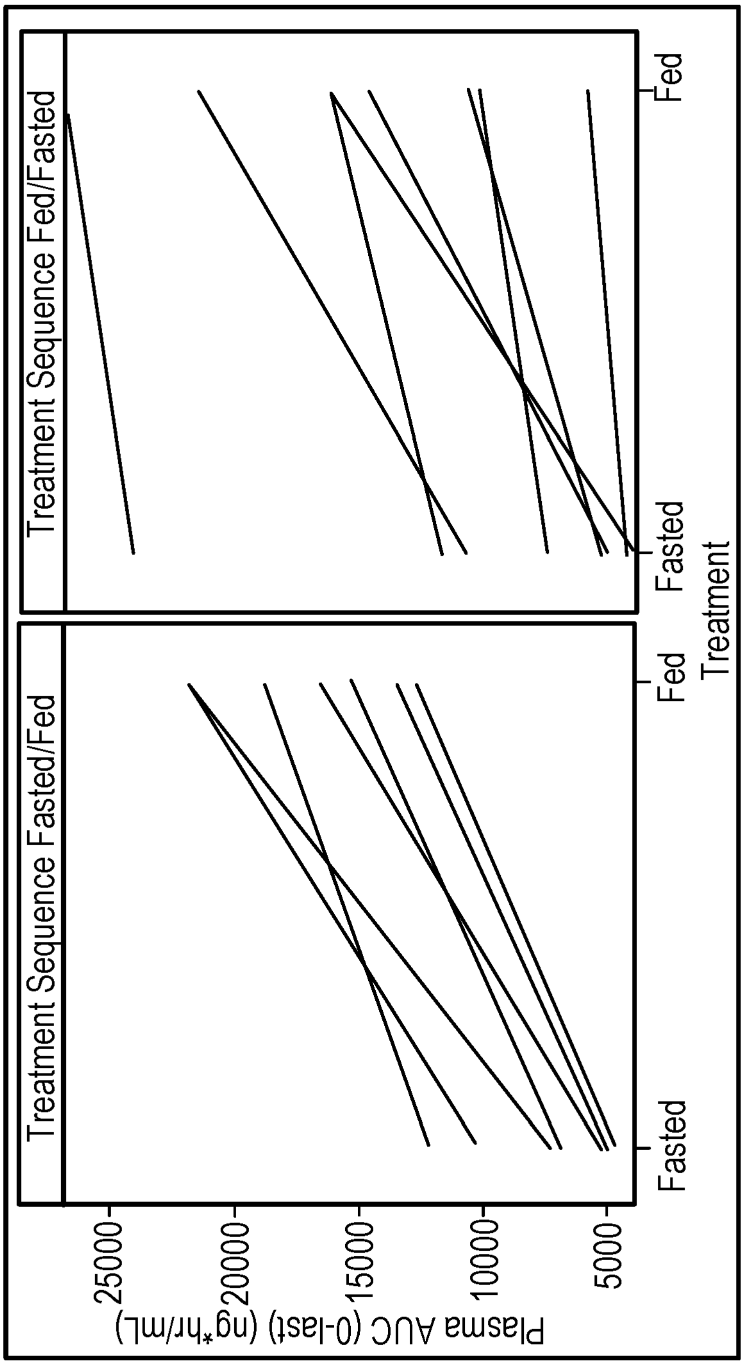
Treatment Sequence Fasted/Fed
Treatment Sequence Fed/Fasted
Plasma AUC (0-last) (ng*hr/mL)
25000
20000
15000
10000
5000
Fasted
Fed
Fasted
Fed
Treatment

FIG. 9A

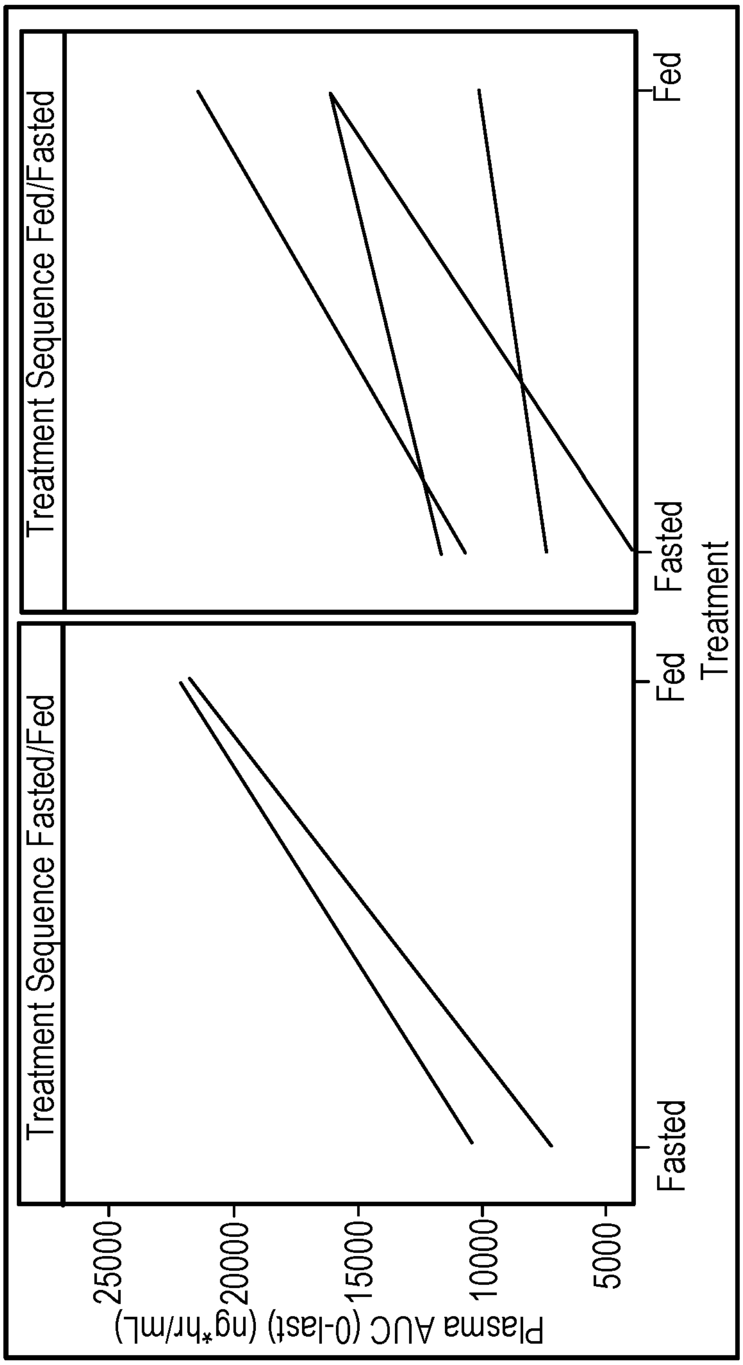
Treatment Sequence Fasted/Fed
Treatment Sequence Fed/Fasted
Plasma AUC (0-last) (ng*hr/mL)
25000
20000
15000
10000
5000
Fasted
Fed
Fasted
Fed
Treatment

FIG. 9B

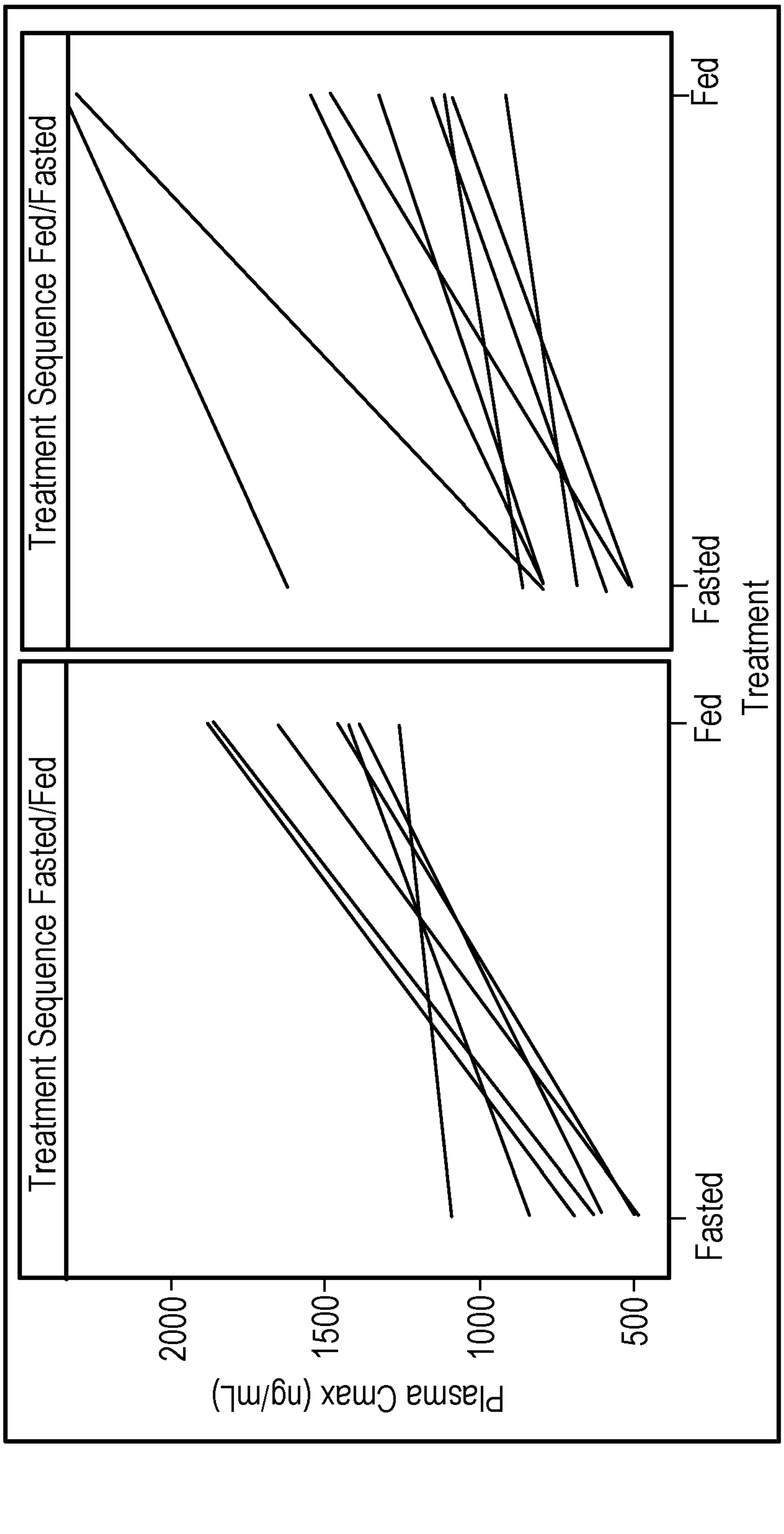
Treatment Sequence Fasted/Fed
Treatment Sequence Fed/Fasted
Plasma Cmax (ng/mL)
2000
1500
1000
500
Fasted
Fed
Fasted
Fed
Treatment

FIG. 9C

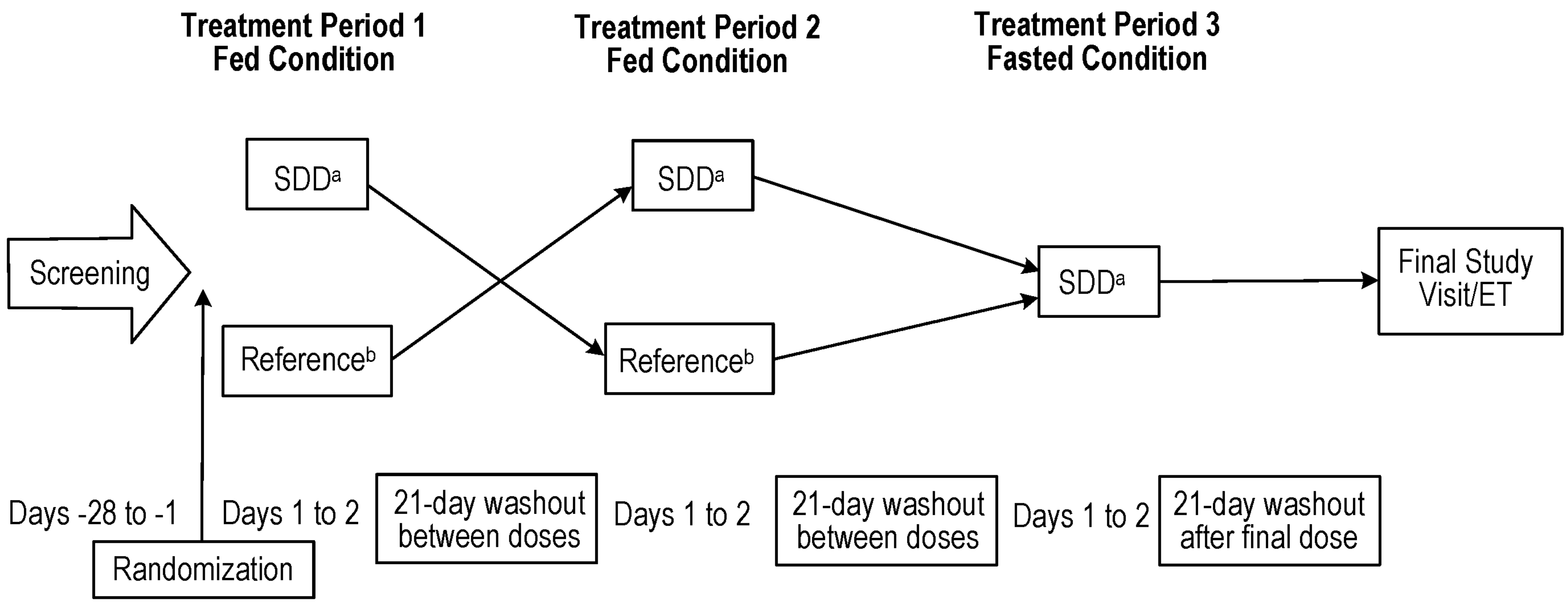


a SDD=Compound of Formula (I) spray dried dispersion (suspension or capsule formulation).
b Reference formulation=Compound of Formula (I) encapsulated, lipid semi-solid.

FIG. 10

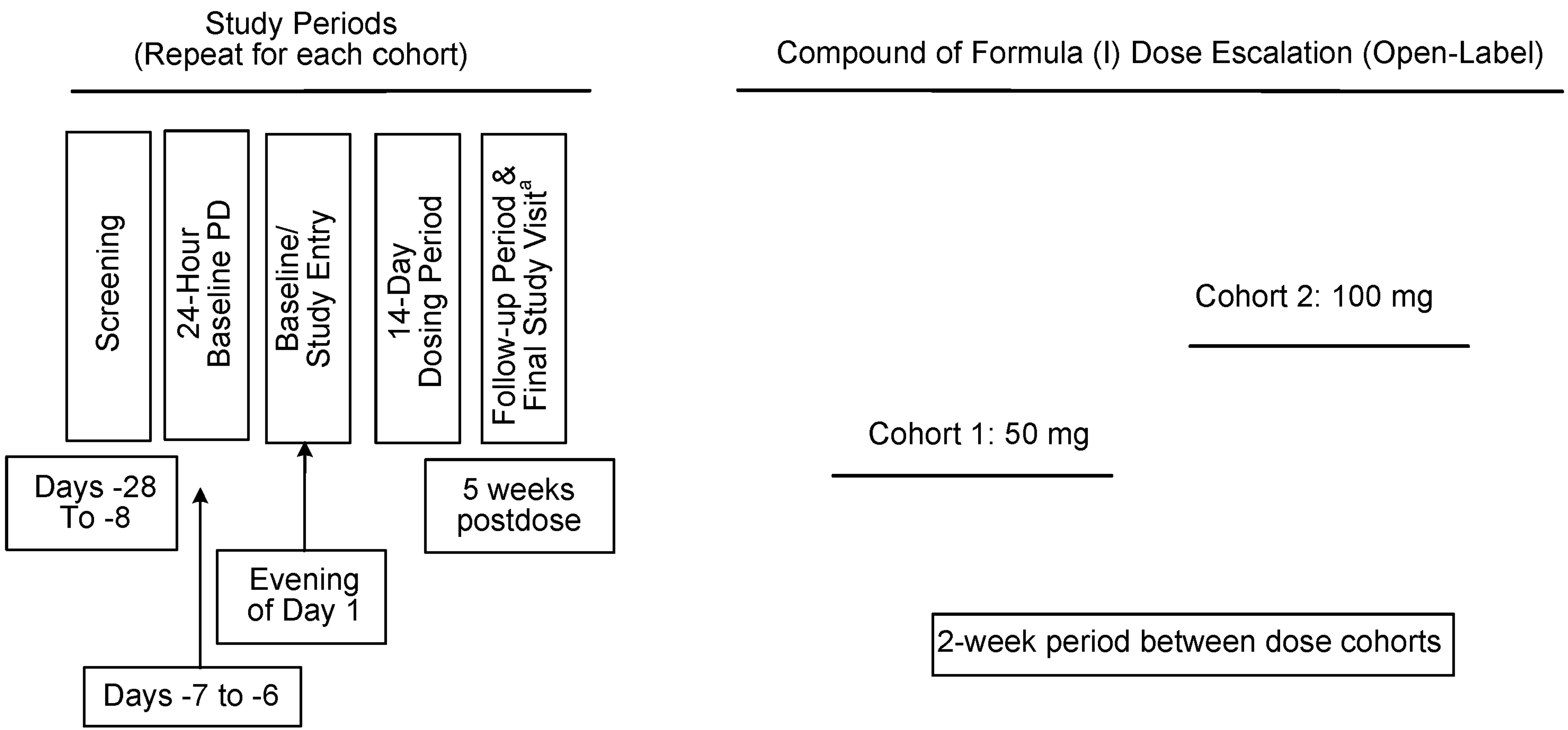


a Visit on Days 21 (+3 days), 28 (+3 days), and 35 (+3 days); final study visit on Day 49 (+7 days) or early termination.
PD = pharmacodynamic.

FIG. 11

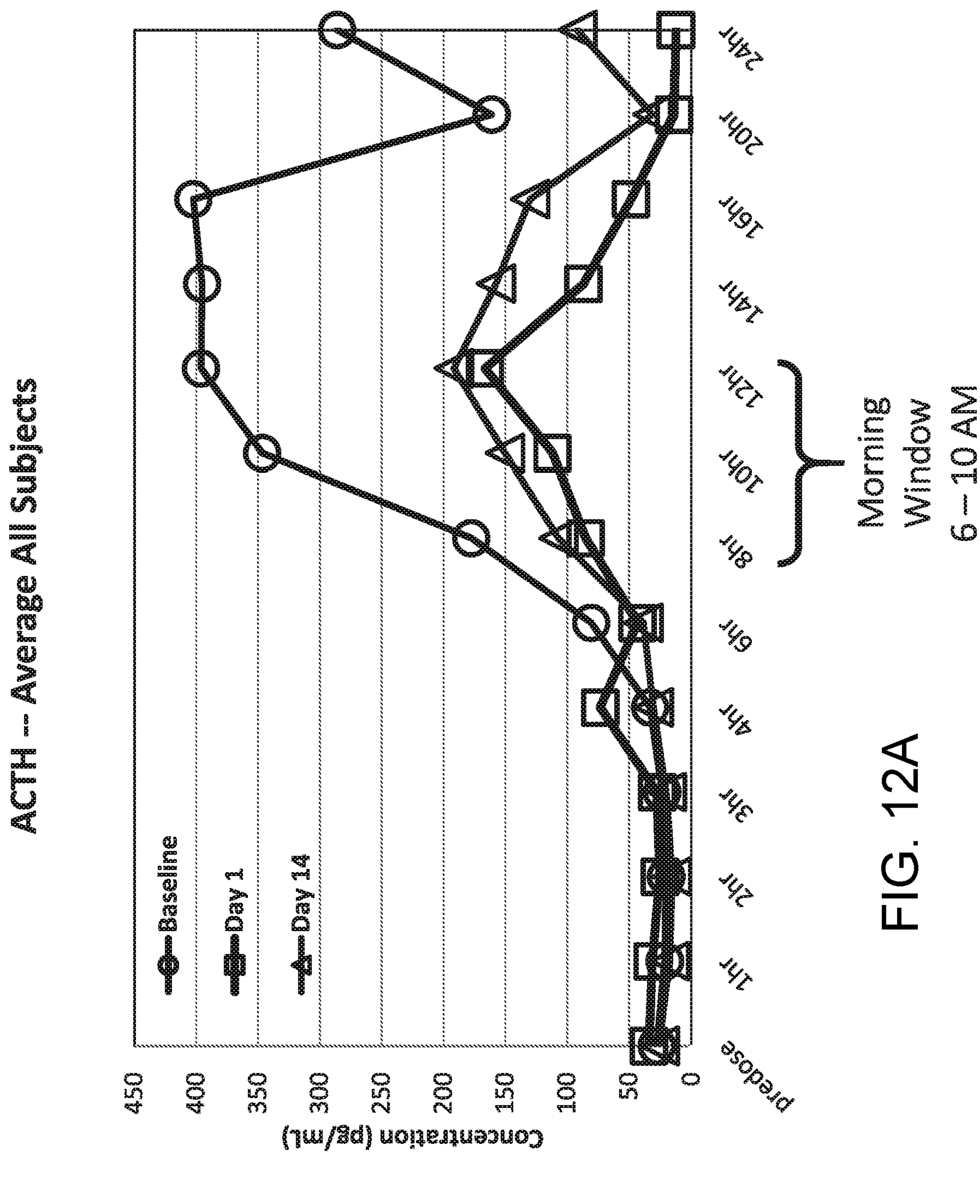
ACTH -- Average All Subjects
Baseline
Day 1
Day 14
Concentration (pg/mL)
450
400
350
300
250
200
150
100
50
0
predose
1hr
2hr
3hr
4hr
6hr
8hr
10hr
12hr
14hr
16hr
20hr
24hr
Morning Window 6 – 10 AM

FIG. 12A

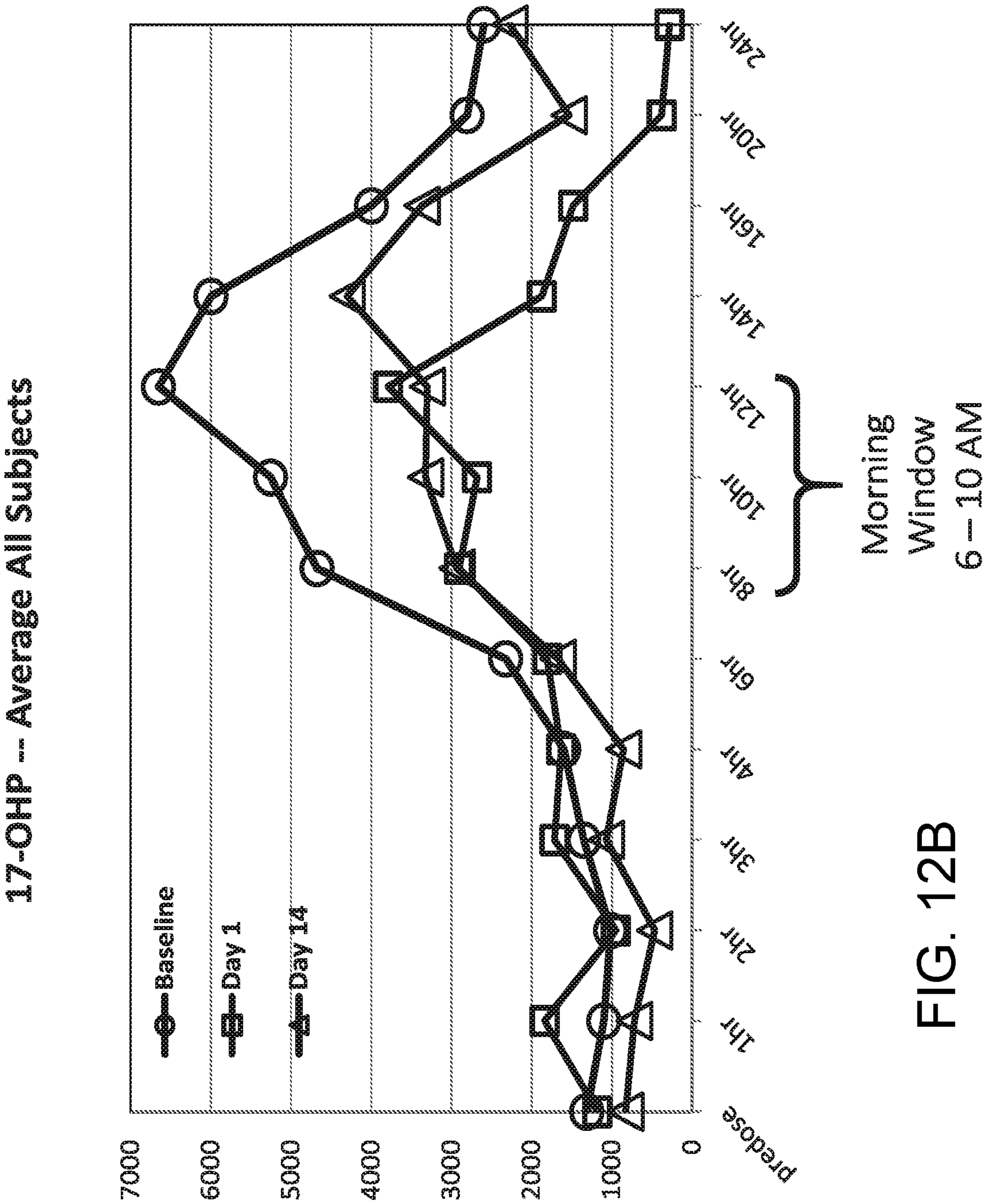
17-OHP -- Average All Subjects
Baseline
Day 1
Day 14
Concentration (pg/mL)
7000
6000
5000
4000
3000
2000
1000
0
predose
1hr
2hr
3hr
4hr
6hr
8hr
10hr
12hr
14hr
16hr
20hr
24hr
Morning
Window
6 – 10 AM

FIG. 12B

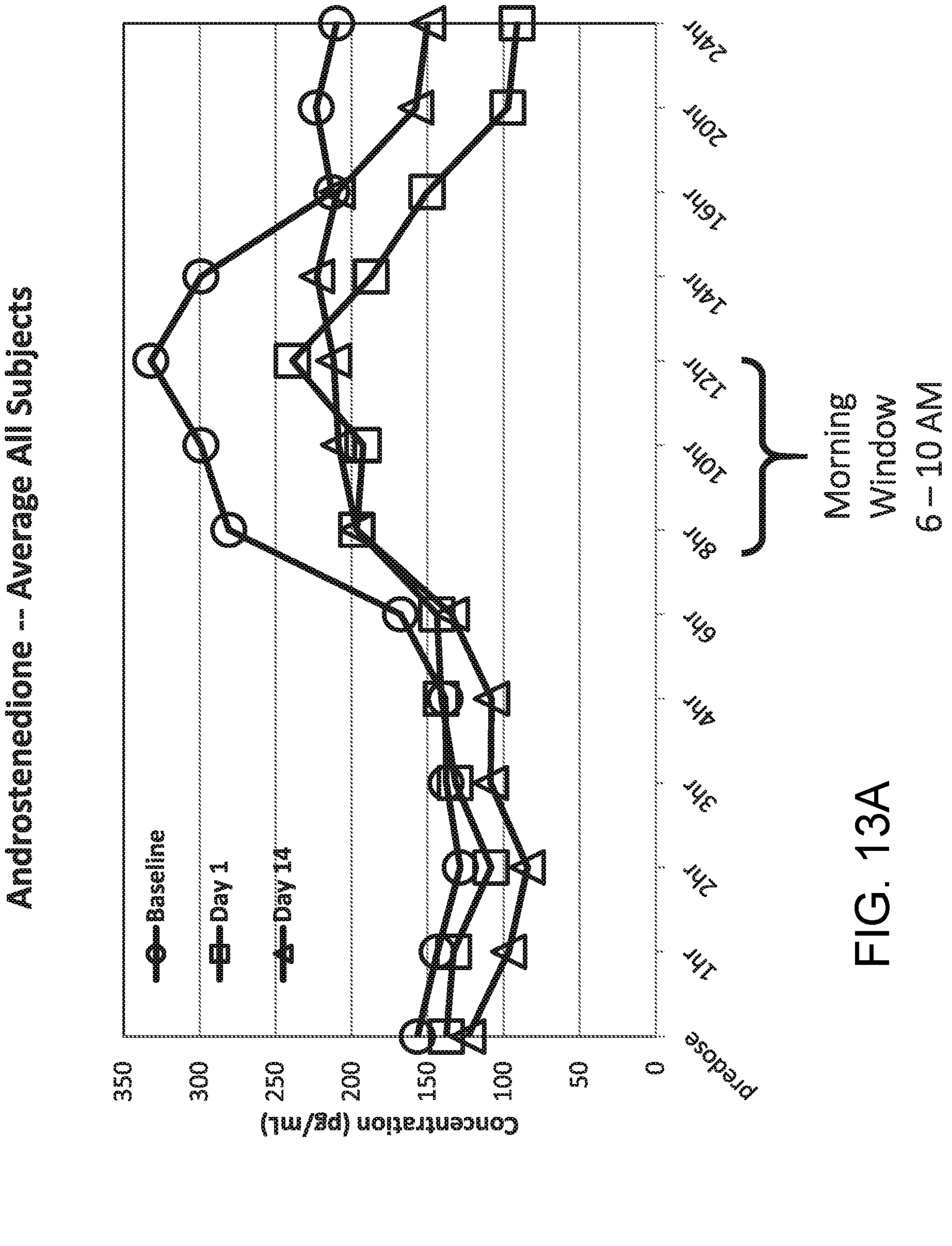
Androstenedione -- Average All Subjects
Baseline
Day 1
Day 14
Concentration (pg/mL)
350
300
250
200
150
100
50
0
predose
1hr
2hr
3hr
4hr
6hr
8hr
10hr
12hr
14hr
16hr
20hr
24hr
Morning
Window
6 – 10 AM

FIG. 13A

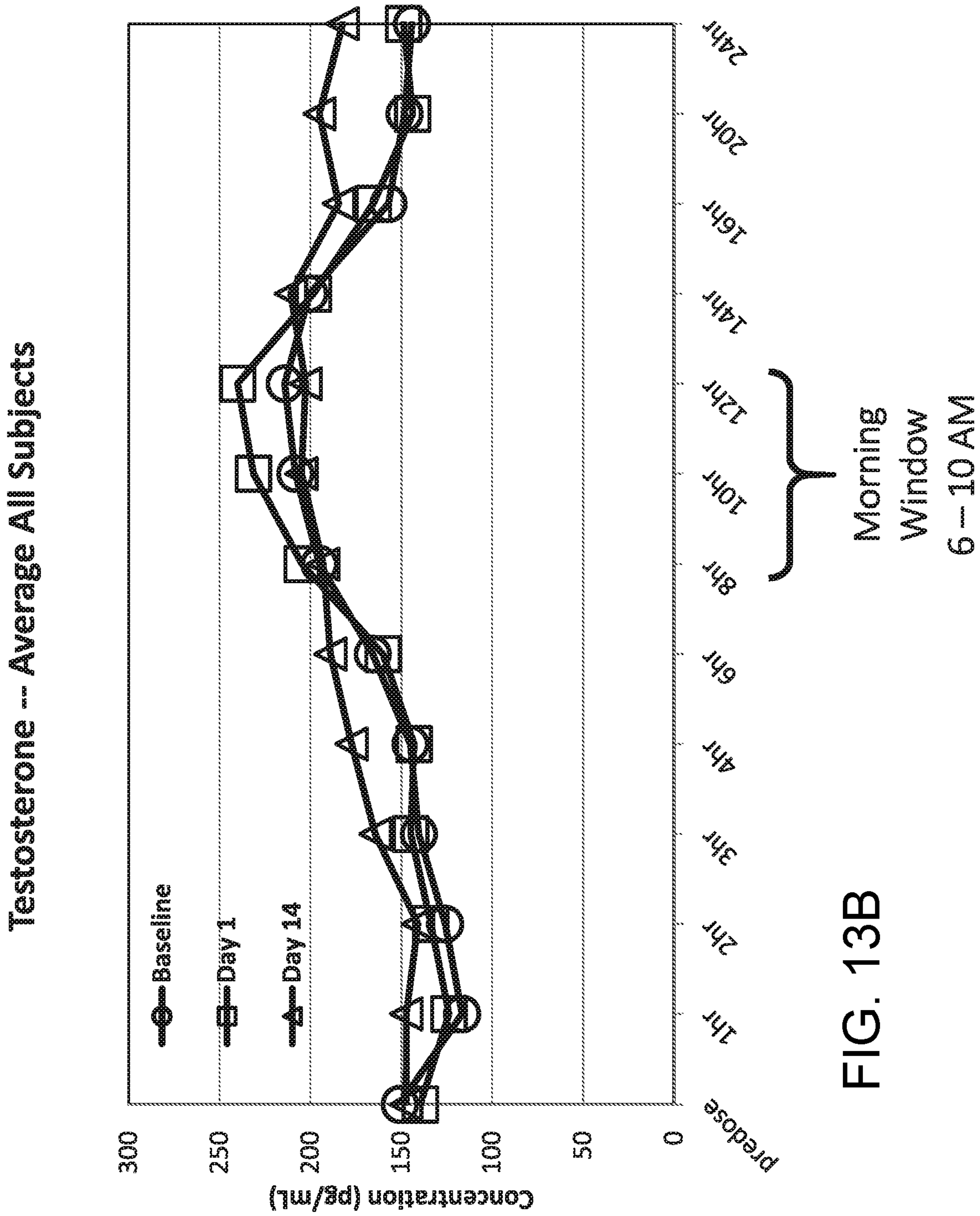
Testosterone -- Average All Subjects
Baseline
Day 1
Day 14
Concentration (pg/mL)
300
250
200
150
100
50
0
predose
1hr
2hr
3hr
4hr
6hr
8hr
10hr
12hr
14hr
16hr
20hr
24hr
Morning
Window
6 – 10 AM

FIG. 13B

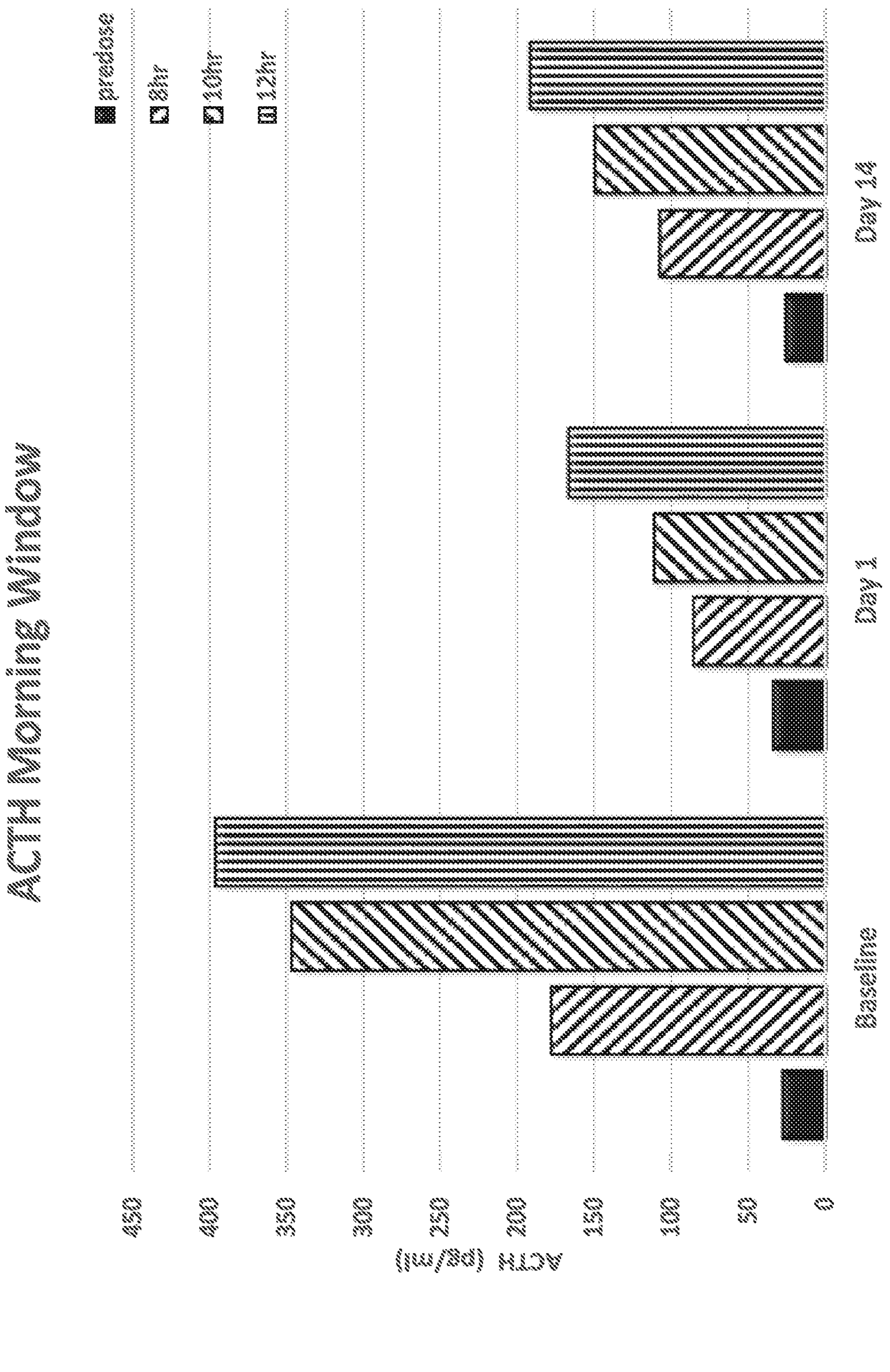
ACTH Morning Window
predose
8hr
10hr
12hr
ACTH (pg/ml)
0
50
100
150
200
250
300
350
400
450
Baseline
Day 1
Day 14

FIG. 14A

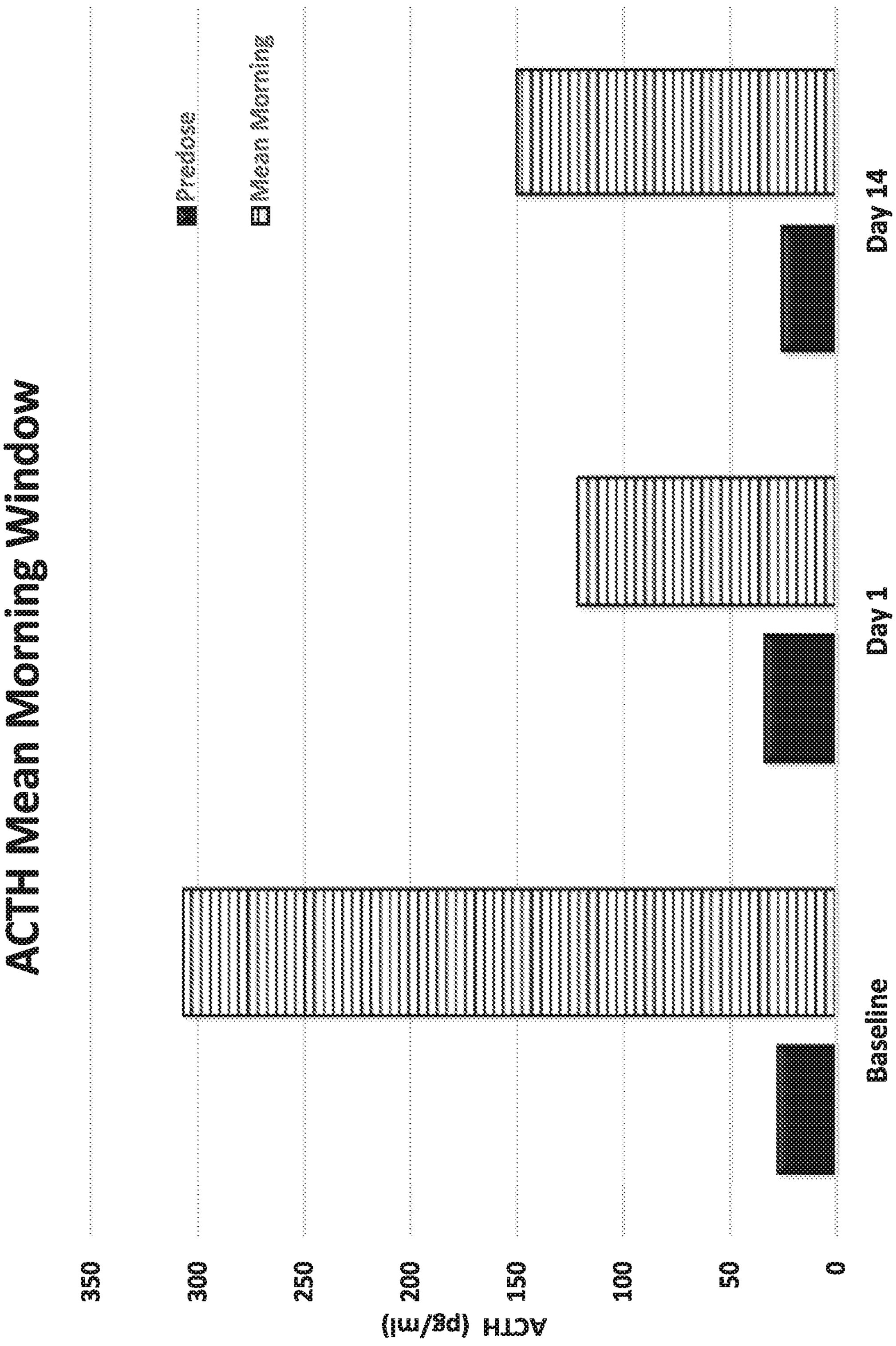
ACTH Mean Morning Window
ACTH (pg/ml)
350
300
250
200
150
100
50
0
Baseline
Day 1
Day 14
Predose
Mean Morning

FIG. 14B

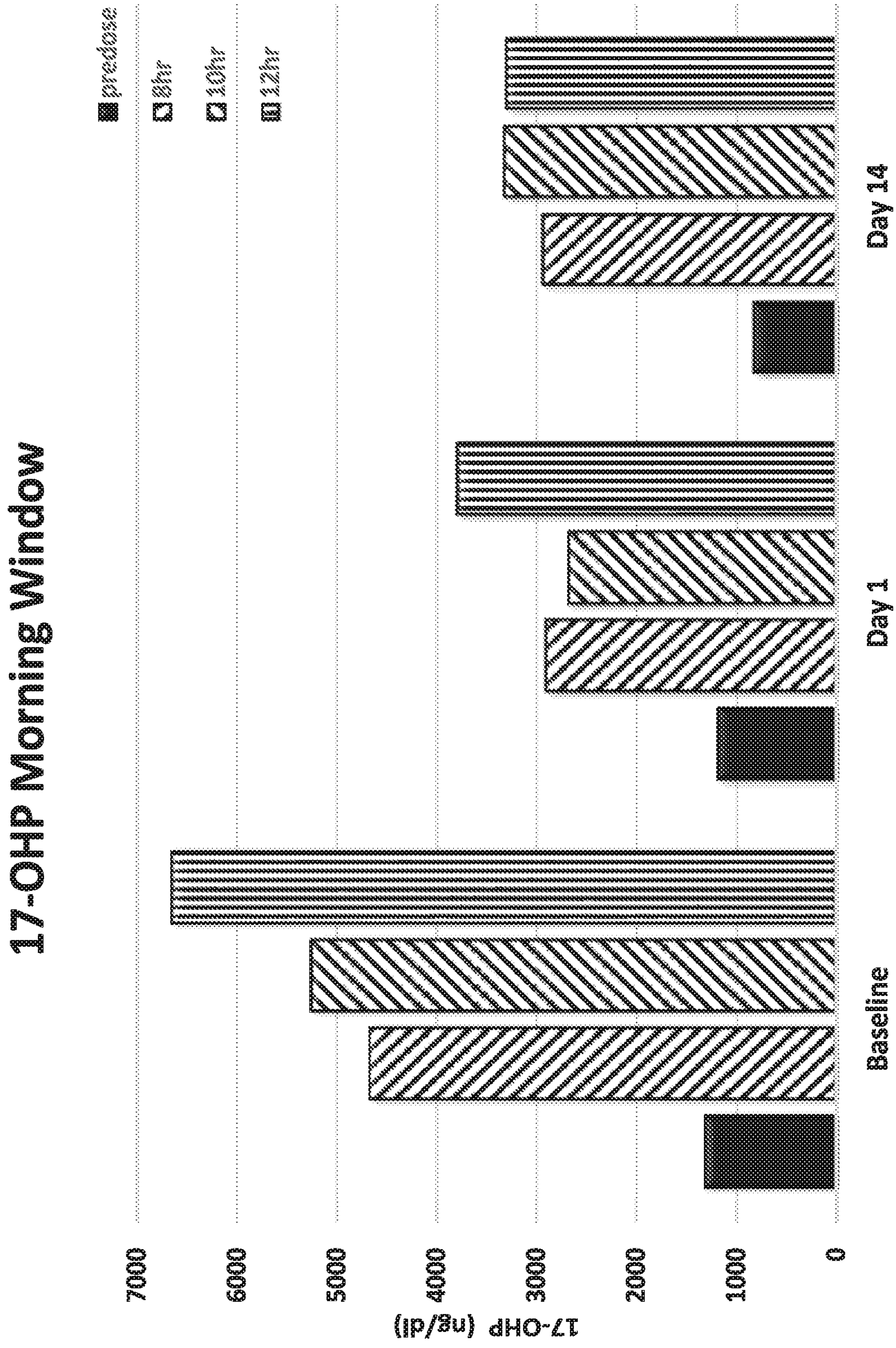
17-OHP Morning Window
17-OHP (ng/dl)
7000
6000
5000
4000
3000
2000
1000
0
Baseline
Day 1
Day 14
predose
8hr
10hr
12hr

FIG. 15A

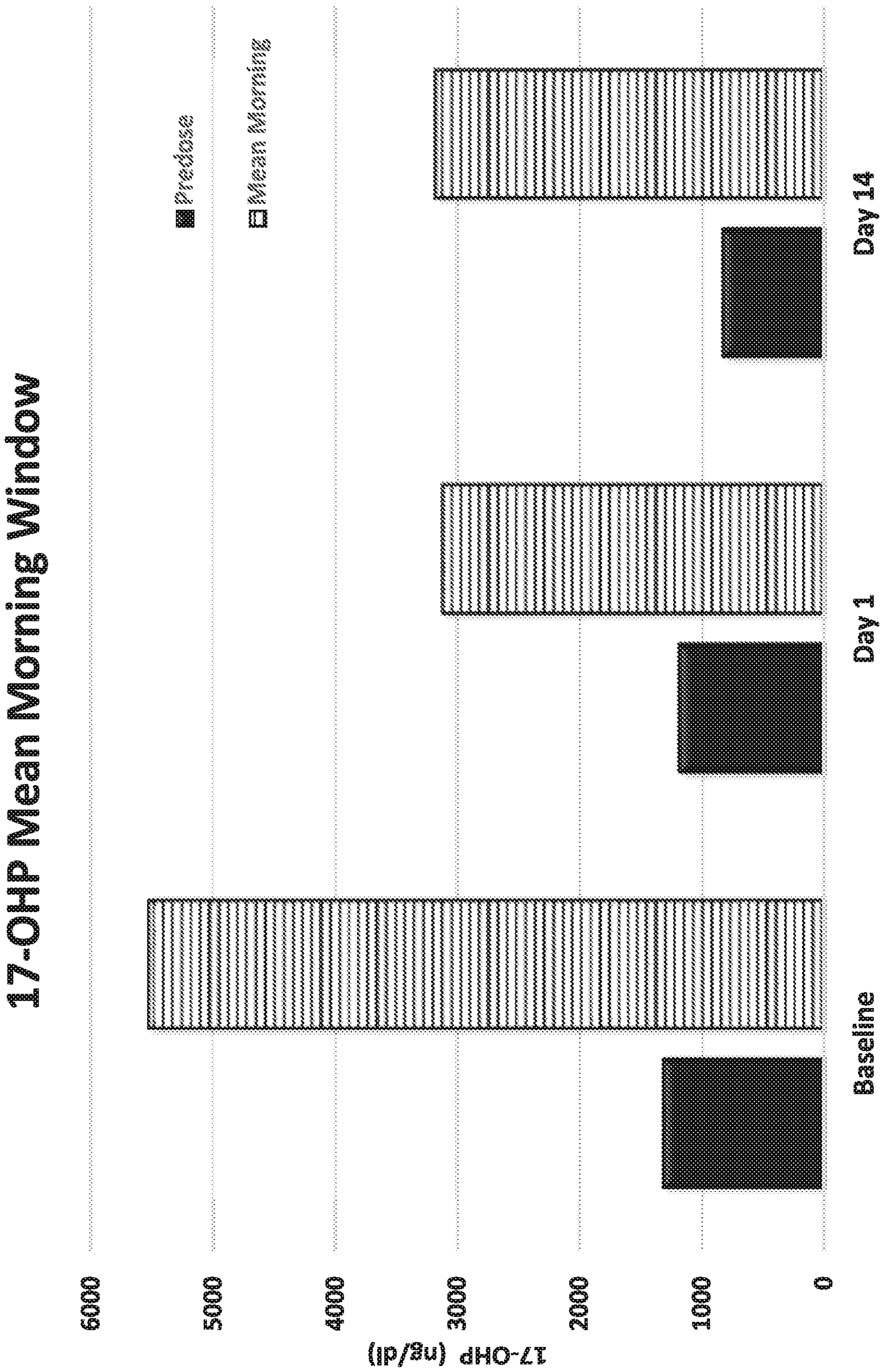
17-OHP Mean Morning Window
17-OHP (ng/dl)
6000
5000
4000
3000
2000
1000
0
Baseline
Day 1
Day 14
Predose
Mean Morning

FIG. 15B

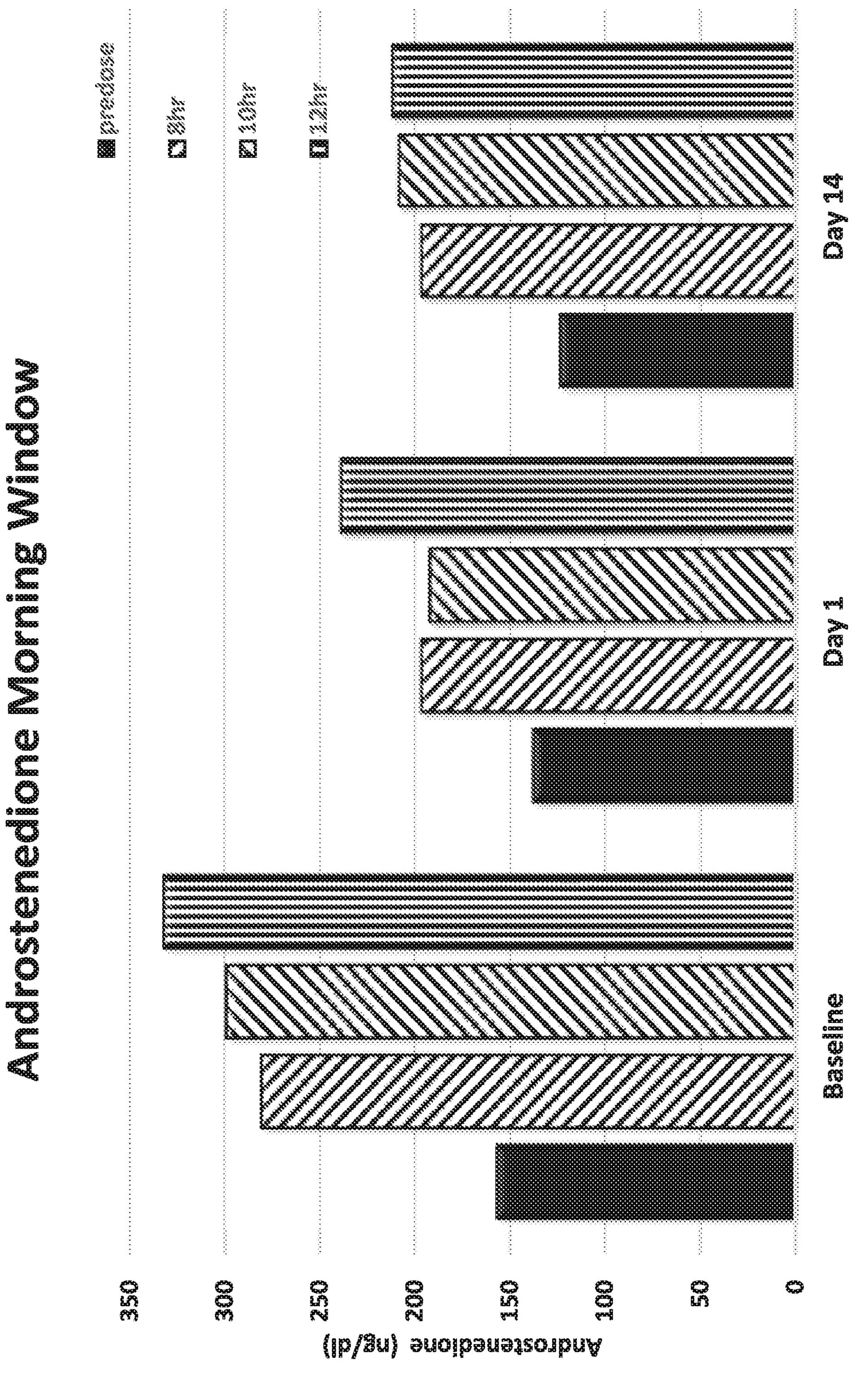
Androstenedione Morning Window
Androstenedione (ng/dl)
350
300
250
200
150
100
50
0
predose
8hr
10hr
12hr
Baseline
Day 1
Day 14

FIG. 16A

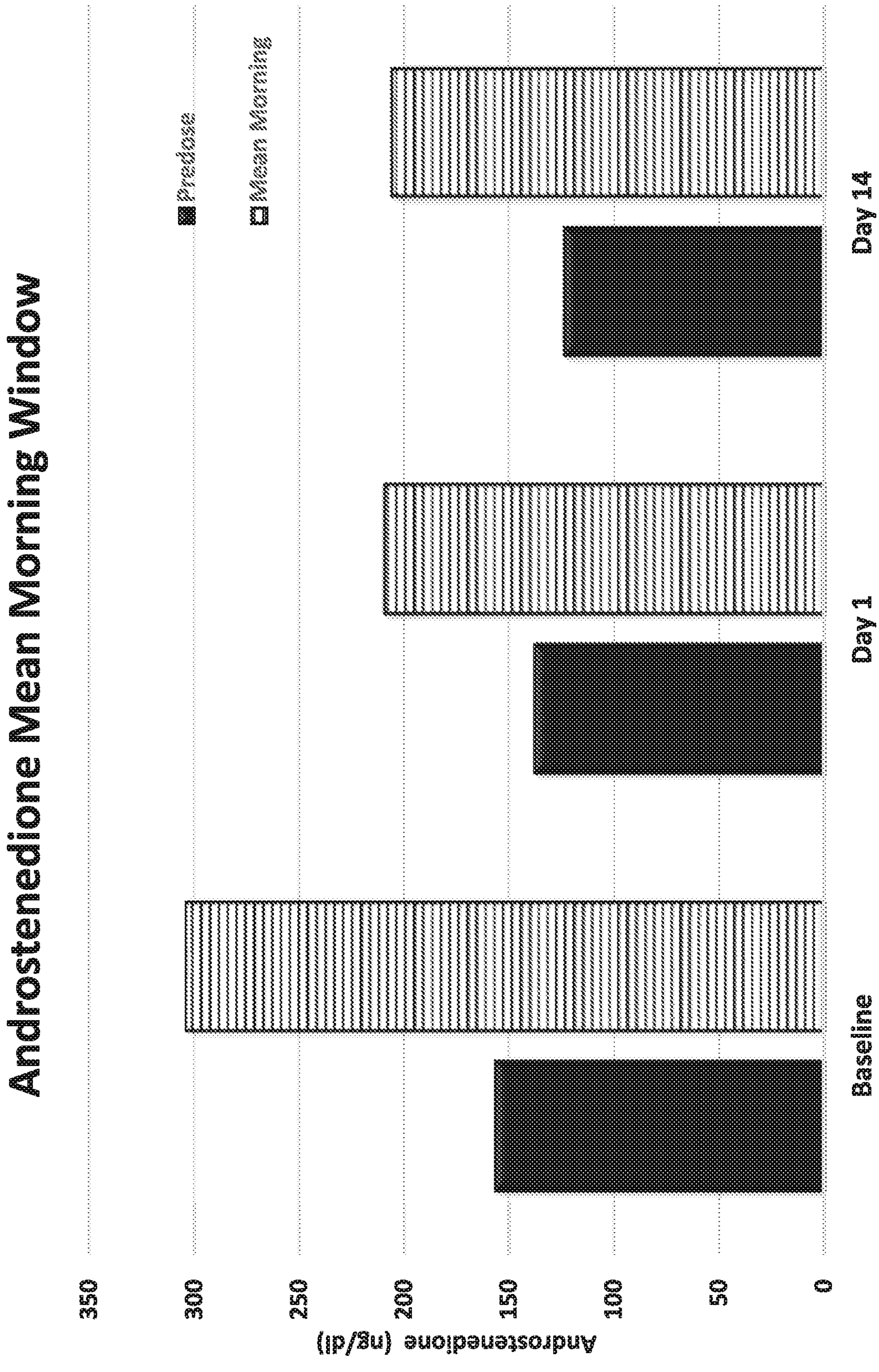
Androstenedione Mean Morning Window
Androstenedione (ng/dl)
350
300
250
200
150
100
50
0
Baseline
Day 1
Day 14
Predose
Mean Morning

FIG. 16B

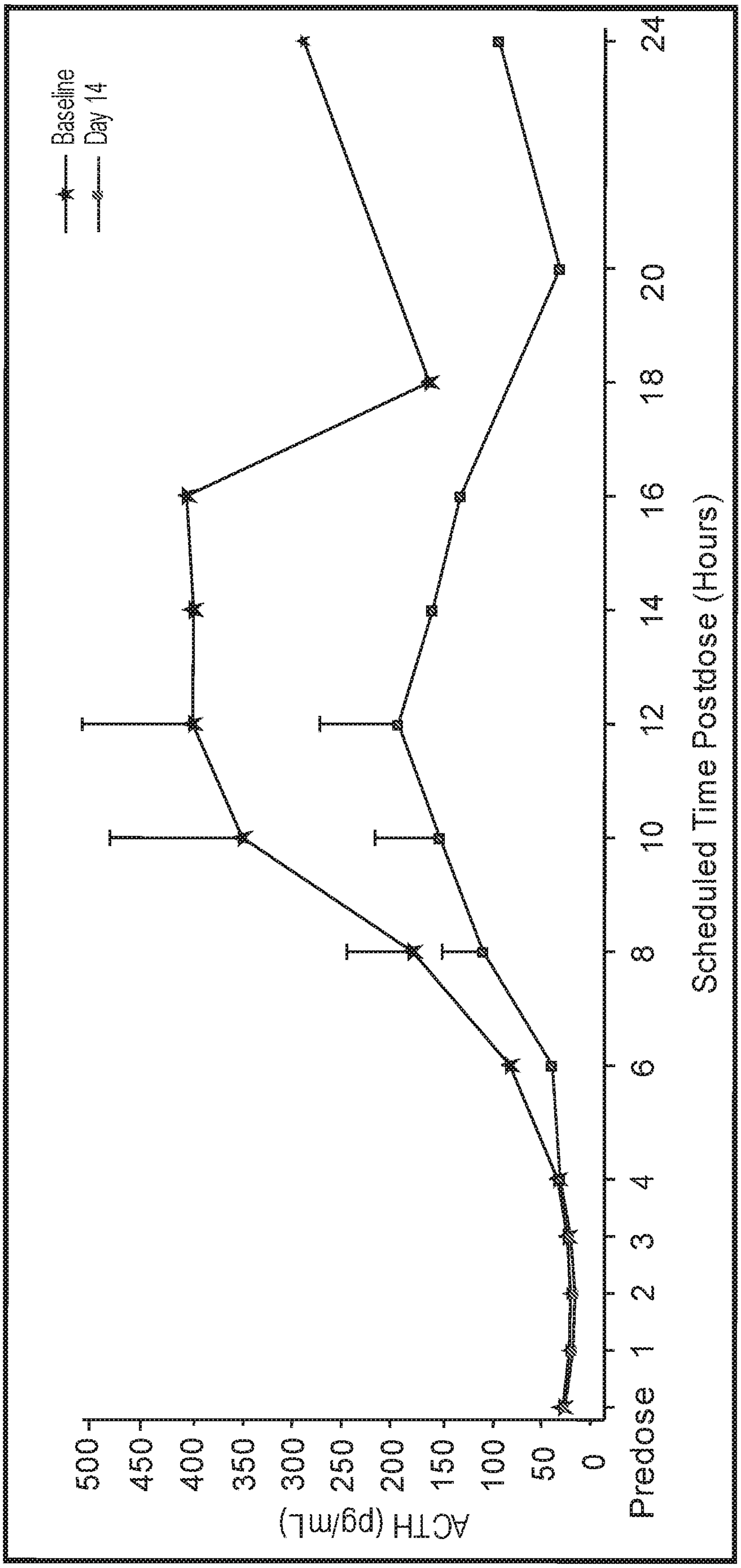
Baseline
Day 14
ACTH (pg/mL)
500
450
400
350
300
250
200
150
100
50
0
Predose
1
2
3
4
6
8
10
12
14
16
18
20
24
Scheduled Time Postdose (Hours)

FIG. 17A

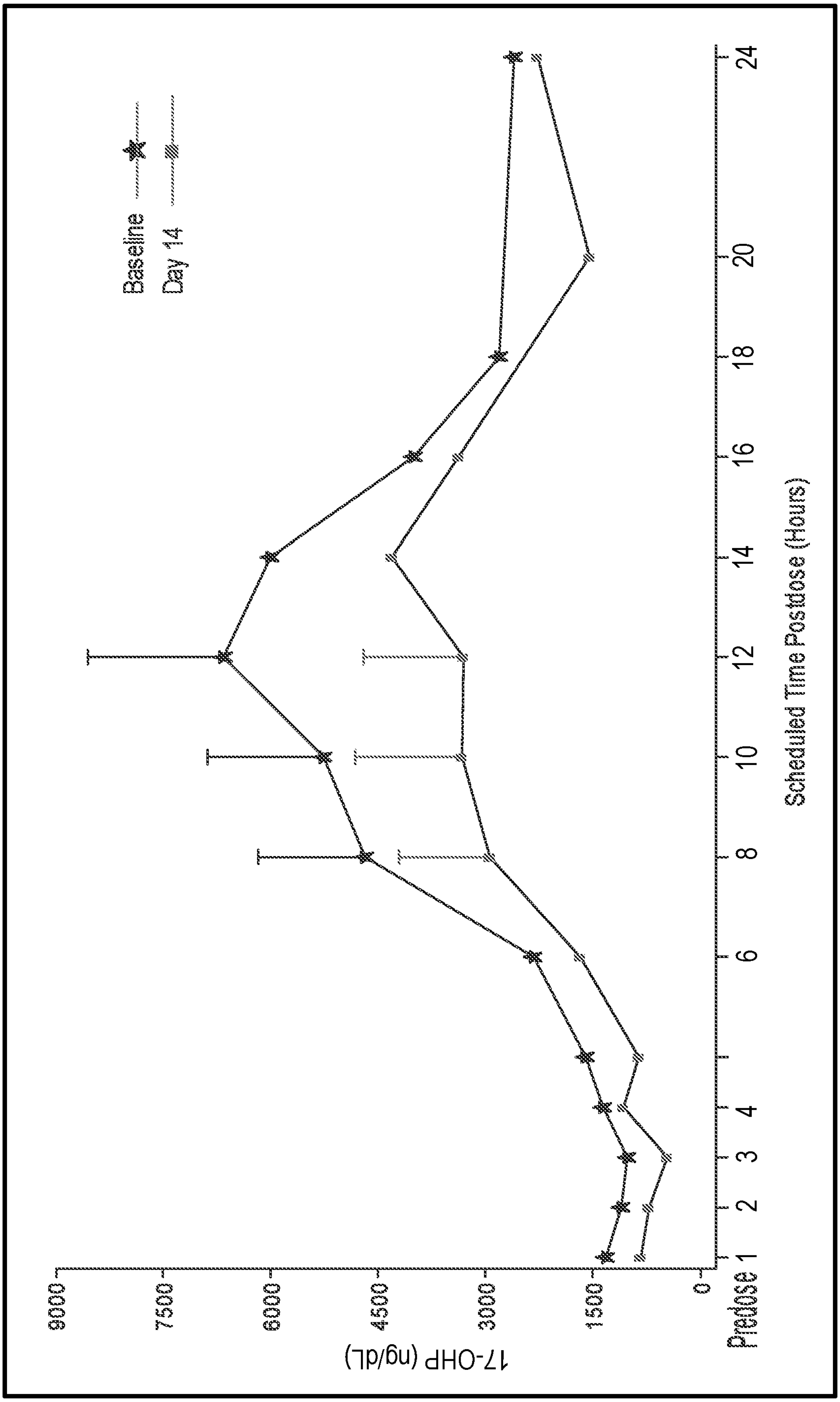

17-OHP (ng/dL)
9000
7500
6000
4500
3000
1500
0
Predose 1
2
3
4
6
8
10
12
14
16
18
20
24
Scheduled Time Postdose (Hours)
Baseline
Day 14


FIG. 17B

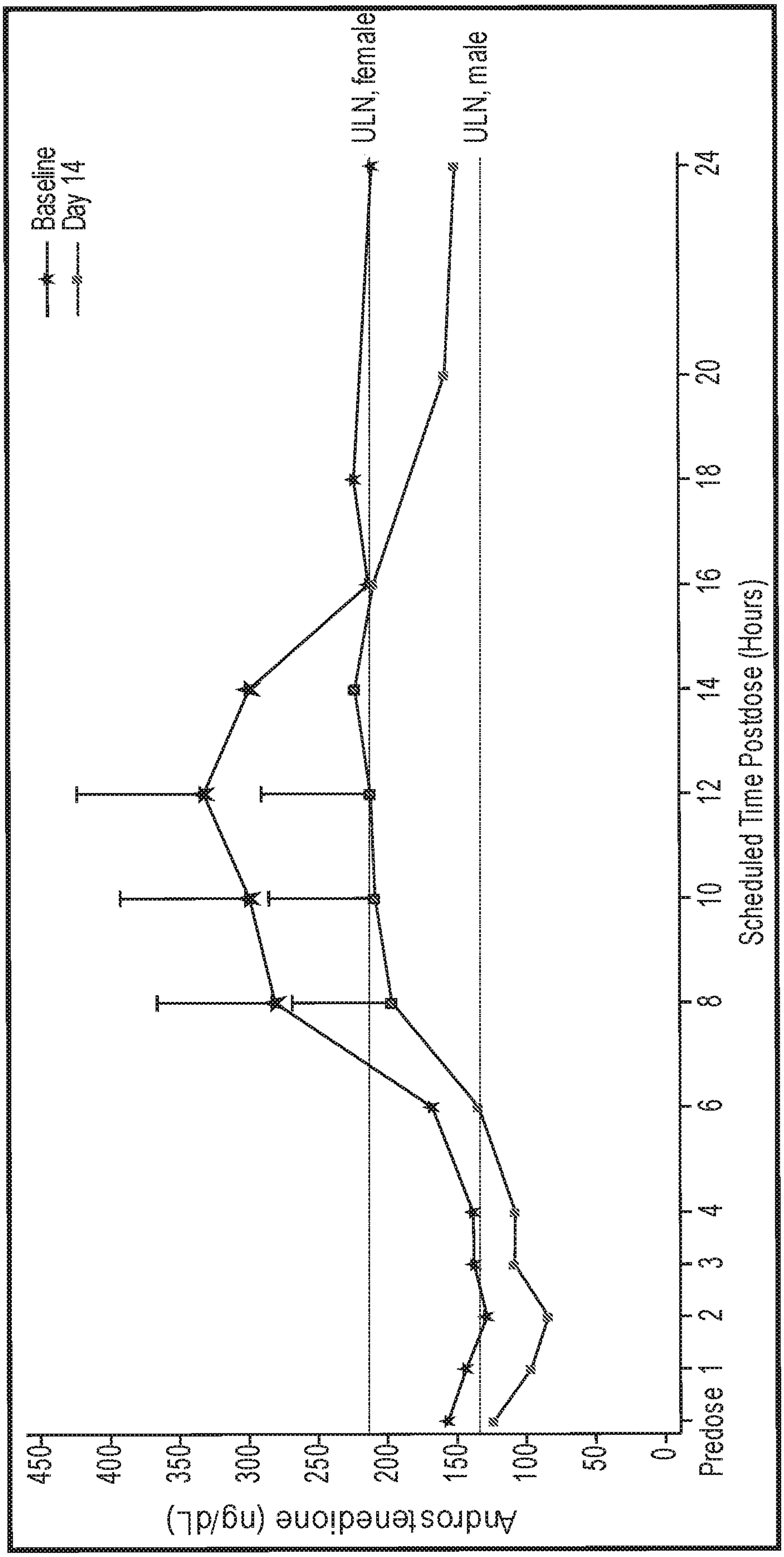

Baseline
Day 14
ULN, female
ULN, male
Androstenedione (ng/dL)
450
400
350
300
250
200
150
100
50
0
Predose
1
2
3
4
6
8
10
12
14
16
18
20
24
Scheduled Time Postdose (Hours)


FIG. 17C

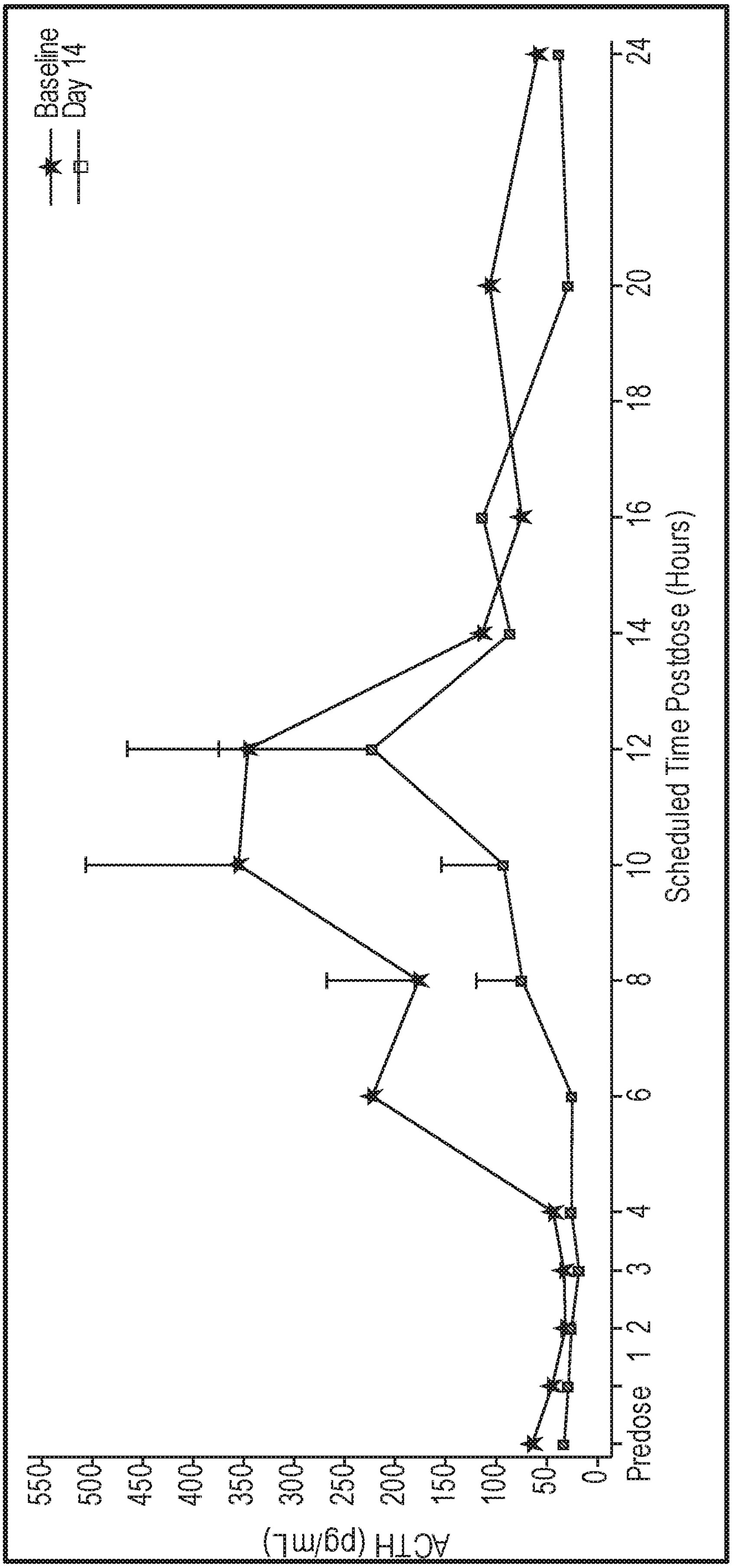
ACTH (pg/mL)
0
50
100
150
200
250
300
350
400
450
500
550
Predose
1
2
3
4
6
8
10
12
14
16
18
20
24
Scheduled Time Postdose (Hours)
Baseline
Day 14

FIG. 18A

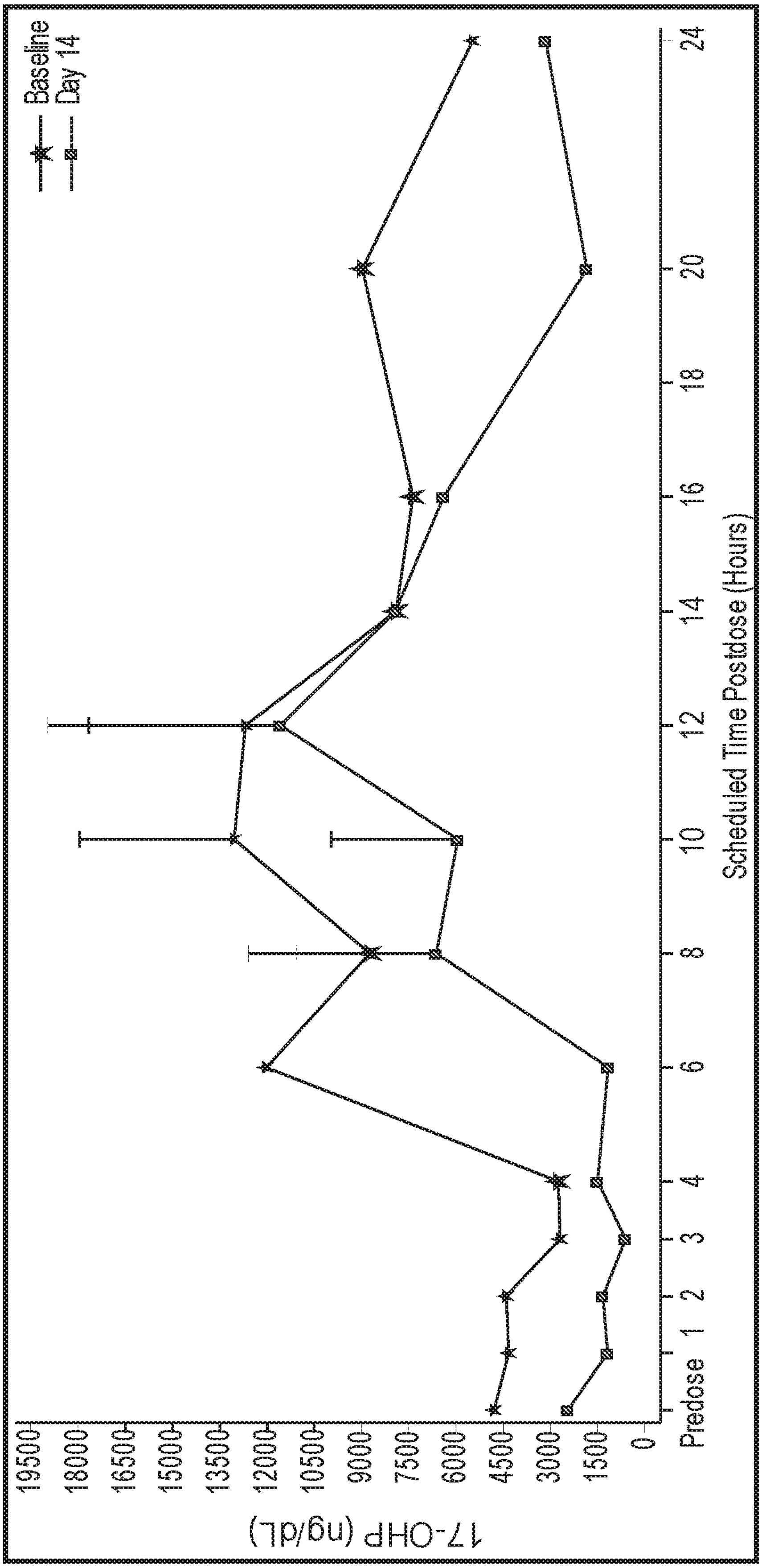
17-OHP (ng/dL)
19500
18000
16500
15000
13500
12000
10500
9000
7500
6000
4500
3000
1500
0
Predose
1
2
3
4
6
8
10
12
14
16
18
20
24
Scheduled Time Postdose (Hours)
Baseline
Day 14

FIG. 18B

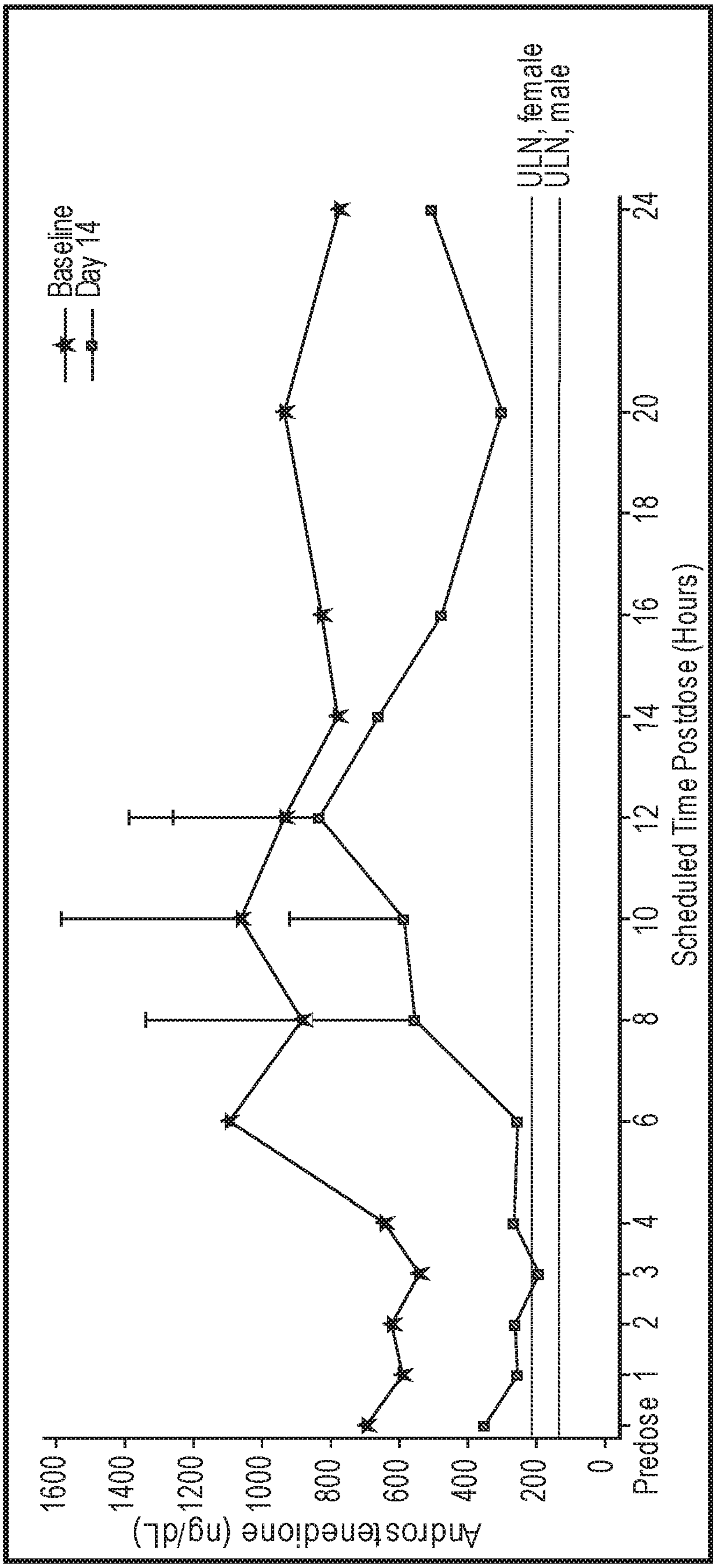
Baseline
Day 14
ULN, female
ULN, male
Androstenedione (ng/dL)
1600
1400
1200
1000
800
600
400
200
0
Predose
1
2
3
4
6
8
10
12
14
16
18
20
24
Scheduled Time Postdose (Hours)

FIG. 18C

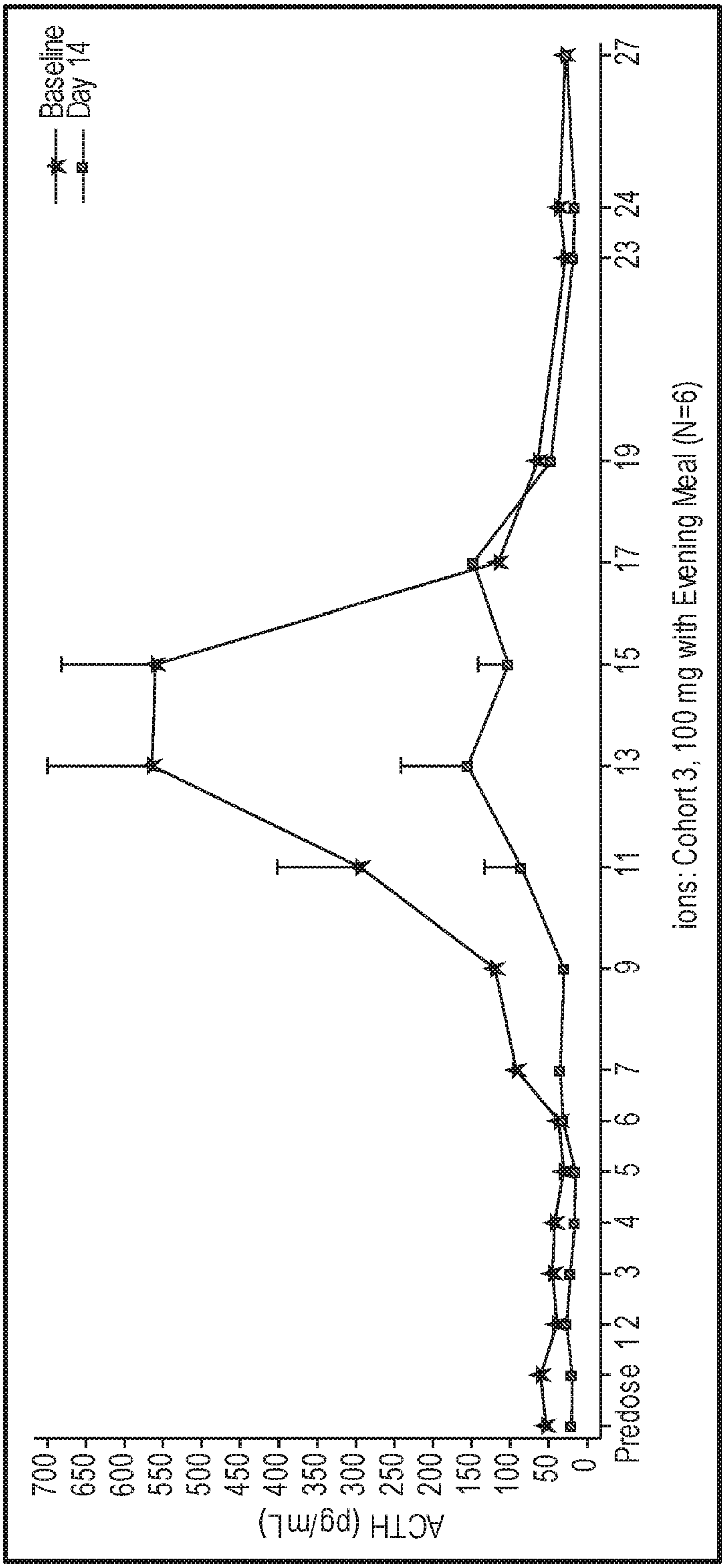

Baseline
Day 14
ACTH (pg/mL)
700
650
600
550
500
450
400
350
300
250
200
150
100
50
0
Predose 1 2 3 4 5 6 7 9 11 13 15 17 19 23 24 27
ions: Cohort 3, 100 mg with Evening Meal (N=6)


FIG. 19A

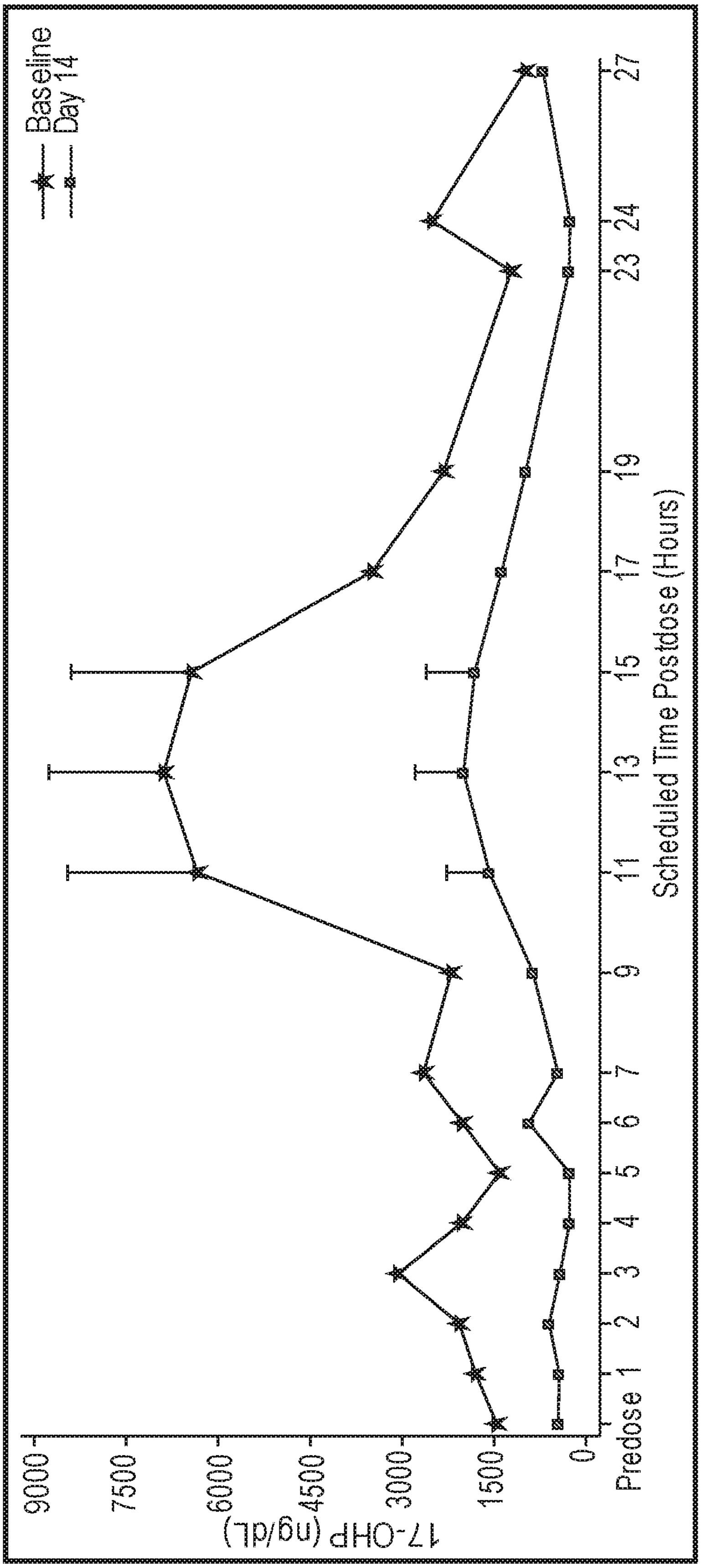
Baseline
Day 14
17-OHP (ng/dL)
9000
7500
6000
4500
3000
1500
0
Predose
1
2
3
4
5
6
7
9
11
13
15
17
19
23
24
27
Scheduled Time Postdose (Hours)

FIG. 19B

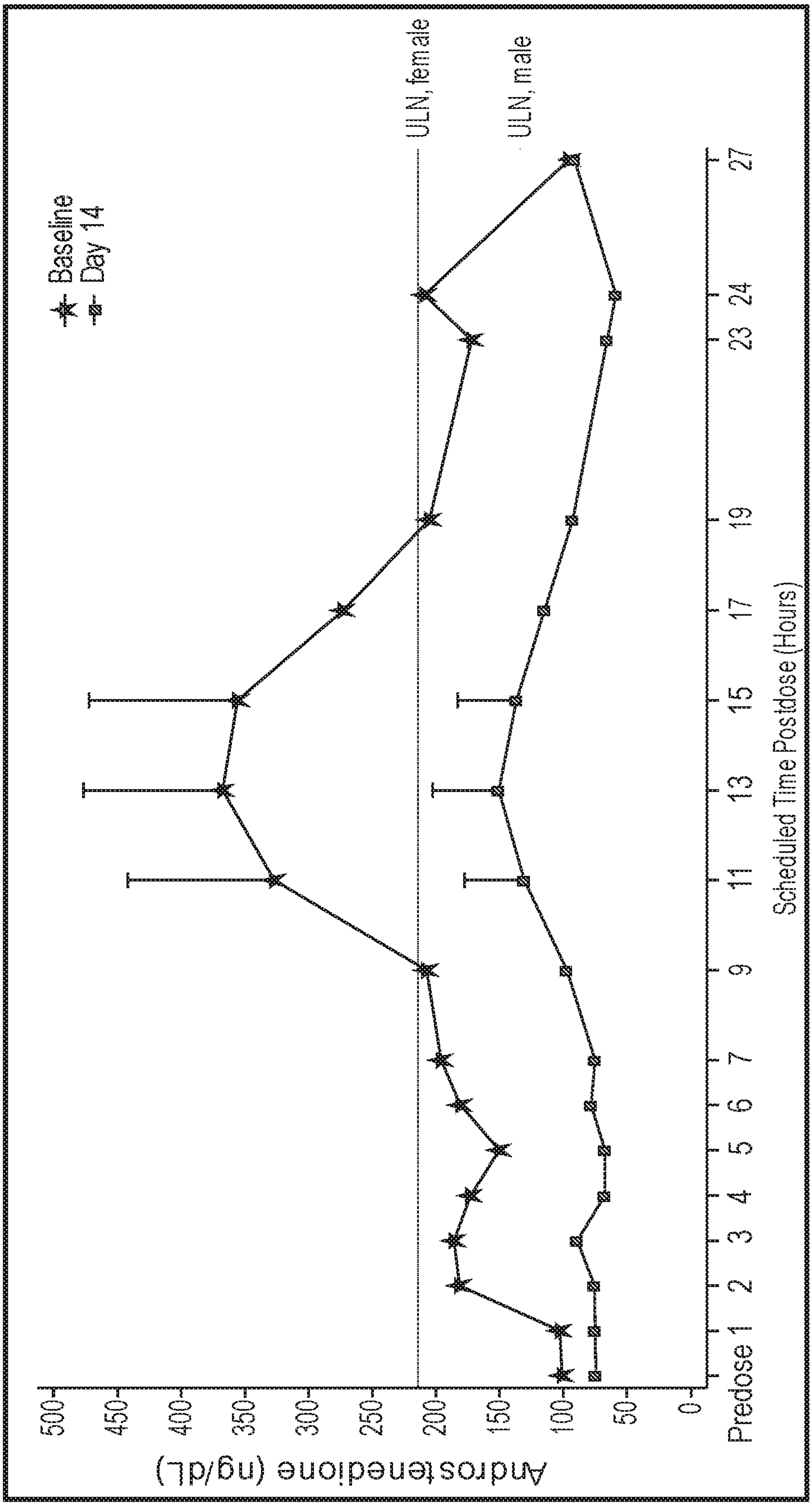
Baseline
Day 14
ULN, female
ULN, male
Androstenedione (ng/dL)
500
450
400
350
300
250
200
150
100
50
0
Predose 1 2 3 4 5 6 7 9 11 13 15 17 19 23 24 27
Scheduled Time Postdose (Hours)

FIG. 19C

**Manufacturing Process Flow Diagram:**

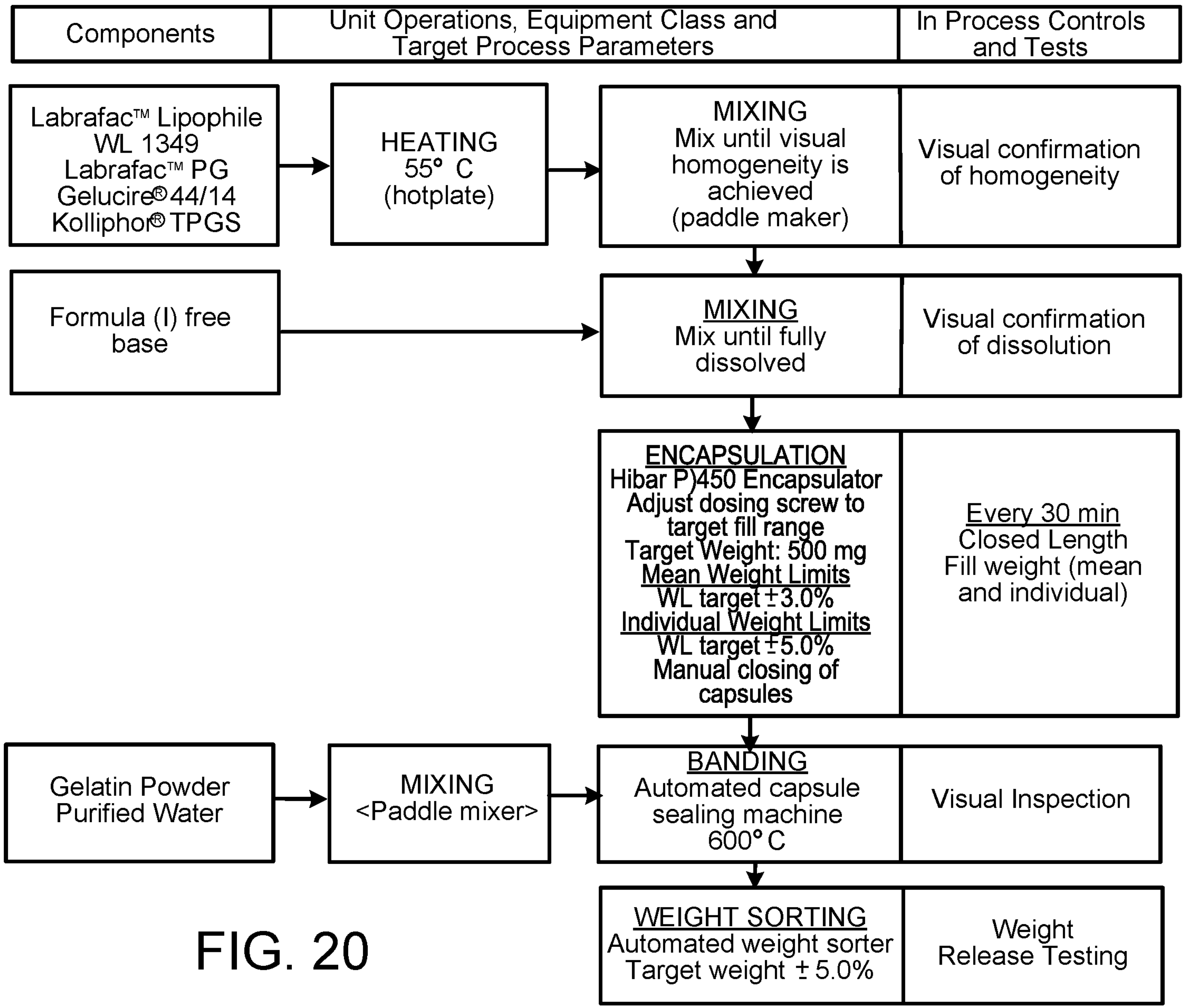


FIG. 20

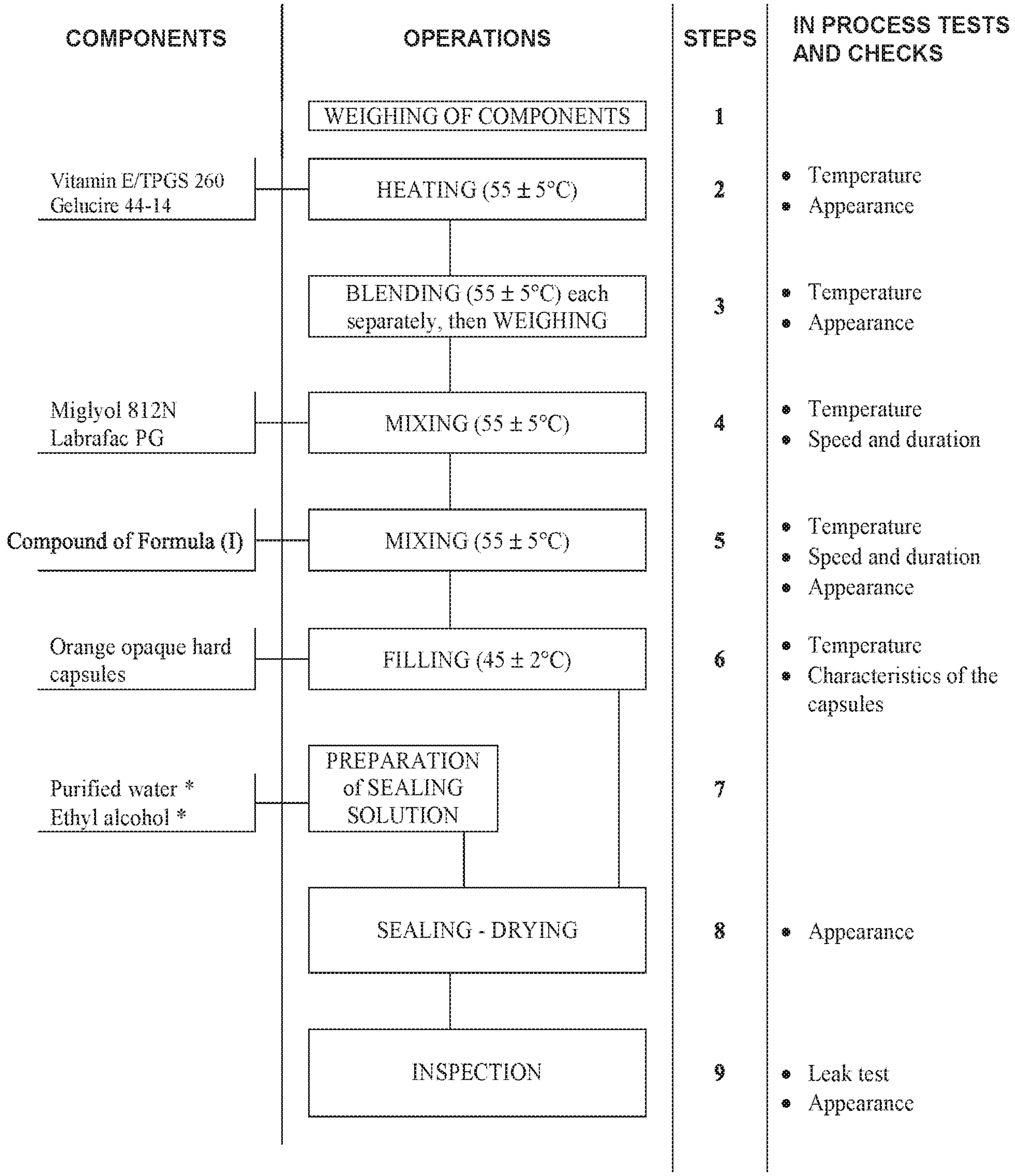


* Can be contained in hydro-alcoholic solution ready for manufacturing use

FIG. 21

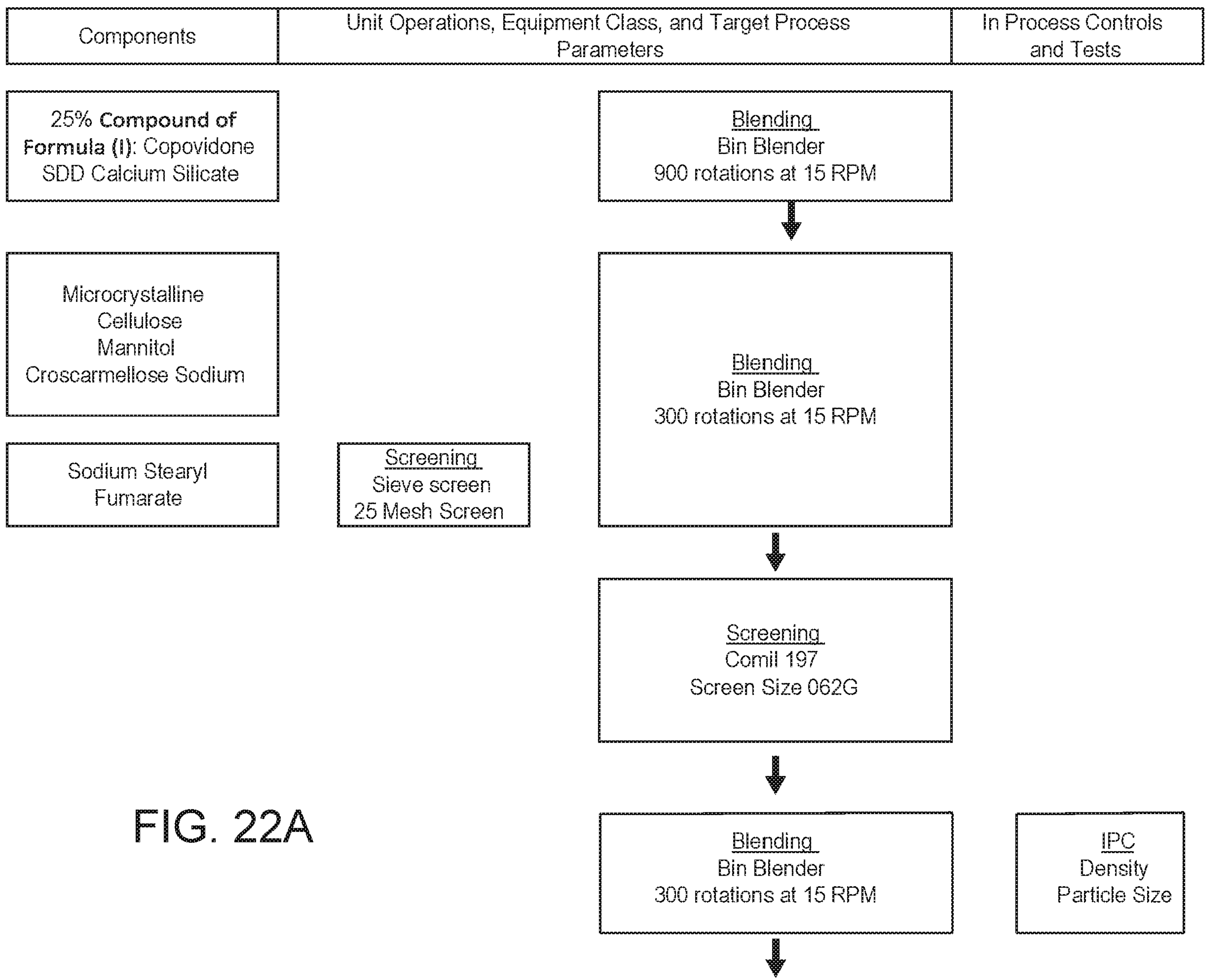
Components
Unit Operations, Equipment Class, and Target Process Parameters
In Process Controls and Tests
25% **Compound of Formula (I)**: Copovidone SDD Calcium Silicate
Blending
Bin Blender
900 rotations at 15 RPM
Microcrystalline Cellulose
Mannitol
Croscarmellose Sodium
Blending
Bin Blender
300 rotations at 15 RPM
Sodium Stearyl Fumarate
Screening
Sieve screen
25 Mesh Screen
Screening
Comil 197
Screen Size 062G
Blending
Bin Blender
300 rotations at 15 RPM
IPC
Density
Particle Size

FIG. 22A

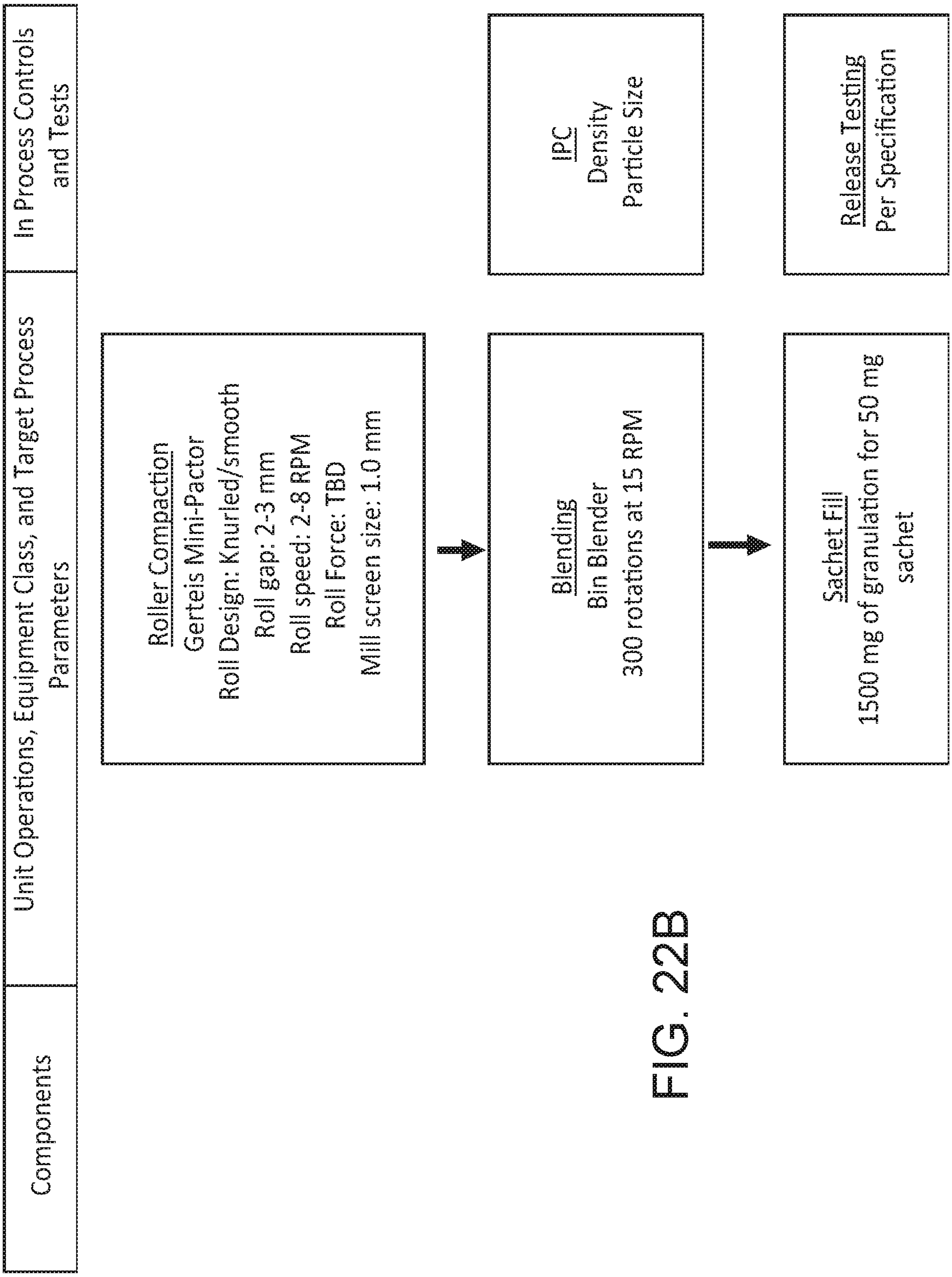
Components
Unit Operations, Equipment Class, and Target Process Parameters
In Process Controls and Tests
Roller Compaction
Gerteis Mini-Pactor
Roll Design: Knurled/smooth
Roll gap: 2-3 mm
Roll speed: 2-8 RPM
Roll Force: TBD
Mill screen size: 1.0 mm
Blending
Bin Blender
300 rotations at 15 RPM
IPC
Density
Particle Size
Sachet Fill
1500 mg of granulation for 50 mg sachet
Release Testing
Per Specification

FIG. 22B

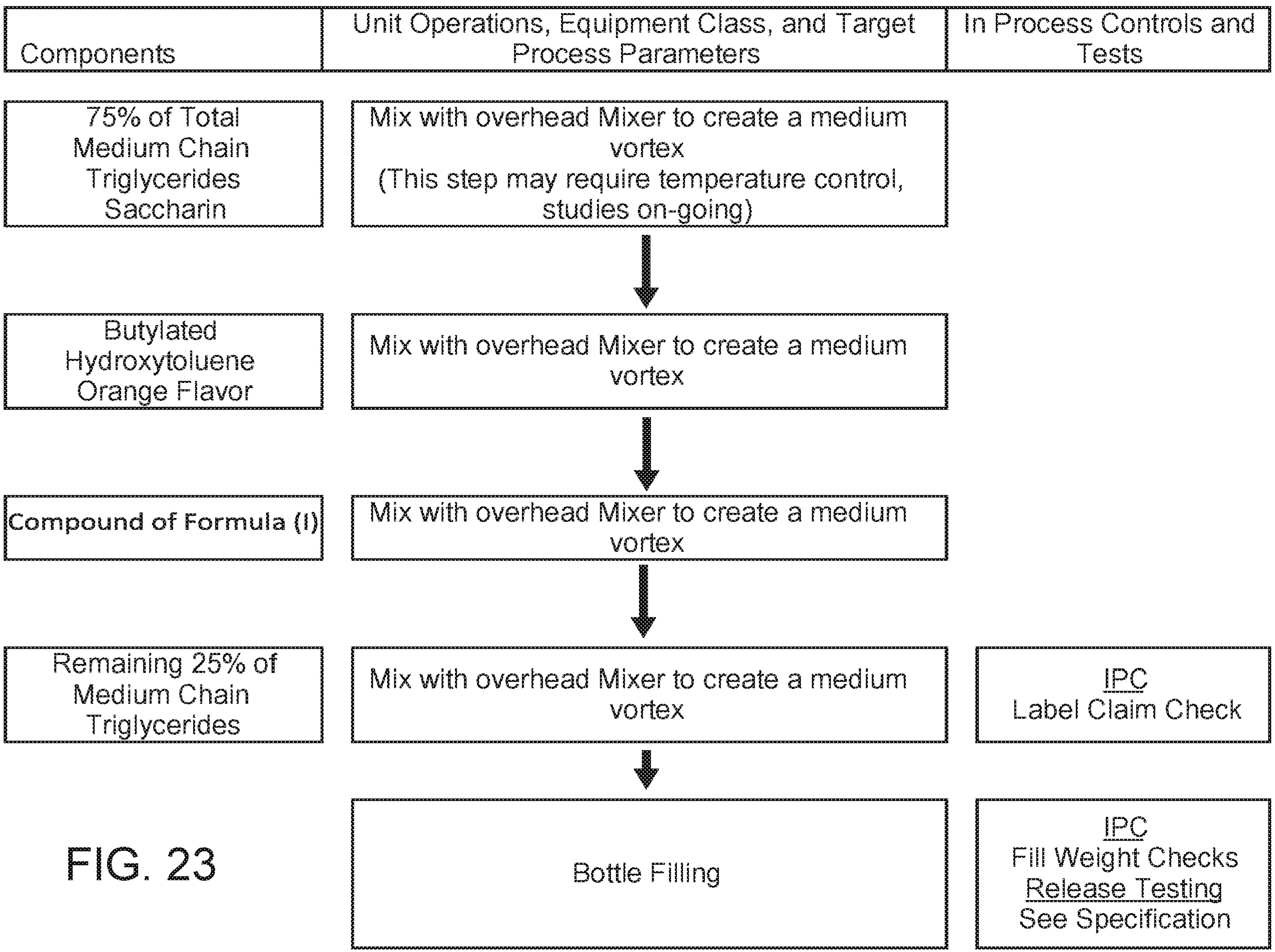
Components
Unit Operations, Equipment Class, and Target Process Parameters
In Process Controls and Tests
75% of Total Medium Chain Triglycerides Saccharin
Mix with overhead Mixer to create a medium vortex (This step may require temperature control, studies on-going)
Butylated Hydroxytoluene Orange Flavor
Mix with overhead Mixer to create a medium vortex
Compound of Formula (I)
Mix with overhead Mixer to create a medium vortex
Remaining 25% of Medium Chain Triglycerides
Mix with overhead Mixer to create a medium vortex
IPC
Label Claim Check
Bottle Filling
IPC
Fill Weight Checks
Release Testing
See Specification

FIG. 23

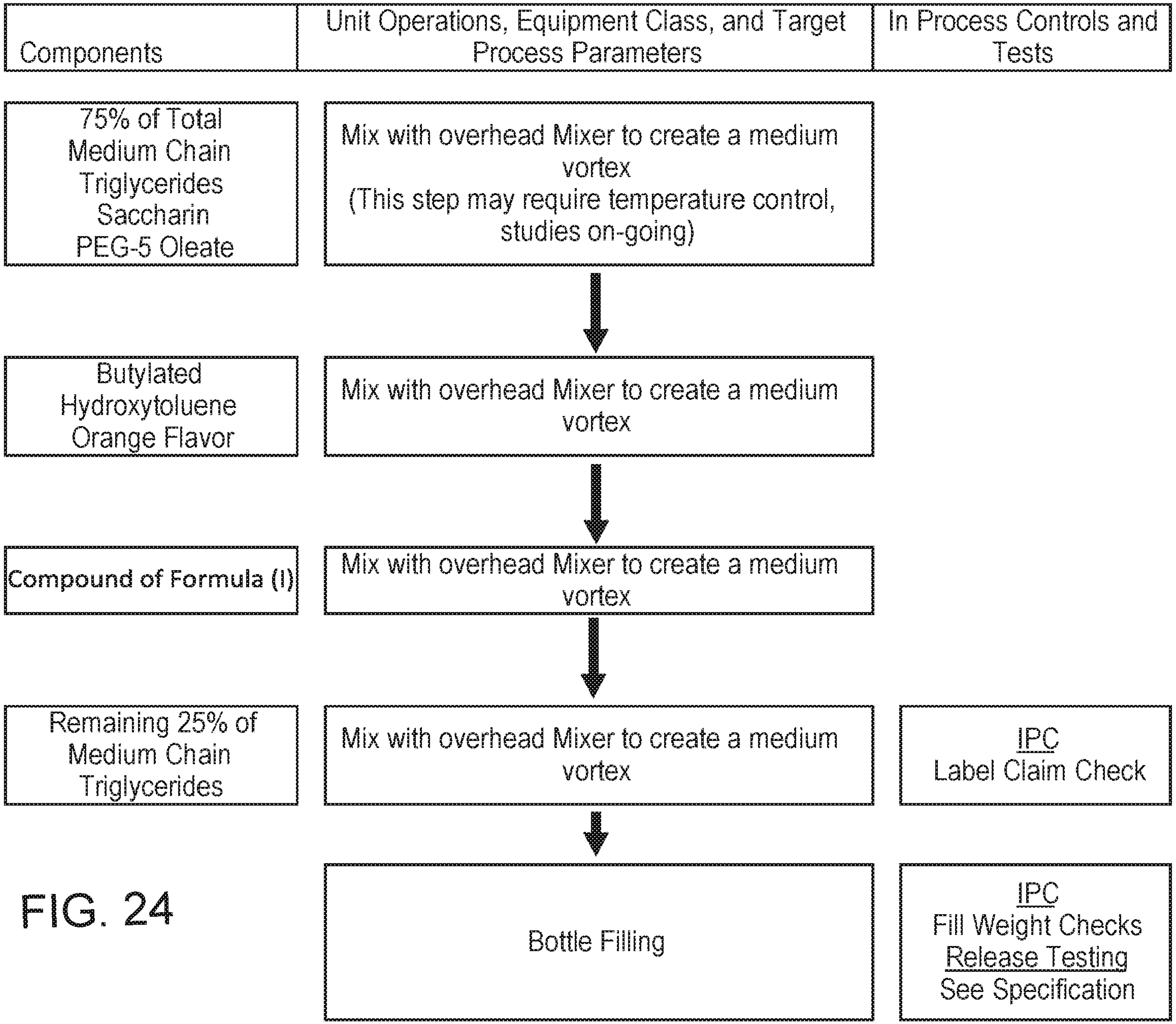
Components
Unit Operations, Equipment Class, and Target Process Parameters
In Process Controls and Tests
75% of Total Medium Chain Triglycerides Saccharin PEG-5 Oleate
Mix with overhead Mixer to create a medium vortex (This step may require temperature control, studies on-going)
Butylated Hydroxytoluene Orange Flavor
Mix with overhead Mixer to create a medium vortex
Compound of Formula (I)
Mix with overhead Mixer to create a medium vortex
Remaining 25% of Medium Chain Triglycerides
Mix with overhead Mixer to create a medium vortex
IPC Label Claim Check
Bottle Filling
IPC Fill Weight Checks Release Testing See Specification

FIG. 24

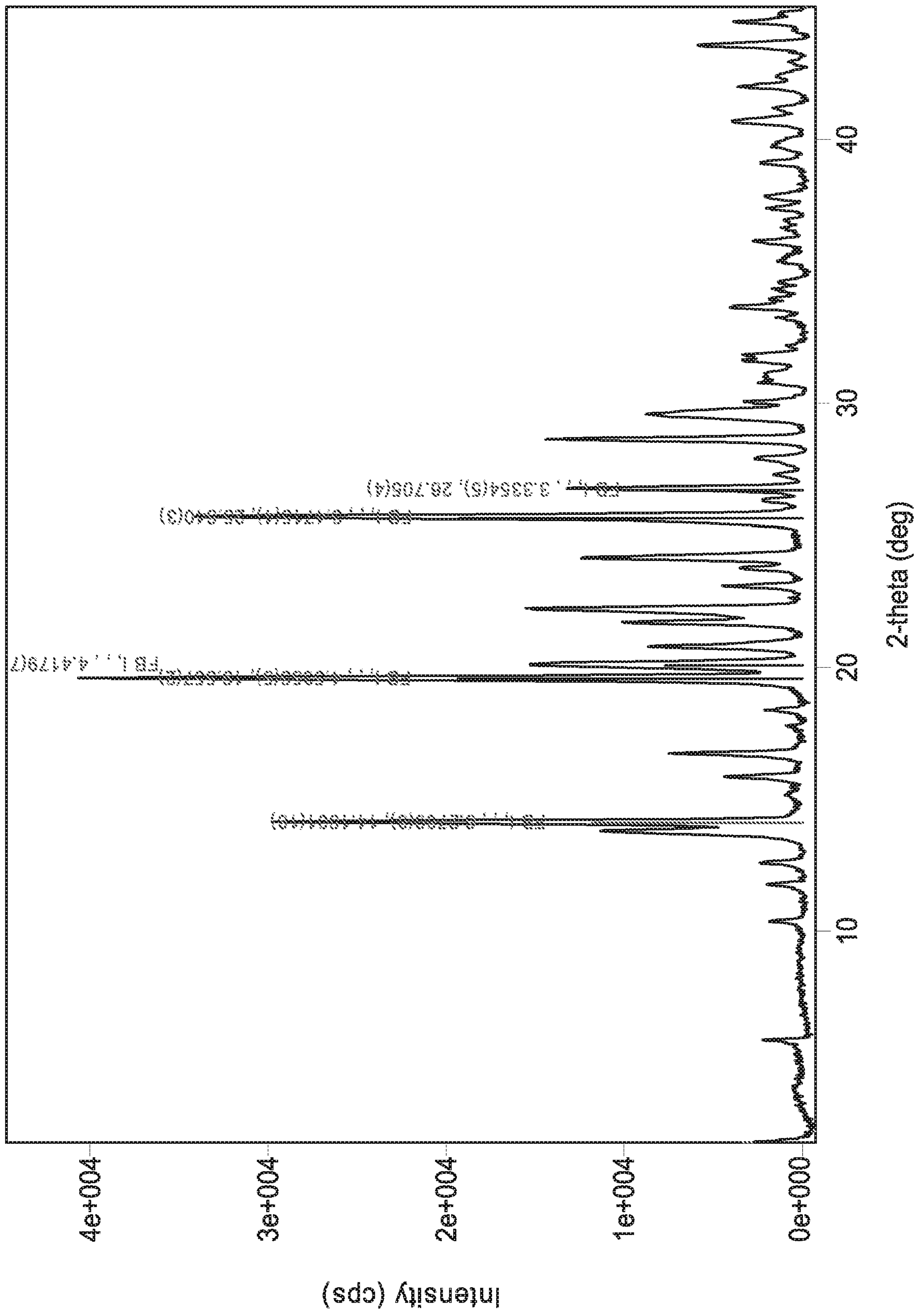
Intensity (cps)
4e+004
3e+004
2e+004
1e+004
0e+000
10
20
30
40
2-theta (deg)

FIG. 25

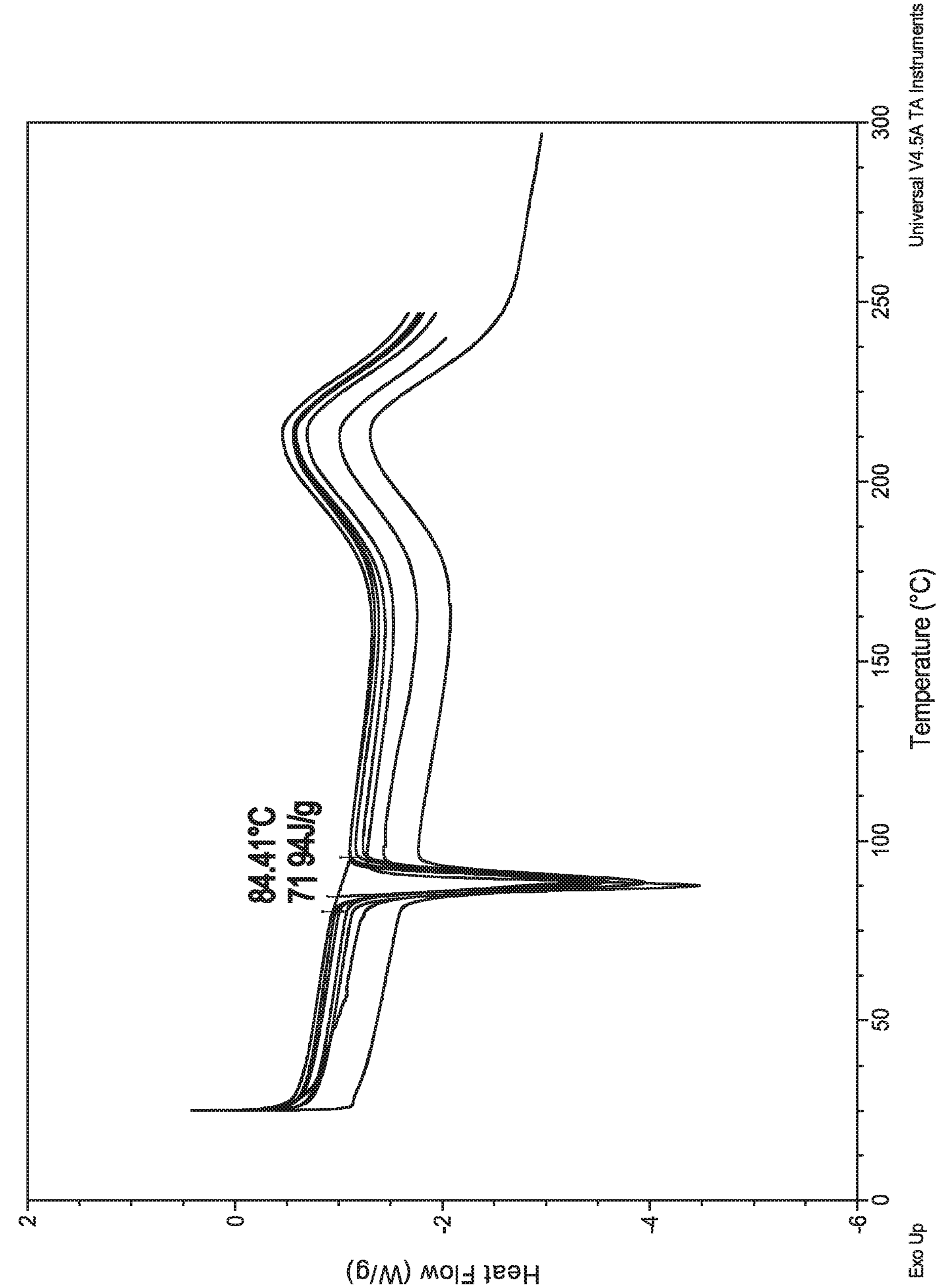
84.41°C
71.94J/g
Heat Flow (W/g)
Exo Up
Temperature (°C)
Universal V4.5A TA Instruments
2
0
-2
-4
-6
0
50
100
150
200
250
300

FIG. 26

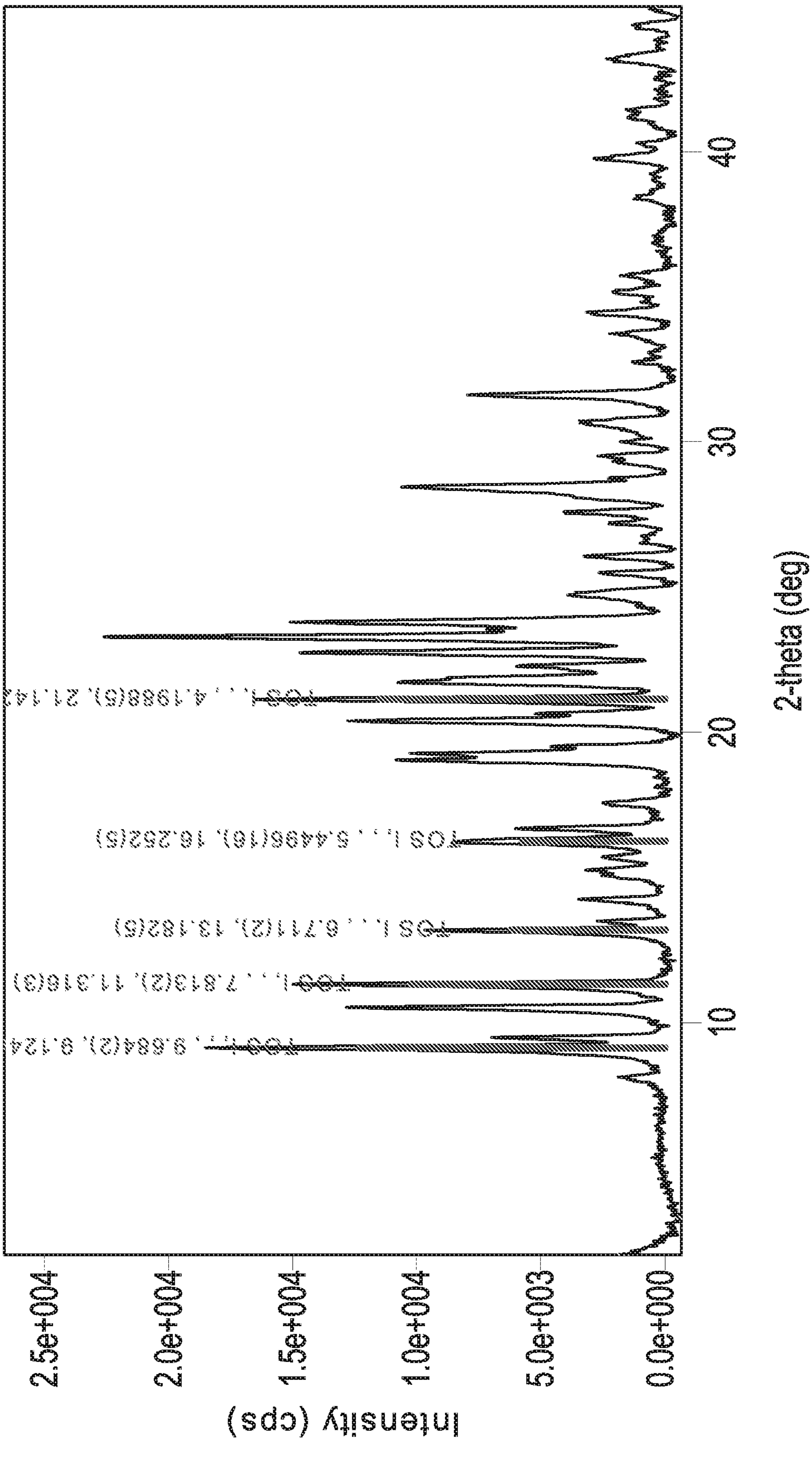
2.5e+004
2.0e+004
1.5e+004
1.0e+004
5.0e+003
0.0e+000
Intensity (cps)
10
20
30
40
2-theta (deg)

FIG. 27

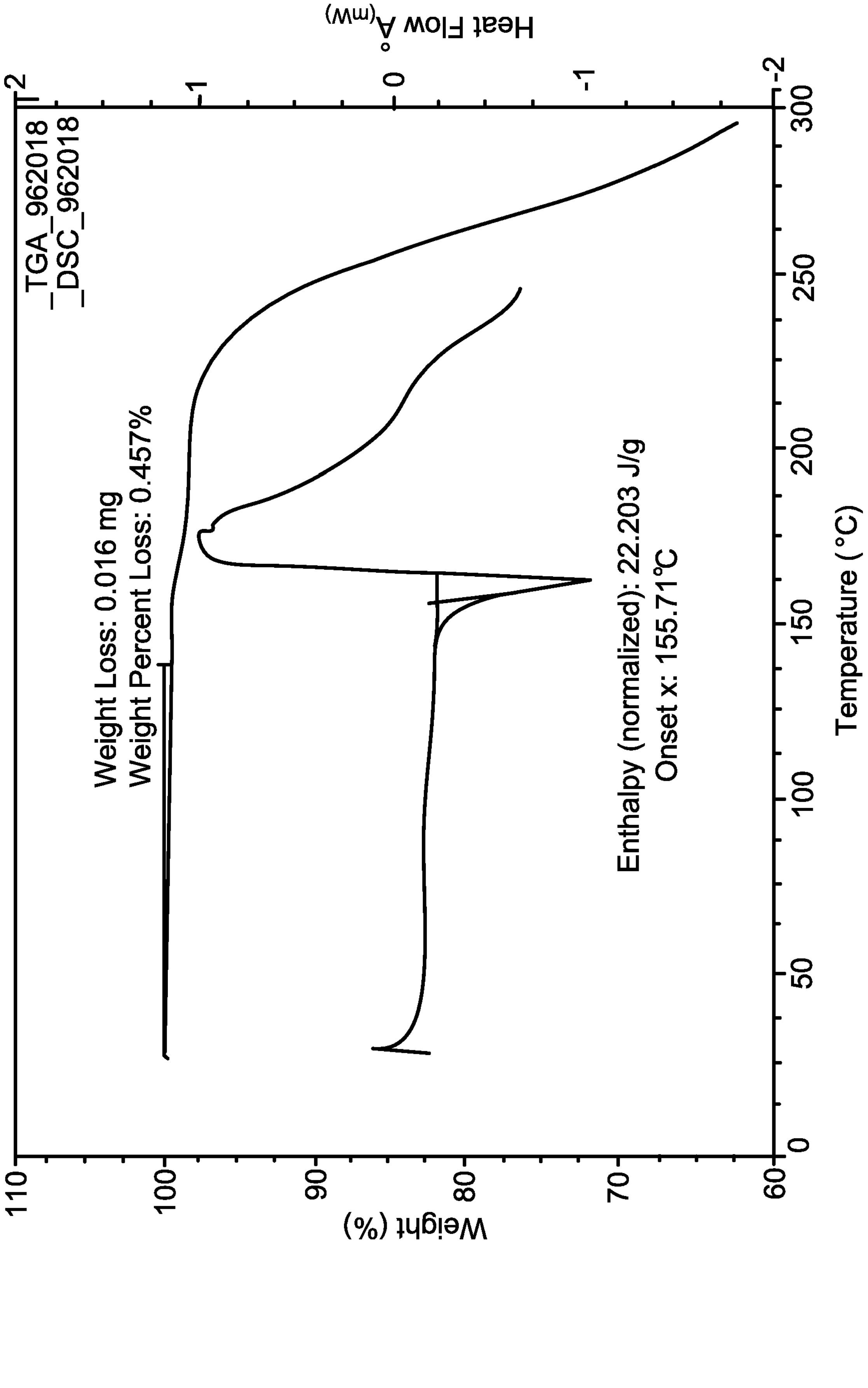
_TGA_962018
_DSC_962018
Weight Loss: 0.016 mg
Weight Percent Loss: 0.457%
Enthalpy (normalized): 22.203 J/g
Onset x: 155.71°C
Heat Flow Å(mW)
2
1
0
-1
-2
Weight (%)
110
100
90
80
70
60
Temperature (°C)
0
50
100
150
200
250
300

FIG. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6586456 B
- US 8314249 B
- WO 2015112642 A1


### Non-patent literature cited in the description


- **TRAKAKIS et al.** An update to 21-hydroxylase deficient congenital adrenal hyperplasia. *Gynecol. Endocrinol.*, 2010, vol. 26 (1), 63-71
- **HERTZBERG et al.** Birth prevalence rates of newborn screening disorders in relation to screening practices in the United States. *J. Pediatr.*, 2011, vol. 159 (4), 555-560
- **CHENG** ; **SPEISER**. Treatment outcomes in congenital adrenal hyperplasia. *Adv. Pediatr.*, 2012, vol. 59 (1), 269-281
- **MERKE** ; **POPPAS**. Management of adolescents with congenital adrenal hyperplasia. *Lancet Diabetes Endocrinol.*, 2013, vol. 1 (4), 341-352
- **ELNECAVE et al.** Bone mineral density in girls with classical congenital adrenal hyperplasia due to CYP21 deficiency. *J. Pediatr. Endocrinol. Metab.*, 2008, vol. 21 (12), 1155-1162
- **KING et al.** Long-term corticosteroid replacement and bone mineral density in adult women with classical congenital adrenal hyperplasia. *J. Clin. Endocrinol. Metab.*, 2006, vol. 91 (3), 865-869
- **MIGEON** ; **WISNIEWSKI**. Congenital adrenal hyperplasia owing to 21-hydroxylase deficiency. Growth, development, and therapeutic considerations. *Endocrinol. Metab. Clin. North Am.*, 2001, vol. 30 (1), 193-206
- **WHITE et al.** *J. Pediatr.*, 2013, vol. 163, 10-12
- **SPEISER et al.** *Int. J. Pediatr. Endocrinol.*, 2010, vol. 2010, 494173
- **KIM et al.** *Semin. Reprod. Med.*, 2009, vol. 27 (4), 316-21
- **FINKIELSTAIN et al.** *J. Clin. Endocrinol Metab.*, 2012, vol. 97 (12), 4429-38
- **ELNECAVE et al.** *J. Pediatr. Endocrinol. Metab.*, 2008, vol. 21, 1155-62
- **KING et al.** *J. Clin. Endocrinol. Metab.*, 2006, vol. 91 (3), 8656-59
- **MIGEON et al.** *Endocrinol. Metab. Clin. North Am.*, 2001, vol. 30, 193-206
- **SPEISER, P.W. et al.** Journal of Clinical Endocrinology and Metabolism. *J. Clin. Endocrinol. Metab.*, November 2018, vol. 103 (11), 1-46
- **VALE et al.** *Science*, 1981, vol. 213, 1394-1397
- Goodman and Gilman's: The Pharmacological Basis of Therapeutics. The McGraw-Hill Companies, 2010
- **BERHMAN et al.** Textbook of Pediatrics. W.B. Saunders Company, 1996
- **RUDOLPH et al.** Rudolph's Pediatrics,. McGraw-Hill, 2002
- **AVERY et al.** Pediatric Medicine. Williams & Wilkins, 1994
- **ORAY, M. et al.** Expert Opinion on Drug Safety. *Long-term effect of glucocorticoids*, 2016
- Assessing the Effects of Food on Drugs in INDs and NDAs - Clinical Pharmacology Considerations Guidance for Industry. *U.S. Department of Health and Human Services, Food and Drug Administration, Center for Drug Evaluation and Research (CDER)*, February 2019
- **STEWART et al.** *Mol. Pharm.*, 2017, vol. 14, 2032-2046